# EUROPEAN PATENT SPECIFICATION

(11) **EP 4 308 122 B1**
(45) Date of publication and mention of the grant of the patent: **22.07.2026**
(21) Application number: 22713948.2
(22) Date of filing: 14.03.2022
(51) Int. Cl.: A61K 31/5025, A61P 35/00, A61P 37/06, C07D 471/04, C07D 519/00

(54) **HETEROCYCLIC DERIVATIVES AS JANUS KINASE INHIBITORS**
HETEROCYCLISCHE DERIVATE ALS JANUS-KINASEHEMMER
DÉRIVÉS HÉTÉROCYCLIQUES COMME INHIBITEURS DE JANUS KINASE

(30) Priority: 15.03.2021 EP 21162515; 23.12.2021 EP 21217274
(43) Date of publication of application: 24.01.2024
(73) Proprietor: Chiesi Farmaceutici S.p.A., 43122 Parma (IT)
(72) Inventor: ACCETTA, Alessandro, 43122 Parma (IT); RANCATI, Fabio, 43122 Parma (IT); RIZZI, Andrea, 43122 Parma (IT); CUZZOLIN, Alberto, 43122 Parma (IT); MESIC, Milan, 43122 Parma (IT); ZADRAVEC, Rahela, 43122 Parma (IT); ZIHER, Dinko, 43122 Parma (IT); ELENKOV, Ivaylo, 43122 Parma (IT)
(74) Representative: Chiesi Farmaceutici S.p.A.
(86) International application number: PCT/EP2022/056548
(87) International publication number: WO 2022/194779

(56) References cited:
- WO-A1-2008/052734
- WO-A1-2012/069202
- HOWELL MICHAEL D. ET AL: "Targeting the Janus Kinase Family in Autoimmune Skin Diseases", FRONTIERS IN IMMUNOLOGY, vol. 10, 1 January 2019 (2019-01-01), pages 2342, XP055863286, Retrieved from the Internet <URL:https://www.ncbi.nlm.nih.gov/pmc/articles/PMC6794457/pdf/fimmu-10-02342.pdf> DOI: 10.3389/fimmu.2019.02342

## Description

### FIELD OF THE INVENTION

The present invention relates to chemical compounds that are derivatives useful as JAK inhibitors, such as JAK 1, useful for the treatment of various inflammatory disease including asthma, COPD and other respiratory diseases.

### BACKGROUND OF THE INVENTION

The JAK family consists of non-receptor tyrosine protein kinases and has four main members, JAK1, JAK2, JAK3, and TYK2. More than 50 cytokines and growth factors bind to type I and II receptors noncovalently associated with different combinations of JAK kinases. The signalling triggered by the ligands consists in tyrosine phosphorylation of receptors by JAK and recruitment of one or more STATs proteins. Tyrosine-phosphorylated STATs dimerize and are then transported into the nucleus through the nuclear membrane to regulate specific genes. JAKs have seven homology domains (the JAK homology domain, JH). Starting from the carboxyl terminus, JH1 is the first JH, known as the kinase domain, and is composed of approximately 250 amino acid residues. JH1 encodes a kinase protein that constitutes the kinase structure domain that phosphorylates a substrate; JH2 is a pseudokinase domain which regulates the activity of the kinase domain. JAK3 is expressed in the bone marrow and lymphatic system, as well as endothelial cells and vascular smooth muscle cells; other members are expressed in almost all tissues (Hu X et al., Signal Transduct Target Ther. 2021, 26;6(1):402). Many cellular processes are downstream JAK/STAT signalling: hematopoiesis, immune balance, tissue repair, inflammation, apoptosis, and adipogenesis. Different biological responses are regulated by specific pairing of JAK isoforms. JAK1/JAK3 combination mediates IL-2, -4, -7, -9, -15, and -21 signalling that is relevant for growth/maturation of lymphoid cells, differentiation/homeostasis of T-cells/NK cells, B-cell class switching and other inflammatory processes. Combinations of JAK1/TYK2-JAK1/JAK2, regulate the signal associated with the innate immune response, such as IL-6 and the type I interferons, involved into naïve T cell differentiation, T cell homeostasis, granulopoiesis and other inflammatory processes. (Howell MD et al., Front. Immunol. 2019, 10, 2342). JAK2 frequently associates with itself (JAK2/ JAK2) controlling the signalling of various cytokines and growth factors, such as IL-3, IL-5, granulocyte macrophage colony-stimulating factor (GM-CSF), erythropoietin (EPO), and thrombopoietin (TPO) (Hodge et al., Clin Exp Rheumatol 2016; 34(2):318-28).

Genetically modified mouse models and human diseases prove the importance of JAK/STAT pathways in immune fitness. In particular, overexpression or mutations involving some JAK isoforms as well as aberrant JAK/STAT signalling drive malignancies of hematopoietic and lymphoid tissues as well as inflammatory disorders. Currently, several Food and Drug Administration (FDA)- and/or EU- approved JAK inhibitors are in clinical use. Two (ruxolitinib and fedratinib) small molecules are in use for hematologic disorders as myelofibrosis and polycythemia vera; six JAK inhibitors (tofacitinib, baricitinib, ruxololitinib, filgotinib, upadicitinib and delgocitinib in Japan) result in use for immune-mediated disorders as rheumatoid arthritis, polyarticular juvenile idiopathic arthritis, atopic dermatitis, ulcerative colitis and acute graft-versus-host disease. Moreover, some of these drugs as well as others are currently under phase II and III of clinical trials for indications that span from autoimmune diseases (lupus, vitiligo, etc.), inflammatory bowel disease to Non-Hodgkin lymphoma and COVID-19 (Hu X. et al., Sig Transduct Target Ther 2021, 6: 402).

The small molecules targeting JAK/STAT represent an attractive option also for the therapy of fibrotic disorders. In fact, inflammatory cytokines (IL-4, IL-3, IL-6, IL-11, IL-31, etc) and growth factors (FGF, VEGF, etc.) involved in the fibrotic processes activate JAK/STAT pathway. Ruxolitinib tested in a bleomycin-induced fibrosis mouse model ameliorated the fibrotic lesions in lung, and reduced levels of fibrotic molecular markers (Zhang, Y et al., Ann. Rheum. Dis. 2017, 76, 1467-1475) while tofacitinib acted as a preventive agent in experimental dermal and pulmonary fibrosis (Wang, W et al., Scleroderma Relat. Disord. 2020, 5, 40-50). In patients, some case reports were studied. A single-case report corroborated the efficacy and safety of tofacitinib in combination with nintedanib in the management of an aggressive interstitial lung disease with poor prognosis (Conca, W et al., Front. Pharmacol. 2020, 11, 5857619). Baricitinib was demonstrated to be a safe immune modulator that reduces the biomarkers' levels of lung fibrosis and inflammation in RA patients, including a subgroup with interstitial lung disease (D'Alessandro M et al., Int. Immunopharmacol. 2020, 86, 106748).

In COVID-19, there are some JAK inhibitors undergoing clinical trials, and they are tofacitinib, baricitinib, and ruxolitinib. Baricitinib and ruxolitinib were associated with a reduced risk of mortality. They reduced the use of invasive mechanical ventilation and had a borderline impact on the admission rate of the intensive care unit and the incidence of acute respiratory distress syndrome (ARDS). (Wijaya, I. et al. Clin. Epidemiol. Glob. Health 2021, 11, 100755). Ruxolitinib also was tested in COVID-19 patients and improved the clinical symptoms and chest computed tomography images (Cao Y. et al., J. Allergy Clin. Immunol. 2020 146, 137-146).

Asthma can be included in the plethora of immune-mediated diseases for which pathogenesis is characterized by an essential role of JAK/STAT signalling. Asthma is a chronic inflammatory disease of the airways due to a complex interplay between immune response, genetic susceptibility and nonspecific external stimuli like cold, allergens and exercise leading to hyperresponsiveness, remodelling of the airways, ultimately contributing to airflow limitation. Severe asthma affects 5% to 15% of the population with adult asthma (which is 300 million people worldwide) and represents a public health issue associated with increased mortality, increased hospitalizations, significant burden of symptoms, health care costs, and missed work and school (Steve NG et al., J Allergy Clin Immunol 2021;148:953-63). Severe asthma represents a subset of difficult-to-treat asthma and occurs in patients whose disease remains uncontrolled despite the use of high doses of inhaled corticosteroids (ICSs) combined with long-acting β-agonists or other controllers. To date, four types of biologics are licensed for severe asthma, i.e. omalizumab (anti-immunoglobulin E) antibody, mepolizumab and reslizumab (anti-interleukin [IL]-5antibody), benralizumab (anti-IL-5 receptor alpha antibody) and dupilumab (anti-IL-4 receptor alpha antibody). Despite their efficacy, many patients continue to experience exacerbations or uncontrolled disease, indicating a need for more novel therapies (Israel E, Reddel HK. N Engl J Med 2017; 377:965-76).

Recently, the better understanding of asthma pathobiology brought to a shift from a phenotypic classification system to the introduction of the "endotype" concept. According to the latter, classification is performed on the basis of pathophysiologic mechanisms and clinical biomarkers associated with a given patient (Wenzel SE et a., Am J Respir Crit Care Med 2021;203:809-21). There are two major endotypes in asthma: type 2 and non-type 2. The type 2 pathway is defined by activation of cytokines derived from TH2 cells and group 2 innate lymphoid cells (ILC2s); these include IL-4, IL-5, and IL-13 that cause airway inflammation by activating eosinophils, B cells, airway epithelial cells, and other cell types. Biomarkers of type 2 asthma include blood/sputum eosinophilia and elevated levels of fractional exhaled nitric oxide (FENO) and IgE. The type 2-low pathway is characterized by absence of type 2-high cytokines and biomarkers, and it manifests either increased levels of neutrophils in the airways or a paucigranulocytic profile, with normal levels of airway neutrophils and eosinophils. Type 2-low asthma is currently not well understood, and it likely encompasses multiple distinct endotypes. Potential mediators and/or biomarkers of T2 low endotypes under investigation include IL-6, IL-17A/F, IL-23, Type I interferons, CXCL10, TNF, alarmins (TSLP, IL-25, IL-33), IL-1β, IL-8, IFN-γ (Hinks TSC et al., ERJ 2021, 57 (1) 2000528).

Almost all the mediators mentioned above both for T2 and T2-low endotypes activate JAK/STAT pathway, here the rationale for the potential use of JAK inhibitors in both endotypes of severe asthma. Targeting simultaneously several cytokines by JAK inhibitors may offer advantage over the biologics (for no-responder patients) and standard therapies (for patients who remain uncontrolled) considering their administration on top of ICS.

Despite strong rationale of JAK inhibitors in asthma, safety concerns may arise by administration of systemic inhibitors or may limits administration into particular asthma subjects such as children. Considering that Asthma is a lung restricted disease, inhalatory route of administration for a JAK inhibitor may offers the advantage of therapeutic efficacy while limiting systemic exposure and correlated side effects. To date, some companies are developing inhaled JAK inhibitor for asthma treatment. Astrazeneca pipeline include AZD-0449 (completed Phase I clinical trial) and AZD-4604 (ongoing Phase I clinical trial); Theravance Biopharma is starting a new preclinical program on TD-8236 inhaled JAK inhibitor and Kinaset/Vectura is developing VR588 (ongoing Phase I clinical trial) as inhalatory compound. Many preclinical studies sponsored by the companies mentioned above demonstrated the efficacy of JAK inhibitors in the modulation of asthma. In the preclinical phase of drug development, JAK1/3 inhibitor R256 (now referred as AZD0449) orally given showed be effective in decreasing airway resistance, BAL eosinophilia, mucus production and if administered during sensitization, also TH2 cytokine responses (Ashino S et al., J Allergy Clin Immunol 2014;133:1162-74). iJak-381 from Genentech given as dry powder reduced BAL eosinophilia, CCL11, airway resistance, and Muc5AC in OVA-challenged mice. Moreover, it reduced BAL eosinophilia, neutrophilia, CCL11, and CXCL1 in a in mouse model of chronic exposure to AAH allergens (Dengler HS et al., Sci Transl Med 2018;10:eaao2151). Moreover, an oral JAK inhibitor as Tofacitinib, formulated for being administered as aerosol, reduced eosinophils count in a house dust mite mouse model of asthma (Younis US et al., AAPS PharmSci-Tech 2019;20:167).

Another respiratory disease that could benefit from lung restricted JAK inhibition is Chronic obstructive pulmonary disease (COPD), an inflammatory disease of the lung, most commonly resulting from cigarette smoke exposure, characterised by a largely irreversible and progressive airflow limitation. Despite inflammatory cytokines are drivers of chronic airway inflammation and some of them trigger JAK/STAT activation (IL-6, IFN-γ, IL-2, etc.), the role of this pathway in COPD pathogenesis is poorly characterized. Phosphorylated-STAT4+ cells (Di Stefano A et al., Eur Respir J. 2004 Jul; 24(1):78-85) were found to be increased in COPD compared to non-smokers healthy controls. In another study, phosphorylated-STAT3+ and phosphorylated-STAT1+ cells counts were higher in lung biopsies of COPD patients than non-smokers controls while it was not possible to reproduce previous data on phosphorylated-STAT4 molecule (Yew-Booth L et al., Eur Respir J 2015; 46(3):843-5). These data might also suggest a therapeutic use of JAK inhibitors also in COPD disease.

In view of the number of pathological responses, which are mediated by JAK enzymes, there is a continuing need for inhibitors of JAK enzymes which can be useful in the treatment of many disorders and particularly respiratory diseases.

Thus, the finding of novel and potent JAK inhibitor suitable for local administration to the lungs for treatment of asthma and respiratory disease still remains an important need.

### SUMMARY OF THE INVENTION

Accordingly, it is one object of the present invention to provide compounds of formula (I)

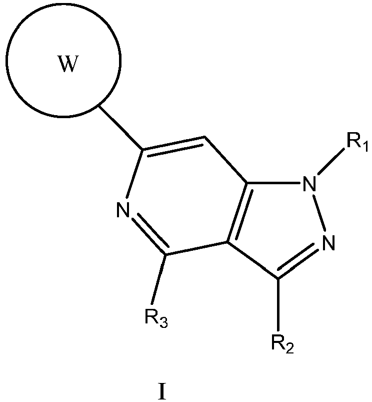

Wherein W, R₁, R₂, R₃ are as defined in the detailed description of the invention; or a pharmaceutically-acceptable salt thereof, that are useful as JAK kinase inhibitors.

It is another object of the present invention to provide pharmaceutical compositions comprising such compounds, for use in the prevention and/or treatment of respiratory diseases, and processes and intermediates useful for preparing such compounds.

In one aspect, the present invention provides a compound of formula (I) for use as a medicament. In one aspect the present invention provides the use of a compound of the invention for the manufacture of a medicament.

In a further aspect, the present invention provides the use of a compound of the invention for the preparation of a medicament for the treatment of any disease associated with JAK enzyme mechanisms.
the compound of the present invention can be used in a method for prevention and/or treatment of any disease associated with JAK enzyme mechanisms as above defined, said method comprises administering to a patient in need of such treatment a therapeutically effective amount of a compound of the invention.

In a Particular aspect the compounds of the invention are used alone or combined with other active ingredients suitable to be administered for the prevention and/or treatment of a pulmonary disease including asthma, Chronic obstructive pulmonary disease (COPD), cystic fibrosis (CF), interstitial lung diseases and idiopathic pulmonary fibrosis (IPF), acute lung injury and acute respiratory distress syndrome (ARDS).

### DETAILED DESCRIPTION OF THE INVENTION

### Definitions

The term "Pharmaceutically acceptable salts" refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts of the invention comprise ions of alkali or alkaline earth metals such as potassium, sodium, calcium or magnesium. Those obtained by reacting the main compound, functioning as a base, with an inorganic or organic acid to form a salt comprise, for example, salts of hydrochloric, hydrobromic, sulfuric, phosphoric, methanesulfonic, camphorsulfonic, acetic, oxalic, maleic, fumaric, succinic and citric acids.

Many organic compounds can form complexes with solvents in which they are reacted or from which they are precipitated or crystallized. These complexes are known as "solvates" which are a further object of the invention. Polymorphs and crystalline forms of compounds of formula (I), or of pharmaceutically acceptable salts, or solvates thereof are a further object of the invention.

The term "Halogen" or "halogen atoms" includes fluorine, chlorine, bromine, and iodine atom; meaning Fluoro, Chloro, Bromo, Iodo as substituent.

The term "(C₁-C₆)Alkyl" refers to straight-chained or branched alkyl groups wherein the number of carbon atoms is in the range 1 to 6. Particular alkyl groups are for example methyl, ethyl, n-propyl, isopropyl, t-butyl, 3-methylbutyl and the like.

The expressions "(C₁-C₆)Haloalkyl" refer to the above defined "(C₁-C₆)alkyl" groups wherein one or more hydrogen atoms are replaced by one or more halogen atoms, which can be the same or different from each other. Examples include halogenated, poly-halogenated and fully halogenated alkyl groups wherein all of the hydrogen atoms are replaced by halogen atoms, e.g. trifluoromethyl or difluoro methyl groups.

By way of analogy, the terms "(C₁-Cₓ) hydroxyalkyl" or "(C₁-Cₓ) aminoalkyl" refer to the above defined "(C₁-Cₓ) alkyl" groups wherein one or more hydrogen atoms are replaced by one or more hydroxy (OH) or amino group respectively.

The definition of aminoalkyl encompasses alkyl groups (i.e. "(C₁-C₆)alkyl" groups) substituted by one or more amino groups (-NR₄R₅). An example of aminoalkyl is a mono-aminoalkyl group such as R₄R₅N-(C₁-C₆)alkyl, or -(CH₂)ₘNR₄R₅. wherein R₄ and R₅ and m are as defined in the detailed description of the invention.

With reference to the substituent R₄ and R₅ as above defined, it is here further explained that when either R₄ and R₅ are taken together with the nitrogen atom they are linked to form a 5 to 6 membered heterocyclic radical, at least one further ring carbon atom in the said heterocyclic radical may be replaced by at least one heteroatom or hetero-group (e.g. N, NH, S or O) or may bear an -oxo (=O) substituent group. The said heterocyclic radical might be further optionally substituted on the available points in the ring, namely on a carbon atom, or on an heteroatom or hetero-group available for substitution. Thus, Examples of said heterocycle radicals are 1-pyrrolidinyl, 1-piperidinyl, 1-piperazinyl, 4-morpholinyl, piperazin-4yl-2-one, 4-methylpiperazine-1-yl.

The term "(C₃-C₁₀)cycloalkyl" likewise "(C₃-C₆)cycloalkyl" refers to saturated cyclic hydrocarbon groups containing the indicated number of ring carbon atoms. Examples include cyclopropyl, cyclobutyl, cyclopentyl, cyclohexyl and cycloheptyl, and polycyclic ring systems such as adamantan-yl.

The expression "Aryl" refers to mono, bi- or tri-cyclic carbon ring systems, which have 6 to 20, preferably from 6 to 15 ring atoms, wherein at least one ring is aromatic. The expression "heteroaryl" refers to mono-, bi- or tri-cyclic ring systems with 5 to 20, preferably from 5 to 15 ring atoms, in which at least one ring is aromatic and in which at least one ring atom is a heteroatom (e.g. N, S or O).

Examples of aryl or heteroaryl monocyclic ring systems include, for instance, phenyl, thienyl, pyrrolyl, pyrazolyl, imidazolyl, isoxazolyl, oxazolyl, isothiazolyl, thiazolyl, pyridinyl, pyrimidinyl, pyrazinyl, pyridazinyl, triazinyl, furanyl radicals and the like.

Examples of aryl or heteroaryl bicyclic ring systems include naphthalenyl, biphenylenyl, purinyl, pteridinyl, pyrazolopyrimidinyl, benzotriazolyl, benzoimidazole-yl, quinolinyl, isoquinolinyl, indolyl, isoindolyl, indazolyl, benzothiopheneyl, benzodioxinyl, dihydrobenzodioxinyl, indenyl, dihydro-indenyl, dihydrobenzo[1,4]dioxinyl, benzothiazole-2-yl, dihydrobenzodioxepinyl, benzooxazinyl, 1,2,3,4-tetrahydroisoquinoline-6-yl, 4,5,6,7-tetrahydrothiazolo[4,5-c]pyridine, 4,5,6,7-tetrahydrobenzo[d]thiazol-2-yl, 5,6,7,8-tetrahydro-1,7-naphthyridine, radicals and the like.

Examples of aryl or heteroaryl tricyclic ring systems include fluorenyl radicals as well as benzocondensed derivatives of the aforementioned heteroaryl bicyclic ring systems.

The derived expression "(C₃-C₁₀)heterocycloalkyl" likewise "(C₃-C₆) heterocycloalkyl" refers to saturated or partially unsaturated mono, bi- or tri- cycloalkyl groups of the indicated number of carbons, in which at least one ring carbon atom is replaced by at least one heteroatom (e.g. N, NH, S or O) and/or may bear an -oxo (=O) substituent group (e.g. C(=O), S(=O)₂). Said heterocycloalkyl (i.e. heterocyclic radical or group) is further optionally substituted on the available points in the ring, namely on a carbon atom, or on an heteroatom available for substitution. Examples of heterocycloalkyl are represented by: oxetanyl, tetrahydro-furanyl, pyrrolidinyl, imidazolidinyl, thiazolidinyl, piperazinyl, piperidinyl, morpholinyl, thiomorpholinyl, dihydro- or tetrahydro-pyridinyl, tetrahydropyranyl, pyranyl, 2H- or 4H-pyranyl, dihydro- or tetrahydrofuranyl, dihydroisoxazolyl, pyrrolidin-2-one-yl, dihydropyrrolyl, 5-oxopyrrolidin-3-yl, (1R,5S,6r)-3-oxabicyclo[3.1.0]hexan-6-yl, 1,1-dioxidothiomorpholino, octahydrocyclopenta[c]pyrrol-5-yl, 4,5,6,7-tetrahydropyrazolo[1,5-a]pyrazin-2-yl; 4,5,6,7-tetrahydrothiazolo[5,4-c]pyridin-2-yl radicals and the like.

The term "Aryl(C₁-C₆)alkyl" refers to an aryl ring linked to a straight-chained or branched alkyl group wherein the number of constituent carbon atoms is in the range from 1 to 6, e.g. phenylmethyl (i.e. benzyl), phenylethyl or phenylpropyl.

Likewise the term "Heteroaryl(C₁-C₆)alkyl" refers to an heteroaryl ring linked to a straight-chained or branched alkyl group wherein the number of constituent carbon atoms is in the range from 1 to 6, e.g. furanylmethyl.

The term "alkanoyl", refers to HC(O)- or to alkylcarbonyl groups (e.g. (C₁-C₆)alkylC(O)-) wherein the group "alkyl" has the meaning above defined. Examples include formyl, acetyl, propanoyl, butanoyl.

The term "(C₁-C₁₀) alkoxy" or "(C₁-C₁₀) alkoxyl", likewise "(C₁-C₆) alkoxy" or "(C₁-C₆) alkoxyl" etc., refers to a straight or branched hydrocarbon of the indicated number of carbons, linked to the rest of the molecule through an oxygen bridge. "(C₁-C₆)Alkylthio" refers to the above hydrocarbon linked through a sulfur bridge. Likewise the term "(C₁-C₆)Alkylthio" refers to the above defined haloalkyl, linked through a sulfur bridge. Examples of (C₁-C₆)Alkylthio and (C₁-C₆)Alkylthio are respectively methylthio, (difluoromethyl)thio.

The derived expression "(C₁-C₆)haloalkoxy" or "(C₁-C₆)haloalkoxyl" refers to the above defined haloalkyl, linked through an oxygen bridge.. Example of (C₁-C₆)haloalkoxy is difluoromethoxy, trifluoromethoxy.

Likewise derived expression "(C₃-C₆)heterocycloalkyl-(C₁-C₆)alkyl" and "(C₃-C₆)cycloalkyl-(C₁-C₆)alkyl" refer to the above defined heterocycloalkyl and cycloalkyl groups linked to the rest of the molecule via an alkyl group of the indicated number of carbons, for example piperidin-4-yl-methyl, cyclohexylethyl.

The derived expression "(C₁-C₆)alkoxy (C₁-C₆)alkyl" refers to the above defined alkoxy group linked to the rest of the molecule via an alkyl group of the indicated number of carbons, for example methoxymethyl.

Likewise "(C₁-C₆)haloalkoxy(C₁-C₆)alkyl" refers to the above defined (C₁-C₆)haloalkoxy" group linked to the rest of the molecule via an alkyl group of the indicated number of carbons, for example difluoromethoxypropyl.

Likewise "(C₁-C₆)alkoxycarbonyl" refers to the above defined alkoxy group linked to the rest of the molecule via an carbonyl group.

And "(C₁-C₆)alkoxycarbonyl-(C₁-C₆)alkyl" refers to the above defined alkoxy group linked to the rest of the molecule via an carbonyl group further enchained with an alkyl group of the indicated number of carbons, for example methoxycarbonylmethyl.

And "(C₁-C₆)alkoxycarbonyl-(C₁-C₆)alkylthio consequently refer to enchained groups like methoxycarbonylmethylthio

An oxo moiety is represented by (O) as an alternative to the other common representation, e.g. (=O). Thus, in terms of general formula, the carbonyl group is herein preferably represented as -C(O)- as an alternative to the other common representations such as -CO-, -(CO)- or -C(=O)-. In general the bracketed group is a lateral group, not included into the chain, and brackets are used, when deemed useful, to help disambiguating linear chemical formulas; e.g. the sulfonyl group -SO₂- might be also represented as-S(O)₂- to disambiguate e.g. with respect to the sulfinic group -S(O)O-.

When a numerical index the statement (value) "p is zero" or "p is 0" means that the substituent or group bearing the index p (e.g. (R)p) is absent, that is to say no substituent, other than H when needed, is present. Likewise when the index is attached to a bridging divalent group (e.g. (CH₂)n) the statement "n in each occurrence is zero..." or "n is 0" means that the bridging group is absent, that is to say it is a bond.

Whenever basic amino or quaternary ammonium groups are present in the compounds of formula (I), physiological acceptable anions, selected among chloride, bromide, iodide, trifluoroacetate, formate, sulfate, phosphate, methanesulfonate, nitrate, maleate, acetate, citrate, fumarate, tartrate, oxalate, succinate, benzoate, p-toluenesulfonate, pamoate and naphthalene disulfonate may be present. Likewise, in the presence of acidic groups such as COOH groups, corresponding physiological cation salts may be present as well, for instance including alkali or alkaline earth metal ions.

Compounds of formula (I) when they contain one or more stereogenic center, may exist as optical stereoisomers.

Where the compounds of the invention have at least one stereogenic center, they may accordingly exist as enantiomers. Where the compounds of the invention possess two or more stereogenic centers, they may additionally exist as diastereoisomers. It is to be understood that all such single enantiomers, diastereoisomers and mixtures thereof in any proportion are encompassed within the scope of the present invention. The absolute configuration (R) or (S) for carbon bearing a stereogenic center is assigned on the basis of Cahn-Ingold-Prelog nomenclature rules based on groups' priorities.

"Single stereoisomer", "single diastereoisomer" or "single enantiomer", when reported near the chemical name of a compound indicate that the isomer was isolated as single diastereoisomer or enantiomer (e.g via chiral chromatography) but the absolute configuration at the relevant stereogenic center was not determined/assigned.

Atropisomers result from hindered rotation about single bonds where the steric strain barrier to rotation is high enough to allow for the isolation of the conformers (Bringmann G et al, Angew. Chemie Int. Ed. 44 (34), 5384-5427, 2005. doi:10.1002/anie.200462661).

Oki defined atropisomers as conformers that interconvert with a half-life of more than 1000 seconds at a given temperature (Oki M, Topics in Stereochemistry 14, 1-82, 1983).

Atropisomers differ from other chiral compounds in that in many cases they can be equilibrated thermally whereas in the other forms of chirality isomerization is usually only possible chemically.

Separation of atropisomers is possible by chiral resolution methods such as selective crystallization. In an atropo-enantioselective or atroposelective synthesis one atropisomer is formed at the expense of the other. Atroposelective synthesis may be carried out by use of chiral auxiliaries like a Corey Bakshi Shibata (CBS) catalyst, an asymmetric catalyst derived from proline, or by approaches based on thermodynamic equilibration when an isomerization reaction favors one atropisomer over the other.

Racemic forms of compounds of formula (I) as well as the individual atropisomers (substantially free of its corresponding enantiomer) and stereoisomer-enriched atropisomer mixtures are included in the scope of the present invention.

The invention further concerns the corresponding deuterated derivatives of compounds of formula (I). In the context of the present invention, deuterated derivative means that at least one position occupied by a hydrogen atom is occupied by deuterium in an amount above its natural abundance. Preferably, the percent of deuterium at that position is at least 90%, more preferably at least 95%, even more preferably 99%.

All preferred groups or embodiments described above and here below for compounds of formula (I) may be combined among each other and apply as well *mutatis mutandis.*

As above mentioned, the present invention refers to compounds of general formula (I), acting as JAK inhibitors, to processes for the preparation thereof, pharmaceutical compositions comprising them either alone or in combination with one or more active ingredient, in admixture with one or more pharmaceutically acceptable carriers.

In a first aspect the present invention is directed to a class of compounds derivatives of formula I

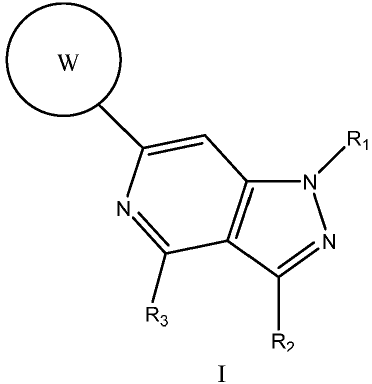

Wherein,
W is a heteroaryl selected from pyrazolo[1,5-a]pyrimidin-3-yl, imidazo[1,2-b]pyridazin-3-yl and (3-oxo-3,4-dihydropyrazin-2-yl)amino;
R₁ is selected in the group of pyridinyl, piperidinyl, phenyl or benzyl substituted by at least 2 or 3 groups, independently selected from
   halogen, preferably Cl and F,
   -OH,
   -CN
   -NO₂
   -(CH₂)ₘNR₄R₅, which is preferably -NH₂
   (C₁-C₆)alkyl,
   (C₁-C₆)hydroxyalkyl,
   (C₁-C₆)alkoxy, preferably methoxy,
   (C₁-C₆)alkylthio-
   (C₁-C₆)haloalkyl,
   (C₁-C₆)haloalkoxy, preferably difluoromethoxy
   (C₁-C₆)haloalkylthio-
   and R₁, particularly preferably when it is phenyl, it is optionally substituted by at least one further group of formula K 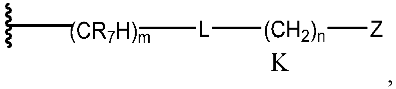 in meta position with respect to the point of attachment of R₁ with the rest of the molecule;
   **L** is absent or is a divalent group selected from O, S, S(O)₂, (CO), C(O)O, O(O)C, C(O)N(R₆), N(R₆)C(O), NHCONH, N(R₆)S(O)₂, S(O)₂N(R₆);
   Z is selected from the group consisting of H, -OH, -CN, -NO₂, (C₁-C₆)alkyl, (C₁-C₆)hydroxyalkyl, (C₁-C₆)haloalkyl, (C₁-C₆)alkoxy, (C₁-C₆)alkoxy (C₁-C₆)alkyl, (C₁-C₆)alkoxycarbonyl, -(CH₂)ₘNR₄R₅, -C(O)NH(R₆), (C₃-C₈)cycloalkyl, aryl, heteroaryl and (C₃-C₆)heterocycloalkyl; wherein said
   (C₃-C₈)cycloalkyl, aryl, heteroaryl and (C₃-C₆)heterocycloalkyl are further optionally substituted by one or more substituents selected from the group consisting of
   (C₁-C₁₀)alkyl, (C₁-C₆)alkoxy, alkanoyl, (C₁-C₆)alkoxycarbonyl, oxo, - C(O)NH(R₆), (C₁-C₆)alkoxy(C₁-C₆)alkyl;
   **R₂ and R₃** (C₁-C₆)alkyl, preferably methyl, and a group of formula J 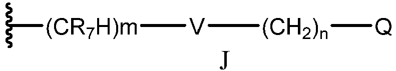
   wherein
   V is absent or is a divalent group selected from O, S, S(O)₂, C(O), C(O)O, O(O)C, C(O)N(R₆), N(R₆)C(O); N(R₆)-(CH₂)ₘ-N(R₆), -N(R₆)-,
   **Q** is selected from the group consisting of H, -CN, -OH, (C₁-C₆)alkyl, (Ci-C₆)hydroxyalkyl, (C₁-C₆)haloalkyl, (C₁-C₆)alkoxy, (C₁-C₆)alkoxycarbonyl, hydroxycarbonyl, -(CH₂)ₘNR₄R₅, -C(O)NR₄R₅, -N(R₆)C(O)R₆, -CH(CN)NR₄R₅, (C₃-C₈)cycloalkyl, aryl, heteroaryl and (C₃-C₆)heterocycloalkyl; wherein said (C₃-C₈)cycloalkyl, aryl, heteroaryl and (C₃-C₆)heterocycloalkyl are further optionally substituted by one or more substituents selected from the group consisting of -OH, oxo, (C₁-C₁₀)alkyl, (C₁-C₁₀)alkyl-S(O)₂-O-, alkanoyl, (C₁-C₆)hydroxyalkyl, (C₁-C₆)alkoxy(C₁-C₆)alkyl, (C₁-C₆)alkoxycarbonyl, (C₁-C₆)alkoxycarbonyl-NH-, -N(R₆)(CH₂)ₘC(O)NR₄R₅, -NR₄R₅, (C₃-C₆)heterocycloalkyl;
   wherein **n and m are in each occurrence independently** 0 or an integer selected from 1, 2, 3 and 4;
   **R₄ and R₅**, the same or different, are selected from the group consisting of
   -H,
   (C₁-C₆)alkyl,
   (C₁-C₆)haloalkyl,
   (C₁-C₆)hydroxyalkyl,
   alkanoyl, (C₁-C₆)alkoxycarbonyl, and
   (C₃-C₆)heterocycloalkyl;
   R₆ is in each occurrence independently selected from the group consisting of H, (C₁-C₆)alkyl, (C₁-C₆)hydroxyalkyl, and alkanoyl,
   R₇ is in each occurrence independently selected from the group consisting of H, (C₁-C₆)alkyl, -NR₄R₅
   or a pharmaceutically acceptable salt or solvate thereof.

In a preferred embodiment the present invention provides compounds of formula (I) further having R₁ which is phenyl substituted by 2 or 3 groups, independently selected from
halogen, preferably Cl and F,
-OH,
(C₁-C₆)alkoxy, preferably methoxy,
(C₁-C₆)alkylthio- preferably methylthio
(C₁-C₆)haloalkoxy, preferably difluoromethoxy
and all the other variables are as defined above.

The said preferred compounds showed advantageously balanced profile for inhalatory admistration.

In another preferred embodiment the present invention provides compounds of formula (I) further having R₂ which is a group J and R₁ which is a substituted phenyl, as represented in formula (Ib) 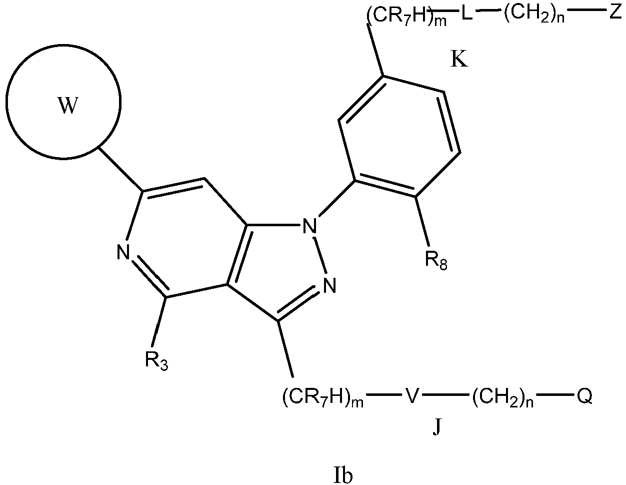 wherein
**R₈** is selected in the group consisting of
(C₁-C₆)alkoxy,
(C₁-C₆)haloalkoxy;
**L** is selected from the divalent group consisting of O, S, S(O)₂, C(O)O, O(O)C, C(O)N(R₆), N(R₆)C(O);
Z is selected from the group consisting of H, (C₁-C₆)alkyl, (C₁-C₆)hydroxyalkyl, (C₁-C₆)haloalkyl, (C₁-C₆)alkoxy, (C₁-C₆)alkoxycarbonyl, -(CH₂)ₘNR₄R₅, -C(O)NH(R₆),
(C₃-C₈)cycloalkyl, aryl, heteroaryl and (C₃-C₆)heterocycloalkyl optionally substituted by one or more substituents selected from the group consisting of
(C₁-C₁₀)alkyl, alkanoyl, (C₁-C₆)alkoxycarbonyl, -C(O)NH(R₆), (C₁-C₆)alkoxy(C₁-C₆)alkyl;
**R₃** is -H or (C₁-C₆)alkyl;
**V** is absent or is selected from the divalent group consisting of O, S, S(O)₂, C(O), C(O)O, O(O)C, C(O)N(R₆), N(R₆)C(O); N(R₆)-(CH₂)ₘ-N(R₆), -N(R₆)-,
**Q** is selected from the group consisting of H, -CN, -OH, (C₁-C₆)alkyl, (C₁-C₆)hydroxyalkyl, (C₁-C₆)haloalkyl, (C₁-C₆)alkoxy, (C₁-C₆)alkoxycarbonyl, - (CH₂)ₘNR₄R₅, -C(O)NR₄R₅, -N(R₆)C(O)R₆, -CH(CN)NR₄R₅,
(C₃-C₈)cycloalkyl, aryl , heteroaryl and (C₃-C₆)heterocycloalkyl optionally substituted by one or more substituents selected from the group consisting of -OH, oxo, (C₁-C₁₀)alkyl, (C₁-C₁₀)alkyl-S(O)₂-O-, alkanoyl, (C₁-C₆)hydroxyalkyl, (C₁-C₆)alkoxy(C₁-C₆)alkyl, (C₁-C₆)alkoxycarbonyl, (C₁-C₆)alkoxycarbonyl-NH-, -N(R₆)(CH₂)ₘC(O)NR₄R₅, -NR₄R₅, (C₃-C₆)heterocycloalkyl;
**n and m are in each occurrence independently** 0 or an integer from 1 to 4;
**R₄ and R₅**, the same or different, are selected from the group consisting of
-H,
(C₁-C₆)alkyl,
(C₁-C₆)haloalkyl,
(C₁-C₆)hydroxyalkyl,
alkanoyl,(C₁-C₆)alkoxycarbonyl, and
(C₃-C₆)heterocycloalkyl;
**R₆ is** (C₁-C₆)alkyl, (C₁-C₆)hydroxyalkyl,
**R₇ is** (C₁-C₆)alkyl,
or pharmaceutically acceptable salts and solvates thereof.

In a preferred embodiment the present invention provides compounds of formula (I) further having W which is (3-oxo-3,4-dihydropyrazin-2-yl)amino, as represented in formula (Ib1) 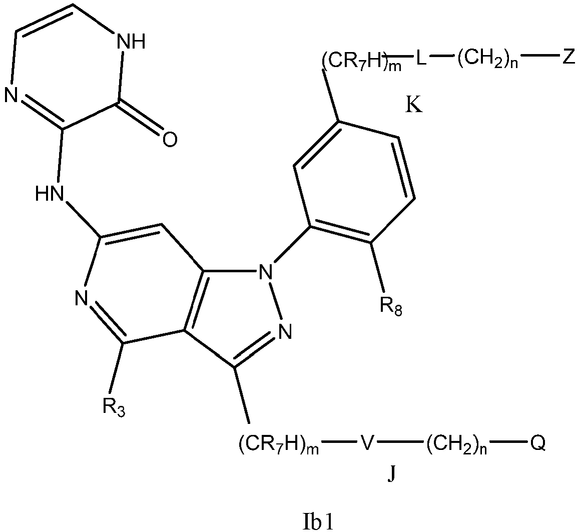 wherein
**R₈** is selected in the group consisting of
(C₁-C₆)alkoxy,
(C₁-C₆)haloalkoxy;
L is selected from the divalent group consisting of O, S, S(O)₂, C(O)O, O(O)C, C(O)N(R₆), N(R₆)C(O);
**Z** is selected from the group consisting of H, (C₁-C₆)alkyl, (C₁-C₆)hydroxyalkyl, (C₁-C₆)haloalkyl, (C₁-C₆)alkoxy, (C₁-C₆)alkoxycarbonyl, -(CH₂)ₘNR₄R₅, -C(O)NH(R₆),
(C₃-C₈)cycloalkyl, aryl, heteroaryl and (C₃-C₆)heterocycloalkyl optionally substituted by one or more substituents selected from the group consisting of
(C₁-C₁₀)alkyl, alkanoyl, (C₁-C₆)alkoxycarbonyl, -C(O)NH(R₆), (C₁-C₆)alkoxy(C₁-C₆)alkyl;
**R₃** is -H or (C₁-C₆)alkyl;
V is absent or is selected from the divalent group consisting of O, S, S(O)₂, C(O), C(O)O, O(O)C, C(O)N(R₆), N(R₆)C(O); N(R₆)-(CH₂)ₘ-N(R₆), -N(R₆)-,
**Q** is selected from the group consisting of H, -CN, -OH, (C₁-C₆)alkyl, (C₁-C₆)hydroxyalkyl, (C₁-C₆)haloalkyl, (C₁-C₆)alkoxy, (C₁-C₆)alkoxycarbonyl, - (CH₂)ₘNR₄R₅, -C(O)NR₄R₅, -N(R₆)C(O)R₆, -CH(CN)NR₄R₅,
(C₃-C₈)cycloalkyl, aryl , heteroaryl and (C₃-C₆)heterocycloalkyl optionally substituted by one or more substituents selected from the group consisting of -OH, oxo, (C₁-C₁₀)alkyl, (C₁-C₁₀)alkyl-S(O)₂-O-, alkanoyl, (C₁-C₆)hydroxyalkyl, (C₁-C₆)alkoxy(C₁-C₆)alkyl, (C₁-C₆)alkoxycarbonyl, (C₁-C₆)alkoxycarbonyl-NH-, -N(R₆)(CH₂)ₘC(O)NR₄R₅, -NR₄R₅, (C₃-C₆)heterocycloalkyl;
**n and m are in each occurrence independently** 0 or an integer from 1 to 4;
**R₄ and R₅,**
-H,
(C₁-C₆)alkyl,
(C₁-C₆)haloalkyl,
(C₁-C₆)hydroxyalkyl,
alkanoyl,(C₁-C₆)alkoxycarbonyl, and
(C₃-C₆)heterocycloalkyl;
**R₆** is in each occurrence independently selected from the group consisting of H, (C₁-C₆)alkyl, (C₁-C₆)hydroxyalkyl,
**R₇** is in each occurrence independently selected from the group consisting of H, (C₁-C₆)alkyl,
or pharmaceutically acceptable salts and solvates thereof.

Thus, a group of particularly preferred compounds are

| | |
|---|---|
| 90 | 3-((1-(5-fluoro-2-methoxyphenyl)-3-methyl-1H-pyrazolo[4,3-c]pyridin-6-yl)amino)pyrazin-2(1H)-one |
| 91 | 3-((1-(5-(difluoromethyl)-2-methoxyphenyl)-3-methyl-1H-pyrazolo[4,3-c]pyridin-6-yl)amino)pyrazin-2(1H)-one |
| 92 | N-(2-hydroxyethyl)-4-methoxy-3-(3-methyl-6-((3-oxo-3,4-dihydropyrazin-2-yl)amino)- 1H-pyrazolo[4,3-c]pyridin-1-yl)benzenesulfonamide |
| 93 | 3-((1-(2-(difluoromethoxy)-5-((2-methoxyethyl)thio)phenyl)-3-methyl-1H-pyrazolo[4,3-c]pyridin-6-yl)amino)pyrazin-2(1H)-one |
| 94 | 3-((1-(2-(difluoromethoxy)-5-(methylsulfonyl)phenyl)-3-methyl-1H-pyrazolo[4,3-c]pyridin-6-yl)amino)pyrazin-2(1H)-one |
| 95 | 3-((1-(2-(difluoromethoxy)-5-((2-methoxyethyl)sulfonyl)phenyl)-3-methyl-1H-pyrazolo[4,3-c]pyridin-6-yl)amino)pyrazin-2(1H)-one |
| 96 | N-(3-(dimethylamino)propyl)-1-(5-fluoro-2-methoxyphenyl)-6-((3-oxo-3,4-dihydropyrazin-2-yl)amino)-1H-pyrazolo[4,3-c]pyridine-3-carboxamide |
| 97 | 3-((3-((3-(dimethylamino)propyl)amino)-1-(5-fluoro-2-methoxyphenyl)-1H-pyrazolo[4,3-c]pyridin-6-yl)amino)pyrazin-2(1H)-one |
| 98 | 3-((1-(5-(difluoromethyl)-2-methoxyphenyl)-3-((2-(dimethylamino)ethyl)amino)-1H-pyrazolo[4,3-c]pyridin-6-yl)amino)pyrazin-2(1H)-one |
| 99 | 3-((1-(5-chloro-2-(difluoromethoxy)phenyl)-3-methyl-1H-pyrazolo[4,3-c]pyridin-6-yl)amino)pyrazin-2(1H)-one |

The said preferred compounds showed balanced profile for inhalatory admistration and preferably inhibitory concentration lower than 50 nM at least on JAK1-2-3.

Another particularly preferred embodiment, not part of the claimed subject-matter, is directed to compounds of formula I 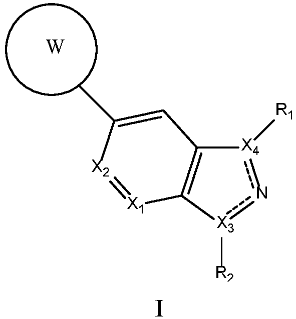
Wherein, X₁ and X₂ are alternatively N or CH; and
X₃ and X₄ are alternatively N or CH, and the two dashed lines indicate that a double bond is accordingly alternatively between X₃=N or between N=X₄,
W is a heteroaryl selected from pyrazolo[1,5-a]pyrimidin-3-yl, imidazo[1,2-b]pyridazin-3-yl
R₁ is selected in the group piperidinyl, phenyl or benzyl optionally substituted by one or more group selected from cyanomethylcarbonyl, difluoromethoxy, Cl and F.
R₂ is methyl or selected from hydroxycarbonylmethyl, metoxycarbonylmethyl, dimethylaminocarbonylmethyl, hydroxymethyl;
or pharmaceutically acceptable salts and solvates thereof.

According to specific embodiments, the present invention provides the compounds of examples 1a-10a (according to the last preferred embodiment described hereabove) and further compounds of examples 1-99, as listed in the table below, and pharmaceutical acceptable salts and solvates thereof.

| **Example** | **Chemical Name** |
|---|---|
| 1a | (1-(5-chloro-2-(difluoromethoxy)phenyl)-6-(pyrazolo[1,5-a]pyrimidin-3-yl)-1H-pyrazolo[4,3-b]pyridin-3-yl)methanol |
| 2a | 2-(3-(5-chloro-2-(difluoromethoxy)phenyl)-5-(pyrazolo[1,5-a]pyrimidin-3-yl)-1H-pyrazolo[3,4-b]pyridin-1-yl)-N,N-dimethylacetamide |
| 3a | methyl 1-(5-chloro-2-(difluoromethoxy)phenyl)-6-(pyrazolo[1,5-a]pyrimidin-3-yl)-1H-pyrazolo[4,3-b]pyridine-3-carboxylate |
| 4a | 2-(1-(5-chloro-2-(difluoromethoxy)phenyl)-6-(pyrazolo[1,5-a]pyrimidin-3-yl)-1H-pyrazolo[4,3-b]pyridin-3-yl)-N,N-dimethylacetamide |
| 5a | 2-(1-(5-chloro-2-(difluoromethoxy)phenyl)-6-(pyrazolo[1,5-a]pyrimidin-3-yl)-1H-pyrazolo[4,3-b]pyridin-3-yl)acetic acid |
| 6a | 1-(5-chloro-2-(difluoromethoxy)phenyl)-6-(imidazo[1,2-b]pyridazin-3-yl)-3-methyl-1H-pyrazolo[4,3-c]pyridine |
| 7a | 1-(5-chloro-2-(difluoromethoxy)phenyl)-3-methyl-6-(pyrazolo[1,5-a]pyrimidin-3-yl)-1H-pyrazolo[4,3-c]pyridine |
| 8a | 1-(4-chloro-2-fluorobenzyl)-6-( imidazo[1,2-b]pyridazin-3-yl )-3-methyl-1H-pyrazolo[4,3-b]pyridine |
| 9a | 1-(5-chloro-2-(difluoromethoxy)phenyl)-3-methyl-6-(pyrazolo[1,5-a]pyrimidin-3-yl)-1H-pyrazolo[4,3-b]pyridine |
| 10a | 3-(3-(3-methyl-6-(pyrazolo[1,5-a]pyrimidin-3-yl)-1H-pyrazolo[4,3-b]pyridin-1-yl)piperidin-1-yl)-3-oxopropanenitrile |
| 1 | 1-(5-fluoro-2-methoxyphenyl)-3-methyl-6-(pyrazolo[1,5-a]pyrimidin-3-yl)-1H-pyrazolo[4,3-c]pyridine |
| 2 | 1-(5-chloro-2-methoxyphenyl)-3-methyl-6-(pyrazolo[1,5-a]pyrimidin-3-yl)-1H-pyrazolo[4,3-c]pyridine |
| 3 | 1-(5-(difluoromethyl)-2-methoxyphenyl)-3-methyl-6-(pyrazolo[1,5-a]pyrimidin-3-yl)-1H-pyrazolo[4,3-c]pyridine |
| 4 | 1-(2-methoxy-5-(trifluoromethyl)phenyl)-3-methyl-6-(pyrazolo[1,5-a]pyrimidin-3-yl)-1H-pyrazolo[4,3-c]pyridine |
| 5 | 1-(5-chloro-2-(methylthio)phenyl)-3-methyl-6-(pyrazolo[1,5-a]pyrimidin-3-yl)-1H-pyrazolo[4,3-c]pyridine |
| 6 | 1-(5-chloro-2-((difluoromethyl)thio)phenyl)-3-methyl-6-(pyrazolo[1,5-a]pyrimidin-3-yl)-1H-pyrazolo[4,3-c]pyridine |
| 7 | 1-(5-chloro-2-cyclopropoxyphenyl)-3-methyl-6-(pyrazolo[1,5-a]pyrimidin-3-yl)-1H-pyrazolo[4,3-c]pyridine |
| 8 | 1-(2,5-dimethoxyphenyl)-3-methyl-6-(pyrazolo[1,5-a]pyrimidin-3-yl)-1H-pyrazolo[4,3-c]pyridine |
| 9 | 3-((4-methoxy-3-(3-methyl-6-(pyrazolo[1,5-a]pyrimidin-3-yl)-1H-pyrazolo[4,3-c]pyridin-1-yl)phenyl)sulfonyl)propan-1-ol |
| 10 | 1-(2-methoxy-5-(propylsulfonyl)phenyl)-3-methyl-6-(pyrazolo[1,5-a]pyrimidin-3-yl)-1H-pyrazolo[4,3-c]pyridine |
| 11 | 1-(5-chloro-2-(difluoromethoxy)phenyl)-3-methyl-6-(pyrazolo[1,5-a]pyrimidin-3-yl)-1H-pyrazolo[4,3-c]pyridine |
| 12 | 4-chloro-2-(3-methyl-6-(pyrazolo[1,5-a]pyrimidin-3-yl)-1H-pyrazolo[4,3-c]pyridin-1-yl)phenol |
| 13 | 1-(2-(difluoromethoxy)-5-(methylthio)phenyl)-3-methyl-6-(pyrazolo[1,5-a]pyrimidin-3-yl)-1H-pyrazolo[4,3-c]pyridine |
| 14 | 4-methoxy-N-methyl-3-(3-methyl-6-(pyrazolo[1,5-a]pyrimidin-3-yl)-1H-pyrazolo[4,3-c]pyridin-1-yl)benzenesulfonamide |
| 15 | N-(2-hydroxyethyl)-4-methoxy-3-(3-methyl-6-(pyrazolo[1,5-a]pyrimidin-3-yl)-1H-pyrazolo[4,3-c]pyridin-1-yl)benzenesulfonamide |
| 16 | 4-methoxy-3-(3-methyl-6-(pyrazolo[1,5-a]pyrimidin-3-yl)-1H-pyrazolo[4,3-c]pyridin-1-yl)-N-(3-(4-methylpiperazin-1-yl)propyl)benzenesulfonamide |
| 17 | 4-methoxy-3-(3-methyl-6-(pyrazolo[1,5-a]pyrimidin-3-yl)-1H-pyrazolo[4,3-c]pyridin-1-yl)-N-((1-methylazetidin-3-yl)methyl)benzene sulfonamide |
| 18 | 4-methoxy-3-(3-methyl-6-(pyrazolo[1,5-a]pyrimidin-3-yl)-1H-pyrazolo[4,3-c]pyridin-1-yl)-N-(2-(4-methylpiperazin-1-yl)ethyl)benzenesulfonamide |
| 19 | 4-methoxy-3-(3-methyl-6-(pyrazolo[1,5-a]pyrimidin-3-yl)-1H-pyrazolo[4,3-c]pyridin-1-yl)-N-(2-morpholinoethyl)benzenesulfonamide |
| 20 | 1-(2-(difluoromethoxy)-5-((2-methoxyethyl)sulfonyl)phenyl)-3-methyl-6-(pyrazolo[1,5-a]pyrimidin-3-yl)-1H-pyrazolo[4,3-c]pyridine |
| 21 | 3-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)-N,N-dimethylpropan-1-amine |
| 22 | 2-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)ethan-1-ol |
| 23 | 1-(2-(difluoromethoxy)-5-((2-(piperidin-1-yl)ethyl)sulfonyl)phenyl)-3-methyl-6-(pyrazolo[1,5-a]pyrimidin-3-yl)-1H-pyrazolo[4,3-c]pyridine |
| 24 | 1-(3-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)pyrrolidin-1-yl)ethan-1-one |
| 25 | 1-(2-(difluoromethoxy)-5-((3-methoxyphenyl)sulfonyl)phenyl)-3-methyl-6-(pyrazolo[1,5-a]pyrimidin-3-yl)-1H-pyrazolo[4,3-c]pyridine |
| 26 | 1-(2-(difluoromethoxy)-5-(methylsulfonyl)phenyl)-3-methyl-6-(pyrazolo[1,5-a]pyrimidin-3-yl)-1H-pyrazolo[4,3-c]pyridine |
| 27 | 3-methoxy-4-(3-methyl-6-(pyrazolo[1,5-a]pyrimidin-3-yl)-1H-pyrazolo[4,3-c]pyridin-1-yl)phenol |
| 28 | 2-(1-(5-chloro-2-(difluoromethoxy)phenyl)-6-(pyrazolo[1,5-a]pyrimidin-3-yl)-1H-pyrazolo[4,3-c]pyridin-3-yl)acetonitrile |
| 29 | (1-(5-chloro-2-(difluoromethoxy)phenyl)-6-(pyrazolo[1,5-a]pyrimidin-3-yl)-1H-pyrazolo[4,3-c]pyridin-3-yl)methanol |
| 30 | 1-(1-(5-chloro-2-(difluoromethoxy)phenyl)-6-(pyrazolo[1,5-a]pyrimidin-3-yl)-1H-pyrazolo[4,3-c]pyridin-3-yl)-N,N-dimethylmethanamine |
| 31 | 1-(1-(5-chloro-2-(difluoromethoxy)phenyl)-6-(pyrazolo[1,5-a]pyrimidin-3-yl)-1H-pyrazolo[4,3-c]pyridin-3-yl)-N-methylmethanamine |
| 32 | (1-(5-chloro-2-(difluoromethoxy)phenyl)-6-(pyrazolo[1,5-a]pyrimidin-3-yl)-1H-pyrazolo[4,3-c]pyridin-3-yl)methanamine |
| 33 | 1-(5-chloro-2-(difluoromethoxy)phenyl)-N-methyl-6-(pyrazolo[1,5-a]pyrimidin-3-yl)-1H-pyrazolo[4,3-c]pyridine-3-carboxamide |
| 34 | 1-(5-chloro-2-methoxyphenyl)-N-((1s,3s)-3-hydroxycyclobutyl)-6-(pyrazolo[1,5-a]pyrimidin-3-yl)-1H-pyrazolo[4,3-c]pyridine-3-carboxamide |
| 35 | (1-(5-chloro-2-(difluoromethoxy)phenyl)-6-(pyrazolo[1,5-a]pyrimidin-3-yl)-1H-pyrazolo[4,3-c]pyridin-3-yl)(1,1-dioxidothiomorpholino)methanone |
| 36 | 1-(5-chloro-2-(difluoromethoxy)phenyl)-N-((1s,3s)-3-(dimethylamino)cyclobutyl)-6-(pyrazolo[1,5-a]pyrimidin-3-yl)-1H-pyrazolo[4,3-c]pyridine-3-carboxamide |
| 37 | 1-(2-(difluoromethoxy)-5-(methylthio)phenyl)-N-(3-(dimethylamino)propyl)-6-(pyrazolo[1,5-a]pyrimidin-3-yl)-1H-pyrazolo[4,3-c]pyridine-3-carboxamide |
| 38 | N-(3-(dimethylamino)propyl)-1-(5-fluoro-2-methoxyphenyl)-6-(pyrazolo[1,5-a]pyrimidin-3-yl)-1H-pyrazolo[4,3-c]pyridine-3-carboxamide |
| 39 | 1-(5-chloro-2-methoxyphenyl)-6-(pyrazolo[1,5-a]pyrimidin-3-yl)-1H-pyrazolo[4,3-c]pyridin-3-amine |
| 40 | N-(1-(5-chloro-2-methoxyphenyl)-6-(pyrazolo[1,5-a]pyrimidin-3-yl)-1H-pyrazolo[4,3-c]pyridin-3-yl)acetamide |
| 41 | 1-(5-chloro-2-methoxyphenyl)-N-methyl-6-(pyrazolo[1,5-a]pyrimidin-3-yl)-1H-pyrazolo[4,3-c]pyridin-3-amine |
| 42 | 1-(5-chloro-2-methoxyphenyl)-N,N-dimethyl-6-(pyrazolo[1,5-a]pyrimidin-3-yl)-1H-pyrazolo[4,3-c]pyridin-3-amine |
| 43 | 1-(2-methoxy-5-(methylsulfonyl)phenyl)-N-(2-morpholinoethyl)-6-(pyrazolo[1,5-a]pyrimidin-3-yl)-1H-pyrazolo[4,3-c]pyridin-3-amine |
| 44 | N1-(1-(5-(difluoromethyl)-2-methoxyphenyl)-6-(pyrazolo[1,5-a]pyrimidin-3-yl)-1H-pyrazolo[4,3-c]pyridin-3-yl)-N2,N2-dimethylethane-1,2-diamine |
| 45 | N1-(1-(2-methoxy-5-methylphenyl)-6-(pyrazolo[1,5-a]pyrimidin-3-yl)-1H-pyrazolo[4,3-c]pyridin-3-yl)-N2,N2-dimethylethane-1,2-diamine |
| 46 | N1-(1-(2-(difluoromethoxy)-5-(methylthio)phenyl)-6-(pyrazolo[1,5-a]pyrimidin-3-yl)-1H-pyrazolo[4,3-c]pyridin-3-yl)-N3,N3-dimethylpropane-1,3-diamine |
| 47 | 1-(4-(1-(5-chloro-2-methoxyphenyl)-6-(pyrazolo[1,5-a]pyrimidin-3-yl)-1H-pyrazolo[4,3-c]pyridin-3-yl)piperazin-1-yl)ethan-1-one |
| 48 | 1-(5-chloro-2-methoxyphenyl)-3-(piperazin-1-yl)-6-(pyrazolo[1,5-a]pyrimidin-3-yl)-1H-pyrazolo[4,3-c]pyridine |
| 49 | (1-(1-(5-chloro-2-methoxyphenyl)-6-(pyrazolo[1,5-a]pyrimidin-3-yl)-1H-pyrazolo[4,3-c]pyridin-3-yl)azetidin-3-yl)methanol |
| 50 | 1-(5-chloro-2-methoxyphenyl)-3-methoxy-6-(pyrazolo[1,5-a]pyrimidin-3-yl)-1H-pyrazolo[4,3-c]pyridine |
| 51 | 1-(5-chloro-2-methoxyphenyl)-N,N-dimethyl-6-(pyrazolo[1,5-a]pyrimidin-3-yl)-1H-pyrazolo[4,3-c]pyridin-4-amine |
| 52 | 1-(5-chloro-2-methoxyphenyl)-N-methyl-6-(pyrazolo[1,5-a]pyrimidin-3-yl)-1H-pyrazolo[4,3-c]pyridin-4-amine |
| 53 | N-(2-((1-(5-chloro-2-methoxyphenyl)-6-(pyrazolo[1,5-a]pyrimidin-3-yl)-1H-pyrazolo[4,3-c]pyridin-4-yl)amino)ethyl)acetamide |
| 54 | 2-((1-(5-chloro-2-methoxyphenyl)-6-(pyrazolo[1,5-a]pyrimidin-3-yl)-1H-pyrazolo[4,3-c]pyridin-4-yl)amino)-N-methylacetamide |
| 55 | N-(1-(5-chloro-2-methoxyphenyl)-6-(pyrazolo[1,5-a]pyrimidin-3-yl)-1H-pyrazolo[4,3-c]pyridin-4-yl)acetamide |
| 56 | 1-(5-chloro-2-methoxyphenyl)-6-(pyrazolo[1,5-a]pyrimidin-3-yl)-1H-pyrazolo[4,3-c]pyridin-4-amine |
| 57 | 1-(5-chloro-2-methoxyphenyl)-6-(pyrazolo[1,5-a]pyrimidin-3-yl)-1H-pyrazolo[4,3-c]pyridin-4-ol |
| 58 | 2-fluoro-5-methoxy-4-(3-methyl-6-(pyrazolo[1,5-a]pyrimidin-3-yl)-1H-pyrazolo[4,3-c]pyridin-1-yl)phenol |
| 59 | 2-chloro-5-methoxy-4-(3-methyl-6-(pyrazolo[1,5-a]pyrimidin-3-yl)-1H-pyrazolo[4,3-c]pyridin-1-yl)phenol |
| 60 | 5-methoxy-2-methyl-4-(3-methyl-6-(pyrazolo[1,5-a]pyrimidin-3-yl)-1H-pyrazolo[4,3-c]pyridin-1-yl)phenol |
| 61 | 1-(2-chloro-5-methoxypyridin-4-yl)-3-methyl-6-(pyrazolo[1,5-a]pyrimidin-3-yl)-1H-pyrazolo[4,3-c]pyridine |
| 62 | (4-chloro-2-(3-methyl-6-(pyrazolo[1,5-a]pyrimidin-3-yl)-1H-pyrazolo[4,3-c]pyridin-1-yl)phenyl)methanol |
| 63 | 1-(5-bromo-2-(difluoromethoxy)phenyl)-3-methyl-6-(pyrazolo[1,5-a]pyrimidin-3-yl)-1H-pyrazolo[4,3-c]pyridine |
| 64 | 4-(difluoromethoxy)-3-(3-methyl-6-(pyrazolo[1,5-a]pyrimidin-3-yl)-1H-pyrazolo[4,3-c]pyridin-1-yl)benzonitrile |
| 65 | 1-(2-(difluoromethoxy)-5-methylphenyl)-3-methyl-6-(pyrazolo[1,5-a]pyrimidin-3-yl)-1H-pyrazolo[4,3-c]pyridine |
| 66 | 4-(difluoromethoxy)-3-(3-methyl-6-(pyrazolo[1,5-a]pyrimidin-3-yl)-1H-pyrazolo[4,3-c]pyridin-1-yl)-N-(thiazol-2-yl)benzamide |
| 67 | 2-((4-(difluoromethoxy)-3-(3-methyl-6-(pyrazolo[1,5-a]pyrimidin-3-yl)-1H-pyrazolo[4,3-c]pyridin-1-yl)phenyl)thio)-N-methylacetamide |
| 68 | 2-((4-(difluoromethoxy)-3-(3-methyl-6-(pyrazolo[1,5-a]pyrimidin-3-yl)-1H-pyrazolo[4,3-c]pyridin-1-yl)phenyl)thio)acetamide |
| 69 | 2-((4-(difluoromethoxy)-3-(3-methyl-6-(pyrazolo[1,5-a]pyrimidin-3-yl)-1H-pyrazolo[4,3-c]pyridin-1-yl)phenyl)thio)-N-(2-hydroxyethyl)acetamide |
| 70 | 2-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)acetamide |
| 71 | 1-(5-(cyclopropylthio)-2-(difluoromethoxy)phenyl)-3-methyl-6-(pyrazolo[1,5-a]pyrimidin-3-yl)-1H-pyrazolo[4,3-c]pyridine |
| 72 | 1-(2-(difluoromethoxy)-5-((tetrahydro-2H-pyran-4-yl)thio)phenyl)-3-methyl-6-(pyrazolo[1,5-a]pyrimidin-3-yl)-1H-pyrazolo[4,3-c]pyridine |
| 73 | 1-(2-(difluoromethoxy)-5-(oxetan-3-ylthio)phenyl)-3-methyl-6-(pyrazolo[1,5-a]pyrimidin-3-yl)-1H-pyrazolo[4,3-c]pyridine |
| 74 | 1-(2-(difluoromethoxy)-5-(piperidin-4-ylthio)phenyl)-3-methyl-6-(pyrazolo[1,5-a]pyrimidin-3-yl)-1H-pyrazolo[4,3-c]pyridine |
| 75 | 1-(4-((4-(difluoromethoxy)-3-(3-methyl-6-(pyrazolo[1,5-a]pyrimidin-3-yl)-1H-pyrazolo[4,3-c]pyridin-1-yl)phenyl)thio)piperidin-1-yl)ethan-1-one |
| 76 | 1-((1-(5-chloro-2-(difluoromethoxy)phenyl)-6-(pyrazolo[1,5-a]pyrimidin-3-yl)-1H-pyrazolo[4,3-c]pyridin-3-yl)methyl)azetidin-3-amine |
| 77 | N-((1r,3r)-3-aminocyclobutyl)-1-(5-chloro-2-methoxyphenyl)-6-(pyrazolo[1,5-a]pyrimidin-3-yl)-1H-pyrazolo[4,3-c]pyridine-3-carboxamide |
| 78 | 1-(5-chloro-2-methoxyphenyl)-N-((1r,3r)-3-(methyl(2-(methylamino)-2-oxoethyl)amino)cyclobutyl)-6-(pyrazolo[1,5-a]pyrimidin-3-yl)-1H-pyrazolo[4,3-c]pyridine-3-carboxamide |
| 79 | 1-(5-chloro-2-methoxyphenyl)-N-((1r,3r)-3-morpholinocyclobutyl)-6-(pyrazolo[1,5-a]pyrimidin-3-yl)-1H-pyrazolo[4,3-c]pyridine-3-carboxamide |
| 80 | 1-(5-chloro-2-methoxyphenyl)-N-((1r,3r)-3-(dimethylamino)cyclobutyl)-6-(pyrazolo[1,5-a]pyrimidin-3-yl)-1H-pyrazolo[4,3-c]pyridine-3-carboxamide |
| 81 | N-((1s,3s)-3-aminocyclobutyl)-1-(5-chloro-2-(difluoromethoxy)phenyl)-6-(pyrazolo[1,5-a]pyrimidin-3-yl)-1H-pyrazolo[4,3-c]pyridine-3-carboxamide |
| 82 | N-(2-(dimethylamino)ethyl)-1-(2-methoxy-5-(methylsulfonyl)phenyl)-6-(pyrazolo[1,5-a]pyrimidin-3-yl)-1H-pyrazolo[4,3-c]pyridine-3-carboxamide |
| 83 | N-(3-(dimethylamino)propyl)-1-(2-methoxy-5-(methylsulfonyl)phenyl)-6-(pyrazolo[1,5-a]pyrimidin-3-yl)-1H-pyrazolo[4,3-c]pyridine-3-carboxamide |
| 84 | 1-(2-methoxy-5-(methylsulfonyl)phenyl)-N-(3-morpholinopropyl)-6-(pyrazolo[1,5-a]pyrimidin-3-yl)-1H-pyrazolo[4,3-c]pyridine-3-carboxamide |
| 85 | 1-(2-methoxy-5-(methylsulfonyl)phenyl)-N-methyl-6-(pyrazolo[1,5-a]pyrimidin-3-yl)-1H-pyrazolo[4,3-c]pyridin-3-amine |
| 86 | N-(3-(3-((2-(dimethylamino)ethyl)amino)-6-(pyrazolo[1,5-a]pyrimidin-3-yl)-1H-pyrazolo[4,3-c]pyridin-1-yl)-4-methoxyphenyl)methanesulfonamide |
| 87 | N-(1-(5-chloro-2-methoxyphenyl)-6-(pyrazolo[1,5-a]pyrimidin-3-yl)-1H-pyrazolo[4,3-c]pyridin-3-yl)-2-(methylamino)acetamide |
| 88 | N-(3-(dimethylamino)propyl)-1-(5-fluoro-4-hydroxy-2-methoxyphenyl)-6-(pyrazolo[1,5-a]pyrimidin-3-yl)-1H-pyrazolo[4,3-c]pyridine-3-carboxamide |
| 89 | 1-(1-(2-methoxy-5-(methylsulfonyl)phenyl)-6-(pyrazolo[1,5-a]pyrimidin-3-yl)-1H-pyrazolo[4,3-c]pyridin-3-yl)-N-methylmethanamine |
| 90 | 3-((1-(5-fluoro-2-methoxyphenyl)-3-methyl-1H-pyrazolo[4,3-c]pyridin-6-yl)amino)pyrazin-2(1H)-one |
| 91 | 3-((1-(5-(difluoromethyl)-2-methoxyphenyl)-3-methyl-1H-pyrazolo[4,3-c]pyridin-6-yl)amino)pyrazin-2(1H)-one |
| 92 | N-(2-hydroxyethyl)-4-methoxy-3-(3-methyl-6-((3-oxo-3,4-dihydropyrazin-2-yl)amino)-1H-pyrazolo[4,3-c]pyridin-1-yl)benzenesulfonamide |
| 93 | 3-((1-(2-(difluoromethoxy)-5-((2-methoxyethyl)thio)phenyl)-3-methyl-1H-pyrazolo[4,3-c]pyridin-6-yl)amino)pyrazin-2(1H)-one |
| 94 | 3-((1-(2-(difluoromethoxy)-5-(methylsulfonyl)phenyl)-3-methyl-1H-pyrazolo[4,3-c]pyridin-6-yl)amino)pyrazin-2(1H)-one |
| 95 | 3-((1-(2-(difluoromethoxy)-5-((2-methoxyethyl)sulfonyl)phenyl)-3-methyl-1H-pyrazolo[4,3-c]pyridin-6-yl)amino)pyrazin-2(1H)-one |
| 96 | N-(3-(dimethylamino)propyl)-1-(5-fluoro-2-methoxyphenyl)-6-((3-oxo-3,4-dihydropyrazin-2-yl)amino)-1H-pyrazolo[4,3-c]pyridine-3-carboxamide |
| 97 | 3-((3-((3-(dimethylamino)propyl)amino)-1-(5-fluoro-2-methoxyphenyl)-1H-pyrazolo[4,3-c]pyridin-6-yl)amino)pyrazin-2(1H)-one |
| 98 | 3-((1-(5-(difluoromethyl)-2-methoxyphenyl)-3-((2-(dimethylamino)ethyl)amino)-1H-pyrazolo[4,3-c]pyridin-6-yl)amino)pyrazin-2(1H)-one |
| 99 | 3-((1-(5-chloro-2-(difluoromethoxy)phenyl)-3-methyl-1H-pyrazolo[4,3-c]pyridin-6-yl)amino)pyrazin-2(1H)-one |

The compounds of the invention, including all the compounds hereabove listed, can be prepared from readily available starting materials using general methods and procedures as described in the experimental part below or by using slightly modified processes readily available to those of ordinary skill in the art. Although a particular embodiment of the present invention may be shown or described herein, those skilled in the art will recognize that all embodiments or aspects of the present invention can be prepared using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures. The general schemes and detailed procedures are described in the PREPARATION OF INTERMEDIATES AND EXAMPLES sections below.

As herein described in detail, the compounds of the invention are inhibitors of kinase activity, in particular. inhibiting JAK kinase activity for the treatment of JAK-dependent diseases.

In one aspect the invention provides compounds according to the invention, i.e. a compound of formula (I) or a pharmaceutical composition thereof, for use as a medicament, preferably for the prevention and /or treatment of respiratory and specifically pulmonary disease.

In a further aspect the invention provides the use of a compound (I), or a pharmaceutically acceptable salt thereof, in the manufacture of a medicament for the treatment of disorders associated with JAK mechanisms, particularly for the treatment of disorders such as respiratory and pulmonary diseases.

In particular the invention provides compounds of formula (I) for use in the prevention and /or treatment of pulmonary disease selected from the group consisting of asthma, chronic obstructive pulmonary disease COPD, idiopathic pulmonary fibrosis (IPF)acute lung injury and acute respiratory distress syndrome (ARDS).

Preferred is the use of the compounds of the invention for the prevention of the aforesaid disorders.

Equally preferred is the use of the compounds of the invention for the treatment of the aforesaid disorders.

Generally speaking, compounds which are JAK inhibitors may be useful in the treatment of many disorders associated with JAK enzyme mechanisms.

In one embodiment, the disorder that can be treated by the compound of the present invention is selected from the group consisting of asthma, chronic obstructive pulmonary disease (COPD) and interstitial lung disease such as idiopathic pulmonary fibrosis (IPF), acute lung injury and acute respiratory distress syndrome (ARDS).

In a further embodiment, the disorder is selected from asthma and chronic obstructive pulmonary disease (COPD).

As used in the present description, " effective amount" in reference to a compound of formula (I) or a pharmaceutically acceptable salt thereof or other pharmaceutically-active agent means an amount of the compound sufficient to treat the patient's condition but low enough to avoid serious side effects and it can nevertheless be routinely determined by the skilled artisan. The compounds of formula (I) or pharmaceutically acceptable salts thereof may be administered in effective amounts once or according to a dosing regimen wherein a number of doses are administered at varying intervals of time for a given period of time. Typical daily dosages may vary depending upon the particular route of administration chosen.

The invention also provides pharmaceutical compositions of compounds of formula (I) in admixture with one or more pharmaceutically acceptable carrier or excipient, for example those described in Remington's Pharmaceutical Sciences Handbook, XVII Ed., Mack Pub., N.Y., U.S.A.

The present invention is also directed to the compounds of the invention and their pharmaceutical compositions for medical use in various route of administration.

Administration of the compounds of the invention and their pharmaceutical compositions may be accomplished according to patient needs, for example, orally, nasally, parenterally (subcutaneously, intravenously, intramuscularly, intrasternally and by infusion), by inhalation, rectally, vaginally, topically, locally, transdermally, and by ocular administration.

Various solid oral dosage forms can be used for administering compounds of the invention including such solid forms as tablets, gelcaps, capsules, caplets, granules, lozenges and bulk powders. The compounds of the present invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like. Time release capsules, tablets and gels are also advantageous.

Various liquid oral dosage forms can also be used for administering compounds of the invention, including aqueous and non-aqueous solutions, emulsions, suspensions, syrups, and elixirs. Such dosage forms can also contain suitable known inert diluents such as water and suitable known excipients such as preservatives, wetting agents, sweeteners, flavorants, as well as agents for emulsifying and/or suspending the compounds of the invention. The compounds of the present invention may be formulated as injectable composition, for example to be injected intravenously, in the form of an isotonic sterile solution. Other preparations are also possible.

Suppositories for rectal administration of the compounds of the invention can be prepared by mixing the compound with a suitable excipient such as cocoa butter, salicylates and polyethylene glycols.

Formulations for vaginal administration can be in the form of cream, gel, paste, foam, or spray formula containing, in addition to the active ingredient, such as suitable carriers, are also known.

For topical administration the pharmaceutical composition can be in the form of creams, ointments, liniments, lotions, emulsions, suspensions, gels, solutions, pastes, powders, sprays, and drops suitable for administration to the skin, eye, ear or nose. Topical administration may also involve transdermal administration via means such as transdermal patches.

For the treatment of the diseases of the respiratory tract, the compounds according to the invention, as above said, may be administered by inhalation.

Inhalable preparations include inhalable powders, propellant-containing metering aerosols or propellant-free inhalable formulations and may be administered through a suitable inhalation device which may be respectively selected from dry powder inhaler, pressurized metered dosed inhaler, or a nebulizer.

For administration as a dry powder, single- or multi-dose inhalers known from the prior art may be utilized. In that case the powder may be filled in gelatine, plastic or other capsules, cartridges or blister packs or in a reservoir.

A diluent or carrier, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in the form of solutions or suspensions in an aqueous, alcoholic or hydroalcoholic medium and they may be delivered by jet or ultrasonic nebulizers known from the prior art or by soft-mist nebulizers such as Respimat^{©},a registered trademark of Boehringer Ingelheim Pharmaceuticals (Wachtel, H., Kattenbeck, S., Dunne, S. et al. Pulm Ther (2017) 3: 19.

The compounds of the invention, regardless of the route of administration, can be administered as the sole active agent or in combination (i.e. as co-therapeutic agents administered in fixed dose combination or in combined therapy of separately formulated active ingredients) with other pharmaceutical active ingredients.

The compounds of the invention can be administered as the sole active agent or in combination with other pharmaceutical active ingredients including those currently used in the treatment of respiratory disorders, and known to the skilled person, such as beta2-agonists, antimuscarinic agents, corticosteroids mitogen-activated kinases (P38 MAP kinases) inhibitors, nuclear factor kappa-B kinase subunit beta inhibitors (IKK2), human neutrophil elastase (HNE) inhibitors, phosphodiesterase 4 (PDE4) inhibitors, leukotriene modulators, non-steroidal anti-inflammatory agents (NSAIDs) and mucus regulators).

The invention is also directed to a kit comprising the pharmaceutical compositions of compounds of the invention alone or in combination with or in admixture with one or more pharmaceutically acceptable carriers and/or excipients and a device, which may be a single- or multi-dose dry powder inhaler, a metered dose inhaler or a nebulizer.

The dosages of the compounds of the invention depend upon a variety of factors including the particular disease to be treated, the severity of the symptoms, the route of administration, the frequency of the dosage interval, the particular compound utilized, the efficacy, toxicology profile, and pharmacokinetic profile of the compound.

A pharmaceutical composition comprising a compound of the invention suitable to be administered by inhalation is in various respirable forms, such as inhalable powders (DPI), propellant-containing metering aerosols (PMDI) or propellant-free inhalable formulations (e.g. UDV).

The invention is also directed to a device comprising the pharmaceutical composition comprising a compound according to the invention, which may be a single- or multi-dose dry powder inhaler, a metered dose inhaler and a nebulizer particularly soft mist nebulizer.

The following examples illustrate the invention in more detail.

The features of the invention will become apparent in the course of the following descriptions of exemplary embodiments which are given for illustration of the invention and are not intended to be limiting thereof.

### PREPARATION OF INTERMEDIATES AND EXAMPLES 1a-10a

The following compounds of Example 1a-10a reported in table 1 below were prepared and characterized as follows:

**Table 1**

| **Example No.** | **Structure** | **Chemical Name** | **Biochemical Potency JAK 1 (pIC50)** | **Cell Based assay PBMC (IL-2 stimulated pSTAT5)** | **1H-NMR** | **LC-MS** |
|---|---|---|---|---|---|---|
| 1a | 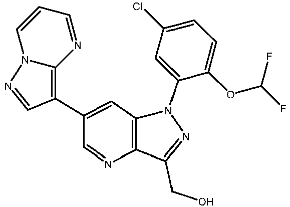 | (1-(5-chloro-2-(difluoromethoxy)p henyl)-6-(pyrazolo[1,5-a]pyrimidin-3-yl)-1H-pyrazolo[4,3-b]pyridin-3-yl)methanol | 8.1 | 7.3 | 1H-NMR (500 MHz, dmso-d6) δ: 9.47 (d, J = 1.8 Hz, 1H); 9.23 (dd, J = 7.1, 1.6 Hz, 1H); 9.00 (s, 1H); 8.72 (dd, J = 4.0, 1.7 Hz, 1H); 8.53 (d, J = 1.8 Hz, 1H); 7.81 (d, J = 2.5 Hz, 1H); 7.72 (dd, J = 8.8, 2.5 Hz, 1H); 7.59 (d, J = 8.8 Hz, 1H); 7.31 (t, J = 73 Hz, 1H); 7.18 (dd, J = 6.9, 4.0 Hz, 1H); 5.40 (t, J = 5.5 Hz, 1H); 4.92 (d, J = 5.3 Hz, 2H). | [MH]+ m/z 443.1/445.1 |
| 3a | 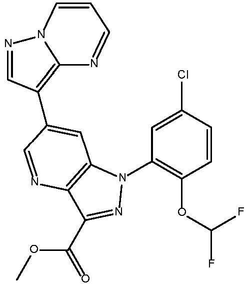 | methyl 1-(5-chloro-2-(difluoromethoxy)p henyl)-6-(pyrazolo[1,5-a]pyrimidin-3-yl)-1H-pyrazolo[4,3-b]pyridine-3-carboxylate | 8.1 | not yet available | 1H-NMR (500 MHz, dmso-d6) δ: 9.65 (d, J = 1.8 Hz, 1H); 9.24 (dd, J = 6.9, 1.6 Hz, 1H); 9.03 (s, 1H); 8.74 (dd, J = 4.0, 1.5 Hz, 1H); 8.59 (d, J = 4.0, 1.8 Hz, 1H); 7.99 (d, J = 2.6 Hz, 1H); 7.84 (dd, J = 8.8, 2.6 Hz, 1H); 7.64 (d, J = 8.8 Hz, 1H); 7.28 (t, J = 73 Hz, 1H); 7.20 (dd, J = 6.9, 4.0 Hz, 1H); 3.98 (s, 3H). | [MH]+ m/z 471.1/473.1 |
| 4a | 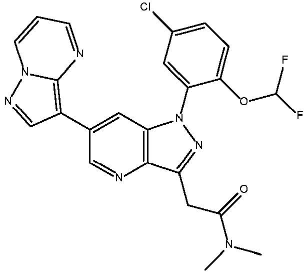 | 2-(1-(5-chloro-2-(difluoromethoxy)p henyl)-6-(pyrazolo[1,5-a]pyrimidin-3-yl)-1H-pyrazolo[4,3-b]pyridin-3-yl)-N,N-dimethylacetamide | 7.8 | 6.5 | 1H-NMR (500 MHz, dmso-d6) δ: 9.43 (d, J = 1.8 Hz, 1H); 9.23 (dd, J = 7.0, 1.7 Hz, 1H); 8.99 (s, 1H); 8.72 (dd, J = 4.0, 1.8Hz, 1H); 8.53 (d, J = 1.8 Hz, 1H); 7.78 (d, J = 2.6 Hz, 1H); 7.72 (dd, J = 8.9, 2.6 Hz, 1H); 7.58 (d, J = 8.9 Hz, 1H); 7.31 (t, J = 73 Hz, 1H); 7.18 (dd, J = 6.9, 4.0 Hz, 1H); 4.15 (s, 2H); 3.17 (s, 3H); 2.87 (s, 3H). | [MH]+ m/z 498.1/500.1 |
| 5a | 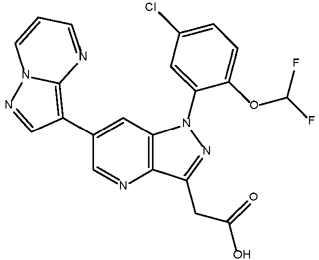 | 2-(1-(5-chloro-2-(difluoromethoxy)p henyl)-6-(pyrazolo[1,5-a]pyrimidin-3-yl)-1H-pyrazolo[4,3-b]pyridin-3-yl)acetic acid | 7.7 | 5.2 | 1H-NMR (500 MHz, dmso-d6) δ: 9.44 (d, J = 1.6 Hz, 1H); 9.23 (dd, J = 7.0, 1.7 Hz, 1H); 8.99 (s, 1H); 8.73 (dd, J = 4.0, 1.8Hz, 1H); 8.54 (d, J = 1.6 Hz, 1H); 7.79 (d, J = 2.6 Hz, 1H); 7.72 (dd, J = 8.9, 2.6 Hz, 1H); 7.58 (d, J = 8.9 Hz, 1H); 7.55 (bs, 1H); 7.32 (t, J = 73 Hz, 1H); 7.18 (dd, J = 6.9, 4.0 Hz, 1H); 7.11 (bs, 1H); 6.64 (bs, 1H); 4.02 (s, 2H). | [MH]+ m/z 471.6/473.5 |
| 6a | 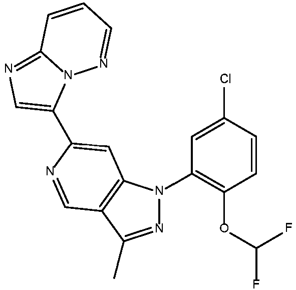 | 1-(5-chloro-2-(difluoromethoxy)p henyl)-6-( imidazo[1,2-b]pyridazin-3-yl)-3-methyl-1H-pyrazolo[4,3-c]pyridine | 8.4 | not yet available | 1H-NMR (600 MHz, dmso-d6) δ: 9.27 (d, J = 1.1 Hz, 1H); 8.70 (dd, J = 4.5, 1.7 Hz, 1H); 8.60 (d, J = 1.1 Hz, 1H); 8.58 (s, 1H); 8.29 (dd, J = 9.1, 1.7 Hz, 1H); 7.85 (d, J = 2.6 Hz, 1H); 7.74 (dd, J = 9.0, 2.6 Hz, 1H); 7.60 (d, J = 9.0, 1H); 7.36 (dd, J = 9.0, 4.3 Hz, 1H); 7.29 (t, J = 73 Hz, 1H); 2.69 (s, 3H). | [MH]+ m/z 427.0/429.0 |
| 7a | 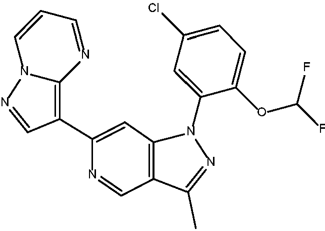 | 1-(5-chloro-2-(difluoromethoxy)p henyl)-3-methyl-6-(pyrazolo[1,5-a]pyrimidin-3-yl)-1H-pyrazolo[4,3-c]pyridine | 8.5 | 7.4 | 1H-NMR (600MHz, dmso-d6) δ: 9.22 (dd, J =6.9, 1.6 Hz, 1H); 9.19 (d, J = 1.1 Hz, 1H); 8.90 (s, 1H); 8.70 (dd, J = 4.0, 1.6 Hz, 1H); 8.34 (d, J = 1.1 Hz, 1H); 7.83 (d, J = 2.7 Hz, 1H); 7.73 (dd, J =8.9, 2.7 Hz, 1H); 7.59 (d, J = 8.9 Hz, 1H); 7.24 (t, J = 73 Hz, 1H); 7.16 (dd, J = 6.9, 4.0 Hz, 1H); 2.65 (s, 3H). | RT = 3.87, [MH]+ m/z 427.2/429.2 |
| 8a | 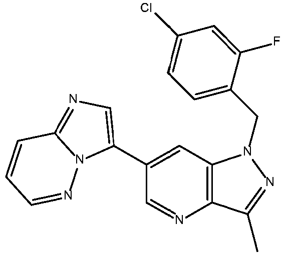 | 1-(4-chloro-2-fluorobenzyl)-6-( imidazo[1,2-b]pyridazin-3-yl )-3-methyl-1H-pyrazolo[4,3-b]pyridine | 6.7 | 6.1 | 1H-NMR (500 MHz, dmso-d6) δ: 9.19 (d, J = 1.1 Hz, 1H); 8.92 (d, J = 1.2 Hz, 1H); 8.72 (dd, J = 4.5, 1.0 Hz, 1H); 8.48 (s, 1H); 8.28 (dd, J = 9.0, 1.3 Hz, 1H); 7.47 (dd, J = 10.7, 1.2 Hz, 1H); 7.37 (dd, J = 9.1, 4.5 Hz, 1H); 7.27-7.32 (m, 2H); 5.69 (s, 2H); 2.54 (s, 3H). | [MH]+ m/z 393.2/395.2 |
| 9a | 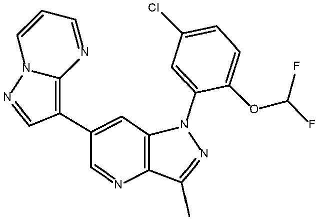 | 1-(5-chloro-2-(difluoromethoxy)p henyl)-3-methyl-6-(pyrazolo[1,5-a]pyrimidin-3-yl)-1H-pyrazolo[4,3-b]pyridine | 7.8 | 7.3 | 1H-NMR (600MHz, CDCl3) δ: 9.26 (d, J = 1.6 Hz, 1H); 8.76 (dd, J = 7.0, 1.6 Hz, 1H); 8.62 (dd, J = 4.0, 1.8 Hz, 1H); 7.58 (s, 1H); 8.45 (d, J = 1.6 Hz, 1H); 7.72 (d, J = 2.5 Hz, 1H); 7.46 (dd, J = 8.8, 2.4 Hz, 1H); 7.43 (d, J = 8.8 Hz, 1H); 6.95 (dd, J = 7.0, 4.0 Hz, 1H); 6.47 (t, J = 73 Hz, 1H); 2.79 (s, 3H). | [MH]+ m/z 427.5/429.4 |
| 10a | 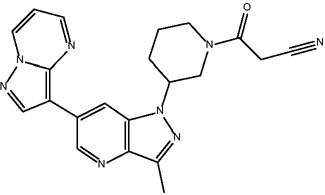 | 3-(3-(3-methyl-6-(pyrazolo[1,5-a]pyrimidin-3-yl)-1H-pyrazolo[4,3-b]pyridin-1-yl)piperidin-1-yl)-3-oxopropanenitrile | 6.0 | 5.2 | 1H- NMR (400 MHz, 353 K, dmso-d6) δ: 9.29 (d, J = 1.8 Hz, 1H); 9.12 (dd, J = 7.0, 1.6 Hz, 1H); 8.83 (s, 1H); 8.72 (dd, J = 4.1, 1.8 Hz, 1H); 8.62 (bs, 1H); 7.13 (dd, J = 7.1, 4.1 Hz, 1H); 4.70 (m, 1H); 3.87-4.06 (m, 3H); 3.01 (m, 3H); 2.56 (s, 3H); 2.21-2.23 (m, 2H); 1.88-1.97 (m, 1H); 1.64-1.78 (m, 1H). | [MH]+ m/z 401.6 |

The compound of Example 1a was prepared according to the following scheme:

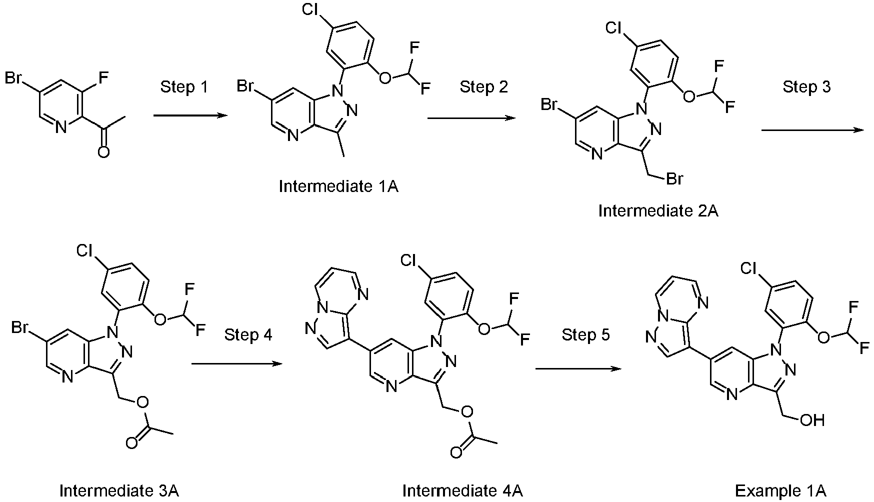

### Step 1

### Intermediate 1A: (6-bromo-1-[5-chloro-2-(difluoromethoxy)phenyl]-3-methyl-pyrazolo[4,3-b]pyridine)

A round-bottom flask was charged with 1-(5-bromo-3-fluoro-2-pyridyl)ethanone (2.00 g, 9.2 mmol), [5-chloro-2-(difluoromethoxy)phenyl]hydrazine hydrochloride (2.47 g, 10 mmol) and potassium carbonate (3.80 g, 28 mmol) in dimethylformammide (16 mL) and the reaction mixture stirred at 85 °C for 1.5 hours, then 120 °C for 4 h. After cooling to room temperature, the reaction mixture was diluted with ethyl acetate (80 mL), washed with aqueous saturated NaCl (3 x 30 mL) and the organic layer dried over Na₂SO₄. Solvent was partially removed under reduced pressure, and a solid was formed from the crude by standing at room temperature. The solid was filtered, washed with petrol ether and then dried to give the title product (1.797 g). ES⁺ m/z 388.0/390.0/392.0 [MH]⁺

### Step 2

### Intermediate 2A: (6-bromo-3-(bromomethyl)-1-[5-chloro-2-(difluoromethoxy)phenyl]pyrazolo[4,3-b]pyridine)

In a round-bottom flask under nitrogen were charged Intermediate 1A (200 mg, 0.51 mmol) and 1,2-dichloroethane (4.0 mL), then N-Bromosuccinimide (110 mg, 0.62 mmol) and AIBN (2,2'-Azobis(2-methylpropionitrile) (17 mg, 0.1 mmol) were added. The reaction mixture was heated at 80 °C for 2 h, then cooled to room temperature and quenched with water (10 mL). The resulting mixture was extracted with dichloromethane (3x5 mL), and the combined organics washed with aqueous saturated NaCl (10 mL) and dried over Na₂SO₄. After evaporation under reduced pressure, the crude was purified by SPE (solid phase extraction) on silica gel to give the title compound (134 mg). ES⁺ m/z 465.9/467.9/469.9/491.9 [MH]⁺

### Step 3

### Intermediate 3A: ([6-bromo-1-[5-chloro-2-(difluoromethoxy)phenyl]pyrazolo[4,3-b]pyridin-3-yl]methyl acetate)

A vial charged with Intermediate 2A (140 mg, 0.30 mmol), dimethylformamide (1.5 mL) and potassium acetate (103 mg, 1.0 mmol was heated at 60 °C for 1.5 hours. Reaction mixture was diluted with water (10 mL) and extracted with ethyl acetate (3x5 mL). Combined organics were washed with aqueous saturated NaCl (2x5 mL), dried over Na₂SO₄ and evaporated to dryness. The crude product was used in the next synthetic steps without further purification. ES⁺ m/z 446.0/448.0/450.0 [MH]⁺.

### Step 4

### Intermediate 4A: ([1-[5-chloro-2-(difluoromethoxy)phenyl]-6-pyrazolo[1,5-a]pyrimidin-3-yl-pyrazolo[4,3-b]pyridin-3-yl]methyl acetate)

Intermediate 3A (83 mg, 0.16 mmol) in THF (1 mL) and potassium phosphate tribasic solution (0.50 M, 0.65 mL, 0.33 mmol) in water were degassed with nitrogen for 10 min, then 3-(4,4,5,5-tetramethyl-1,3,2-dioxaborolan-2-yl)pyrazolo[1,5-a]pyrimidine (44 mg, 0.18 mmol) and XPhos-Pd-G3 ((2-Dicyclohexylphosphino-2',4',6'-triisopropyl-1,1'-biphenyl)[2-(2'-amino-1,1'-biphenyl)]palladium(II) methanesulfonate) (6.9 mg, 0.0082 mmol) were added. The reaction mixture was heated at 55 °C for 1.5 hours, cooled to room temperature and diluted with dichloromethane (10 mL) and water (10 mL).Aqueous layer was extracted with dichloromethane (4x5 mL), then combined organics washed with water (10 mL) and dried over Na₂SO₄. Solvent was removed under reduced pressure and crude residue purified by SPE (solid phase extraction) on silica gel to afford the title compound (66 mg). ES⁺ m/z 485.1.1/487.1 [MH]⁺.

### Step 5

### Example 1a: ([1-[5-chloro-2-(difluoromethoxy)phenyl]-6-pyrazolo[1,5-a]pyrimidin-3-yl-pyrazolo[4,3-b]pyridin-3-yl]methanol)

A round-bottom flask was charged with Intermediate 4A (95 %, 48 mg, 0.094 mmol) and methanol (5 mL), then K₂CO₃ (0.039 g, 0.28 mmol) was added and the mixture stirred at room temperature overnight. Solvent was evaporated and the crude residue purified by SPE (solid phase extraction) on silica gel to afford the title compound (30 mg). ES⁺ m/z 443.1/445.1 [MH]⁺.

The compounds of example 2a-10a were prepared in a similar manner to Example 1a, following the same synthetic sequence; modification of reaction conditions reactants or solvent used can be readily determined by those skilled in the art by routine optimization procedures.

### PHARMACOLOGICAL ACTIVITY OF THE COMPOUNDS OF THE INVENTION (1a-10a).

### Biochemical Potency JAK1 (Data displayed into the table 1 as pIC50)

The objective of this study was to assess the activity of novel JAK inhibitors measuring the capability of compounds to inhibit JAK1 kinase activity in a biochemical time-resolved fluorescence resonance energy transfer (TR-FRET) LANCE assay. In LANCE Ultra kinase assay in the presence of JAK1 kinase and ATP (corresponding to Km), the ULight peptide substrate (LANCE Ulight-JAK-1 (Tyr1023) Peptide, Perkin Elmer, TRF0121) was phosphorylated. It was then captured by a Eu-anti-phospho-substrate antibody (LANCE Eu-W1024 Anti-phosphotyrosine (PT66), Perkin Elmer, AD0069), which brought the Eu-chelate donor and ULight acceptor dyes into close proximity. Upon excitation at 320 nm, the Eu-chelate transfers its energy to the ULight dye, resulting in a fluorescent light emission at 665 nm. Inhibitors were tested at 11 consecutive 5-fold dilutions starting from 30 µM (30 µM - 3 pM) in duplicate. Calculation of IC50 data, curves and QC analysis were made using Excel tools and GraphPadPrism software. QC criteria parameters: Z' ≥ 0.5, Hill Slope range 0.5 to 5, S:B > 2.

In addition to enzymatic potency, the effects of the inhibitors against JAK1/JAK3 activity in a cellular assay was characterized against IL-2 induced phosphorylation of STAT5 level in human peripheral blood mononuclear cells (PBMCs).

### Cell Based assay PBMC (IL-2 stimulated pSTATS) (Data displayed into the table as pIC50)

PBMC have been isolated from human healthy volunteers. Cells were seeded in wells and treated with compounds and rh IL-2. After 30 min incubation cells were lysed and pSTAT5 determined by PathScan phospho-stat5 (Tyr694) ELISA (Cell signaling). Inhibitors were tested at 11 consecutive 5-fold dilutions starting from 30 µM (30 µM - 3 pM) in duplicate. Calculation of IC50 data, curves and QC analysis were made using Excel tools and GraphPadPrism software. QC criteria parameters: Z' ≥ 0.35, Hill Slope range 0.5 to 5, S:B > 2.

### NMR spectra

NMR spectra were recorded on a Bruker Avance III 600 (5 mm RT inverse probehead), Bruker DRX 500, Bruker Avance AV 400 (5 mm RT direct probehead) and Bruker DPX 300 spectrometers using standard Bruker pulse sequences. DMSO-d₆ or CDCl3 were used as solvents and TMS as the internal standard unless in the latter case where solvent residual peak was used. All experiments were recorded at 25 °C, unless stated differently.

LC-MS spectra were recorded on Acquity UPLC coupled with SQD mass spectrometer. **Chromatographic Columns:** Acquity UPLC BEH C18 (50mm x 2.1mm i.d., 1.7µm packing diameter), or Acquity UPLC BEH C18 (50mm x 2.1mm i.d., 1.7µm packing diameter), column temperature 40 °C. Mobile phase: A = = 0.1% v/v solution of formic acid in water, B = 0.1% v/v solution of formic acid in acetonitrile or A = 10 mM aqueous solution of NH4HCO3 (adjusted to pH 10 with ammonia) and B = Acetonitrile. Analytical samples were dissolved in mixture of water:acetonitrile (1:1). If necessery about 10 % of dmso was used in order to improve solubility.

### PREPARATION OF INTERMEDIATES AND EXAMPLES 1-99

Processes of preparation described below and reported in the following schemes should not be viewed as limiting the scope of the synthetic methods available for the preparation of the compounds of the invention.

Those skilled in the art will recognize that all embodiments or aspects of the present invention (including examples 1a to 10a) can be prepared using the methods described herein or easily adapted by using other known methods, reagents and starting materials.

In some cases a step is needed in order to mask or protect sensitive or reactive moieties, generally known protective groups (PG) could be employed, in accordance with general principles of chemistry (Protective group in organic syntheses, 3rd ed. T. W. Greene, P. G. M. Wuts).

Compounds of formula (I), here reported again for clarity, including all here above listed, can be usually prepared according to the procedures shown in the schemes below. Where a specific detail or step differs from the general schemes it has been detailed in the specific examples, and/or in additional schemes.

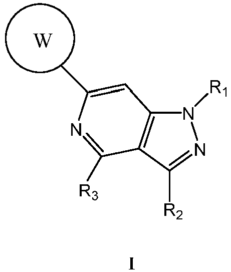

Compounds of formula **(I)** can be prepared according to *scheme 1.* Compound **IV** is an intermediate where general group r₁, r₂ , r₃ and w can be converted into R₁, R₂ R₃ and W respectively by mean of procedures well known to those skilled in the art such as protective groups deprotection or functional group conversion that may involve more than one step. Said procedures can be applied to one or more of those groups (r₁, r₂ , r₃ and w) to allow the conversion of intermediate **IV** into compound of general formula **I** and they are detailed in the experiemental section for specific examples. It is apparent that in the case such conversions are not needed (when r₁, r₂ , r₃ and w correspond to R₁, R₂ R₃ and W respectively), any general approach described below for the preparation of intermediate **IV** will provide a compound of general formula **I.**

Compound of formula **I** (or intermediate **IV)** can be obtained by direct introduction of W through a metal/palladium catalyzed cross coupling reaction such as Suzuki coupling, Stille coupling, Buchwald-Hartwig or similar (Strategic application of named reactions in organic synthesis, L. Kurti, B. Czako, Ed. 2005) by reaction of intermediate **II** with intermediate **III**.

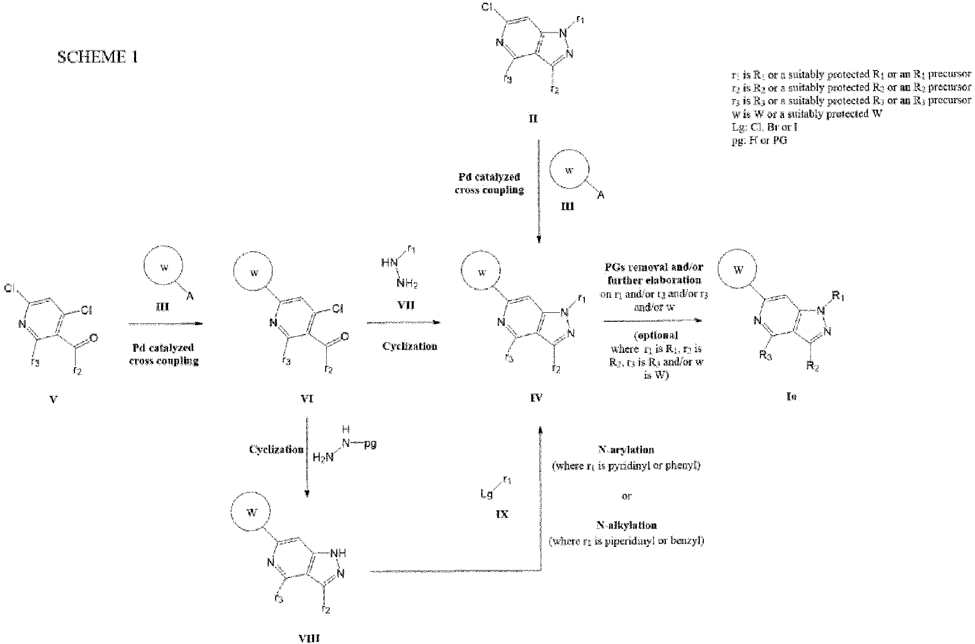

For example, a suitable palladium catalyzed cross coupling for introducing W, when it is an pyrazolo[1,5-a]pyrimidin-3-yl, is a Suzuki coupling. Suzuki coupling can be performed by reacting intermediate **II** with the corresponding boronic acid or boron pinacolate (intermediate **III,** where w is pyrazolo[1,5-a]pyrimidin-3-yl and A is dihydroxyboryl or 4,4,5,5-tetramethyl-1,3,2-dioxaborolanyl) in the presence of a Pd catalyst such as tetrakistriphenylphosphinepalladium(0), PdCl₂(dppf)₂, or a ligand-palladacycle precatalyst such as XPhos-Pd-G3 [(2-Dicyclohexylphosphino-2',4',6'-triisopropyl-1,1'-biphenyl)[2-(2'-amino-1,1'-biphenyl)]palladium(II) methanesulfonate], in an organic solvent such as 1,4-dioxane, THF, 1,2-dimethoxyethane, 2-propanol or DMF, with or without water, in the presence of an inorganic base such as an alkaline carbonate (for example Cs₂CO₃ or K₂CO₃) or an inorganic phosphate (for example K₃PO₄), under heating (typically in the range of 50-100°C) for few hours (typically 1 to 3h). Boronic acid and boronic pinacolate esters are generally commercially available or may be readily prepared by those skilled in the art starting from commercially available reagents. For synthetic convenience, primary/secondary amines and phenols that may be present in intermediates of Suzuki coupling need to be protected with suitable protective groups. A suitable protective group for protecting phenolic OH can be benzyl type protective groups such as PMB group (para-methoxybenzyl) or ether type such as MOM (monomethoxymethyl). PMB groups can be easily removed by treating corresponding PMB protected intermediate **IV** in acidic conditions with an organic or an inorganic strong acid. For example, PMB can be deprotected by treating the intermediates with trifluoroacetic acid neat or in mixture with an organic solvent such as DCM, THF or similar, typically at room temperature for few hours (typically 1 h). A suitable protective group for protecting primary and secondary amines, eventually present in r₂/r₃ groups, can be carbamate type protective groups such as Boc (tert-butoxycarbonyl). Boc group can be easily removed by treating Boc protected intermediate **IV** in acidic conditions with an organic or an inorganic strong acid. For example, Boc group can be cleaved by treating the intermediates with trifluoroacetic acid neat or in mixture with an organic solvent such as DCM, DCE, THF or similar, typically at room temperature for few hours (typically 1 to 3 h).

A suitable palladium catalyzed cross coupling for introducing **W** when it is an imidazo[1,2-b]pyridazin-3-yl, is a Stille coupling that can be performed by reacting intermediate **II** with the corresponding stannane (intermediate **III,** where w is imidazo[1,2-b]pyridazin-3-yl and A is tributylstannyl or a trimethylstannyl) in the presence of an appropriate palladium catalyst (such as Pd(PPh₃)2Cl₂) in a polar organic solvent (for example DMF or 1,4-dioxane with or without additives (like base or lithium salt). Stannanes are generally commercially available or may be readily prepared by those skilled in the art starting from commercially available reagents.

In another approach, **W** when it is an imidazo[1,2-b]pyridazin-3-yl can be introduced by a direct CH arylation by reacting intermediate **II** with the corresponding heterocycle (intermediate **III,** where w is imidazo[1,2-b]pyridazin-3-yl and A is H) in the presence of an appropriate palladium catalyst (such as Pd(Oac)₂) and a suitable phosphine (such as PCy₃ HBF₄ or CyJohnPhos) in a organic solvent (for example DMF, 1,4-dioxane or toluene) with a base (such as Cs₂CO₃ or K₂CO₃), with or without carboxilic acid additive (for example pivalic acid) by heating at temperature around 110°C.

A suitable palladium catalyzed cross coupling for introducing **W,** when it is an (3-oxo-3,4-dihydropyrazin-2-yl)amino, is a Buchwald-Hartwig coupling. For synthetic convenience the carbonyl group of (3-oxo-3,4-dihydropyrazin-2-yl)amino need to be masked as an alkoxy group (such as methoxy group) and removed at the end of the synthesis from intermediate **IV.** Intermediate **II** and intermediate **III** (where w is 3-methoxypyrazin-2-aminyl and A is H) can be reacted to give intermediate **IV** (where w is 3-methoxypyrazin-2-aminyl) in the presence of a suitable ligand palladacyle system such as RuPhos-Pd-G3 (2-Dicyclohexylphosphino-2',6'-diisopropoxy-1,1'-biphenyl)[2-(2'-amino-1,1'-biphenyl)]palladium(II) methanesulfonate) or in general a suitable Pd source (for example Pd₂(dba)₃ or Pd(OAc)₂) with a suitable biphenylphosphine ligand type (RuPhos, X-Phos, or similar), in the presence of a strong organic base such as sodium tert-butoxyde or an inorganic base such as Cs₂CO₃, in an organic solvent such 1,4-dioxane, THF or toluene, under heating at high temperature (typically 80-120°C), for few hours (typically 1-5 h). Methoxy group in intermediate **IV** can be demethylated to give compound of formula **I** (where W is (3-oxo-3,4-dihydropyrazin-2-yl)amino) by treatment with TMS-Cl (trimethylsilyl chloride) and sodium iodide in acetonitrile for 1 to 5 h at 60 - 100°C. The above described process may provide at least one non limiting synthetic route for the preparation of example 1 to 60, example 76, example 85 and 87 to 98 and one non

limiting synthetic route for the preparation of intermediate **IV,** where r₁, r₂, r₃ and/or w are independently precursors of R₁, R₂, R₃ and/or W.

In another approach, compound of formula **I** (or intermediate **IV)** can be prepared by means of a cyclization reaction ofintermediate **VI** with intermediate **VII.** Cyclization reaction can be performed by heating (typically 60-170°C) the required reagents in an polar organic solvent such as N-methyl pirrolidone (NMP), dimethylacetamide (DMA) or 1,2-dimethoxyethane (DME) for few hours (typically 1 to 5 h) or longer. Intermediate **VI** can be prepared from intermediate **V** and intermediate **III** through a palladium catalyzed cross coupling using similar conditions to those described above for reaction of intermediate **II** with intermediate **III.** This approach provides at least one non limiting synthetic route for the preparation of example 62, 63 and intermediates of formula **IV.**

In an alternative way, compound of formula **I** (or intermediate **VI)** can be prepared by N-arylation (when r₁/R₁ is pyridinyl or phenyl) or N-alkylation (when r₁/R₁ is piperidinyl or benzyl) of intermediate **VIII** with intermediate **IX.** An N-arylation can be performed by using copper catalyzed Ullmann type reaction. An Ullmann reaction between a NH heteroaryl and an aryl/heteroaryl halide (chloride, bromide or iodide) can be performed in the presence of a suitable copper(I) catalyst/promoter such as CuI, Cu₂O or CuTC (copper thiophene carboxylate), ligandless or with a suitable ligand such as N,N-dimethylglicine, proline or dimethylcyclohexane-1,2-diamine (DMCHA), in the presence of an inorganic base such as K₂CO₃ or Cs₂CO₃, by heating (typically 90-150°C) in a polar organic solvent such as DMSO, DMF or DMA, for few hours or longer (typically 3-12h). Intermediate **VIII** can be prepared from cyclization of intermediate **V** with hydrazine (or a protected derivative) using similar conditions to those described for reaction of intermediate **VI** with intermediate **VII.** This alternative process may provide at least one non limiting synthetic route for the preparation of example 61 and intermediates of formula **IV.**

In another approach, compound of formula **I** can be obtained by further elaboration of specific functional groups present in r₁, r₂, r₃ of intermediate **IV** (prepared according to scheme 1), by means of the funtctional group transformation reported in table 1 thus providing at least one non limiting synthetic route for the preparation of examples reported into the table.

**Table 1**

| Starting Materials | Products | Reaction type / steps | Examples |
|---|---|---|---|
| r₁/R₁ 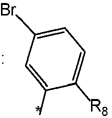 | R₁: 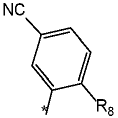 | Cyanation with Zn(CN)₂ | example 64 |
| | R₁: 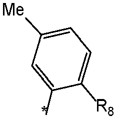 | Suzuki coupling with 1,3,5,2,4,6-trioxatriborinane | example 65 |
| | R₁: 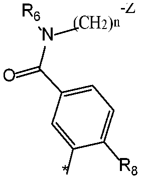 | 1) Carbonylation | example 66 |
| | | 2) Amide coupling with H-N(R₆)-(CH2)ₙ-Z | |
| | R₁: 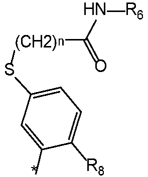 | 1) Pd catalyzed S-coupling with H-S(CH₂)ₙ-COOMe | example 67 to 69 |
| | | 2) Amide coupling with H-H-NH(R₆) | |
| r₁/R₁: 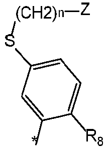 | R₁: 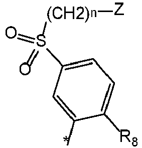 | S-oxidation | Example 70 |
| r₁/R₁ 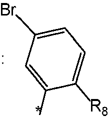 | R₁: 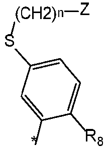 | 1) Pd catalyzed C-S coupling with tris(propan-2-yl)silanethiol (TIPS-SH) | example 71 to 73 (Step 1 to 3), |
| | | 2) TIPS deprotecion (optional) | |
| | | 3) Cu(I) catalyzed Cham-Lam with Z-B(OH)₂ (when n is 0) or alkylation with x-(CH₂)ₙ-Z (x is Cl, Br, I) | example 74 (step 1-4) |
| | | | and |
| | | | example 75 (step 1 to 5) |
| | | 4) Boc deprotection | |
| | | 5) Acetylation | |
| r₂/R₂: 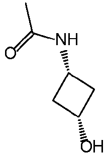 | R₂: 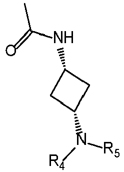 when R₄=R₅= H or Me | 1) Mesylation with Ms-Cl | example 77 (step 1 to 3), |
| | | 2) Nucleophilic substitution with sodium azide | |
| | | 3) Staudinger reduction | example 80 (step 1 to 4) |
| | | 4) Dimethylation with Eschweiler-Clarke | |
| r₂/R₂: 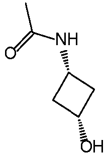 | R₂: 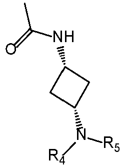 | 1) Mesylation with Ms-Cl | example 78 to 79 |
| | | 2) Nucleophilic substitution with -NR₄R₅ | |
| 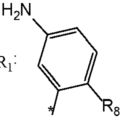 | 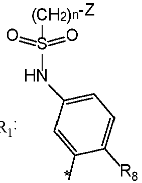 | 1) Coupling with Cl-S(O)₂(CH₂)nZ | example 86 |
| r₂/R₂: 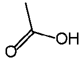 | R₂: 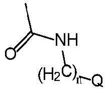 | Amide coupling with H₂N-(CH₂)ₙQ | example 81 |
| r₂/R₂: 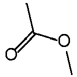 | R₂: 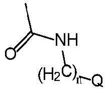 | Amidation with H₂N-(CH₂)ₙQ | example 82 to 84 |

Preparations of intermediate II are detailed in *scheme 2.*

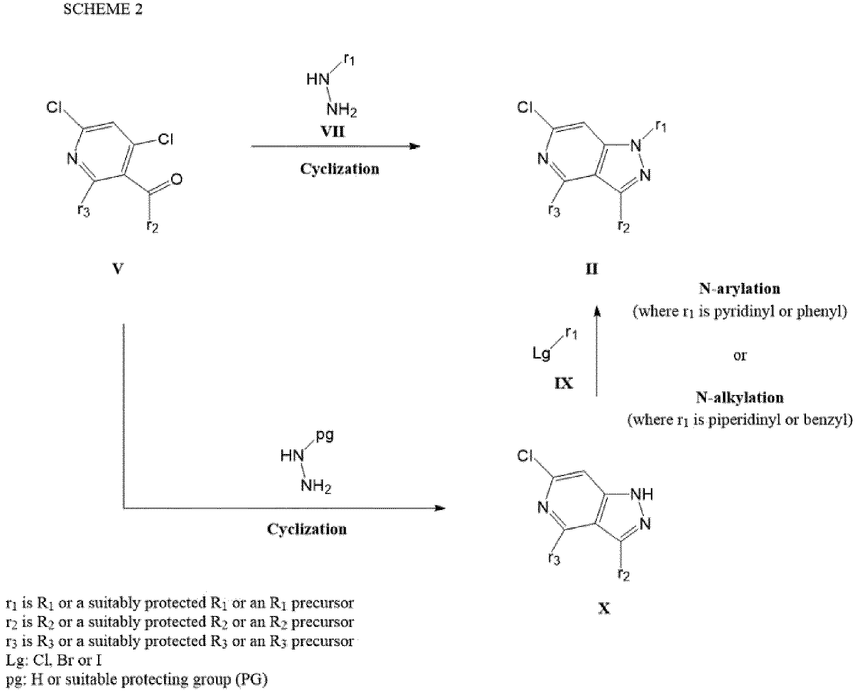

Intermediate II can be obtained from cyclization of intermediate V with intermediate VII using similar conditions to those described in scheme 1 for intermediate VI with intermediate VII.

In another approach, intermediate II can be obtained from intermediate X and intermediate IX analogously to what described for N-arylation/N-alkylation of intermediate II and intermediate III. Intermediate X can be obtained from intermediate **V** by cyclization with hydrazine (or a protected derivative) analogously to what previously described for intermediate **VI** with intermediate **VII** in scheme 1.

In another approach, intermediates **II** can be obtained from further elaboration of r₁ and/or r₂ and/or r₃ groups by general accepted methods and in accordance with principles of chemistry. In the following schemes, the most common transformations that can be used to obtain specific intermediates **II** have been detailed. For sake of clarity they were labelled with an additional letter index.

Intermediate of formula **IIa,** when r₁ is phenyl and K is -S(O)₂NR₄R₅, can be obtained by further elaboration of an intermediate of formula **IIa'** or formula **IIa"** as shown in scheme 3.

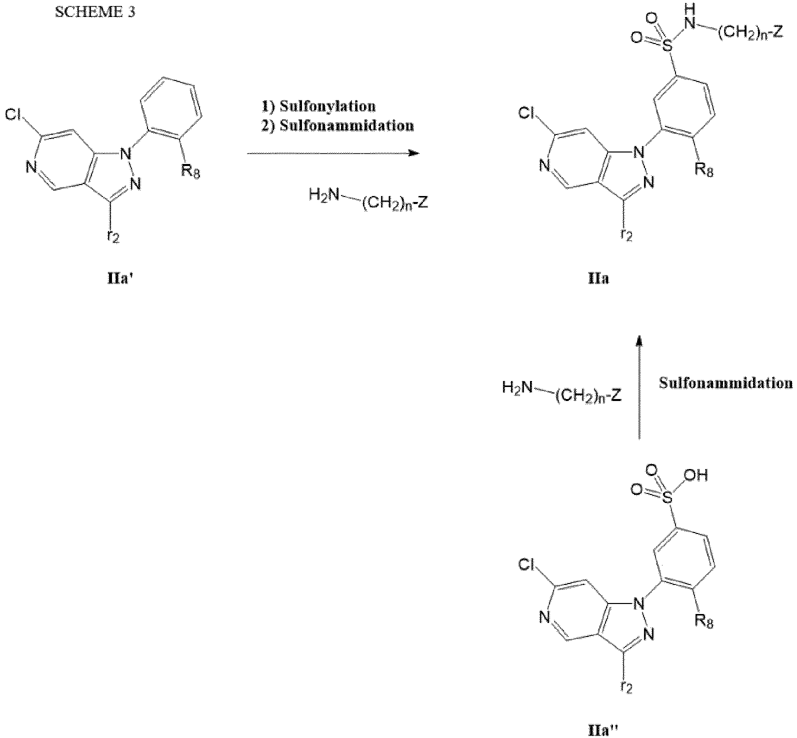

Intermediate **IIa'** can undergo a reaction of sulfonylation by treatment with chlorosulphonic acid and SO₂Cl₂ at temperature typically from 0°C to 5°C, for few hours (typically 1-3 h) to give an intermediate sulfonyl chloride. The sulfonyl chloride can be reacted with the correspondig amine H₂N-(CH₂)ₙ-Z, in the presence of a base such as triethylamnine (TEA) or pyridine, in an orgnaic solvent like DCM or THF, typically at RT for few hours (typically 1 to 3 h). Alternatively, intermediate **IIa"** can be activated to give an intermediate sulfonyl chloride by treatment with SO₂Cl₂ in an organic solvent like DMF, at temperature typically from 0°C to 5°C , followed by the treatment with a large excess of the corresponding amine H₂N-(CH₂)n-Z (typically 10 to 30 eq.) to give intermediate **IIa.**

In another approach showed in scheme 4, intermediate of formula **IIb** (when r₂ is H and r₃ is -NH(CH2)ₙQ or -O(CH2)ₙQ) can be obtained by displacement of the chlorine by nucleophilic substitution of intermediate **IIb'** with the respective amine (H₂N-(CH₂)ₙ-Q) or alchol intermediates (HO-(CH₂)ₙ-Q). The reaction can be carried out by treating the reagents in an high boiling organic solvent like NMP or DMA by heating at temperature around 150°C. Intermediate **IIb** when r₃ is -NH(CH₂)ₙQ can be alternatively prepared by means of a Pd catalyzed N-arylation under starting from intermediate **IIb'** and H₂N-(CH₂)ₙ-Q, in the presence of a suitable catalytic system like Pd₂(dba)₃/Xantphos or an alternative suitable Pd source/Buchwald type phosphine and a base like Cs₂CO₃, in an organic solvent such 1,4-dioxane by heating at temperature around 100°C for time up to 24 h. In some cases, r₃ group of intermediate **IIb** may been further elaborated by general accepted methods, for example by hydrolyzing esters moieties in acid and then into amides by amide couplings.

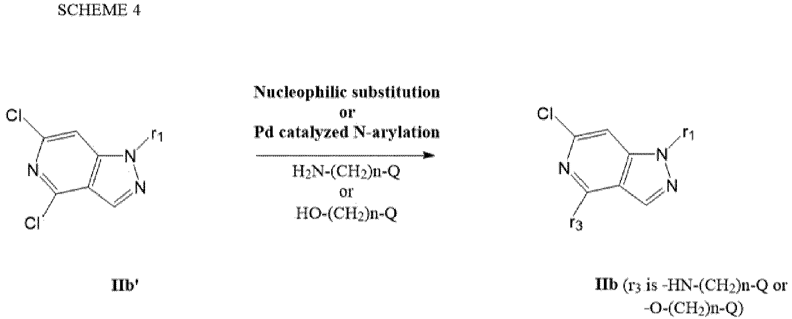

In different approach reported in scheme 5, intermediates of formula **IIc** (when r₂ is -CH₂CN), intermediate **IId** (when r₂ is -CH₂OH) and intermediate **IIe** (when r₂ is - CH₂NR₄R₅) can be prepared in a two step process from intermediate **IIc'.** In the the first step, methyl group of intermediate **IIc'** can be selectively brominated by reaction with NBS (N-bromosuccinimide), in the presence of a radical initiator like AIBN (azobisisobutyronitrile) and in a suitable inert organic solvent like tetrachloromethane to give intermediate **IIc"**. In the second step, nucleophilic substitution of bromine of intermediate **IIc"** can give intermediates **IIc, IId** and **IIe** by reaction with their corresponding nucleophiles: sodium cyanide, potassium acetate/water and amine HNR₆(CH₂)ₙ-Q.

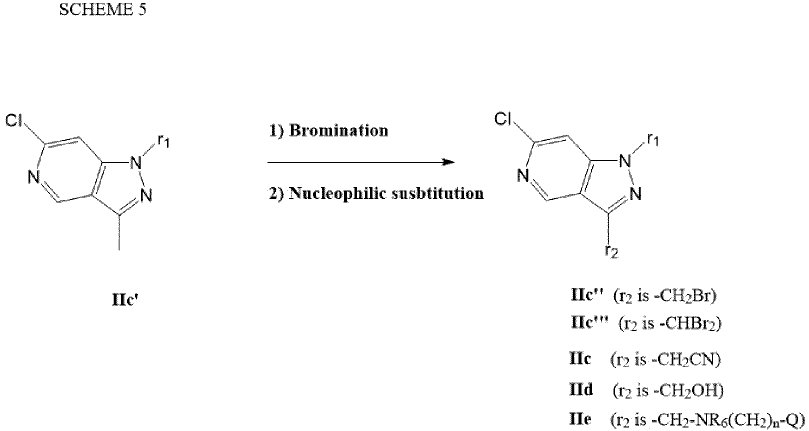

In an alternative approach reported in scheme 6, intermediates of formula **IIg** (when r₂ is -C(O)NR₆-(CH₂)ₙ-Q) may be obtained from intermediate **IIf** by means of amide coupling with the corresponding amine HNR₆-(CH₂)ₙ-Q. An amide coupling can be performed by reacting the amine and the acid in an organic solvent like DMF, DCM, or THF, in the presence of a coupling agent like HATU((1-[Bis(dimethylamino)methylene]-1H-1,2,3-triazolo[4,5-b]pyridinium 3-oxide hexafluorophosphate), HBTU (O-(Benzotriazol-1-yl)-N,N,N',N'-tetramethyluronium hexafluorophosphate) or COMU ((1-Cyano-2-ethoxy-2-oxoethylidenaminooxy)dimethylamino-morpholino-carbenium) and in the presence of an organic base like TEA, DIEA or pyridine.

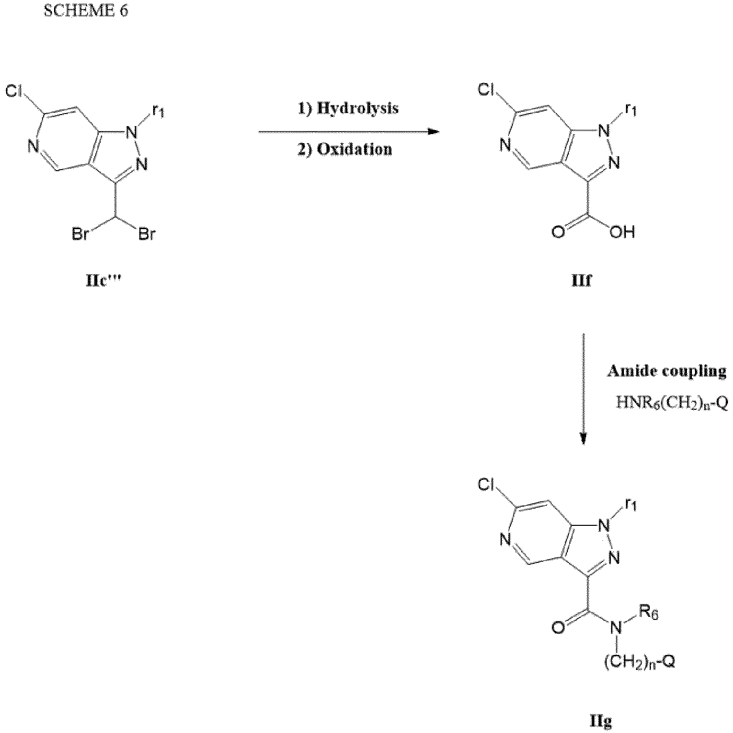

Intermediate **IIf** can be obtained from intermediate **IIc‴** by a two step process. In the fist step, intermediate **IIc‴** can be hydrolyzed to give the corresponding aldehyde in a mixture of water and a water organic miscible solvent like DMSO or DMF by heating at temperature around 100°C. In the second step, the aldehyde can be oxidized to the corresponding acid with an oxidant like sodium chlorite, in the presence of a inorganic phosphate salt like sodium dihydrogenphosphate, with an additive like 2-methyl-2-butene in a mixture of water and organic solvent such THF. In a different way, intermediate **IIf** can be obtained from intermediate **IId** by a two steps oxidation that involves first alchol oxidation to aldehyde by treatment with DMP (Dess-Martin periodinane), and subsequently oxidation of aldehyde to acid **IIf** as described above.

Intermediate **IIc‴** can be obtained in the same reaction and concomitantly to the synthesis of intermediate **IIc"** showed in scheme 5.

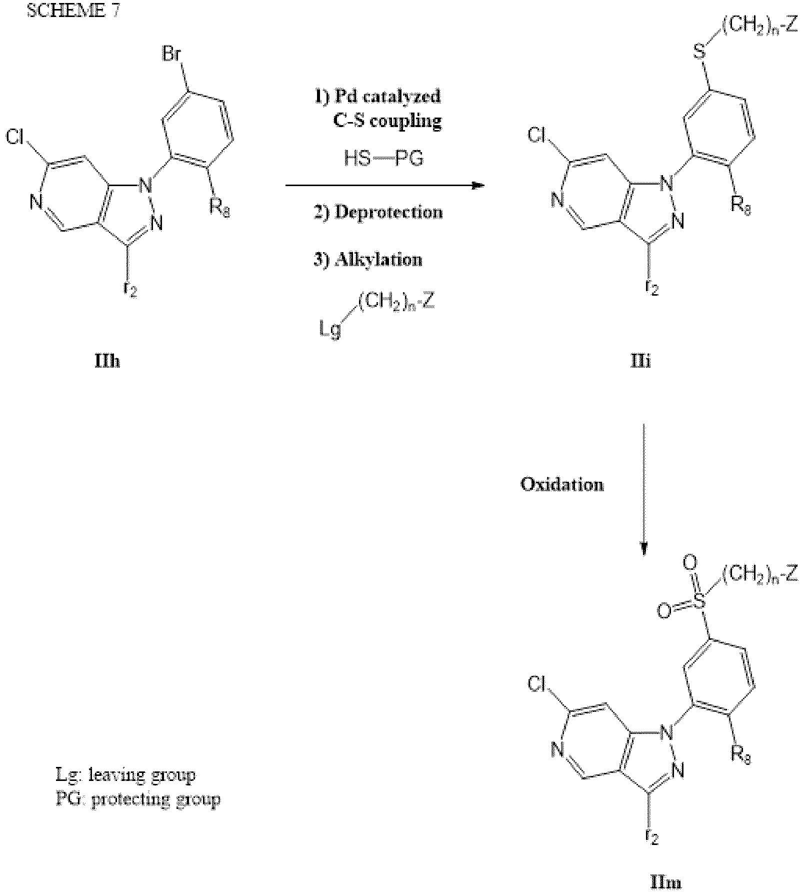

In an alternative approach reported in scheme 7, intermediates of formula **IIi** (when r₁ is phenyl and K is -S(CH₂)ₙ-Z) and intermediate **IIm** (when r₁ is phenyl and K is - S(O)₂(CH₂)ₙ-Z) can be obtained from intermediate **IIh**. Intermediate **IIi** can be obtained from intermediate **IIh** by a three step process as follow. In the first step, bromine of intermediate **IIh** is replaced with S-PG by introducing a suitable protected source of hydrogen sulfide (HS-PG), for example HS-TIPS (triisopropylsilanethiol), by a C-S palladium catalyzed coupling. C-S coupling can be performed by reacting aryl bromide **IIh** and HS-PG in the presence of a suitable catalytic system like as Pd₂(dba)₃ / Xantphose or another suitable palladium source / phosphine source, in an organic solvent as toluene or DMA, in the presence of a strong base like sodium hydride or sodium terbutoxide, at temperature up to 100°C. TIPS group can be partially deprotected during the palladium catalyzed C-S coupling and/or deprotected by treating the mixture with an acid such as hydrochloric acid to give the corresponding thiophenol derivative of intermediate **IIh.** In the third step, thiophenol of intermediate **IIh** can be alkylated by Lg-(CH₂)ₙ-Z (Lg is leaving group, for example Cl, Br or tosyl) by reacting those intermediates in the presence of a base such K₂CO₃ or Cs₂CO₃ and of sodium iodide as additive, in an organic solvent as acetone or acetonitrile, by heating at reflux temperature. In another settings, when n is 0 and Z is an aryl or heteoaryl, intermediate **IIi** can be obtained from reaction the corresponding free or TIPS-protected thiophenol with an aryl/heteroaryl halide by a palladium catalyzed C-S coupling as described above. In some cases, intermediate **IIi** can be directly obtained from intermediate **IIh** by a palladium catalyzed C-S coupling with HS-(CH₂)ₙ-Z.

Intermediate **IIm** can be obtained from the corresponding intermediates **IIi** by oxidation of the thioether moiety to sulfone using an oxidant like m-CPBA (meta chloroperbenzoic acid) or another suitable peroxide, in an organic solvent like DCM at temperature around 0°C.

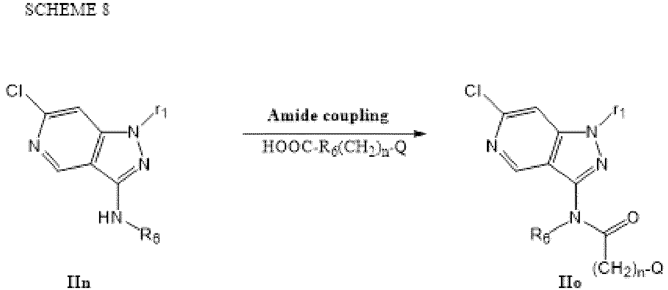

In another approach described in scheme 8, intermediate **IIo** can be obtained from intermediate **IIn** with an amide coupling by reacting amine and acid in the same condition to what described for conversion of intermediate **IIf** into intermediate **IIg** in scheme 6. Intermediate **IIn** can be obtained as reported in scheme 2. In an alternative way, intermediate **IIn** (when R₆ is H) can be obtained by Curtious rearrangements of the corresponding carboxylic acid intermediate **IIf** by reaction with DPPA (diphenyl phosphoryl amide), with a base like TEA or DIPEA, by heating (up to 100-120°C) in an organic solvent such *tert*-butanol to give N-Boc protected intermediate **IIn** (when R₆ is H). N-Boc protected intermediate **IIn** (when R₆ is H) can be cleaved to give free amine or used for introducing R₆ and then cleaved to give intermediate **IIn**.

In another approach, intermediate X can be obtained from further elaboration of r₂ and/or r₃ groups by general accepted methods. In the following schemes (scheme 9 and scheme 10), it were detailed the most common transformations that can be used to obtain intermediates **Xa** and **Xb.** groups.

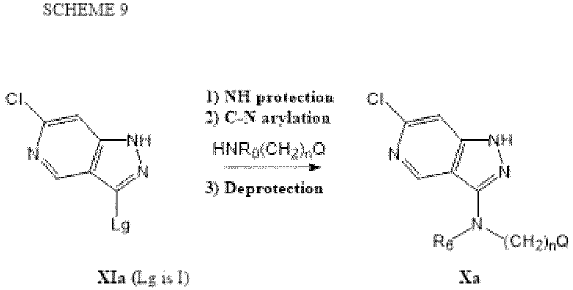

As reported in scheme 9, intermediate **Xa** can be obtained from intermediate **XIa** in a three step process. First, for synthetic convenience, heterocyclic NH need to be protected with a suitable protecting group before C-N arylation. THP (tetrahydropyranyl) may be represent a suitable protective group and it can be introduced on intermediate **XIa** by reaction with dihydropyran in the presence of a sulphonic acid as methanesulfonic acid or p-toluensulfonic acid, in an organic solvent as DCM or THF, at reflux temperatures or lower. In the second step, the C-N arylation of THP protected **XIa,** can be carried out by using copper catalyzed Ullmann reaction or a palladium catalyzed C-N arylation. Copper catalyzed Ullmann type reaction can be conducted as described in scheme 1 for the reaction of intermediate **VIII** and intermediate **IX.** A palladium catalyzed C-N arylation can be carried out similarly to what described for the conversion of intermediate **IIb'** to intermediate **IIb** in scheme 4. In the last step, deprotection of THP group can be carried out by treating correspondig intermediate with an acid like trifluoroacetic acid or hydrochloric acid, in an organic solvent as isopropanol, 1,4-dioxane, DCM or THF with or without a scavenger like triethylsilane.

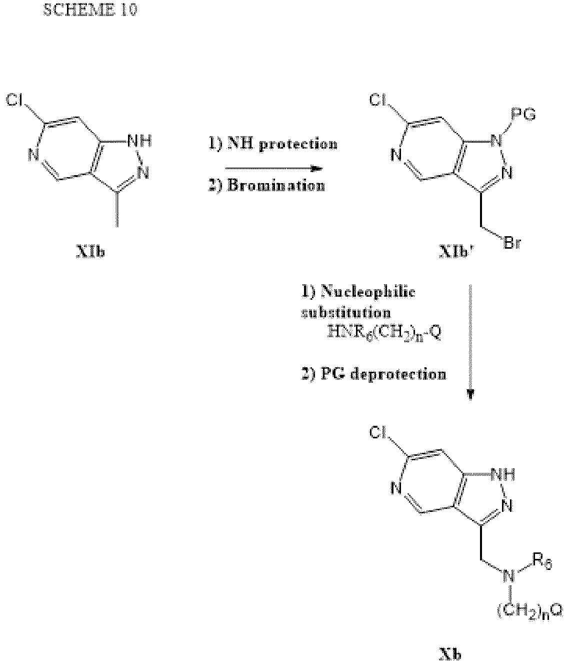

Intermediate Xb can be obtained from intermediate XIb' in a two step process that involve nucleophilic substitution of bromine of intermediate XIb' with an amine of formula HNR6(CH2)n-Q followed by deprotection of PG. Intermediate XIb' can be obtained from intermediate XIb in a two step process that involve PG insertion and bromination similarly to what described in scheme 5. A suitable protective group that can be used for protecting NH of intermediate XIb during above mentioned transformation is trityl group. A trityl group can be inserted by reaction of substrate with trityl chloride, in the presence of a hydride such as sodium hydride in an organic solvent as THF or dioxane. Removal of trityl group can be carried out by treating corresponding substrate with an acid such as trifluoroacetic acid, in a solvent as DCM or THF, with or without a scavenger like triethylsilane.

Starting intermediates reported in all above schemes, unless their process for preparation have been detailed here and/or into experimental sections, are commercially available or may be readily prepared by those skilled in the art starting from commercially available reagents using common accepted methods.

### General Experimental details

Chemical Names of the compounds were generated with Structure To Name Enterprise 10.0 Cambridge Software or latest.

Purification by 'chromatography' or 'flash chromatography' refers to purification using a Biotage SP1, or Interchim puriFlash purification system, or equivalent MPLC system using a pre-packed polypropylene column containing stationary phase (cartridge). Where products were purified using an Si cartridge, this refers to an Interchim pre-packed polypropylene column (or equivalent) containing unbounded activated silica with spherical particles with average size of 15 µm or Isolute^{®} pre-packed polypropylene column (or equivalent) containing unbounded activated silica with irregular particles with average size of 50 µm. When 'NH-silica' and 'C18-silica' are specified, they refer respectively to aminopropyl chain bonded silica and octadecyl carbon chain (C18)-bonded silica. Fractions containing the required product (identified by TLC and/or LCMS analysis) were pooled and concentrated in vacuo. Where an SCX cartridge was used, 'SCX cartridge' refers to a Bond Elut^{®} pre-packed polypropylene column (or equivalent) containing a non-end-capped propylsulphonic acid functionalised silica strong cation exchange sorbent.

Where preparative HPLC - MDAP was used for purification (MDAP-mass directed automatic purification) fractions containing the desired product were pooled and the solvent removed by evaporation or alternatively lyophilised. Wherein MDAP is used, method reference where reported in the description of examples.

### NMR Methods

NMR spectra were obtained on a Bruker Avance III 600 (5 mm RT inverse probe head), Bruker DRX 500, Bruker Avance AV 400 (5 mm RT direct probehead) or Bruker DPX 300 spectrometers using standard Bruker pulse sequences. DMSO-d6 or CDCl₃ were used as solvents and tetramethylsilane as the internal standard unless in the latter case where solvent residual peak was used. All experiments were recorded at 298 K, unless stated differently. Chemical shifts are reported as δ values in ppm relative to tetramethylsilane. Coupling constants (J values) are given in hertz (Hz) and multiplicities are reported using the following abbreviation: s=singlet, d=doublet, t=triplet, q=quartet, m=multiplet, br=broad, nd=not determined.

### LCMS Methods

### Method 1

Acquity UPLC coupled with SQD mass spectrometer; Column: Acquity BEH C18 (50mm x 2.1mm i.d., 1.7µm), mobile phase A: 0.1% (v/v) formic acid in water, mobile phase B: 0.1% (v/v) formic acid in acetonitrile;

| Gradient - Time | Flow (mL/min) | A% | B% |
|---|---|---|---|
| 0.00 | 0.9 | 97 | 3 |
| 1.50 | 0.9 | 3 | 97 |
| 1.90 | 0.9 | 3 | 97 |
| 2.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1000 AMU.

### Method 2

Acquity UPLC coupled with SQD mass spectrometer; Column: Acquity BEH C18 (50mm x 2.1mm i.d., 1.7µm), mobile phase A: 10 mM aqueous solution of ammonium bicarbonate (adjusted to pH 10 with ammonia), mobile phase B: acetonitrile;

| Gradient - Time | Flow (mL/min) | A% | B% |
|---|---|---|---|
| 0.00 | 0.9 | 97 | 3 |
| 1.50 | 0.9 | 3 | 97 |
| 1.90 | 0.9 | 3 | 97 |
| 2.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1000 AMU.

### Method 3

Acquity UPLC coupled with SQD mass spectrometer; Column: Acquity BEH C18 (50mm x 2.1mm i.d., 1.7µm), mobile phase A: 0.1% (v/v) formic acid in water, mobile phase B: 0.1% (v/v) formic acid in acetonitrile

| Gradient - Time | Flow (mL/min) | A % | B% |
|---|---|---|---|
| 0.00 | 0.9 | 97 | 3 |
| 3.20 | 0.9 | 3 | 97 |
| 3.90 | 0.9 | 3 | 97 |
| 4.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1500 AMU.

### Method 4

Acquity UPLC coupled with SQD mass spectrometer; Column: Acquity BEH C18 (50mm x 2.1mm i.d., 1.7µm), mobile phase A: 10 mM aqueous solution of ammonium bicarbonate (adjusted to pH 10 with ammonia), mobile phase B: acetonitrile;

| Gradient - Time | Flow (mL/min) | A % | B % |
|---|---|---|---|
| 0.00 | 0.9 | 97 | 3 |
| 3.20 | 0.9 | 3 | 97 |
| 3.90 | 0.9 | 3 | 97 |
| 4.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1500 AMU.

### Method 5

Acquity UPLC coupled with SQD mass spectrometer; Column: Acquity BEH C18 (50mm x 2.1mm i.d., 1.7µm), mobile phase A: 0.1% (v/v) formic acid in water, mobile phase B: 0.1% (v/v) formic acid in acetonitrile;

| Gradient - Time | Flow (mL/min) | A % | B% |
|---|---|---|---|
| 0.00 | 0.6 | 97 | 3 |
| 0.50 | 0.6 | 97 | 3 |
| 7.00 | 0.6 | 3 | 97 |
| 7.50 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1500 AMU.

### Method 6

Acquity UPLC coupled with SQD mass spectrometer; Column: Acquity BEH C18 (50mm x 2.1mm i.d., 1.7µm), mobile phase A: 10 mM aqueous solution of ammonium bicarbonate (adjusted to pH 10 with ammonia), mobile phase B: acetonitrile;

| Gradient - Time | Flow (mL/min) | A % | B% |
|---|---|---|---|
| 0.00 | 0.6 | 97 | 3 |
| 0.50 | 0.6 | 97 | 3 |
| 7.00 | 0.6 | 3 | 97 |
| 7.50 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1500 AMU.

### Method 7

AGILENT LC 1260 Infinity with SFC and Agilent 6540 UHD Accurate-Mass Q-TOF LC/MS; Column: Acquity BEH C18 (100mm x 2.1mm i.d., 1.7µm), mobile phase A: 0.1% (v/v) formic acid in water, mobile phase B: 0.1% (v/v) formic acid in acetonitrile;

| Gradient - Time | Flow (mL/min) | A % | B% |
|---|---|---|---|
| 0.00 | 0.5 | 97 | 3 |
| 8.00 | 0.5 | 0 | 100 |
| 10.00 | 0.5 | 97 | 3 |
| 12.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1500 AMU.

### Method 8

AGILENT LC 1260 Infinity with SFC and Agilent 6540 UHD Accurate-Mass Q-TOF LC/MS; Column: Acquity UPLC BEH C18 (100mm x 2.1mm i.d., 1.7µm), mobile phase A: 0.05 % (v/v) aqueous ammonia, mobile phase B: acetonitrile;

| Gradient - Time | Flow (mL/min) | A % | B% |
|---|---|---|---|
| 0.00 | 0.5 | 97 | 3 |
| 8.00 | 0.5 | 0 | 100 |
| 10.00 | 0.5 | 97 | 3 |
| 12.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1000 AMU.

### Method Prep 1

Agilent 1290 Infinity II Purification System; Column: Waters XBridge^{®} (C18, 100 mm x 19 mm i.d., 5 µm), mobile phase A: 0.1% (v/v) ammonia in water, mobile phase B: acetonitrile;

| Gradient - Time | Flow (mL/min) | A % | B% |
|---|---|---|---|
| 0.00 | 30 | 55 | 45 |
| 0.80 | 30 | 55 | 45 |
| 5.00 | 30 | 25 | 75 |
| 5.01 | 30 | 0 | 100 |
| 5.90 | 30 | 0 | 100 |
| 5.95 | 30 | 60 | 40 |

### Method Prep 2

Agilent 1290 Infinity II Purification System; Column: Waters XBridge^{®} (C18, 100 mm x 19 mm i.d., 5 µm), mobile phase A: 0.1% (v/v) ammonia in water, mobile phase B: acetonitrile;

| Gradient - Time | Flow (mL/min) | A % | B% |
|---|---|---|---|
| 0.00 | 30 | 60 | 40 |
| 0.80 | 30 | 60 | 40 |
| 5.00 | 30 | 35 | 65 |
| 5.01 | 30 | 0 | 100 |
| 5.90 | 30 | 0 | 100 |
| 5.95 | 30 | 60 | 40 |

In the procedures that follow, some of the starting materials are identified through an "Intermediate" or "Example" number with indications on step name. This is provided merely for assistance to the skilled chemist. When reference is made to the use of a "similar" or "analogous" procedure, as will be appreciated by those skilled in the art, such a procedure may involve minor variations, for example reaction temperature, reagent/solvent amount, reaction time, work-up or chromatographic purification conditions.

The stereochemistry of the compounds in the Examples, where indicated, has been assigned on the assumption that absolute configuration at resolved stereogenic centres of starting materials is maintained throughout any subsequent reaction conditions.

Unless otherwise stated, where absolute configuration (R) or (S) is reported in the compound name, ee% has to be considered equal or greater than 90%.

All solvents and commercial reagents were used as received. Where the preparation of starting materials is not described, these are commercially available, known in the literature, or readily obtainable by those skilled in the art using standard procedures.

### Abbreviations

AIBN = Azobisisobutyronitrile; Boc₂O = Di-tert-butyl dicarbonate; tBuXPhos = 2-di-tert-butylphosphino-2',4',6'-triisopropylbiphenyl; aq. = aqueous; DABAL-Me₃ = Bis(trimethylaluminum)-1,4-diazabicyclo[2.2.2]octane adduct; DAST = Diethylaminosulfur trifluoride; DBU = 1,8-Diazabicyclo[5.4.0]undec-7-ene; DCC = Dicyclohexylcarbodiimine; DCE = 1,2-Dichloroethane; DCM = Dichloromethane; DIPEA = N,N-Diisopropylethylamine; DMAP = 4-dimethylaminopyridine; DMCHDA = *trans*-*N*,*N'*-Dimethylcyclohexane-1,2-diamine; DMF = N,N-Dimethylformamide; DMP = Dess-Martin Periodinane; DMSO = Dimethylsulfoxide; DPPA = diphenyl phosphoryl azide; EEDQ = 2-Ethoxy-1-ethoxycarbonyl-1,2-dihydroquinoline; EtOAc = Ethyl acetate; HATU=(1-[Bis(dimethylamino)methylene]-1H-1,2,3-triazolo[4,5-b]pyridinium 3-oxid hexafluorophosphate), HBTU=(2-(1H-benzotriazol-1-yl)-1,1,3,3-tetramethyluronium hexafluorophosphate; LCMS = Liquid chromatography-mass spectrometry; LiHMDS = Lithium bis(trimethylsilyl)amide; mCPBA = 3-Chloroperbenzoic acid; MW = microwave; NBS = N-Bromosuccinimide; PE = petroleum ether; 1H-NMR = Proton nuclear magnetic resonance; RM = Reaction mixture; Rt = Retention time; RT = Room temperature; RuPhos Pd-G3 = (2-dicyclohexylphosphino-2',6'-di-isopropoxy-1,1'-biphenyl)(2'-amino-1,1'-biphenyl-2-yl)palladium(II) methanesulfonate; sat. = saturated; TEA = Triethylamine; TFA - Trifluoroacetic acid; THF = Tetrahydrofuran; Xphos-Pd-G3 - (2-Dicyclohexylphosphino-2',4',6'-triisopropyl-1,1'-biphenyl)[2-(2'-amino-1,1'-biphenyl)]palladium(II) methanesulfonate

### PREPARATION OF INTERMEDIATES

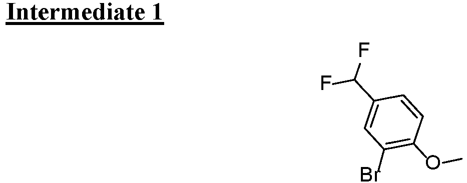

### 2-Bromo-4-(difluoromethyl)-1-methoxybenzene (Intermediate 1)

To a solution of 3-bromo-4-methoxy-benzaldehyde (1.0 g, 4.7 mmol) in anhydrous DCM (10 mL), DAST (1.2 mL, 9.3 mmol) was added dropwise at 0 °C, RM warmed up to RT and stirred overnight. RM was quenched at 0 °C by slowly addition of sat. aq. NaHCO₃ and extracted with DCM (3x15 mL). Combined organic layers were passed through phase separator and solvent evaporated. The residue was purified by flash chromatography on a Si cartridge by eluting with 0-20% EtOAc in cyclohexane to afford the title product (797 mg).
LCMS (Method 2): Rt = 1.14 min
¹H-NMR (300 MHz, DMSO-*d₆*) δ: 7.77 (s, 1H), 7.57 (d, J=8.6 Hz, 1H), 7.22 (d, J=8.7 Hz, 1H), 6.96 (t, J=56.4 H7, 1H), 3.89 (s, 3H)

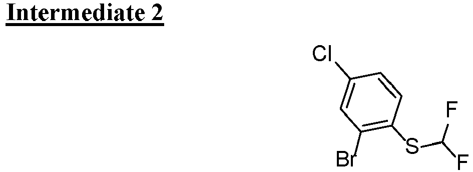

### (2-Bromo-4-chlorophenyl)(difluoromethyl)sulfane (Intermediate 2)

2-Bromo-4-chloro-benzenethiol (100 mg, 0.447 mmol), sodium chlorodifluoroacetate (157 mg, 1.03 mmol) and Cs₂CO₃ (204 mg, 0.626 mmol) were suspended in DMF (1 mL) and stirred at 100 °C for 2 h. RM was cooled to RT. Water (10 mL) was added and product was extracted with EtOAc (2x15 mL). Combined organic layers were washed with sat. aq. NaHCO₃ (3x5mL), water (5 mL) and sat. aq. NaCl (5 mL). Organic layer was dried over Na₂SO₄ and evaporated under reduced pressure to afford desired product (160 mg) that was used in the next steps without further purification.
LCMS (Method 2): Rt = 1.37 min
¹H-NMR (300 MHz, *CDCl₃*) δ: 7.69 (d, J=2.3 Hz, 1H), 7.58 (d, J=8.4 Hz, 1H), 7.32 (dd, J=8.4, 2.4 Hz, 1H), 6.85 (t, J=56.3 Hz, 1H)

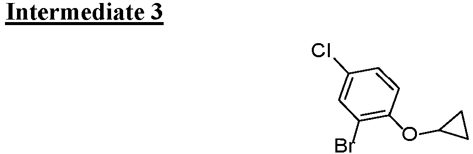

### 2-Bromo-4-chloro-1-cyclopropoxybenzene (Intemediate 3)

N-Bromosuccinimide (52.8 mg, 0.3 mmol) was added portionwise to 1-chloro-4-(cyclopropoxy)benzene (40.0 µL, 0.3 mmol) in 1,1,1,3,3,3-hexafluoropropan-2-ol (2.0 mL) and RM stirred at RT overnight. Reaction was quenched with sat. aq. NaHCO₃ and extracted with EtOAc (4 times). Combined organic layers were passed through a phase separator and concentrated *in vacuo.* Crude product was purified by flash chromatography on a Si cartridge by eluting with 0-20% EtOAc in cyclohexane to give the title product (52 mg).

LCMS (Method 1): Rt = 1.38 min.

¹H-NMR (300 MHz, DMSO-*d₆*) δ: 7.67 (d, J=2.4 Hz, 1H), 7.45 (dd, J=8.7, 2.3. Hz, 1H), 7.39 (dd, J=8.8 Hz, 1H), 3.94-3.97 (m, 1H), 0-80-08.7 (m, 2H), 0.68-0.71 (m, 2H).

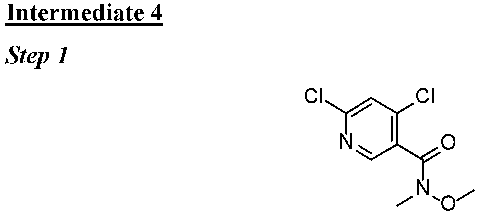

### 4,6-Dichloro-N-methoxy-N-methylnicotinamide (Intermediate 4-1)

To a suspension of 4,6-dichloropyridine-3-carboxylic acid (15.0 g, 78 mmol) in anhydrous DCM (225 mL) cooled at 5 °C, DMF (4.5 mL, 59 mmol) was added, followed by dropwise addition of oxalyl chloride (6.6 mL, 78 mmol). RM was stirred for 20 h reaching RT, then solvents were removed under reduced pressure and the residue azeotroped with toluene (20 mL). The residue was taken in DCM (40 mL) and added dropwise to a mixture of N,O-Dimethylhydroxylamine hydrochloride (11 g, 117 mmol) in DCM (100 mL) and TEA (10.8 mL, 78 mmol) at 5 °C. RM was stirred overnight at RT, quenched with sat. aq. NaHCO₃ (70 mL) and organic layer washed with water (3x25 mL), dried over Na₂SO₄ and solvent removed under reduced pressure to afford the title product (16.1 g) that was used in the next synthetic steps without further purification.
LCMS (Method 1): Rt = 0.82 min
¹H-NMR (300 MHz, *CDCl₃*) δ: 8.34 (s, 1H), 7.42 (s, 1H), 3.47 (s, 3H), 3.36 (s, 3H)

### Step 2

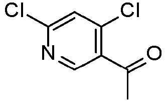

### 1-(4,6-Dichloropyridin-3-yl)ethan-1-one (Intermediate 4)

To a mixture of intermediate 4-1 (25 g, 105 mmol) and THF (150 mL) at 0-5 °C, MeMgBr (3.0 M in diethyl ether, 79 mL, 238 mmol) was added dropwise over 1 h and RM stirred for further 1 h. RM was quenched with sat. aq. NH₄Cl (100 mL) and stirred for 10 min. The aqueous layer was extracted with EtOAc (100 mL), washed with water (100 mL), sat. aq. NaCl (100 mL), dried over Na₂SO₄ and evaporated under reduced pressure. The residue was purified by bulb-to-bulb distillation (91 °C/2x10⁻¹ mbar) to afford desired product (15.8 g).
LCMS (Method 2): Rt = 0.89 min
¹H-NMR (600 MHz, *CDCl₃*) δ: 8.59 (s, 1H), 7.42 (s, 1H), 2.66 (s, 3H)

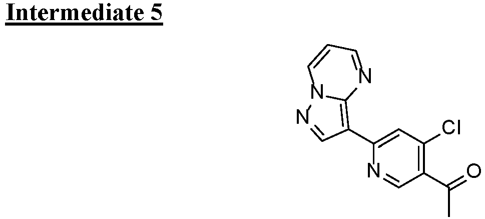

### 1-(4-Chloro-6-(pyrazolo[1,5-a]pyrimidin-3-yl)pyridin-3-yl)ethan-1-one (Intermediate 5)

Intermediate 4 (5.00 g, 26.3 mmol) was dissolved in 1,2-Dimethoxyethane (35 mL) under argon, then added with Pd(PPh₃)₄ (608 mg, 0.526 mmol). After 10 min, 3-(4,4,5,5-tetramethyl-1,3,2-dioxaborolan-2-yl)pyrazolo[1,5-a]pyrimidine (6.45 mg, 26.3 mmol) in 2-propanol (35 mL) and 2M aq. K₂CO₃ (23.7 mL, 47.4 mmol) were added and RM stirred at 95 °C for 1 h. RM was cooled to RT, diluted with water (120 mL), and the formed precipitate collected by filtration, washed with acetonitrile (2 x 50 mL) and dried at 45 °C for 1 h to afford the title compound (5 g) that was used in the next steps without further purification.

LCMS (Method 2): Rt = 0.89 min, ES⁺ *m*/*z* 272.9/274.9 [M+H]⁺

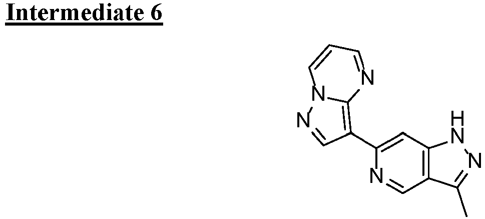

### 3-Methyl-6-(pyrazolo[1,5-a]pyrimidin-3-yl)-1H-pyrazolo[4,3-c]pyridine (Intermediate 6)

Intermediate 5 (500 mg, 1.83 mmol) in NMP (10 mL) and hydrazine monohydrochloride (126 mg, 1.83 mmol) were heated at 100 °C overnight. After cooling to RT, RM was quenched with sat. aq. NaHCO₃ and extracted with EtOAc (4x15 mL). Combined organic layers were washed with sat. aq. NaCl (5x20 mL), dried over Na₂SO₄ and concentrated *in vacuo.* The crude material was triturated with DCM to afford the desired product (167 mg).

LCMS (Method 2): Rt = 0.64 min, ES⁺ *m*/*z* 251.0 [M+H]⁺

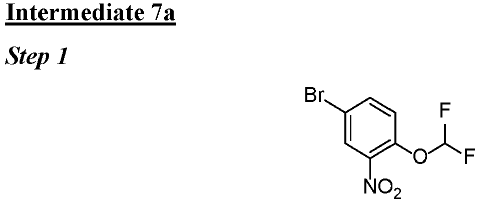

### 4-Bromo-1-(difluoromethoxy)-2-nitrobenzene (Intermediate 7a-1)

4-Bromo-2-nitro-phenol (22 g, 14 mmol), chlorodifluoroacetic acid sodium salt (35 g, 232 mmol) and Cs₂CO₃ (46 g, 141 mmol) were suspended in DMF/water (250/25 mL) and stirred at 100 °C for 1.5 h. RM was concentrated *in vacuo,* diluted with water (200 mL) and extracted with EtOAc (2x200 mL). Combined organic layers were washed with sat. aq. NaHCO₃ (3x150 mL), sat. aq. NaCl (150 mL), dried over Na₂SO₄ and evaporated under reduced pressure to afford the title product (25.9 g) that was used in the next steps without further purification.
LCMS (Method 2): Rt = 1.14 min
¹H-NMR (400 MHz, *CDCl₃*) δ: 8.04 (d, J=2.4 Hz, 1H), 7.71(dd, J=8.8, 2.5 Hz, 1H), 7.28 (dt, J=8.8, 1.0 Hz, 1H), 6.58 (t, J=73 Hz, 1H)

### Step 2

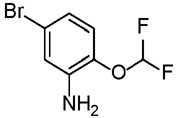

### 5-Bromo-2-(difluoromethoxy)aniline (Intermediate 7a)

Intermediate 7a-1 (25.9 g, 96.6 mmol) was dissolved in acetic acid (200 mL), then iron (8.10 g, 145 mmol) was added portionwise and RM stirred at 90 °C for 3 h. After cooling to RT, RM was diluted with DCM (700 mL) and washed with sat. aq. NaHCO₃ (2x800 mL). Organic layer was filtered through a bed of diacetomatous earth, washed with sat. aq. NaCl (400 mL), dried over Na₂SO₄ and evaporated under reduced pressure. The crude material was dissolved in DCM (400 mL), washed with aq. 10 % w/w Na₂CO₃ (3x200 mL), dried over Na₂SO₄, and evaporated under reduced pressure. The residue was purified by distillation (60 °C / 5.9x10⁻² mbar) to give the title product (15.5 g).
LCMS (Method 2): Rt = 1.05 min
¹H-NMR (400 MHz, *CDCl₃*) δ: 6.92-6.85 (m, 2H), 6.79 (dd, J=8.5, 2.5 Hz, 1H), 6.41 (t, J=73.0 Hz, 1H), 3.97 (br s, 2H)

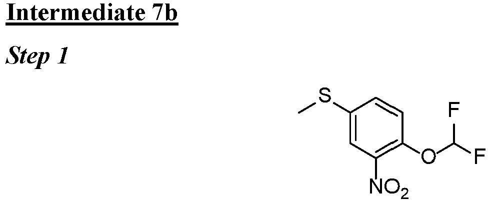

### (4-(Difluoromethoxy)-3-nitrophenyl)(methyl)sulfane (Intermediate 7b-1)

Intermediate 7a-1 (2.81 g, 11.0 mmol) in dry toluene (45 mL) was added with sodium thiomethoxide (2.06 g, 29 mmol) and RM degassed prior to the addition of Xantphos Pd-G3 (498 mg, 0.53 mmol). RM was stirred at 85 °C overnight. After cooling to RT, RM was diluted with EtOAc (200 mL) and washed with sat. aq. NaCl (2x100 mL) and water (3x100 mL). The organic layer was dried over Na₂SO₄, solvent removed under reduced pressure, and the residue purified by flash chromatography on Si cartridge by eluting with 0-10 % EtOAc in PE to afford the title product (471 mg).
LCMS (Method 2): Rt = 1.15 min
¹H-NMR (300 MHz, *CDCl₃*) δ: 7.70 (d, J=2.5 Hz, 1H), 7.42 (dd, J=8.7, 2.5 Hz, 1H), 7.29 (dt, J=8.7, 1.0 Hz, 1H), 6.56 (t, J=73.1 Hz, 1H), 2.52 (s, 1H)

### Step 2

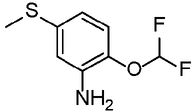

### 2-(Difluoromethoxy)-5-(methylthio)aniline (Intermediate 7b)

Intermediate 7b was prepared in a similar manner to intermediate 7a *(step 2)* starting from intermediate 7b-1.
LCMS (Method 2): Rt = 1.00 min
¹H-NMR (300 MHz, *CDCl₃*) δ: 6.93 (d, J=8.7 Hz, 1H), 6.66 (d, J=2.3 Hz, 1H), 6.58 (dd, J=8.6, 2.3 Hz, 1H), 6.40 (t, J=74.1 Hz, 1H), 3.84 (bs, 2H), 2.42 (s, 1H)

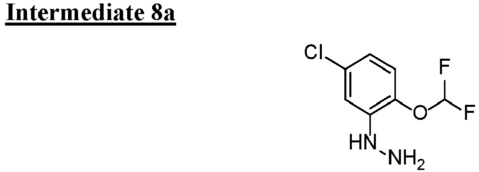

### (5-Chloro-2-(difluoromethoxy)phenyl)hydrazine hydrochloride (Intermediate 8a)

To a solution of conc. HCl (aq. 37% w/w, 45 mL) at 0°C, 5-chloro-2-(difluoromethoxy)aniline (12.8 g, 66.1 mmol) was added dropwise under vigorous stirring (keeping the temperature < 5 °C), then followed by a solution of NaNO₂ (5.93 g, 86.0 mmol) in water (45 mL). RM was stirred at 0 °C for 90 min, followed by a dropwise addition of a solution of tin (II) chloride (37.6 g, 198 mmol) in conc. HCl (aq. 37% w/w, 45 mL) keeping the temperature <5 °C. RM was stirred overnight at 4 °C. RM was diluted with sat. aq. NaCl (100 mL), pH adjusted to 10 using aq. 20% w/w NaOH, and filtered through pad of diacemateous earth, followed by washings with water (2 x100 mL) and DCM (6x100 mL). Organic layer was separated, washed with water (200 mL), dried over Na₂SO₄ and solvent removed under reduced pressure affording the first crop of crude product. A second crop of crude product was obtained by further washing of diacematateous earth pad with DCM. Combined crops were dissolved in 1,4-dioxane (100 mL) and treated with 4N HCl in 1,4-dioxane (9.64 mL, 38.53 mmol) at RT for 30 min to form a precipitate that was collected by filtration, washed with 1,4-dioxane and dried to give the title compound (13 g) that was used in the next steps without further purification.
LCMS (Method 2): Rt = 0.95 min, ES⁻ *m*/*z* 207.0/209.0 [M-H]⁻

### Preparation of intermediates 8b to 8e

The following intermediates were prepared in a similar manner to **intermediate** 8a from the indicated starting materials.

| Intermediate | Structure | Starting material | LCMS |
|---|---|---|---|
| Intermediate 8b | 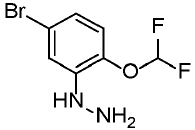 | Intermediate 7a | (Method 2): Rt = 0.97 min, ES⁻ *m*/*z* 251.0/253.0 [M-H]⁻ |
| Intermediate 8c | 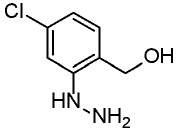 | 2-amino-4-chlorophenyl)methanol | (Method 2): Rt = 0.64 min, ES⁻ *m*/*z* 171.0/173.0 [M-H]⁻ |
| Intermediate 8d | 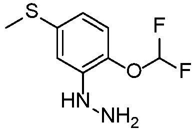 | Intermediate 7b | (Method 2): Rt = 0.90 min, ES⁻ *m*/*z* 219.0 [M-H]⁻ |
| Intermediate 8e | 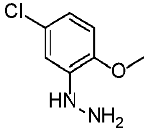 | 5-chloro-2-methoxy-aniline | (Method 2): Rt = 0.80 min, ES⁺ *m*/*z* 173.3/175.1 [M+H]⁺ |

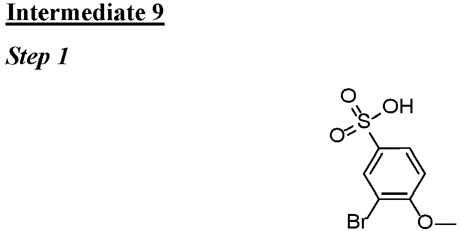

### 3-Bromo-4-methoxybenzenesulfonic acid (Intermediate 9-1)

To a mixture of 3-bromo-4-methoxy-benzenesulfonyl chloride (3 g, 10.5 mmol) in 1,4-dioxane (6 mL), water was added (6 mL) and RM refluxed for 3 h. RM was evaporated to dryness to afford the title product (2.8 g) that was used in the next steps without further purification.
LCMS (Method 1): Rt = 0.52 min, ES⁻ *m*/*z* 264.7/266.7[M-H]⁻

### Step 2

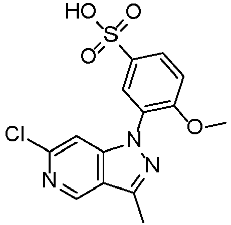

### 3-(6-Chloro-3-methyl-1H-pyrazolo[4,3-c]pyridin-1-yl)-4-methoxybenzenesulfonic acid (Intermediate 9)

Copper(I)iodide (1.13 g, 6.0 mmol), *N*,*N-*Dimethylglycine (1.23 g, 12 mmol), K₂CO₃ (1.65 g, 12 mmol), intermediate 9-1 (2.8 g, 10 mmol) and 6-chloro-3-methyl-1*H-*pyrazolo[4,3-*c*]pyridine (1 g, 6 mmol) in DMSO (15 mL) were stirred at 100 °C overnight under argon. After cooling to RT, RM was diluted with aq. 1M HCl (15 mL) and filtrate to remove undissolved solids. Aqueous extract was washed with DCM (3x15 mL) and then freeze dried. The lyophilized residue was purified by flash chromatography on C18 silica by gradient eluition from 5 to 99% acetonitrile in water (+0.1% v/v HCOOH) to afford the desired product (480 mg).

LCMS (Method 1): Rt = 0.63 min, ES⁺ *m*/*z* 353.9/355.8 [M+H]⁺

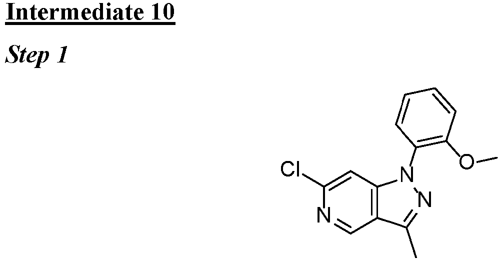

### 6-Chloro-1-(2-methoxyphenyl)-3-methyl-1H-pyrazolo[4,3-c]pyridine (Intermediate 10-1)

Copper(I)iodide (511 mg, 2.69 mmol), *N*,*N*-dimethylglycine (554 mg, 5.37 mmol), K₂CO₃ (1.48 mg, 10.7 mmol), 1-bromo-2-methoxy-benzene (2 mL, 16.1 mmol) and 6-chloro-3-methyl-1*H*-pyrazolo[4,3-c]pyridine (900 mg, 5.37 mmol) in DMSO (20 mL) were stirred at 100 °C overnight under argon. After cooling to RT, RM was diluted with EtOAc (100 mL), washed with 15% w/w aq. ammonia (3x100 mL) and sat. aq. NaCl (5x50 mL). Combined organic layers were dried over Na₂SO₄, concentrated *in vacuo,* and the residue purified by flash chromatography on a Si cartridge by eluting with 0-15 % EtOAc in DCM to afford the desired compound (950 mg).
LCMS (Method 2): Rt = 1.09 min, ES⁺ *m*/*z* 273.9/275.7[M+H]⁺

### Step 2

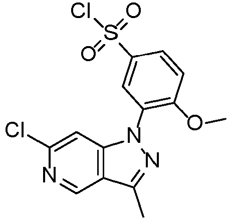

### 3-(6-Chloro-3-methyl-1H-pyrazolo[4,3-c]pyridin-1-yl)-4-methoxybenzenesulfonyl chloride (Intermediate 10)

Intermediate10-1 (500 mg, 1.8 mmol) was cooled in an ice bath, then ClSO₃H (2.8 mL, 42 mmol) was slowly added under argon. RM was stirred for 1 h at 0-5 °C, added with SOCl₂ (0.56 mL, 7.6 mmol) and stirred for further 1 h. After quenching RM in a water/ice mixture, the precipitate formed was collected by filtration, washed with ice cold water and dried to afford the title product (620 mg) that was used in the next steps without further purification.

LCMS (Method 1): Rt = 1.18 min, ES⁺ *m*/*z* 371.4/373.6/375.5 [M+H]⁺.

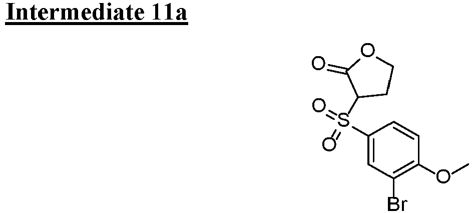

### 3-((3-Bromo-4-methoxyphenyl)sulfonyl)dihydrofuran-2(3H)-one (Intermediate 11a)

3-bromo-4-methoxy-benzenesulfonyl chloride (500 mg, 1.75 mmol), Na₂SO₃ (441 mg, 3.50 mmol) and NaHCO₃ (294 mg, 3.50 mmol) in water (3.75 mL) were stirred at RT for 1.5 h. Tetrabutylammonium bromide (35.0 mg, 0.109 mmol) and 3-bromotetrahydrofuran-2-one (321 µL, 3.50 mmol) were added. RM was stirred at 70 °C for 1.5 h and then partitioned between DCM and water. Organic layer was dried over Na₂SO₄ and concentrate under reduced pressure. The residue was chromatographed on silica by eluting with EtOAc/hexanes (1:1) to afford the title product (160 mg).

LCMS (Method 2): Rt = 0.91 min, ES⁻ *m*/*z=333.1*/*335.1* [M-H]⁻

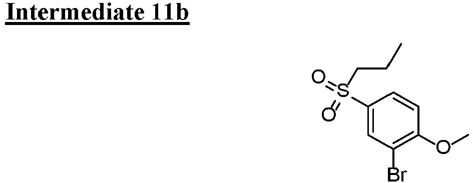

### 2-Bromo-1-methoxy-4-(propylsulfonyl)benzene (Intermediate 11b)

Title compound was prepared on a similar manner to intermediate 11a starting from 3-bromo-4-methoxy-benzenesulfonyl chloride and 1-iodopropane.
LCMS (Method 2): Rt = 1.03 min
¹H-NMR (300 MHz, *CDCl₃*) δ: 8.05 (d, J=2.3 Hz, 1H), 7.81 (dd, J=8.5, 2.3 Hz, 1H), 6.99 (d, J=8.6 Hz, 1H), 3.96 (s, 3H), 3.00-3.05 (m, 2H), 1.66-1.79 (m, 2H), 0.98 (t, J=7.1 Hz, 3H)

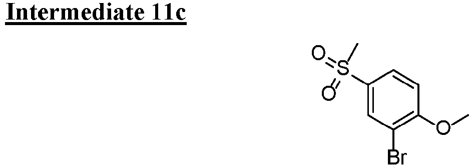

### 2-Bromo-1-methoxy-4-(methylsulfonyl)benzene (Intermediate 11c)

The title compound was prepared on a similar manner to intermediate 11a starting from 3-bromo-4-methoxy-benzenesulfonyl chloride and iodomethane.
LCMS (Method 2): Rt = 0.86 min
¹H-NMR (300 MHz, DMSO-*d₆*) δ: 8.07 (d, J=2.2 Hz, 1H), 7.90 (dd, J=8.7, 2.2 Hz, 1H), 7.33 (d, J=8.7 Hz, 1H), 3.99 (s, 3H), 3.21 (s, 3H)

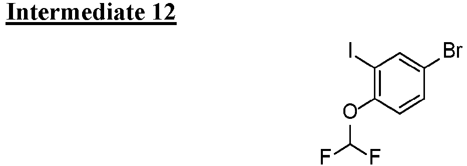

### 4-Bromo-1-(difluoromethoxy)-2-iodobenzene (Intermediate 12)

4-bromo-2-iodo-phenol (5.00 g, 16.7 mmol), sodium chlorodifluoroacetate (5.87 g, 38.5 mmol) and Cs₂CO₃ (7.63 mg, 23.4 mmol) in DMF (25 mL) were stirred at 100 °C for 2 h. After cooling to RT, RM was poured into water (250 mL) and filtered. Filtrate was extracted with EtOAc (2x50 mL). Combined organic layers were washed with sat. aq. NaHCO₃ (3x40mL), water (40 mL), sat. aq. NaCl (40 mL), dried over MgSO₄ and solvent evaporated under reduced pressure to give the desired product (4.50 g) that was used in the next steps without further purification.
LCMS (Method 2): Rt = 1.31 min
¹H-NMR (300 MHz, *CDCl₃*) δ: 7.96 (d, J=2.3 Hz, 1H), 7.44 (dd, J=8.7, 2.4 Hz, 1H), 7.02 (d, J=8.7 Hz, 1H), 6.48 (t, J=73.0 Hz, 1H)

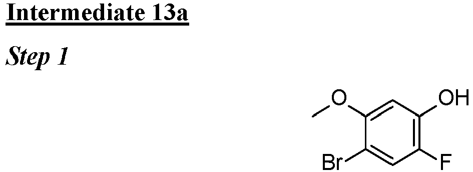

### 4-Bromo-2-fluoro-5-methoxyphenol (Intermediate 13a-1)

To a mixture of 2-fluoro-5-methoxy-phenol (400 mg, 2.81 mmol) in hexafluoroisopropanol (11.3 mL), NBS (501 mg, 2.81 mmol) was added and RM stirred at RT for 1 h. RM was quenched with sat. aq. NaHCO₃ and extracted with EtOAc. Organic layer was washed with sat. aq. NaHCO₃ (3x15 mL), sat. aq. NaCl, dried over Na₂SO₄ and evaporated under reduced pressure to give the title product (28mg) that was used in the next synthetic step without further purification.
LCMS (Method 1): Rt = 0.92 min, ES⁻ 219.0/221.1 [M-H]⁻

### Step 2

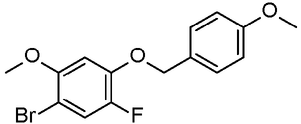

### 1-Bromo-5-fluoro-2-methoxy-4-((4-methoxybenzyl)oxy)benzene (Intermediate 13a)

To a mixture of intermediate 13a-1 (724 mg, 3.28 mmol) in DMF (6.3 mL) at 0°C, PMB-Cl (577 µL, 4.26 mmol) and anhydrous K₂CO₃ (1.36 g, 9.83 mmol) were added. RM was stirred 0 °C at for 1 h and at RT overnight. RM was diluted with EtOAc (25 mL), washed with sat. aq. NaHCO₃ (3x15 mL) and sat. aq. NaCl (15 mL). Organic layer was dried over Na₂SO₄ and concentrated *in vacuo.* The residue was purified by flash chromatography on a Si cartridge by eluting with 0-10 % EtOAc in cyclohexane to give the title product (567 mg).
LCMS (Method 2): Rt = 1.35 min
¹H-NMR (300 MHz, *CDCl₃*) δ: 7.32 (m, 2H), 7.25 (d, J=10.2 Hz, 1H), 6.89 (m, 2H), 6.56 (d, J=7.1 Hz, 1H), 5.06 (s, 2H), 3.80 (s, 3H), 3.78 (s, 3H)

### Intermediates 13b-c

The following intermediates were prepared in a similar manner to intermediate 13a from the indicated starting materials.

| Intermediate | Structure | *Step 1* | | *Step 2* | |
|---|---|---|---|---|---|
| | | Starting Material | Name of intermediate Step 1 / LCMS | ¹H-NMR | LCMS |
| Intermediate 13b | 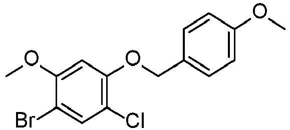 | 2-Chloro-5-methoxy -phenol | 4-bromo-2-chloro-5-methoxyphenol | (500 MHz, *CDCl₃*) δ: 7.52 (s, 1H), 7.38 (d, J=8.3 Hz, 2H), 6.93 (d, J=8.3 Hz, 2H), 6.55 (s, 1H), 5.09 (s, 2H), 3.83 (bs, 6H) | (Method 2): Rt = 1.43 min |
| | | | (Method 1): Rt = 1.02 min, ES⁻ *m*/*z* 235.1/237.0/239.0 [M-H]⁻ | | |
| Intermediate 13c | 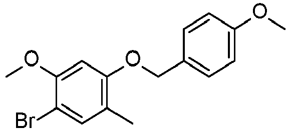 | 4-bromo-5-methoxy -2-methylph enol | 2-Chloro-5-methoxy-phenol | (500 MHz, CDCl3) δ: 7.52 (s, 1H), 7.38 (d, J=8.3 Hz, 2H), 6.93 (d, J=8.3 Hz, 2H), 6.55 (s, 1H), 5.09 (s, 2H), 3.83 (bs, 6H) | (Method 2): Rt = 1.41 min |
| | | | (Method 1): Rt = 0.98 min, ES+ m/z 217.0/219.0 [M+H]+ | | |

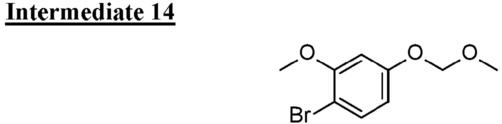

### 1-bromo-2-methoxy-4-(methoxymethoxy)benzene (Intermediate 14) (Intermediate 14)

A solution of 4-bromo-3-methoxyphenol (160 mg, 0.67 mmol) in DMF (400 µl) was added to a suspension of NaH (60% mineral oil dispersion, 48.2 mg, 1.21 mmol) in DMF (1.2 mL) at 0 °C. RM was stirred for 30 min followed by addition of chloro(methoxy) methane (91.0 µl, 1.14 mmol). RM was stirred for 2 h at RT, then quenched with water (10 mL) and extracted with diethyl ether (10 mL). Organic layer was washed with sat. aq. NaCl (2 x 10 mL), dried over MgSO₄ and concentrated under reduced pressure. The residue was purified by flash chromatography on Si cartridge by eluting with 0-50 % EtOAc in cyclohexane to afford the title product. (202 mg).
LCMS (Method 2): Rt = 1.14 min
1H-NMR (300 MHz, CDCl3) δ: 7.38 (d, J=9.0 Hz, 1H), 6.60 (d, J=2.7 Hz, 1H), 6.54 (dd, J=8.7, 2.7 Hz, 1H), 5.14 (s, 2H), 3.85 (s, 3H), 3.46 (s, 1H).

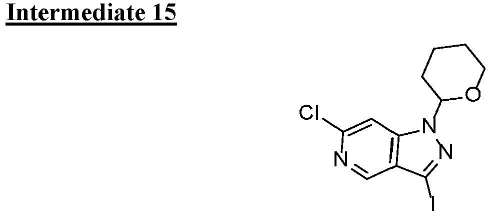

### 6-Chloro-3-iodo-1-(tetrahydro-2H-pyran-2-yl)-1H-pyrazolo[4,3-c]pyridine (Intermediate 15)

Dihydropyran (3.4 mL, 37.4 mmol) and methanesulfonic acid (0.16 mL, 2.5 mmol) were added to 6-chloro-3-iodo-1H-pyrazolo[4,3-c]pyridine (3.48 g, 11.1 mmol) in DCM (33 mL) and THF (16.6 mL). RM was stirred at 40 °C for 2 h, then at RT overnight. Solvents were evaporated and the residue purified by flash chromatography on a Si cartridge by eluting with 0-100% of EtOAc in cyclohexane to afford the title product (1.48 g).

LCMS (Method 2): Rt = 1.18 min, ES⁺ *m*/*z* 364.0/366.0 [M+H]⁺

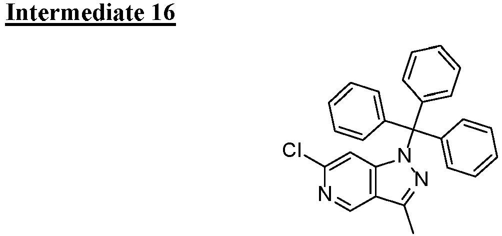

### 6-Chloro-3-methyl-1-trityl-1H-pyrazolo[4,3-c]pyridine (Intermediate 16)

Sodium hydride (60.0 % disperssion in mineral oil, 1.00 g, 25.0 mmol) was added portionwise to 6-chloro-3-methyl-1H-pyrazolo[4,3-c]pyridine (2.50 g, 14.9 mmol) in dry THF (50.0 mL). RM was stirred at 0°C for 1 h, then and trityl chloride (5.10 g, 18.3 mmol) added portionwise and RM stirred overnight at RT. RM was quenched at 0 - 5 °C with sat. aq. NH₄Cl, and THF removed under reduced pressure. The remaining mixture was extracted with EtOAc (3x). Combined organic layers were concentrated and the formed precipitate collected by filtration, washed with EtOAc and dried to afford the title product (3.77 g).

LCMS (Method 2): Rt = 1.51, ES⁺ *m*/*z* 410.2/421.1 [M+H]⁺

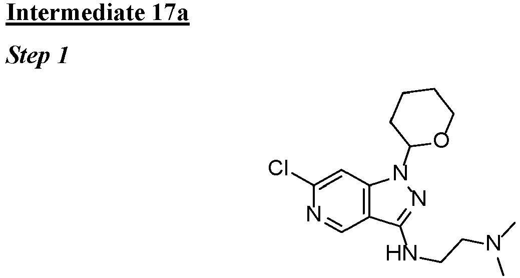

### N1-(6-Chloro-1-(tetrahydro-2H-pyran-2-yl)-1H-pyrazolo[4,3-c]pyridin-3-yl)-N2,N2-dimethylethane-1,2-diamine (Intermediate 17a-1)

L-proline (85.5 mg, 0.74 mmol) and copper(I)iodide (94.3 mg, 0.5 mmol) were added to a mixture of intermediate 15 (900 mg, 2.48 mmol), *N'*,*N'*-dimethylethane-1,2-diamine (1.23 mL, 11.3 mmol), K₂CO₃ (2.05 g, 14.9 mmol) in DMF (8.0 mL) and RM stirred at 110 °C for 2 h under argon atmosphere. After cooling to RT, RM was diluted with water (80 mL) and extracted with EtOAc (3x10 mL). Combined organic layers were washed with sat. aq. NaHCO₃, passed through phase separator and concentrated *in vacuo.* The residue was purified by flash chromatography on a Si cartridge by eluting with 0-100% DCM / MeOH/ NH₄OH (90:9:0.5) in DCM To give title compound (630 mg).
LCMS (Method 1): Rt = 0.62 min, ES⁺ *m*/*z* 323.9/325.9 [M+H]⁺

### Step 2

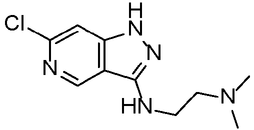

### N1-(6-Chloro-1H-pyrazolo[4,3-c]pyridin-3-yl)-N2,N2-dimethylethane-1,2-diamine (Intermediate 17a)

Triethylsilane (932 µL, 5.84 mmol) was added dropwise to intermediate 17a-1 (630 mg, 1.95 mmol) in DCM (9 mL) /TFA (2.29 mL, 29.9 mmol). RM was stirred at RT for 1 h, then diluted with DCM and extracted with sat. aq. NaHCO₃ (10 mL). Aqueous layer (adjusted at pH 9.6) was further extracted with EtOAc (3x), followed by DCM/iPrOH (1:1). Combined organic layers were passed through a phase separator and evaporated under reduced pressure to afford the title product (396 mg) that was used in the next steps without further purification.
LCMS (Method 1): Rt = 0.39 min, ES⁺ *m*/*z* 240.0/242.0 [M+H]⁺

### Preparation of intermediates 17b to 17d

The following intermediates were prepared in a similar manner to intermediate 17a from the indicated starting materials that replace *N',N'-dimethylethane-1,2-diamine* in step 1.

| Intermediate | Structure | *Step 1* | | *Step 2* |
|---|---|---|---|---|
| | | Starting Material | Name of intermediate Step 1 / LCMS | LCMS |
| Intermediate 17b | 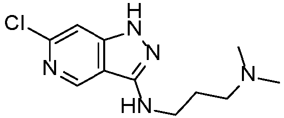 | N',N'-dimethylpropane-1,3-diamine | N1-(6-chloro-1-(tetrahydro-2H-pyran-2-yl)-1H-pyrazolo[4,3-c]pyridin-3-yl)-N3,N3-dimethylpropane-1,3-diamine | (Method 2): Rt = 0.74 min, ES+ m/z 254.1 /256.2 [M+H]+ |
| | | | (Method 2): Rt = 1.12 min, ES+ m/z 338.2 /340.2 [M+H]⁺ | |
| Intermediate 17c | 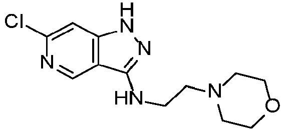 | 2-morpholinoethan-1-amine | 6-chloro-N-(2-morpholinoethyl)-1-(tetrahydro-2H-pyran-2-yl)-1H-pyrazolo[4,3-c]pyridin-3-amine | (Method 2): Rt = 0.59 min, ES+ m/z 282.1 /284.2 [M+H]+ |
| | | | (Method 2): Rt = 0.91 min, ES+ m/z 366.2/368.2 [M+H]⁺ | |
| Intermediate 17d | 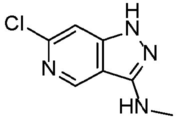 | methylamine hydrochloride | 6-chloro-N-methyl-1-(tetrahydro-2H-pyran-2-yl)-1H-pyrazolo[4,3-c]pyridin-3-amine | (Method 2): Rt = 0.52 min, ES⁻ *m*/*z* 183.0/185.0 [M+H]⁺ |
| | | | (Method 2): Rt = 0.92 min, ES+ m/z 267.1/269.1 [M+H]+ | |

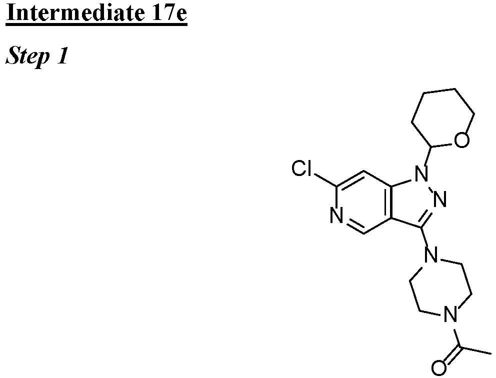

### 1-(4-(6-Chloro-1-(tetrahydro-2H-pyran-2-yl)-1H-pyrazolo[4,3-c]pyridin-3-yl)piperazin-1-yl)ethan-1-one (Intermediate 17e-1)

Intermediate 15 (150 mg, 0.41 mmol), 1-piperazin-1-ylethanone (63.5 mg, 0.495 mmol), Xanthphos (23.9 mg, 0.04 mmol), Pd₂(dba)₃ (11.9 mg, 0.02 mmol), Cs₂CO₃ (269 mg, 0.83 mmol) in 1,4-Dioxane (2.7 mL) were heated at 90 °C for 16 h under nitrogen atmosphere. After cooling to RT, RM was diluted with water (10 mL) and extracted with EtOAc (3x10 mL). Combined organic layers were washed with sat. aq. NaCl (10 mL), dried over anhydrous MgSO₄ and concentrated *in vacuo.* The residue was purified by flash chromatography on a Si cartridge by eluting with 0-50% DCM/MeOH (20:1) in DCM to afford the title product (105.3 mg).
LCMS (Method 2): Rt = 0.94 min, ES⁺ *m*/*z* 364.2/366.2 [M+H]⁺

### Step 2

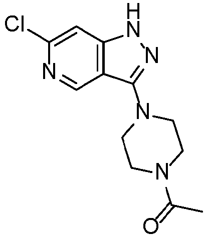

### 1-(4-(6-Chloro-1H-pyrazolo[4,3-c]pyridin-3-yl)piperazin-1-yl)ethan-1-one (Intermediate 17e)

HCl (4M in 1,4-dioxane, 3.7 mL, 14.9 mmol) was added dropwise to a mixture of intermediate 17e-1 (140 mg, 0.331 mmol) in isopropanol (3.7 mL). RM was stirred at RT for 2 h, then partitioned between water (pH adjusted to 8) and DCM (30 mL). Aqueous layer was further extracted with DCM (2 x 15 mL) and combined organic layers were dried over MgSO₄, filtered and evaporated under reduced pressure. The residue was purified by flash chromatography on a 4 cartridge by eluting with 0-50% DCM/MeOH (10:1) in DCM to afford the title product (65.9 mg)

LCMS (Method 2): Rt = 0.61 min, ES⁺ *m*/*z* 280.1/282.1 [M+H]⁺

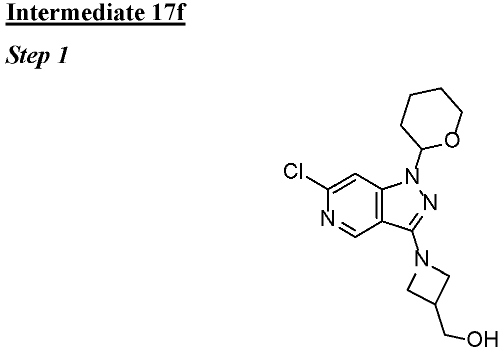

### (1-(6-chloro-1-(tetrahydro-2H-pyran-2-yl)-1H-pyrazolo[4,3-c]pyridin-3-yl)azetidin-3-yl)methanol (Intermediate 17f-1)

Title product was obtained on a similar manner to intermediate 17e-1 starting from intermediate 15 and azetidin-3-ylmethanol hydrochloride.
LCMS (Method 2): Rt = 0.84 min, ES⁺ *m*/*z* 323.1 /325.1 [M+H]⁺

### Step 2

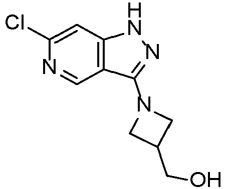

### (1-(6-chloro-1H-pyrazolo[4,3-c]pyridin-3-yl)azetidin-3-yl)methanol (Intermdiate 17f-2)

Title product was obtained in a similar manner to intermediate 17a *(step 2)* starting from intermediate 17f-1.

LCMS (Method 2): Rt = 0.53 min, ES⁺ *m*/*z* 239.1 /240.1 [M+H]⁺

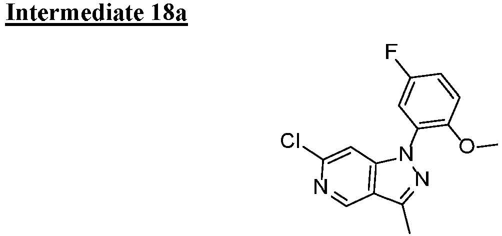

### 6-Chloro-1-(5-fluoro-2-methoxyphenyl)-3-methyl-1H-pyrazolo[4,3-c]pyridine (Intermediate 18a)

Copper (I) iodide (170 mg, 0.89 mmol), *N*,*N*-Dimethylglycine (185 mg, 1.79 mmol), K₂CO₃ (495 mg, 3.58 mmol), 2-bromo-4-fluoro-1-methoxy-benzene (697 µL, 5.4 mmol) and 6-chloro-3-methyl-1H-pyrazolo[4,3-c]pyridine (300 mg, 1.8 mmol) in DMSO (3 mL) were stirred at 100 °C overnight under argon atmosphere. RM was diluted with EtOAc (15 mL) and washed with 15% aq. ammonia (3x15 mL) and sat. aq. NaCl (10 mL). The organic layer was dried over Na₂SO₄ and concentrated *in vacuo.* The residue was purified by flash chromatography on a Si cartridge by eluting with 0-40% EtOAc in cyclohexane to give the desired product (380 mg).
LCMS (Method 2): Rt = 1.10 min, ES⁺ *m*/*z* 292.0/293.9 [M+H]⁺

### Preparation of intermediates 18b to 18y

The following intermediates were prepared in a similar manner to intermediate 18a from the indicated starting materials. When minor modifications on base, solvent, temperature, reaction time, ligand and/or copper were made, they were detailed below in brackets

| Intermediate | Structure | Starting material | LCMS |
|---|---|---|---|
| Intermediate 18b | 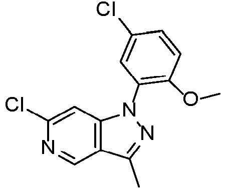 | 2-bromo-4-chloro-1-methoxy-benzene / 6-chloro-3-methyl-1*H*-pyrazolo[4,3-*c*]pyridine | (Method 2): Rt = 1.21 min, ES⁺ *m*/*z* 307.9/309.9 [M+H]⁺ |
| Intermediate 18c | 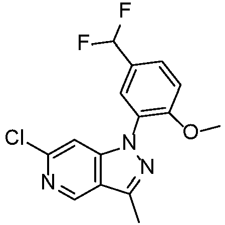 | Intermediate 1 / 6-chloro-3-methyl-1*H-*pyrazolo[4,3-*c*]pyridine | (Method 2): Rt = 1.11 min, ES⁺ *m*/*z* 324.0/325.9 [M+H]⁺ |
| Intermediate 18d | 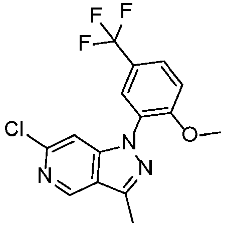 | 2-Bromo-1-methoxy-4-(trifluoromethyl) benzene / 6-chloro-3-methyl-1*H-*pyrazolo[4,3-*c*]pyridine | (Method 2): Rt = 1.22 min, ES⁺ *m*/*z* 342.0/343.9 [M+H]⁺ |
| | | (Reaction temperature: 140 °C) | |
| Intermediate 18e | 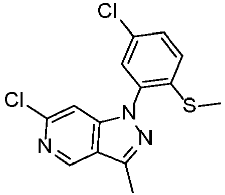 | 2-bromo-4-chloro-1-methylsulfanyl-benzene / 6-chloro-3-methyl-1*H-*pyrazolo[4,3-*c*]pyridine | (Method 2): Rt = 1.28 min, ES⁺ *m*/*z* 324.0/326.0/32 7.9 [M+H]⁺ |
| | | (Reaction solvent: DMF) | |
| Intermediate 18f | 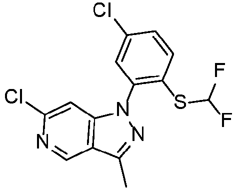 | Intermediate 2 /6-chloro-3-methyl-1*H-*pyrazolo[4,3-*c*]pyridine | (Method 2): Rt = 1.32 min, ES⁺ *m*/*z* 360.0/362.0/ 364.0 [M+H]⁺ |
| | | (Reaction solvent DMF) | |
| Intermediate 18g | 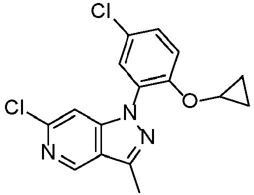 | Intermediate 3 /6-chloro-3-methyl-1*H-*pyrazolo[4,3-*c*]pyridine | (Method 1): Rt = 1.35 min, ES⁺ *m*/*z* 334.1/336.0/ 338.1 [M+H]⁺ |
| Intermediate 18h | 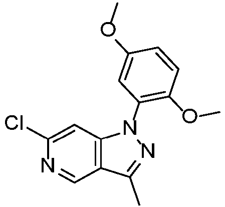 | 2-iodo-1,4-dimethoxy-benzene / 6-chloro-3-methyl-1*H*-pyrazolo[4,3-*c*]pyridine | (Method 2): Rt = 1.11 min, ES⁺ *m*/*z* 304.2/306.1 [M+H]⁺ |
| Intermediate 18i | 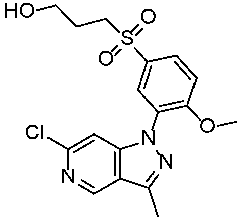 | Intermediate 11a / 6-chloro-3-methyl-1*H-*pyrazolo[4,3-*c*]pyridine | (Method 2): Rt = 0.81 min, ES⁺ *m*/*z* 396.1/398.0 [M+H]⁺ |
| | | (Ligand: trans-N,N'-Dimethylcyclohexan e-1,2-diamine / reaction solvent:DMF) | |
| Intermediate 18j | 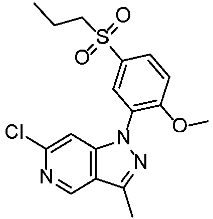 | Intermediate 11b / 6-chloro-3-methyl-1*H-*pyrazolo[4,3-*c*]pyridine | (Method 2): Rt = 1.01 min, ES⁺ *m*/*z* 380.1/382.0 [M+H]⁺ |
| | | (Ligand: trans-N,N'-Dimethylcyclohexan e-1,2-diamine / reaction solvent: DMF) | |
| Intermediate 18k | 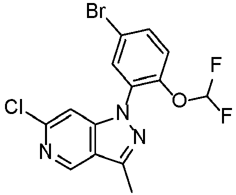 | Intermediate 12/ 6-chloro-3-methyl-1*H-*pyrazolo[4,3-*c*]pyridine | (Method 2): Rt = 1.25 min, ES⁺ *m*/*z* 387.9/389.9/39 1.9 [M+H]⁺ |
| | | (Ligand: 8-quinolinol / Reaction temperature: 105 °C) | |
| Intermediate 181 | 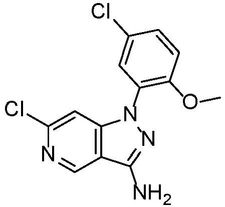 | 2-bromo-4-chloro-1-methoxy-benzene / 3-amino-6-chloro-1*H*-pyrazolo[4,3-*c*]pyridine | (Method 2): Rt = 1.01 min, ES⁺ *m*/*z* 308.9/310.8/31 2.8 [M+H]⁺ |
| | | (Ligand: trans-N,N'-Dimethylcyclohexan e-1,2-diamine / reaction solvent:DMF) | |
| Intermediate 18m | 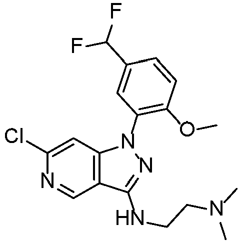 | Intermediate 17a / Intermediate 1 | (Method 2): Rt = 1.09 min, ES⁺ *m*/*z* 396.1/398.1 [M+H]⁺ |
| Intermediate 18n | 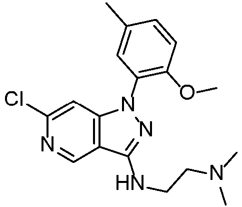 | Intermediate 17a / 2-bromo-1-methoxy-4-methyl-benzene | (Method 1): Rt = 0.81 min, ES⁺ *m*/*z* 360.2/362.1 [M+H]⁺ |
| Intermediate 18o | 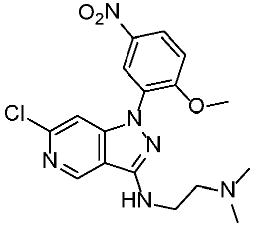 | Intermediate 17a / 2-bromo-1-methoxy-4-nitro-benzene | (Method 1): Rt = 1.04 min, ES⁺ *m*/*z* 391.1/393.1 [M+H]⁺ |
| Intermediate 18p | 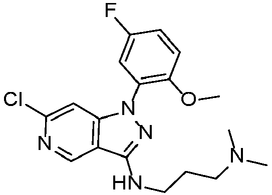 | Intermediate 17b / 2-bromo-4-fluoro-1-methoxy-benzene | (Method 1): Rt = 0.69 min, ES⁺ *m*/*z* 378.1/380.1 [M+H]⁺ |
| Intermediate 18q | 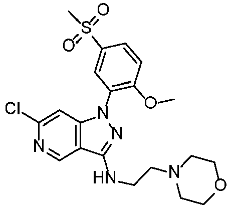 | Intermediate 17c / Intermediate 11c | (Method 2): Rt = 0.80 min, ES⁺ *m*/*z* 466.1/468.1 [M+H]⁺ |
| Intermediate 18r | 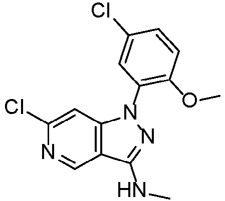 | Intermediate 17d / 2-bromo-4-chloro-1-methoxy-benzene | (Method 2): Rt = 1.14 min, ES⁺ *m*/*z* 323.0/325.0 [M+H]⁺ |
| Intermediate 18s | 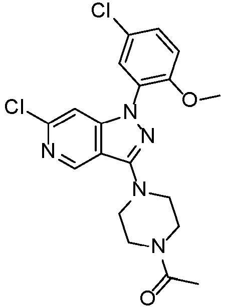 | Intermediate 17e / 2-bromo-4-chloro-1-methoxy-benzene | (Method 2): Rt = 1.12 min, ES⁺ *m*/*z* 420.2/422.2 [M+H]⁺ |
| Intermediate 18t | 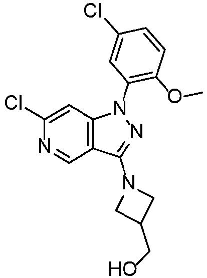 | Intermediate 17f / 2-bromo-4-chloro-1-methoxy-benzene | (Method 2): Rt = 1.04 min, ES⁺ *m*/*z* 379.1/381.0 [M+H]⁺ |
| Intermediate 18u | 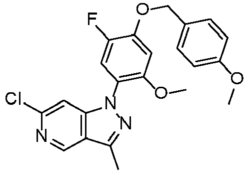 | Intermediate 13a / 6-chloro-3-methyl-1*H-*pyrazolo[4,3-*c*]pyridine | (Method 2): Rt = 1.32 min, ES⁺ *m*/*z* 428.1/430.0 [M+H]⁺ |
| | | (Ligand: trans-N,N'-Dimethylcyclohexane -1,2-diamine / reaction solvent:DMF) | |
| Intermediate 18v | 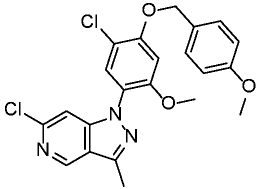 | Intermediate 13b / 6-chloro-3-methyl-1*H-*pyrazolo[4,3-*c*]pyridine | (Method 2): Rt = 1.38 min, ES⁺ *m*/*z* 444.1/446.1/44 8.1 [M+H]⁺ |
| | | (Ligand: trans-N,N'-Dimethylcyclohexan e-1,2-diamine / reaction solvent:DMF) | |
| Intermediate 18w | 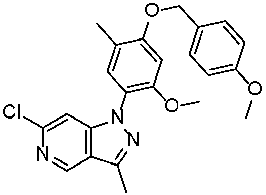 | Intermediate 13c / 6-chloro-3-methyl-1*H-*pyrazolo[4,3-*c*]pyridine | (Method 2): Rt = 1.38 min, ES⁺ *m*/*z* 424.0/425.9 [M+H]⁺ |
| | | (Ligand: trans-N,N'-Dimethylcyclohexan e-1,2-diamine / reaction solvent:DMF) | |
| Intermediate 18x | 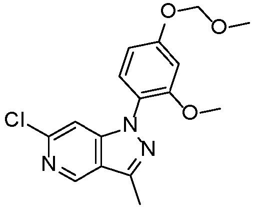 | Intermediate 14 / 6-chloro-3-methyl-1*H-*pyrazolo[4,3-*c*]pyridine | (Method 2): Rt = 1.12 min, ES⁺ *m*/*z* 334.1/336.1 [M+H]⁺ |
| Intermediate 18y | 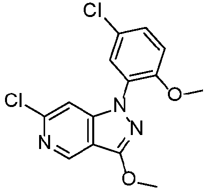 | 6-chloro-3-methoxy-1H-pyrazolo[4,3-c]pyridine / 2-bromo-4-chloro-1-methoxy-benzene | (Method 2): Rt = 1.29 min, ES⁺ *m*/*z 324.1,* 326.1, 328.1 [M+H]⁺ |

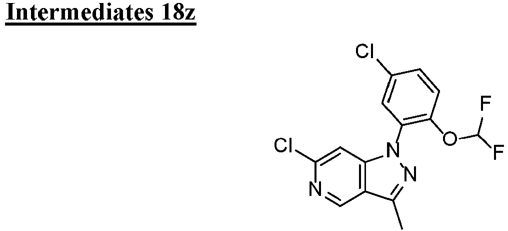

### 6-Chloro-1-(5-chloro-2-(difluoromethoxy)phenyl)-3-methyl-1H-pyrazolo[4,3-c]pyridine (Intermediate 18z)

Intermediate 4 (500 mg, 2.6 mmol), intermediate 8a (680 mg, 2.8 mmol) and NMP (3 mL) were stirred at 60 °C for 1 h and at 120 °C for 5 h. After cooling to RT, RM was poured in water (20 mL) and stirred for 10 min. The precipitate formed was collected by filtration, washed with water and purified by flash chromatography on a Si cartridge by eluting with 0-24 % EtOAc in cyclohexane to afford the title product (242 mg).
LCMS (Method 2): Rt = 1.25 min, ES⁺ *m*/*z* 344.1/346.1/348.1 [M+H]⁺

From the flash chromatography purification, a second pure product was obtained. LC-MS analysis confirmed the structure of des-methyl of intermediate 18z and it was identified as **Intermediate 18aa**

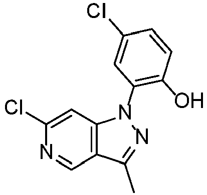

### 4-chloro-2-(6-chloro-3-methyl-1H-pyrazolo[4,3-c]pyridin-1-yl)phenol (Intermediate 18aa)

LCMS (Method 2): Rt = 0.65 min, ES⁺ *m*/*z* 294.1/296.1/298.1 [M+H]⁺

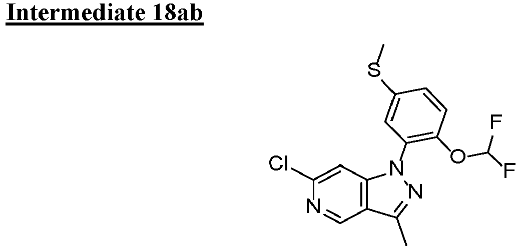

### 6-Chloro-1-(2-(difluoromethoxy)-5-(methylthio)phenyl)-3-methyl-1H-pyrazolo[4,3-c]pyridine (Intermeidate 18ab)

Intermediate 4 (180 mg, 0.95 mmol) and intermediate 8d (243 mg, 0.95 mmol) in NMP (2 mL) were stirred at RT overnight and then at 170 °C under microwave irradiation for 1 h. After cooling to RT, RM was diluted with water (15 mL) and extracted with EtOAc (2x10 mL). Combined organic layers were washed with water (5x15 mL), dried over Na₂SO₄ and solvent removed *in vacuo.* The crude was purified by flash chromatography on a Si cartridge by eluting with 0-8 % EtOAc in cyclohexane to afford the title product (144 mg).

LCMS (Method 2): Rt = 1.26 min, ES⁺ *m*/*z* 356.2/358.1 [M+H]⁺

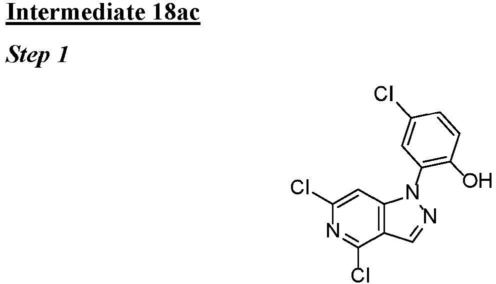

### 4-Chloro-2-(4,6-dichloro-1H-pyrazolo[4,3-c]pyridin-1-yl)phenol (Intermediate 18ac-1)

2,4,6-trichloropyridine-3-carbaldehyde (2 g, 9.5 mmol) and intermediate 8e (1.99 g, 9.0 mmol) in NMP (12 mL) were stirred at RT for 30 min and then at 150 °C under MW irradiation for 7.5 h. After cooling to RT, RM was partitioned between EtOAc (200 mL) and water (200 mL), then aqueous layer further extracted with EtOAc (150 mL). Combined organic layers were washed with water (150 mL), sat. aq. NaCl (150 mL), dried over MgSO₄ and evaporated under reduced pressure to give the title compound (2.6 g) that was used in the next steps without further purification.
LCMS (Method 2): Rt = 1.26 min, ES⁻ *m*/*z* 312.0/314.0/316.0 [M-H]⁻

### Step 2

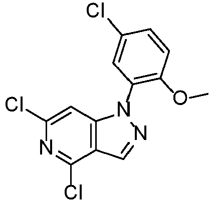

### 4,6-Dichloro-1-(5-chloro-2-methoxyphenyl)-1H-pyrazolo[4,3-c]pyridine (Intermediate 18ac)

K₂CO₃ (2.28 g, 17 mmol) and iodomethane (772 µL, 12 mmol) were added to a mixture of intermediate 18ac-1 (2.6 g, 8.3 mmol) in DMF (6 mL). RM was stirred at RT for nearly 2h, the diluted with EtOAc (20 mL) and washed with sat. aq. NaHCO₃ (2x20 mL). Organic layer was washed with sat. aq. NaCl and evaporated under reduced pressure. The residue was purified by flash chromatography on a Si cartridge by eluting with 0-50% cyclohexane:DCM (3:1) in cyclohexane to afford the the title product (167 mg).
LCMS (Method 2): Rt = 1.38 min
¹H-NMR (300 MHz, *CDCl₃*) δ: 8.30 (d, J=0.9 Hz, 1H), 7.46 (s, 1H), 7.44 (dd, J=9.3, 2.3 Hz, 1H), 7.09 (d, J=0.9 Hz. 1H), 7.02-7.06 (m, 1H), 3.81 (s, 3H).

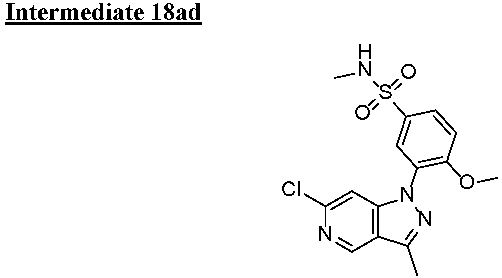

### 3-(6-Chloro-3-methyl-1H-pyrazolo[4,3-c]pyridin-1-yl)-4-methoxy-N-methylbenzenesulfonamide (Intermediate 18ad)

To a cooled mixture (in an ice bath) of intermediate 9 (70 mg, 0.20 mmol) in dry DMF (0.5 mL), SOCl₂ (58 µL, 0.79 mmol) was added and RM stirred at 0-5 °C for 30 min, followed by dropwise addition of methylamine (2.0 M in THF, 2.0 mL, 4.0 mmol). RM was stirred for further 10 min, then diluted with EtOAc (15 mL), washed with sat. aq. NaHCO₃ (3x10 mL) and sat. aq. NaCl (10 mL). Organic layer was dried over Na₂SO₄ and evaporated to dryness. The crude was purified by flash chromatography on a Si cartridge by eluting with 0-40 % EtOAc in DCM to afford the title product (40 mg).

LCMS (Method 1): Rt = 0.91 min, ES⁺ *m*/*z* 367.0/369.0 [M+H]⁺.

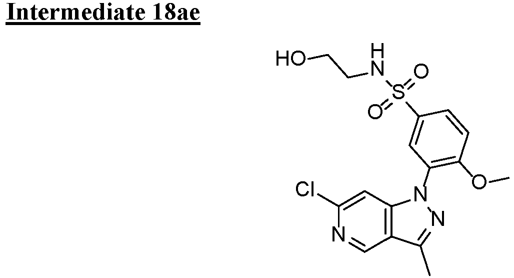

### 3-(6-Chloro-3-methyl-1H-pyrazolo[4,3-c]pyridin-1-yl)-N-(2-hydroxyethyl)-4-methoxybenzenesulfonamide (Intermediate 18ae)

Title product was prepared on a similar manner to intermediate 18ad starting from intermediate 9, ethanolamine and 1 equivalent of TEA.

LCMS (Method 1): Rt = 0.81 min, ES⁺ *m*/*z* 397.0/398.9 [M+H]⁺.

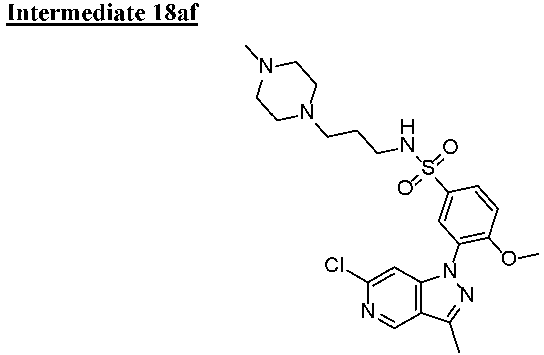

### 3-(6-Chloro-3-methyl-1H-pyrazolo[4,3-c]pyridin-1-yl)-4-methoxy-N-(3-(4-methylpiperazin-1- yl)propyl)benzenesulfonamide (Intermediate 18af)

Intermediate 10 (50 mg, 0.13 mmol) and TEA (56 µL, 0.4 mmol) were added to a solution of 3-(4-methylpiperazin-1-yl)propan-1-amine (22 µL, 0.13 mmol) in dry DCM (1 mL) and RM stirred at RT for 1 h. RM was diluted with EtOAc (10 mL) and washed with sat. aq. NaHCO₃ (3x5 mL) and sat. aq. NaCl (5 mL). Organic layer was dried over Na₂SO₄ and evaporated to dryness. The crude was purified by flash chromatography on a Si cartridge by eluting with 0-90 % DCM/MeOH/NH₄OH (90:9:0.5) in DCM to afford the title product (45 mg).
LCMS (Method 2): Rt = 0.86 min, ES⁺ *m*/*z* 493.1/495.0 [M+H]⁺

### Preparation of intermediates 18ag to 18ai

The following intermediates were prepared in a similar manner to Intermediate 18af from the indicated starting materials. When minor modifications on base, solvent, and/or temperature were made, they were detailed below in brackets

| Intermediate | Structure | Starting material | LCMS |
|---|---|---|---|
| Intermediate 18ag | 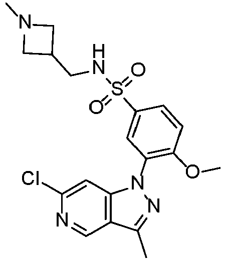 | Intermediate 10 / (1-methylazetidin-3-yl)methanamine | (Method 2): Rt = 0.90 min, ES⁺ *m*/*z* 436.1/438.0 [M+H]⁺ |
| Intermediate 18ah | 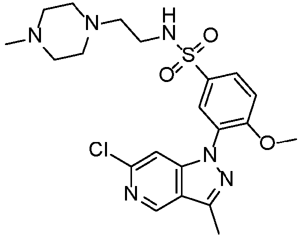 | Intermediate 10 / 2-(4-methylpiperazin-1-yl)ethanamine | (Method 1): Rt = 0.67 min, ES⁺ *m*/*z* 479.1/481.1 [M+H]⁺ |
| | | (Base: pyridine) | |
| Intermediate 18ai | 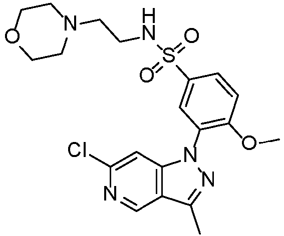 | Intermediate 10 / 2-morpholinoethanamine | (Method 1): Rt = 0.69 min, ES⁺ *m*/*z* 466.1/468.1 [M+H]⁺ |
| | | (Base: pyridine) | |

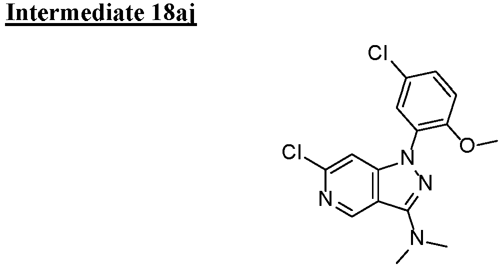

### 6-Chloro-1-(5-chloro-2-methoxyphenyl)-N,N-dimethyl-1H-pyrazolo[4,3-c]pyridin-3-amine (Intermediate 18aj)

To a mixture of intermediate 18r (46 mg, 0.14 mmol) in MeOH (2.8 mL)/THF (2 mL), aq. formaldehyde (37%, 53 µL, 0.71 mmol), acetic acid (4 µL, 0.07 mmol) and Na(CN)BH₃ (18 mg, 0.28) were added and RM stirred at RT overnight. Further 1 equivalent of Na(CN)BH₃, formaldehyde and acetic acid were added and stirring proceed at 40°C for further 6 hours. RM was diluted with water and extracted with EtOAc. Organic layer was washed with sat. aq. NaCl and evaporated under reduced pressure. The residue was purified by flash chromatography on a Si cartridge by eluting with 0-10 % MeOH in EtOAc To afford the title product (42.6 mg).

LCMS (Method 2): Rt = 1.26 min, ES⁺ *m*/*z* 337.1/339.1 [M+H]⁺

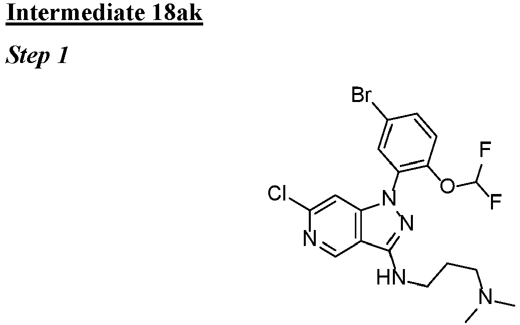

### N1-(1-(5-bromo-2-(difluoromethoxy)phenyl)-6-chloro-1H-pyrazolo[4,3-c]pyridin-3-yl)-N3,N3-dimethylpropane-1,3-diamine (Intermediate 18ak-1)

Title product was prepared on a similar manner to intermediate 18a starting from intermediate 17b and intermediate 12.
LCMS (Method 2): Rt = 1.34, ES⁺ *m*/*z* 474.1/476.1/478.1 [M+H]⁺

### Step 2

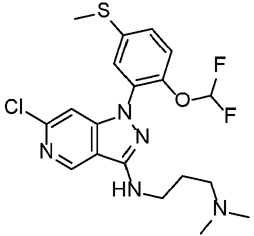

### N1-(6-chloro-1-(2-(difluoromethoxy)-5-(methylthio)phenyl)-1H-pyrazolo[4,3-c]pyridin-3-yl)-N3,N3-dimethylpropane-1,3-diamine (Intermediate 18ak)

Intermediate 18ak-1 (43 mg, 0.09 mmol), sodium thiomethoxide (19 mg, 0.27 mmol), Pd₂(dba)₃ (7.8 mg, 8.6 µmol), Xantphos (10 mg, 18 µmol) and degassed toluene (1 mL) were stirred at 80 °C under nitrogen. After 3h, a second equivalent of Pd₂(dba)₃ (7.8 mg) and Xantphos (10 mg) were added and stirring continued at 80 °C for further 3 h. RM was allowed to cool to RT and formed precipitate filtered off. Filtrate was diluted with EtOAc (10 mL), washed with sat. NaHCO₃ (10 mL) and sat. aq. NaCl (10 mL). Organic layer was evaporated under reduced pressure and the residue purified on a 4 g Si cartridge by eluting with 0-100 %, DCM/MeOH/NH₄OH (90:5:0.5) in DCM to afford the title product (27 mg).

LCMS (Method 2): Rt = 1.38, ES⁺ *m*/*z* 442.2/444.2 [M+H]⁺

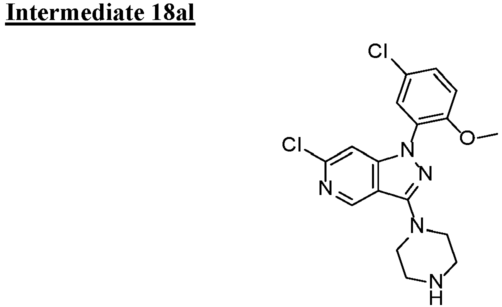

### 6-chloro-1-(5-chloro-2-methoxyphenyl)-3-(piperazin-1-yl)-1H-pyrazolo[4,3-c]pyridine (Intermediate 18al)

To a suspension of intermediate 18s (35 mg, 0.071 mmol) in methanol (1.7 mL), HCl (4.0M in 1,4-dioxane, 1.7 mL, 6.8 mmol) was added dropwise and RM stirred at 60 °C for 2 h. RM was partitioned between water and DCM (30 mL) and aqueous layer (after adjusting pH to 10.5) further extracted with DCM (2 x 15 mL). Combined organic layers were dried over MgSO₄ and evaporated to dryness. The crude was purified by flash chromatography on a Si cartridge by eluting with 0-80 % DCM/MeOH (10:1) in DCM to afford the title product (6.6 mg).

LCMS (Method 2): Rt = 1.09, ES⁺ *m*/*z* 378.2/380.2 [M+H]⁺

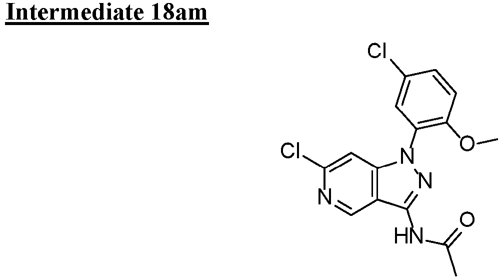

### N-(6-Chloro-1-(5-chloro-2-methoxyphenyl)-1H-pyrazolo[4,3-c]pyridin-3-yl)acetamide (Intermediate 18am)

Intermediate 18l (35.0 mg, 0.113 mmol) was dissolved in acetic anhydride (1 mL) and stirred at RT for 4 h. RM was poured on ice cold water (10 mL), pH adjusted to 7 with sat.aq. NaHCO₃ and extracted with EtOAc. Combined organic layers were washed with sat. aq. NaHCO₃ (2x 3 mL), dried over Na₂SO₄ and concentrated. The residue was purified by flash chromatography on a Si cartridge by eluting with EtOAc/MeOH (9:1) to afford the desired product (29 mg).

LCMS (Method 2): Rt = 1.01, ES⁺ *m*/*z* 351.0/353.0 [M+H]⁺

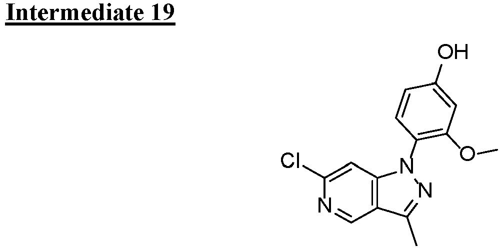

### 4-(6-chloro-3-methyl-1H-pyrazolo[4,3-c]pyridin-1-yl)-3-methoxyphenol (Intermediate 19)

To a solution of intermediate 18x (10.0 mg, 30 µmol) in MeOH (0.25 mL) was added 2M aq. HCl (127 µL, 255 µmol) at RT, then RM stirred for 1 h at 60 °C. RM of two parallel reactions made on the same scale were combined, cooled to RT, diluted with sat. aq. NH₄Cl (2 mL) and stirred for 30 min prior to be extracted with DCM (4x5 mL). Combined organic layers were passed through a phase separator and solvent removed under reduced pressure to afford the title product (10mg) that was used in the next synthetic step without further purification.

LCMS (Method 2): Rt = 0.63, ES⁺ *m*/*z* 290.1/292.1 [M+H]⁺

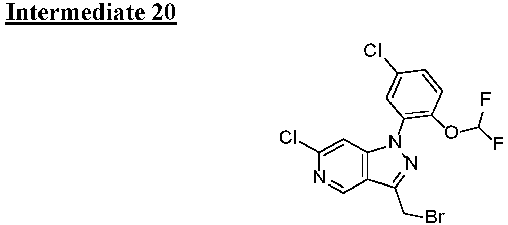

### 3-(bromomethyl)-6-chloro-1-(5-chloro-2-(difluoromethoxy)phenyl)-1H-pyrazolo[4,3-c]pyridine (Intermediate 20)

NBS (2.06 g, 12 mmol) and AIBN (317 mg, 1.9 mmol) were added to a mixture of intermediate 18z (3.2 g, 9.65 mmol) in tetrachloromethane (45 mL) and refluxed for 3 h under nitrogen. A second equivalent of NBS (2.06 g, 12 mmol) and AIBN (317 mg, 1.9 mmol) was added and stirring proceeded overnight. After cooling to RT, formed solids were filtered-off and washed with tetrachloromethane. Combined organic layers were washed with sat. aq. NaCl (80 mL), dried over Na₂SO₄ and evaporated under reduced pressure. The crude product was purified by flash cromatography on a Si cartridge by eluting with 0-100 % DCM in cyclohexane to afford the title compound (550 mg).
LCMS (Method 2): Rt = 1.35, ES⁺ *m*/*z* 422.0/424.0/426.0 [M+H]⁺

From the flash chromatography purification, a second pure product was obtained. LC-MS analysis confirmed the structure of dibromoderivative of intermediate 18z and it was identified as **Intermediate 21.**

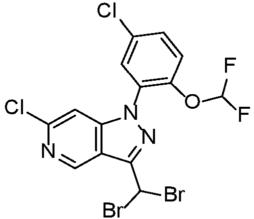

### 6-chloro-1-(5-chloro-2-(difluoromethoxy)phenyl)-3-(dibromomethyl)-1H-pyrazolo[4,3-c]pyridine (Intermediate 21)

LCMS (Method 2): Rt = 1.47, ES⁺ ion cluster with major peaks *m*/*z* 501.8 and 503.8 [M+H]⁺

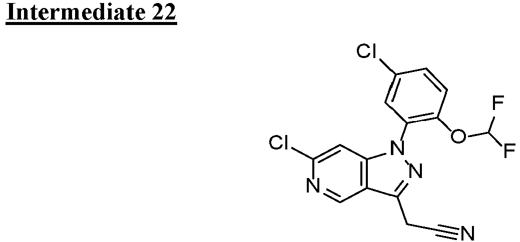

### 2-(6-Chloro-1-(5-chloro-2-(difluoromethoxy)phenyl)-1H-pyrazolo[4,3-c]pyridin-3-yl)acetonitrile (Intermediate 22)

To a mixture of intermediate 20 (2.0 g, 4.7 mmol) and ethanol (45 mL), sodium cyanide (278 mg, 5.7 mmol) in water (4 mL) was added and RM stirred at 80 °C overnight. After cooling to RT, RM was diluted with sat. aq. NaCl (40 mL) and extracted twice with EtOAc (40+20 mL). Combined organic layers were washed with sat. aq. NaCl (20 mL), dried over MgSO₄ and evaporated under reduced pressure. The residue was purified by flash chromatography on a Si cartridge by eluting with 0-30 % EtOAc in cyclohexane to afford the title compound (300 mg).

LCMS (Method 1): Rt = 1.18, ES⁺ *m*/*z* 369.9/372.0 [M+H]⁺

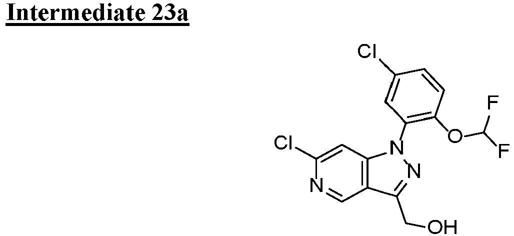

### (6-Chloro-1-(5-chloro-2-(difluoromethoxy)phenyl)-1H-pyrazolo[4,3-c]pyridin-3-yl)methanol

To a mixture of intermediate 20 (780 mg) in DMF (4 mL), potassium acetate (353 mg, 3.6 mmol) was added and RM stirred at 60 °C for 2 h. After cooling to RT, RM was diluted with water (20 mL) and extracted with EtOAc (3x10 mL). Combined organic layers were washed with water (2x10 mL), sat. aq. NaCl (2x10 mL), dried over Na₂SO₄ and solvent removed under reduced pressure. The residue was dissolved in a mixture of methanol (5mL) and water (1mL), added with potassium carbonate (498 mg, 3.6 mmol) and stirred at RT. Volatile solvents were removed under reduced pressure, the residue was dissolved in EtOAc (30 mL), washed with water (2x15 mL) and sat. aq. NaCl (15 mL). Organic layer was dried over Na₂SO₄, solvent removed under reduced pressure and the residue purified by flash chromatography on a Si cartridge by eluting with 0-10 %, DCM/MeOH/NH₄OH (90:5:0.5) in DCM to afford the title product (184 mg).

LCMS (Method 2): Rt = 1.04, ES⁺ *m*/*z* 360.1/362.1/362.1 [M+H]⁺

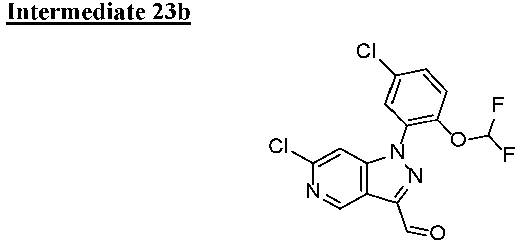

### 6-Chloro-1-(5-chloro-2-(difluoromethoxy)phenyl)-1H-pyrazolo[4,3-c]pyridine-3-carbaldehyde (Intermediate 23b)

Intermediate 21 (1.67 g, 3.3 mmol) in DMSO (10 mL) / water (1.0 mL) was heated at 100 °C for 5 h and at 120 °C for 3 h. After cooling to RT, RM was diluted with water (50 mL) and extracted with EtOAc (2x20 mL). Combined organic layers were washed with water (4x15 mL), sat. aq. NaCl (20 mL), dried over Na₂SO₄ and solvent removed under reduced pressure to give the title product (1.08 g) that was used in the next steps without further purification.

LCMS (Method 2): Rt = 1.24, ES⁺ *m*/*z* 358.1/360.1 [M+H]⁺

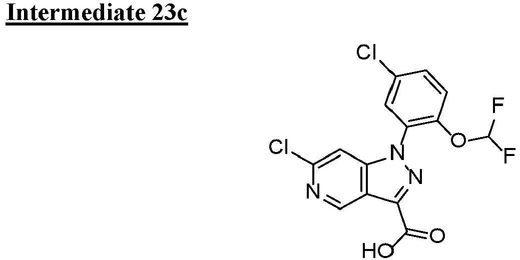

### 6-Chloro-1-(5-chloro-2-(difluoromethoxy)phenyl)-1H-pyrazolo[4,3-c]pyridine-3-carboxylic acid (Intermediate 23c)

To a mixture of intermediate 23b (104 mg, 0.29 mmol) and THF (5 mL), NaClO₂ (263 mg, 2.9 mmol), a solution of NaH₂PO₄ (348 mg, 2.9 mmol) in water (0.8 mL) and 2-methyl-2-butene (1.38 mL, 13 mmol) were added and RM stirred to 40 °C for 1.5 h. After cooling to RT, RM was diluted with water (10 mL), acidified with aq. 1N HCl to pH 2.5, and extracted with DCM (2x10 mL). Combined organic layers were washed with sat. aq. NaCl (10 mL), dried over Na₂SO₄ and solvent removed under reduced pressure to give the desired product (90 mg) that was used in the next steps without further purification.

LCMS (Method 2): Rt = 0.59, ES⁺ *m*/*z* 374.1/376.1 [M+H]⁺

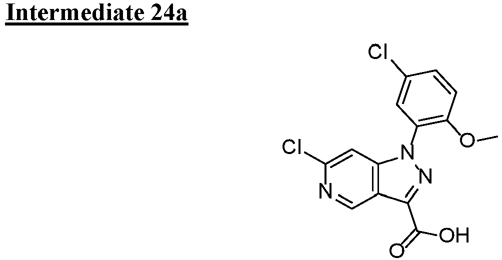

### 6-Chloro-1-(5-chloro-2-methoxyphenyl)-1H-pyrazolo[4,3-c]pyridine-3-carboxylic acid (Intermediate 24a)

Copper(I)thiophene-2-carboxylate (434 mg, 2.28 mmol), Cs₂CO₃ (2.22 g, 6.83 mmol), 2-bromo-4-chloro-1-methoxy-benzene (970 µl, 6.83 mmol) and 6-chloro-1H-pyrazolo[4,3-c]pyridine-3-carboxylic acid (450 mg, 2.28 mmol) in DMSO (6.75 mL) were stirred at 100 °C overnight under argon atmosphere. After cooling to RT, RM was diluted with water and pH adjusted to 3 with aq. 1M HCl. The precipitate formed was collected by filtration, dried and purified by flash chromatography on a Si cartridge eluting with 0-50 % DCM/MeOH/formic acid (90:10:0.3) in DCM to afford the title product (570 mg).

LCMS (Method 1): Rt = 1.01, ES⁺ *m*/*z* 337.9/339.9 [M+H]⁺

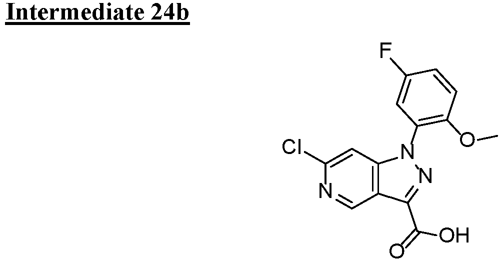

### 6-Chloro-1-(5-fluoro-2-methoxyphenyl)-1H-pyrazolo[4,3-c]pyridine-3-carboxylic acid (Intermediate 24b)

Title compound was prepared in a similar manner to intermediate 24a starting from 2-bromo-4-fluoro-1-methoxy-benzene and 6-chloro-1H-pyrazolo[4,3-c]pyridine-3-carboxylic acid.

LCMS (Method 1): Rt = 0.96, ES⁺ *m*/*z* 321.9/323.9 [M+H]⁺

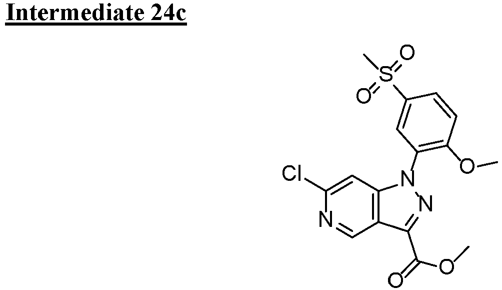

### Methyl 6-chloro-1-(2-methoxy-5-(methylsulfonyl)phenyl)-1H-pyrazolo[4,3-c]pyridine-3-carboxylate (Intermediate 24c)

Copper(I)thiophene-2-carboxylate (501 mg, 2.63 mmol), Cs₂CO₃ (3.42 g, 10.5 mmol), intermediate 11c (1.39 g, 5.25 mmol), 6-chloro-1H-pyrazolo[4,3-c]pyridine-3-carboxylic acid (519 mg, 2.63 mmol in DMSO (9 mL) were stirred at 105 °C for 16 h under nitrogen. After cooling to RT, iodomethane (654 µl, 10.5 mmol) was added and RM stirred at RT for 2h. RM diluted with water (10 mL) and extracted with DCM (3 x 8 mL). Combined organic layers were dried over Na₂SO₄, evaporated to dryness and the residue purified by flash chromatography on a Si cartridge by eluting with 0-50 % EtOAc in DCM to afford the title product (682 mg).

LCMS (Method 2): Rt = 0.94, ES⁺ *m*/*z* 396.1/398.1 [M+H]⁺

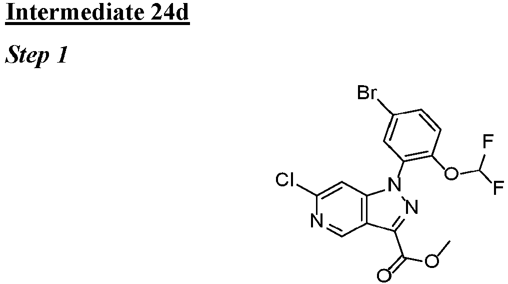

### Methyl 1-(5-bromo-2-(difluoromethoxy)phenyl)-6-chloro-1H-pyrazolo[4,3-c]pyridine-3-carboxylate (Intermediate 24d-1)

Title compound was prepared in a similar manner to intermediate 24c starting from intermediate 12 and 6-chloro-1H-pyrazolo[4,3-c]pyridine-3-carboxylic acid.
LCMS (Method 2): Rt = 1.26, ES⁺ *m*/*z* 432.0/434.0/436.0 [M+H]⁺

### Step 2

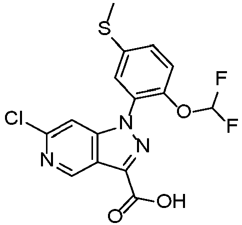

### 6-Chloro-1-(2-(difluoromethoxy)-5-(methylthio)phenyl)-1H-pyrazolo[4,3-c]pyridine-3-carboxylic acid (Intermediate 24d)

Intermediate 24d-1 (50.0 mg, 0.12 mmol), sodium thiomethoxide (24 mg, 0.35 mmol), Pd₂(dba)₃ (10.0 mg, 0.01 mmol), Xantphos (13 mg, 0.02 mmol) in degassed toluene (1.5 mL) were stirred at 80 °C for 3 h under nitrogen. The formed precipitate was collected by filtration, washed with toluene and dried to afford the title compound (68mg) that was used in the next steps without any further purification.

LCMS (Method 2): Rt = 0.62, ES⁺ *m*/*z* 386.1/388.1 [M+H]⁺

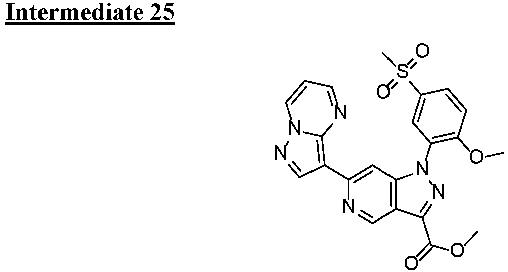

### methyl 1-(2-methoxy-5-(methylsulfonyl)phenyl)-6-(pyrazolo[1,5-a]pyrimidin-3-yl)-1H-pyrazolo[4,3-c]pyridine-3-carboxylate (Intermediate 25)

Intermediate 24c (300 mg, 0.76 mmol), 3-(4,4,5,5-tetramethyl-1,3,2-dioxaborolan-2-yl)pyrazolo[1,5-a]pyrimidine (274 mg, 1.1 mmol), K₃PO₄ (322 mg, 1.5 mmol), XPhos PdG3 (64 mg, 0.076 mmol) in degassed water (3.75 mL) / THF (7.5 mL) were stirred at 70 °C for 2 h under nitrogen. After cooling to RT, RM was diluted with water (10 mL) and extracted with DCM (6 x 10 mL). Combined organic layers were passed through a phase separator and evaporated to dryness. The crude was purified by flash chromatography on a Si cartridge by eluting with 0-40 % DCM/MeOH (20: 1) in DCM to afford the title product (262 mg).

LCMS (Method 2): Rt = 0.84, ES⁺ *m*/*z* 479.1 [M+H]⁺

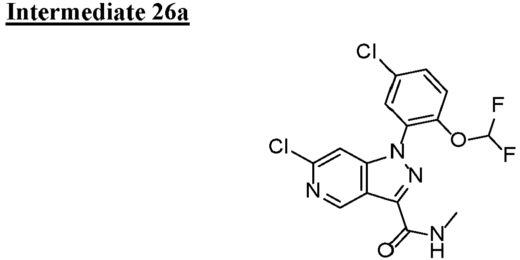

### 6-Chloro-1-(5-chloro-2-(difluoromethoxy)phenyl)-N-methyl-1H-pyrazolo[4,3-c]pyridine-3-carboxamide (Intermediate 26a)

To a mixture of intermediate 23c (25 mg, 0.067 mmol) in DMF (1.5 mL), DIPEA (23 µL, 0.13 mmol), HATU (28 mg, 0.074 mmol) and methylamine (2.0 M in THF, 334 µL, 0.67 mmol) were added and RM stirred at 50 °C overnight. After cooling to RT, RM was diluted with EtOAc (5 mL) and washed with sat. aq. NaHCO₃ (10 mL). Aqueous layer was further extracted with EtOAc (2x5 mL). Combined organic layers were washed with sat. aq. NaCl (10 mL), dried over Na₂SO₄ and evaporated under reduced pressure. The residue was purified by flash chromatography on Si cartridge by eluting with 0-24 % EtOAc in PE to give the title product (12 mg).
LCMS (Method 1): Rt = 1.12, ES⁺ *m*/*z* 387.1/389.1 [M+H]⁺

### Preparation of intermediates 26b to 26f

The following intermediates were prepared in a similar manner to intermediate 26a from the indicated starting materials. When minor modifications on solvent, and/or temperature were made, they were detailed below in brackets.

| Intermediate | Structure | Starting material | LCMS |
|---|---|---|---|
| Intermediate 26b | 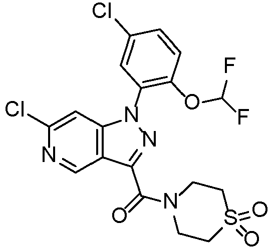 | Intermediate 23c / thiomorpholine 1,1-dioxide | (Method 2): Rt = 1.11 min, ES⁺ *m*/*z* 490.9/492.8 [M+H]⁺ |
| | | (Reaction temperature: RT) | |
| Intermediate 26c | 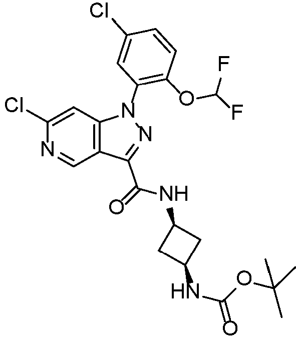 | Intermediate 23c / *tert-*butyl ((1*s,*3*s*)-3-aminocyclobutyl)car bamate | (Method 2): Rt = 1.28 min, ES⁺ *m*/*z* 542.5/544.5 [M+H]⁺ |
| | | (Reaction temperature: RT) | |
| Intermediate 26d | 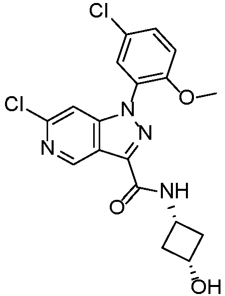 | Intermediate 24a / (1*s*,3*s*)-3-aminocyclobutan-1-ol hydrochloride | (Method 2): Rt = 1.00 min, ES⁺ *m*/*z* 407.1/409.1 [M+H]⁺ |
| | | (Reaction temperature: RT) | |
| Intermediate 26e | 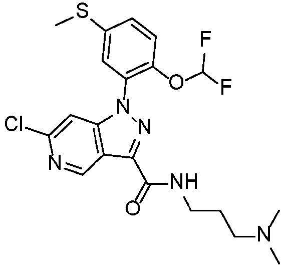 | Intermediate 24d and *N',N'-*dimethylpropane-1,3-diamine | (Method 2): Rt = 1.24 min, ES⁺ *m*/*z* 470.2/472.2 [M+H]⁺ |
| Intermediate 26f | 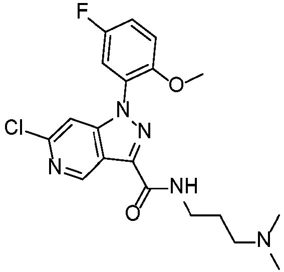 | Intermediate 24b and *N',N'-*dimethylpropane-1,3-diamine | (Method 1): Rt = 0.74 min, ES⁺ *m*/*z* 406.1/408.1 [M+H]⁺ |

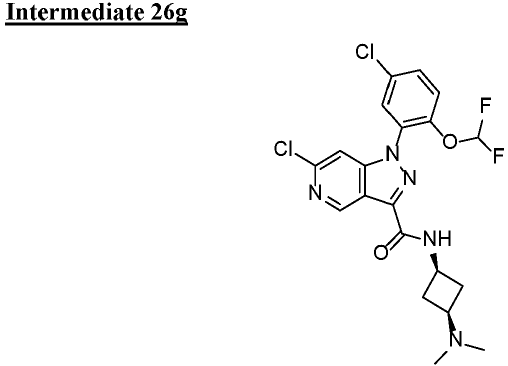

### 6-Chloro-1-(5-chloro-2-(difluoromethoxy)phenyl)-N-((1s,3s)-3-(dimethylamino)cyclobutyl)-1H-pyrazolo[4,3-c]pyridine-3-carboxamide (Intermediate 26g)

To an ice chilled solution of intermediate 26c (276 mg, 0.509 mmol) in dry DCM (6.9 ml), TFA (1.95 ml, 25.4 mmol) was added dropwise. RM was stirred for 1 h reaching RT, then loaded onto an SCX cartridge, washed with methanol and eluted with methanolic ammonia (7M). Relevant fractions were concentrated *in vacuo.* The residue was dissolved in a mixture of formic acid (768 µL, 20.4 mmol) and aqueous formaldehyde (37.0 %, 1.52 mL, 20.4 mmol) and stirred at 60 °C overnight. After cooling to RT, RM was diluted with EtOAc (20 mL), washed with sat. aq. NaHCO₃ (3x15 mL), sat. aq. NaCl (15 mL), dried over Na₂SO₄ and solvent removed under reduced pressure to afford the title product (144 mg) that was used in the next steps without any further purification.

LCMS (Method 2): Rt = 1.15 min, ES⁺ *m*/*z* 470.0/472.0 [M+H]⁺

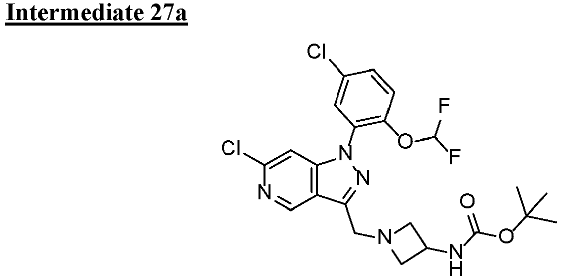

### tert-Butyl (1-((6-chloro-1-(5-chloro-2-(difluoromethoxy)phenyl)-1H-pyrazolo[4,3-c]pyridin-3-yl)methyl)azetidin-3-yl)carbamate (Intermediate 27a)

To a suspension of intermediate 20 (60.0 mg, 0.14 mmol) and *tert*-butyl *N-*(azetidin-3-yl)carbamate hydrochloride (32.6 mg, 0.16 mmol) in tetrahydrofuran (3.5 mL), TEA (59.3 µL, 0.43 mmol) was added. RM was stirred at RT overnight, then partitioned between sat. aq. NH₄Cl and EtOAc. Organic layer was washed with water, sat. aq. NaCl, dried over Na₂SO₄ and evaporated to give the title product (62 mg) that was used in the next steps without further purification.
LCMS (Method 2): Rt = 1.27 min, ES⁺ *m*/*z* 514.1/516.0 [M+H]⁺

### Preparation of intermediates 27b-d

The following intermediates were prepared in a similar manner to intermediate 27a from the indicated starting materials. When minor modifications on solvent, and/or temperature were made, they were detailed below in brackets

| Intermediate | Structure | Starting material | LCMS |
|---|---|---|---|
| Intermediate 27b | 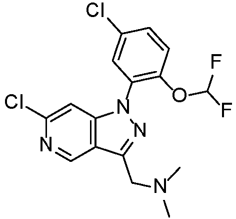 | Intermediate 20 / dimethylamine | (Method 2): Rt = 1.22 min, ES⁺ *m*/*z* 387.4/389.4 [M+H]⁺ |
| Intermediate 27c | 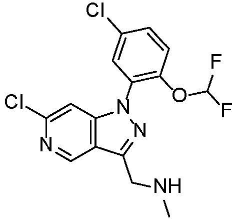 | Intermediate 20 / methylamine | (Method 2): Rt = 1.09 min, ES⁺ *m*/*z* 373.1/375.0[M+H]⁺ |
| Intermediate 27d | 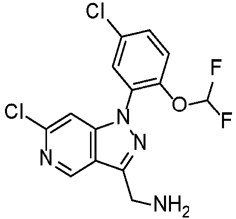 | Intermediate 20 / ammonia in methanol | (Method 2): Rt = 1.09 min, ES⁺ *m*/*z* 359.0/361.0[M+H]⁺ |
| | | (Reaction temperature: 90 °C) | |

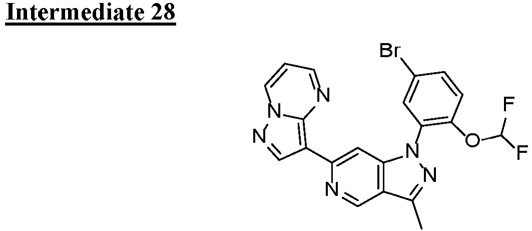

### 1-(5-Bromo-2-(difluoromethoxy)phenyl)-3-methyl-6-(pyrazolo[1,5-a]pyrimidin-3-yl)-1H-pyrazolo[4,3-c]pyridine (Intermediate 28)

Intermediate 5 (2.25 g, 8.25 mmol) and Intermediate 8b (3.92 g, 13.5 mmol) in NMP (35 mL) were stirred at 60 °C for 1h, then at 120 °C for 2 h. RM was cooled to RT and added with water (250 mL) under vigorous stirring. RM was extracted with EtOAc (3x200mL). Combined organic layers were washed with sat. aq. NaHCO₃ (2x200 mL), sat. aq. NaCl (200 mL), and evaporated under reduced pressure. The crude material was triturated with diisopropyl ether and with DCM/MeOH (5:1) and dried to afford the title product (563 mg).

LCMS (Method 2): Rt = 1.21 min, ES⁺ *m*/*z* 471.0/473.1 [M+H]⁺

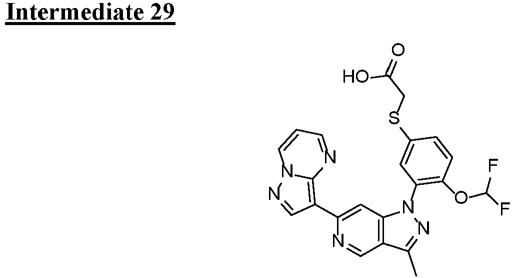

### 2-((4-(difluoromethoxy)-3-(3-methyl-6-(pyrazolo[1,5-a]pyrimidin-3-yl)-1H-pyrazolo[4,3-c]pyridin-1-yl)phenyl)thio)acetic acid (Intermediate 29)

Methyl thioglycolate (251 mg, 2.37 mmol) in toluene (8 mL) was added dropwise to a mixture of NaH (60.0 %, 158 mg, 3.95 mmol) in toluene (10 mL) under nitrogen atmosphere. RM was stirred at RT for 90 min then intermediate 28 (930 mg, 1.97 mmol), Xantphos (143 mg, 0.25 mmol) and Pd₂(dba)₃ (90.4 mg, 0.1 mmol) were added and RM further stirred at 100 °C for 1.5h. After cooling to RT, the formed precipitate was collected by filtration and triturated in water (5 mL), in diisopropyl ether (5 mL) and again in water (5 mL) at pH 5 and dried to afford the title product (0.42 g).

LCMS (Method 2): Rt = 0.57 ES⁺ *m*/*z* 483.3 [M+H]⁺

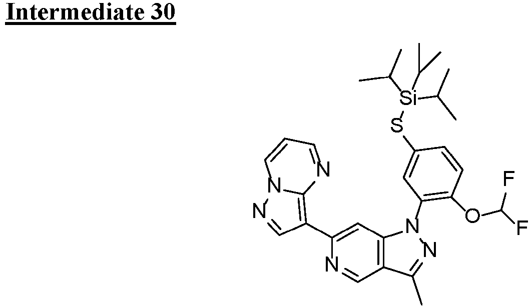

### 1-(2-(Difluoromethoxy)-5-((triisopropylsilyl)thio)phenyl)-3-methyl-6-(pyrazolo[1,5-a]pyrimidin-3-yl)-1H-pyrazolo[4,3-c]pyridine (Intermediate 30)

A solution of tris(propan-2-yl)silanethiol (328 µL, 1.53 mmol) in toluene (2 mL) was added dropwise to a mixture of NaH (60.0 %, 102 mg, 2.55 mmol) in toluene (10 mL) and RM stirred under nitrogen at RT for 1.5h. Intermediate 28 (600 mg, 1.27 mmol), Xantphos (92.1 mg, 0.16 mmol) and Pd₂(dba)₃ (58.3 mg, 0.06 mmol) were added and RM stirred at 100 °C for 45 min. RM was cooled to RT, quenched by addition of water (20 mL), and filtered through a diacematous earth pad thoroughly washed with EtOAc (2x35 mL). Organic layer was separated and washed with water (20 mL), sat. aq. NaCl (30 mL), dried over MgSO₄ and evaporated under reduced pressure. The crude was purified by chromatography on a Si cartridge by eluting with 0-100 % EtOAc in cyclohexane to afford the title product (153 mg).

¹H-NMR (500 MHz, *DMSO-d₆*) δ: 9.27 (d, J=6.7 Hz, 1H), 9.18 (s, 1H), 8.90 (s, 1H), 8.71 (d, J=3.6 Hz, 1H), 8.30 (s, 1H), 7.64 (d, J=1.6 Hz, 1H), 7.53 (dd, J=8.5, 1.7 Hz, 1H), 7.44 (d, J=8.6 Hz, 1H), 7.16 (dd, J=6.9, 4.0 Hz, 1H), 7.13 (t, J=73.0 Hz, 1H), 2.66 (s, 3H), 0.98 (d, J=6.5 Hz, 18H), 0.92 (m, 3H)

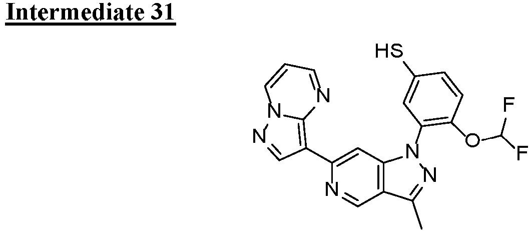

### 4-(Difluoromethoxy)-3-(3-methyl-6-(pyrazolo[1,5-a]pyrimidin-3-yl)-1H-pyrazolo[4,3-c]pyridin-1-yl)benzenethiol (Intermediate 31)

Aq. 37% w/w HCl (75 µL, 2.45 mmol) was added to a mixture of intermediate 30 (150 mg, 0.258 mmol) in EtOH (3 mL) and RM stirred at RT under nitrogen for 2 h. RM was dried under reduced pressure to afford the title product (125 mg) that was used in the next steps without further purification.
¹H-NMR (500 MHz, *DMSO-d₆*) δ: 9.23 (dd, J=6.9,1.6 Hz, 1H), 9.18 (s, 1H), 8.89 (s, 1H), 8.71 (dd, J=4.0, 1.5 Hz, 1H), 8.30 (s, 1H), 7.67 (d, J=1.6 Hz, 1H), 7.53 (dd, J=8.5, 1.7 Hz, 1H), 7.44 (d, J=8.6 Hz, 1H), 7.16 (dd, J=6.9, 4.0 Hz, 1H), 7.13 (t, J=73.0 Hz, 1H), 5.94 (s, 1H), 2.66 (s, 3H)
LCMS (Method 2): RT = 0.50 min, ES⁺ *m*/*z* 425.2 [M+H]⁺

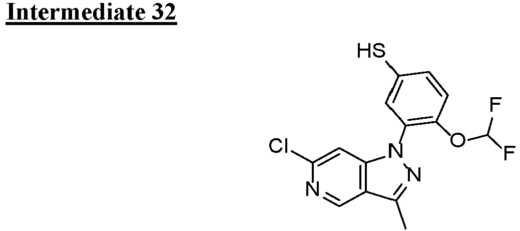

### 3-(6-Chloro-3-methyl-1H-pyrazolo[4,3-c]pyridin-1-yl)-4-(difluoromethoxy)benzenethiol (Intermediate 32)

Tris(propan-2-yl)silanethiol (365 µL, 1.7 mmol) in toluene (2 mL) was added dropwise to a mixture of NaH (60 %, 113 mg, 2.8 mmol) in toluene (4 mL) and RM stirred under argon at RT for 1.5h. Intermediate 18k (551 mg, 1.4 mmol) in toluene (4 mL), Xantphos (103 mg, 0.18 mmol) and Pd₂(dba)₃ (41 mg, 0.071 mmol) were added, and the RM stirred at 100 °C for 45 minutes. After cooling to RT, RM was quenched with sat. aq. NH₄Cl (20 mL) and extracted with EtOAc (100 mL). Organic layer was washed with water (10 mL), sat. aq. NaCl (10 mL), dried over Na₂SO₄ and evaporated under reduced pressure. The crude was purified by flash chromatography on a Si cartridge by eluting with 0-50 % EtOAc in cyclohexane to afford the title compound (198 mg).
LCMS (Method 1): RT = 1.16 min, ES⁺ *m*/*z* 341.7/343.7 [M+H]⁺
¹H-NMR (300 MHz, *CDCl₃*) δ: 8.81 (s, 1H), 7.46 (d, J=2.3 Hz, 1H), 7.36 (dd, J=8.5, 2.1 Hz, 1H), 7.28 (d, J=8.1 Hz, 1H), 7.19 (s, 1H), 6.31 (t, J=73.0 Hz, 1H), 3.59 (s, 1H), 2.66 (s, 3H)

From the flash chromatography purification, a second pure product was obtained. LC-MS analysis confirmed the structure of dibromoderivative of intermediate 18z and it was identified as **Intermediate 33.**

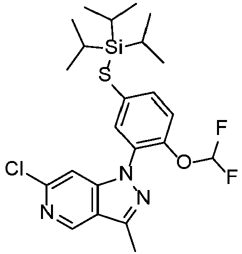

### 3-(6-Chloro-3-methyl-1H-pyrazolo[4,3-c]pyridin-1-yl)-4-(difluoromethoxy)benzenethiol and 6-chloro-1-(2-(difluoromethoxy)-5-((triisopropylsilyl)thio)phenyl)-3-methyl-1H-pyrazolo[4,3-c]pyridine

LCMS (Method 1): RT = 1.80 min, ES⁺ *m*/*z* 341.9/343.9 [M+H]⁺
¹H-NMR (300 MHz, *CDCl₃*) δ: 8.82 (s, 1H), 7.64 (d, J=2.3 Hz, 1H), 7.58 (dd, J=8.5, 2.1 Hz, 1H), 7.26 (d, J=8.1 Hz, 1H), 6.39 (t, J=73.0 Hz, 1H), 2.68 (s, 3H), 1.27 (m, 3H), 1.11 (m, 18H)

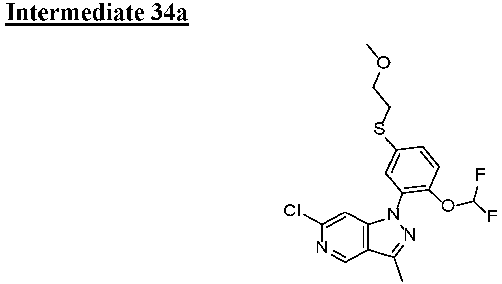

### 6-Chloro-1-(2-(difluoromethoxy)-5-((2-methoxyethyl)thio)phenyl)-3-methyl-1H-pyrazolo[4,3-c]pyridine (Intermediate 34a)

A solution of intermediate 32 (60.0 mg, 0.176 mmol) in acetone (2.6 mL) was added to a mixture of 1-bromo-2-methoxy-ethane (19.8 µL, 0.21 mmol), NaI (26.3 mg, 0.18 mmol) and K₂CO₃ (48.5 mg, 0.351 mmol) in acetone (2.6 mL). RM stirred at 70°C for 75 min under argon atmosphere. After cooling to RT, RM was partitioned between DCM and sat. aq. NaHCO₃. Aqueous phase was extracted with DCM (2x) and combined organic layers dried over Na₂SO₄ and evaporated to dryness to afford the title product (65.0 mg) that was used in the next steps without further purification.
LCMS (Method 1): RT = 1.22 min, ES⁺ *m*/*z* 400.1/402.0 [M+H]⁺

### Preparation of intermediates 34b to 34e

The following intermediates were prepared in a similar manner to intermediate 34a from the indicated starting materials.

| Intermediate | Structure | Starting material | LCMS |
|---|---|---|---|
| Intermediate 34b | 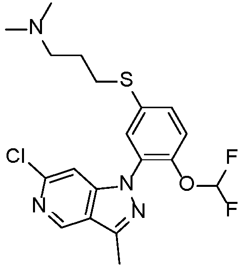 | Intermediate 32 / 3-chloro-*N,N-*dimethyl-propan-1-amine hydrochloride | (Method 1): Rt = 0.82 min, ES⁺ *m*/*z* 427.1/429.0 [M+H]⁺ |
| Intermediate 34c | 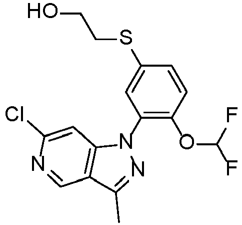 | Intermediate 32 / 2-chloroethanol | (Method 1): Rt = 1.02 min, ES⁺ *m*/*z* 386.0/388.0 [M+H]⁺ |
| Intermediate 34d | 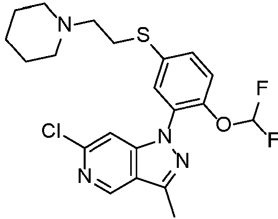 | Intermediate 32 / 1-(2-chloroethyl)piperidine hydrochloride | (Method 1): Rt = 0.79 min, ES⁺ *m*/*z* 452.8/454.7 [M+H]⁺ |
| Intermediate 34e | 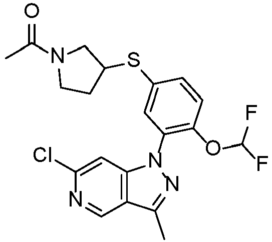 | Intermediate 32 / 1-(3-bromopyrrolidin-1-yl)ethanone | (Method 1): Rt = 1.05 min, ES⁺ *m*/*z* 453.1/455.0 [M+H]⁺ |

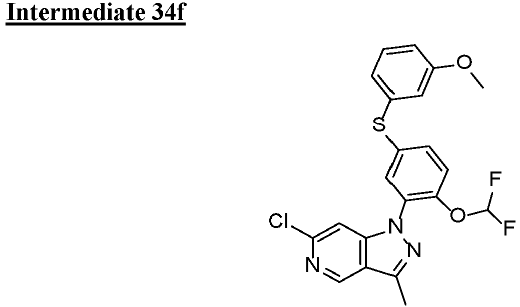

### 6-Chloro-1-(2-(difluoromethoxy)-5-((3-methoxyphenyl)thio)phenyl)-3-methyl-1H-pyrazolo[4,3-c]pyridine (Intermediate 34f)

A mixture of intermediate 33 (200 mg, 0.40 mmol) in i-PrOH (2 mL), CsF (140 mg, 0.92 mmol), Cs₂CO₃ (301 mg, 0.92 mmol), Pd₂(dba)₃ (12 mg, 0.020 mmol) and a degassed solution of 1-bromo-3-methoxy-benzene (118 µL, 0.92 mmol) in i-PrOH (2 mL) were stirred at 100°C overnight. After cooling to RT, RM was evaporated under reduced pressure and the residue purified by flash chromatography on a Si cartridge by eluting with 0-50% EtOAc in cyclohexane to afford the title product (83 mg).

LCMS (Method 1): Rt = 1.43 min, ES⁺ *m*/*z* 448.0/455.0 [M+H]⁺

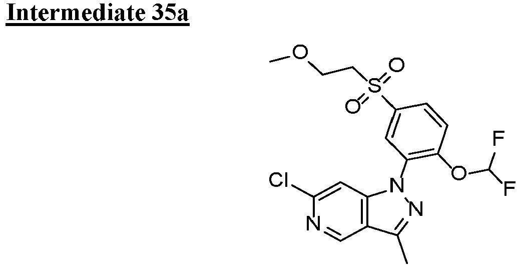

### 6-Chloro-1-(2-(difluoromethoxy)-5-((2-methoxyethyl)sulfonyl)phenyl)-3-methyl-1H-pyrazolo[4,3-c]pyridine (Intermediate 35a)

To an ice-bath cooled mixture of intermediate 34a (64.0 mg, 0.16 mmol) in dry DCM (3.3 mL), mCPBA (55.2 mg, 0.32 mmol) was added and RM stirred at 0°C for 70 min. A second equivalent of *m*CPBA (20 mg, 0.12 mmol) was added and the RM stirred for further 40 min. RM was diluted with DCM and washed with sat. aq. NaHCO₃. Organic layer was dried over Na₂SO₄ and evaporated *in vacuo.* The crude was purified by flash chromatography on a Si cartridge by eluting with EtOAc in DCM to afford the title product (51.0 mg).

LCMS (Method 1): Rt = 1.02 min, ES⁺ *m*/*z* 432.1/434.0 [M+H]⁺

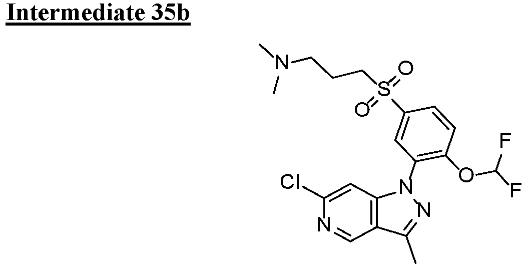

### 3-((3-(6-Chloro-3-methyl-1H-pyrazolo[4,3-c]pyridin-1-yl)-4-(difluoromethoxy)phenyl)sulfonyl)-N,N-dimethylpropan-1-amine (Intermediate 35b)

A solution of Oxone^{®} (263 mg, 0.428 mmol) in water (0.86 mL) was added to a mixture of intermediate 34b (87.0 mg, 0.204 mmol) in MeOH (6.4 mL) and RM stirred at RT for 1 h. RM was diluted with water and extracted twice with DCM, and again after bringing pH of aqueous phase to pH ≈ 7-8. Combined organic layers were dried over Na₂SO₄ and solvent removed *in vacuo.* The residue was purified by flash chromatography on a Si cartridge by eluting with 10% MeOH in DCM to afford the title product (16.0 mg).
LCMS (Method 2): Rt = 1.02 min, ES⁺ *m*/*z* 459.1/461.1 [M+H]⁺

### Preparation of intermediates 35c to 35e

The following intermediates were prepared in a similar manner to intermediate 35b from the indicated starting materials.

| Intermediate | Structure | Starting material | LCMS |
|---|---|---|---|
| Intermediate 35c | 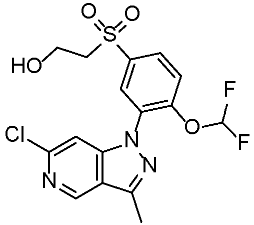 | Intermediate 34c | (Method 1): Rt = 0.89 min, ES⁺ *m*/*z* 418.0/420.0 [M+H]⁺ |
| Intermediate 35d | 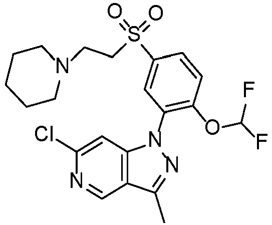 | Intermediate 34d | (Method 1): Rt = 0.75 min, ES⁺ *m*/*z* 485.1/487.1 [M+H]⁺ |
| Intermediate 35e | 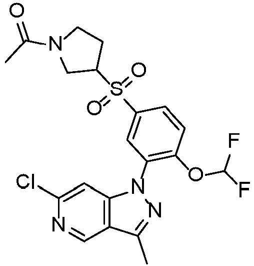 | Intermediate 34e | (Method 1): Rt = 0.90 min, ES⁺ *m*/*z* 484.8/486.7 [M+H]⁺ |
| Intermediate 35f | 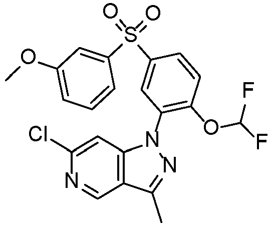 | Intermediate 34f | (Method 1): Rt = 1.22 min, ES⁺ *m*/*z* 480.0/482.0 [M+H]⁺ |
| Intermediate 35g | 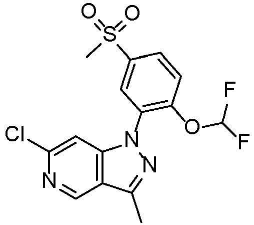 | Intermediate 18ab | (Method 2): Rt = 0.95 min, ES⁺ *m*/*z* 387.8/389.8 [M+H]⁺ |

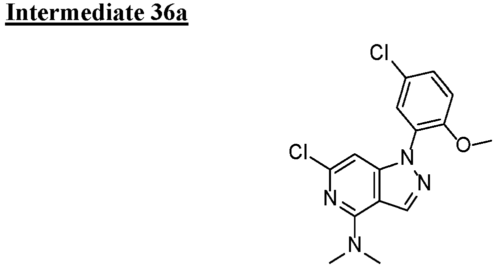

### 6-Chloro-1-(5-chloro-2-methoxyphenyl)-N,N-dimethyl-1H-pyrazolo[4,3-c]pyridin-4-amine (Intermediate 36a)

To a mixture of intermediate 18ac (100 mg, 0.30 mmol), DIPEA (118 mg, 0.91 mmol) in NMP (3 mL), dimethylamine (2.0 M in THF, 457 µL, 0.91 mmol) was added and RM heated under MW irradiation at 120 °C for 30 min. After cooling to RT, RM was partitioned between EtOAc (15 mL) and sat. aq. NaHCO₃ (15 mL). Organic layer was washed with sat. aq. NaCl (10 mL) and concentrated *in vacuo.* The residue was triturated two times in hexane (3 mL), filtered and dried to afford the title product (78 mg).
LCMS (Method 2): Rt = 1.34 min, ES⁺ *m*/*z* 337.1/339.1/341.1 [M+H]⁺

### Preparation of Intermediates 36b to 36c

The following intermediates were prepared in a similar manner to intermediate **36a** from the indicated starting materials.

| Intermediate | Structure | Starting material | LCMS |
|---|---|---|---|
| Intermediate 36b | 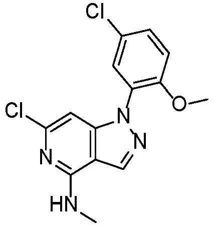 | Intermediate 18ac / methylamine (2.0 M in THF) | (Method 2): Rt = 1.21 min, ES⁺ *m*/*z* 323.1/325.1/327.1 [M+H]⁺ |
| Intermediate 36c | 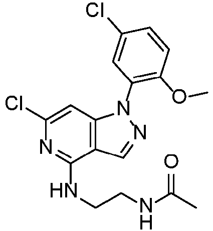 | Intermediate 18ac / *N-*acetylethylenediam ine | (Method 2): Rt = 1.04 min, ES⁺ *m*/*z* 394.1/396.1/398.1 [M+H]⁺ |

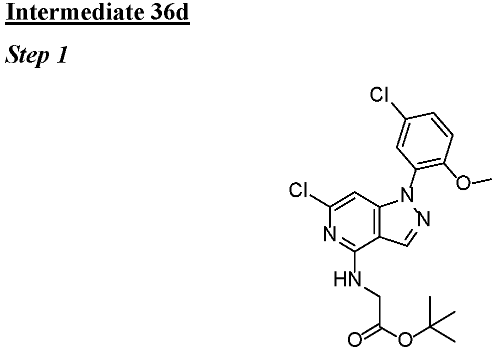

### tert-Butyl (6-chloro-1-(5-chloro-2-methoxyphenyl)-1H-pyrazolo[4,3-c]pyridin-4-yl)glycinate (Intermediate 36d-1)

To a mixture of intermediate 18ac (200 mg, 0.61 mmol), DIPEA (318 µL, 1.83 mmol) in NMP (2 mL), *tert*-Butyl 2-aminoacetate (250 µL, 1.83 mmol) was added and RM heated under MW irradiation at 150 °C for 45 min. After cooling to RT, RM was diluted with EtOAc (20 mL), washed with sat. aq. NaHCO₃ (5x10 mL) and sat. aq. NaCl (10 mL). Organic layer was dried over Na₂SO₄ and concentrated *in vacuo.* The crude was purified by flash chromatography on a Si cartridge by eluting with 0-10 % EtOAc in cyclohexane to afford the title product (172.6 mg).
LCMS (Method 2): Rt = 1.41 min, ES⁺ *m*/*z* 423.1/425.1/427.1 [M+H]⁺

### Step 2

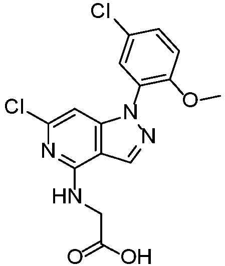

### (6-Chloro-1-(5-chloro-2-methoxyphenyl)-1H-pyrazolo[4,3-c]pyridin-4-yl)glycine trifluoroacetate (Intermediate 36d-2)

To an ice cooled solution of intermediate 36d-1 (120 mg, 0.28 mmol) in dry DCM (2 mL), TFA (2 mL, 26.1 mmol) was added dropwise and RM stirred at RT for 48 h. RM was evaporated under reduced pressure, the residue suspended in diethyl ether and solvent removed (two times) to afford the title product (120 mg) that was used in the next step without further purification.
LCMS (Method 2): Rt = 0.63 min, ES⁺ *m*/*z* 367.0/369.0/371.0 [M+H]⁺

### Step 3

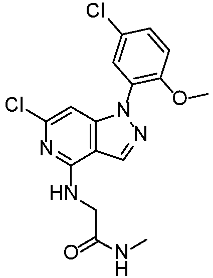

### 2-((6-Chloro-1-(5-chloro-2-methoxyphenyl)-1H-pyrazolo[4,3-c]pyridin-4-yl)amino)-N-methylacetamide (Intermediate 36d)

To a mixture of intermediate 36d-2 (40.0 mg, 0.08 mmol) in dry DMF (0-5 mL), methylamine hydrochloride (16.8 mg, 0.25 mmol) and DIPEA (72.4 µL, 0.42 mmol) were added, followed by HATU (34.8 mg, 0.09 mmol). RM was stirred at RT overnight, then diluted with EtOAc (15 mL) and washed with sat. aq. NaHCO₃ (3x10 mL). Organic layer was washed with sat. aq. NaCl (10 mL), dried over Na₂SO₄ and concentrated *in vacuo.* The crude was purified by flash chromatography on a Si cartridge by eluting with 0-5 % MeOH in DCM to afford the desired product (24 mg).

LCMS (Method 2): Rt = 1.03 min, ES⁺ *m*/*z* 380.0/381.9/383.9 [M+H]⁺.

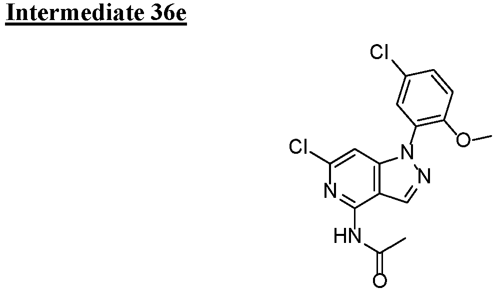

### N-(6-Chloro-1-(5-chloro-2-methoxyphenyl)-1H-pyrazolo[4,3-c]pyridin-4-yl)acetamide (Intermediate 36e)

A mixture of intermediate 18ac (45.0 mg, 0.14 mmol), acetamide (10.5 mg, 0.18 mmol), Pd₂(dba)₃ (4.5 mg, 7.8 µmol), Xantphos (3.96 mg, 6.9 µmol), Cs₂CO₃ (66.9 mg, 0.21 mmol) in previously degassed 1,4-dioxane (1 mL) was heated at 100 °C for 24 h under nitrogen atmosphere. After cooling to RT, RM was diluted with water and the formed precipitate collected by filtration and purified by flash chromatography on a Si cartridge by eluting with 0-15 % DCM/MeOH (99:1) in DCM to afford the title product (28 mg).

LCMS (Method 2): Rt = 1.11 min, ES⁺ *m*/*z* 351.1, 353.1, 355.1 [M+H]⁺.

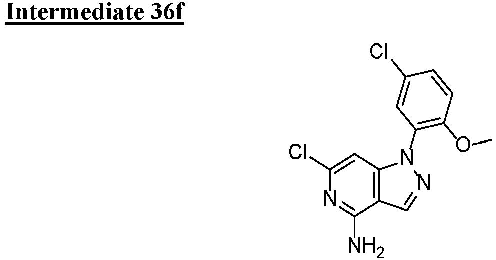

### 6-Chloro-1-(5-chloro-2-methoxyphenyl)-1H-pyrazolo[4,3-c]pyridin-4-amine (Intermediate 36f)

A mixture of Cs₂CO₃ (223 mg, 0.69 mmol) (dried under vacuum at 150 °C for 1 h), intermediate 18ac (150 mg, 0.46 mmol), *tert-*butyl carbamate (64.2 mg, 0.55 mmol), Pd₂(dba)₃ (15.0 mg, 26.1 µmol), Xantphos (13.2 mg, 22.8 µmol) in previously degassed 1,4-dioxane (4mL) were stirred at 90 °C for 24 h under nitrogen atmosphere. After cooling to RT, RM was partitioned between EtOAc (15 mL) and water (10 mL). Organic layer was washed with water (5 mL), sat. aq. NaCl (10 mL) and evaporated under reduced pressure. The residue was purified by flash chromatography on a Si cartridge by eluting with 0-100% (5% MeOH in DCM ) in DCM to afford the title product.

LCMS (Method 2): Rt = 1.06 min, ES⁺ *m*/*z* 309.0/331.0/313.0 [M+H]⁺

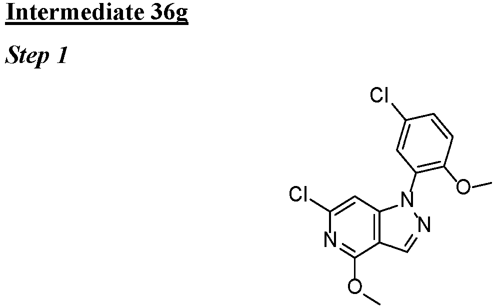

### 6-Chloro-1-(5-chloro-2-methoxyphenyl)-4-methoxy-1H-pyrazolo[4,3-c]pyridine (Intermediate 36g-1)

To a suspension of intermediate 18ac (80 mg, 0.24 mmol) in dry MeOH (2.4 mL), sodium methoxide in MeOH (25 %, 443 µL, 1.9 mmol) was added and RM stirred at 60 °C for 18 h. RM was cooled to RT and quenched in water (10 mL). The formed precipitate was collected by filtration and dried to afford the title product (70 mg).

LCMS (Method 2): Rt = 1.40 min, ES⁺ *m*/*z* 324.1/326.1/328.1 [M+H]⁺.

### Step 2

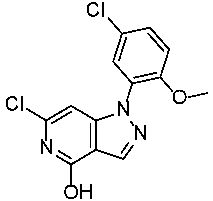

### 6-Chloro-1-(5-chloro-2-methoxyphenyl)-1H-pyrazolo[4,3-c]pyridin-4-ol (Intermediate 36g)

To a suspension of intermediate 36g-1 (38.0 mg, 0.12 mmol) in MeCN (1.5 mL), TMS-Cl (44.4 µL, 0.35 mmol) and NaI (52.7 mg, 0.352 mmol) were added and RM stirred at 85 °C for 15 min. After cooling to RT, RM was partitioned between water (2 mL) and EtOAc (4 mL). The organic layer was washed with 5% (w/w) aq. Na₂S₂O₃ (10 mL), sat. aq. NaCl (5 mL) and evaporated under reduced pressure to give the title product (14 mg).

LCMS (Method 2): Rt = 0.57 min, ES⁺ *m*/*z* 310.1/312.1/314.0 [M+H]⁺.

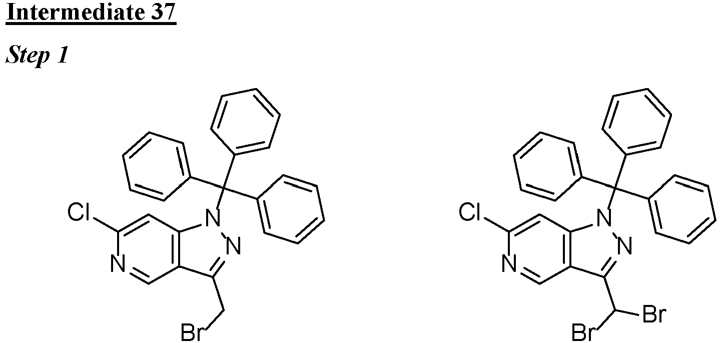

### Mix of 3-(bromomethyl)-6-chloro-1-trityl-1H-pyrazolo[4,3-c]pyridine (Intermediate 37-1a) 6-chloro-3-(dibromomethyl)-1-trityl-1H-pyrazolo[4,3-c]pyridine (Intermediate 37-1b)

To a mixture of intermediate 16 (3.72 g, 9.08 mmol) and benzotrifluoride (120 mL), NBS (1.94 g, 10.9 mmol) and AIBN (298 mg, 1.82 mmol) were added and RM stirred at 80 °C for 3.5 h under argon atmosphere. The precipitate formed was removed by filtration and washed with EtOAc. The filtrate was partitioned between EtOAc and sat. aq. NaCl, organic layer dried over Na₂SO₄ and evaporated to dryness to afford a mix 4:1 of monobrominated (Intermediate 37-1a) and di-brominated product (Intermnediate 37-1b) (total amount 2.57g) that was used in the next steps without any further purification.
LCMS (Method 2): Rt = 1.55 and 1.63 ES⁺ ion cluster peaks with predominant *m*/*z* 490.0, 568.0 [M+H]⁺

### Step 2

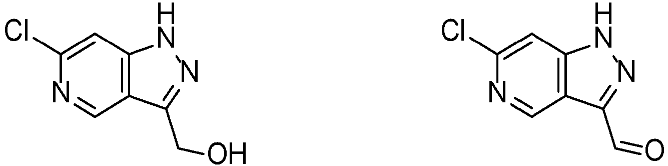

### Mix of (6-Chloro-1H-pyrazolo[4,3-c]pyridin-3-yl)methanol (Intermediate 37) and 6-chloro-1H-pyrazolo[4,3-c]pyridine-3-carbaldehyde (intermediate 37-2)

The mix of intermediate 37-1a and intermediate 37-1b from previous step (2. 00 g) in DMSO (12 mL)/ water (1.2 mL) was stirred vigorously at 100 °C for 10 h. After cooling to RT, RM was diluted with sat. aq. NaHCO₃ (12 mL). The formed precipitate was filtered, washed with water (5 mL) and EtOAc (2 x 10 mL) and discarded. Mother liquors were extracted with EtOAc (3 x 20 mL). Aqueous layer (after adjusting pH to 6.8) was extracted with EtOAc (3 x 20 mL), and further extracted when pH adjusted to 4.8 with EtOAc (7 x 10 mL). Combined organic layers were dried over MgSO₄, filtered, and evaporated to dryness. The crude products (650 mg) was obtained as a mix 1:1 of Intermediate 37 and Intermediate 37-2 and it was used in the next synthetic step without any further purification.
LCMS (Method 2): Rt = 0.34 min and 0.44 min, ES⁺ *m*/*z* 182.1 and 184.1 [M+H]⁺

### Step 3

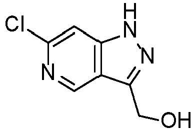

### (6-Chloro-1H-pyrazolo[4,3-c]pyridin-3-yl)methanol (Intermediate 37)

The mix of intermediate 37 and intermediate 37-2 from previous step (from step 2) (630 mg, assumed to be 3.5 mmol) in EtOH (50 mL) was cooled to 0 °C and added with NaBH₄ (66 mg, 1.7 mmol). After stirring for 15 min at 0 °C, RM was quenched with water (20 mL). The formed precipitate was filtered, washed with water (2 x 5 mL) and discarded. The combined filtrate was extracted with EtOAc (2 x 20 mL). Aqueous layer (pH adjusted to 6.8) was further extracted with EtOAc (5 x 20 mL). Combined organic layers were dried over Na₂SO₄, filtered and evaporated to dryness. The residue was purified on a Si cartridge by eluting with 0-90 % DCM/MeOH (20:1) in DCM to afford the title product (450 mg).
LCMS (Method 2): Rt = 0.44, ES⁺ *m*/*z* 184.1 [M+H]⁺

### PREPARATION OF EXAMPLES

### Example 1

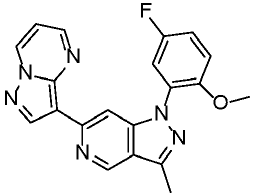

### 1-(5-Fluoro-2-methoxyphenyl)-3-methyl-6-(pyrazolo[1,5-a]pyrimidin-3-yl)-1H-pyrazolo[4,3-c]pyridine (Example 1)

A mixture of intermediate 18a (70.0 mg, 0.240 mmol), 3-(4,4,5,5-tetramethyl-1,3,2-dioxaborolan-2-yl)pyrazolo[1,5-*a*]pyrimidine (82.3 mg, 0.336 mmol), an aqueous solution of K₃PO₄ (0.500 M, 0.960 mL, 0.480 mmol), XPhos Pd G3 (10.2 mg, 0.012 mmol) in degassed THF/water (4.8 mL) was heated at 100 °C for 2h under argon. RM was cooled to RT and quenched with water (5 mL). The precipitate was collected by filtration and submitted to flash chromatography on a Si cartridge by eluting with 0-100 % EtOAc in cyclohexane to afford the title product (72.6 mg).
LCMS (Method 5): Rt = 3.37 min, ES⁺ *m*/*z* 375.2 [M+H]⁺
¹H-NMR (300 MHz, *DMSO-d₆*) δ: 9.22 (dd, J=7.1, 1.7 Hz, 1H), 9.14 (d, J=1.0 Hz, 1H), 8.88 (s, 1H), 8.70 (dd, J=4.1, 1.7 Hz, 1H), 8.27 (d, J=1.0 Hz, 1H), 7.34-7.47 (m, 3H), 7.15 (dd, J=7.1, 4.1 Hz, 1H), 3.83 (s, 3H), 2.64 (s, 3H)

### Example 2 to 57

The following examples were prepared in a similar manner to example 1 from the indicated starting materials. When minor modifications on base, solvent, temperature, ligand and/or palladium source were made, they were stated in brackets.

| Ex. | Structure/Name | Starting material | ¹H-NMR | LCMS |
|---|---|---|---|---|
| Ex. 2 | 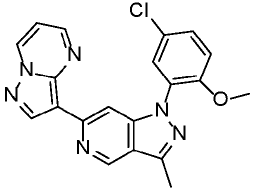 1-(5-chloro-2-methoxyphenyl)-3-methyl-6-(pyrazolo[1,5-*a*]pyrimidin-3-yl)-1*H-*pyrazolo[4,3-c]pyridine | Intermediate 18b | (300 MHz, *DMSO-d₆*) δ: 9.22 (dd, J=7.14, 1.74 Hz, 1H), 9.14 (d, J=1.05 Hz, 1H), 8.88 (s, 1H), 8.70 (dd, J=4.09, 1.65 Hz, 1H), 8.26 (d, J=1.05 Hz, 1H), 7.55-7.63 (m, 2H), 7.36-7.44 (m, 1H), 7.15 (dd, J=7.05, 4.09 Hz, 1H), 3.85 (s, 3H), 2.64 (s, 3H). | (Method 5): Rt = 3.46, ES⁺ *m*/*z* 391.1/393. 0 |
| Ex. 3 | 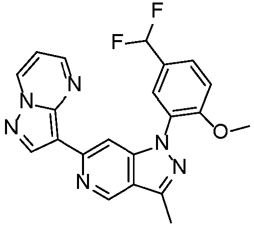 1-(5-(difluoromethyl)-2-methoxyphenyl)-3-methyl-6-(pyrazolo[1,5-*a*]pyrimidin-3-yl)-1*H-*pyrazolo[4,3-*c*]pyridine | Intermediate 18c | (300 MHz, *DMSO-d₆*) δ: 9.22 (dd, J=7.1, 1.7 Hz, 1H), 9.15 (d, J=1.0 Hz, 1H), 8.88 (s, 1H), 8.69 (dd, J=4.1, 1.7 Hz, 1H), 8.27 (d, J=1.0 Hz, 1H), 7.70-7.79 (m, 2H), 7.51 (d, J=8.5 Hz, 1H), 7.15 (dd, J=7.1, 4.1 Hz, 1H), 7.08 (t, J=55.9 Hz, 1H), 3.91 (s, 3H), 2.65 (s, 3H). | (Method 7): Rt = 4.69 min, ES⁺ *m*/*z* 407.1 [M+H]⁺ |
| Ex. 4 | 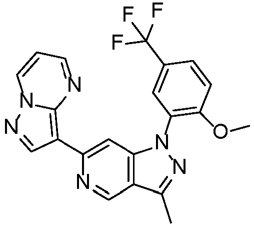 1-(2-methoxy-5-(trifluoromethyl)phenyl)-3-methyl-6-(pyrazolo[1,5-*a*]pyrimidin-3-yl)-1*H-*pyrazolo[4,3-*c*]pyridine | Intermediate 18d | (600 MHz, *DMSO-d₆*) δ: 9.23 (dd, J=7.1, 1.6 Hz, 1H), 9.17 (s, 1H), 8.90 (s, 1H), 8.71 (dd, J=4.0, 1.7 Hz, 1H), 8.30 (s, 1H), 7.93 (dd, J=8.9, 2.1 Hz, 1H), 7.87 (d, J=2.0 Hz, 1H), 7.59 (d, J=8.8 Hz, 1H), 7.17 (dd, J=7.0, 4.0 Hz, 1H), 3.96 (s, 3H), 2.67 (s, 3H). | (Method 7): Rt = 5.27 min, ES⁺ *m*/*z* 425.0 [M+H]⁺ |
| Ex. 5 | 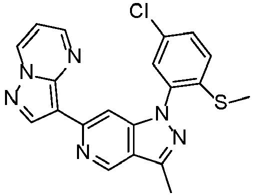 1-(5-chloro-2-(methylthio)phenyl)-3-methyl-6-(pyrazolo[1,5-*a*]pyrimidin-3-yl)-1*H-*pyrazolo[4,3-*c*]pyridine | Intermediate 18e | (600 MHz, *DMSO-d₆*) δ: 9.22 (dd, J=6.97, 1.65 Hz, 1H), 9.19 (d, J=1.10 Hz, 1H), 8.90 (s, 1H), 8.68 (dd, J=4.13, 1.74 Hz, 1H), 8.18 (d, J=1.28 Hz, 1H), 7.68-7.70 (m, 1H), 7.67 (d, J=2.20 Hz, 1H), 7.60 (d, J=8.62 Hz, 1H), 7.15 (dd, J=6.97, 4.22 Hz, 1H), 2.65 (s, 3H), 2.42 (s, 3H). | (Method 6): Rt = 4.65 min, ES⁺*m*/*z* 407.1/409. 1; [M+H]⁺ |
| Ex. 6 | 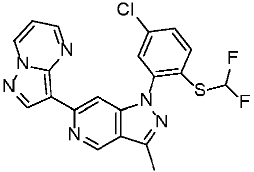 1-(5-chloro-2-((difluoromethyl)thio)ph enyl)-3-methyl-6-(pyrazolo[1,5-*a*]pyrimidin-3-yl)-1*H-*pyrazolo[4,3-*c*]pyridine | Intermediate 18f | (*DMSO-d₆,* | (Method 7): Rt = 5.66 min, ES⁺ *m*/*z* 443.1/445. 0 [M+H]⁺ |
| Ex. 7 | 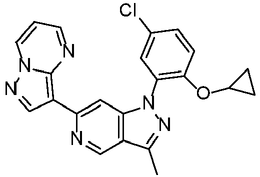 1-(5-chloro-2-cyclopropoxyphenyl)-3-methyl-6-(pyrazolo[1,5-*a*]pyrimidin-3-yl)-1*H-*pyrazolo[4,3-*c*]pyridine | Intermediate 18g | (500 MHz, *DMSO-d₆*) δ: 9.24 (d, J=7.02 Hz, 1H), 9.14 (s, 1H), 8.90 (s, 1H), 8.72 (d, J=3.97 Hz, 1H), 8.16 (s, 1H), 7.67-7.60 (m, 2H), 7.57 (d, J=2.44 Hz, 1H), 7.18 (dd, J=6.87, 4.12 Hz, 1H), 3.97-3.92 (m, 1H), 2.64 (s, 3H), 0.78-0.67 (m, 4H) | (Method 7): Rt = 5.61 min, ES⁺ *m*/*z* 417.1/419. 1 [M+H]⁺ |
| Ex. 8 | 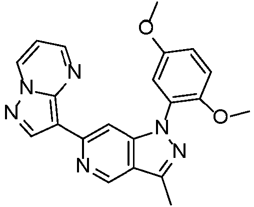 1-(2,5-dimethoxyphenyl)-3-methyl-6-(pyrazolo[1,5-*a*]pyrimidin-3-yl)-1*H-*pyrazolo[4,3-*c*]pyridine | Intermediate 18h | (*DMSO-d₆,-*7.18 (m, 1H), 7.14 (s, 1H), 3.79 (s, 3H), 3.79 (s, 3H), 2.71 (s, 3H). | (Method 6): Rt = 4.08 min, ES⁺ *m*/*z* 387.3 [M+H]⁺ |
| Ex. 9 | 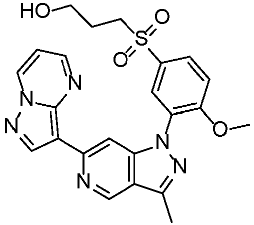 3-((4-methoxy-3-(3-methyl-6-(pyrazolo[1,5-*a*]pyrimidin-3-yl)-1*H-*pyrazolo[4,3-*c*]pyridin-1-yl)phenyl)sulfonyl)propa n-1-ol | Intermediate 18i | (500 MHz, *DMSO-d₆*) δ: 9.24 (br d, J=7.02 Hz, 1H), 9.17 (s, 1H), 8.90 (s, 1H), 8.68-8.75 (m, 1H), 8.32 (s, 1H), 8.04 (dd, J=8.85, 2.14 Hz, 1H), 7.99 (d, J=2.14 Hz, 1H), 7.65 (d, J=8.85 Hz, 1H), 7.17 (dd, J=6.87, 4.12 Hz, 1H), 4.64 (br s, 1H), 4.01 (s, 3H), 3.39-3.46 (m, 2H), 3.35-3.39 (m, 2H; overlapped with water from DMSO), 2.67 (s, 3H), 1.67-1.78 (m, 2H). | (Method 6): Rt = 3.16 min, ES⁺ *m*/*z* 479.1 |
| Ex. 10 | 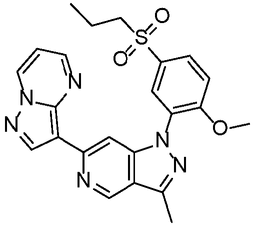 1-(2-methoxy-5-(propylsulfonyl)phenyl)-3-methyl-6-(pyrazolo[1,5-*a*]pyrimidin-3-yl)-1*H-*pyrazolo[4,3-*c*]pyridine | Intermediate 18j | (600 MHz, *DMSO-d₆*) δ: 9.24 (dd, J=7.0, 1.8 Hz, 1H), 9.18 (d, J=1.1 Hz, 1H), 8.90 (s, 1H), 8.71 (dd, J=4.1, 1.7 Hz, 1H), 8.32 (d, J=1.1 Hz, 1H), 8.04 (dd, J=8.8, 2.4 Hz, 1H), 7.99 (d, J=2.2 Hz, 1H), 7.65 (d, J=8.8 Hz, 1H), 7.17 (dd, J=6.9, 4.1 Hz, 1H), 4.01 (s, 3H), 3.36-3.33 (m, 2H), 1.65-1.58 (m, 2H), 0.93 (t, J=7.4 Hz, 3H) | (Method 6): Rt = 3.91 min, ES⁺ *m*/*z* 463.01 [M+H]⁺ |
| Ex. 11 | 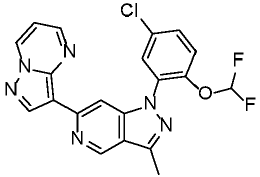 1-(5-chloro-2-(difluoromethoxy)phenyl )-3-methyl-6-(pyrazolo[1,5-*a*]pyrimidin-3-yl)-1*H-*pyrazolo[4,3-*c*]pyridine | Intermediate 18z | (600MHz, *DMSO-d₆*) δ: 9.22 (dd, J =6.9, 1.6 Hz, 1H), 9.19 (d, J=1.1 Hz, 1H), 8.90 (s, 1H), 8.70 (dd, J=4.0, 1.6 Hz, 1H), 8.34 (d, J=1.1 Hz, 1H), 7.83 (d, J=2.7 Hz, 1H), 7.73 (dd, J =8.9, 2.7 Hz, 1H), 7.59 (d, J=8.9 Hz, 1H), 7.24 (t, J=73.0 Hz, 1H), 7.16 (dd, J=6.9, 4.0 Hz, 1H), 2.65 (s, 3H). | (Method 5): Rt = 3.87 min, ES⁺ *m*/*z* 427.2/429. 2 [M+H]⁺ |
| Ex. 12 | 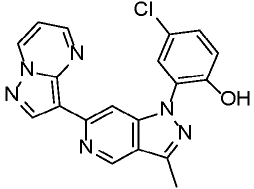 4-chloro-2-(3-methyl-6-(pyrazolo[1,5-*a*]pyrimidin-3-yl)-1*H-*pyrazolo[4,3-*c*]pyridin-1-yl)phenol | Intermediate 18aa | (600MHz, *DMSO-d₆*) δ: 10.5 (bs, 1H), 9.23 (d, J=6.4 Hz, 1H), 9.16 (s, 1H), 8.91 (s, 1H), 8.70 (bs, 1H), 8.26 (s, 1H), 7.51 (s, 1H), 7.44 (d, J=8.7 Hz, 1H), 7.15-7.18 (m, 1H), 2.66 (s, 3H). | (Methid 6): Rt = 2.76 min, ES⁺ *m*/*z* 377.4/379. 3 [M+H]⁺ |
| Ex. 13 | 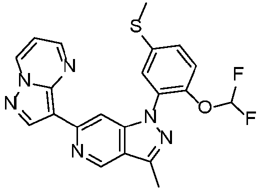 1-(2-(difluoromethoxy)-5-(methylthio)phenyl)-3-methyl-6-(pyrazolo[1,5-*a*]pyrimidin-3-yl)-1*H-*pyrazolo[4,3-*c*]pyridine | Intermediate 18ab | (400 MHz, *DMSO-d₆*) δ: 9.22 (dd, J=1.4, 6.9 Hz, 1H), 9.17 (s, 1H), 8.89 (s, 1H), 8.68 (dd, J=1.5, 4.0 Hz, 1H), 8.32 (s, 1H), 7.58-7.45 (m, 3H), 7.13 (t, J=73.2 Hz, 1H), 7.15 (dd, J=4.2, 7.0 Hz, 1H), 7.18-7.13 (m, 1H), 7.13 (s, 1H), 2.65 (s, 3H), 2.55 (s, 3H) | (Method 6): Rt = 4.57 min, ES⁺ *m*/*z* 439.2 [M+H]⁺ |
| Ex. 14 | 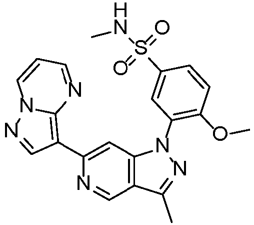 4-methoxy-N-methyl-3-(3-methyl-6-(pyrazolo[1,5-*a*]pyrimidin-3-yl)-1*H-*pyrazolo[4,3-*c*]pyridin-1-yl)benzenesulfonamide | Intermediate 18ad | (300 MHz, *DMSO-d₆*) δ: 9.23 (dd, J=7.0, 1.6 Hz, 1H), 9.17 (d, J=1.0 Hz, 1H), 8.89 (s, 1H), 8.70 (dd, J=4.0, 1.6 Hz, 1H), 8.31 (d, J=0.9 Hz, 1H), 7.90-7.95 (m, 1H), 7.86-7.90 (m, 1H), 7.59 (d, J=8.7 Hz, 1H), 7.49 (br s, 1H), 7.16 (dd, J=7.1, 4.1 Hz, 1H), 3.97 (s, 3H), 2.66 (s, 3H), 2.45 (s, 3H). | (Method 7): Rt = 3.89 min, ES⁺ *m*/*z* 450.1 [M+H]⁺ |
| Ex. 15 | 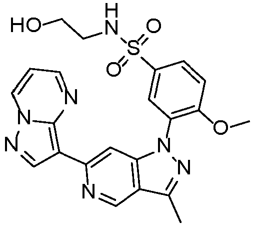 N-(2-hydroxyethyl)-4-methoxy-3-(3-methyl-6-(pyrazolo[1,5-*a*]pyrimidin-3-yl)-1*H-*pyrazolo[4,3-*c*]pyridin-1-yl)benzenesulfonamide | Intermediate 18ae | (600 MHz, *DMSO-d₆*) δ: 9.23 (d, J=6.1 Hz, 1H), 9.18 (s, 1H), 8.90 (s, 1H), 8.70-8.75 (m, 1H), 8.32 (s, 1H), 7.91-7.98 (m, 2H), 7.67 (br s, 1H), 7.59 (d, J=8.8 Hz, 1H), 7.13-7.20 (m, 1H), 4.70-4.75 (m, 1H), 3.98 (s, 3H), 3.31-3.43 (m, 2H), 2.78-2.89 (m, 2H), 2.68 (s, 3H). | (Method 5): Rt = 2.43 min, ES⁺ *m*/*z* 479.8 [M+H]⁺ |
| Ex. 16 | 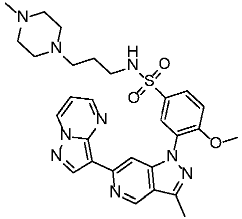 4-methoxy-3-(3-methyl-6-(pyrazolo[1,5-*a*]pyrimidin-3-yl)-1*H-*pyrazolo[4,3-*c*]pyridin-1-yl)-*N*-(3-(4-methylpiperazin-1-yl)propyl)benzenesulfona mide | Intermediate 18af | (500 MHz, *DMSO-d₆*) δ: 9.23 (d, J=7.0 Hz, 1H), 9.17 (s, 1H), 8.89 (s, 1H), 8.74-8.70 (m, 1H), 8.31 (s, 1H), 7.94-7.88 (m, 2H), 7.63 (br s, 1H), 7.59 (d, J=8.5 Hz, 1H), 7.19-7.15 (m, 1H), 3.99 (s, 3H), 2.81 (br s, 2H), 2.68 (s, 3H), 2.23-2.06 (m, 10H), 1.98 (s, 3H), 1.52-1.43 (m, 2H) | (Method 7): Rt = 3.22 min, ES⁺ *m*/*z* 576.3 [M+H]⁺ |
| Ex. 17 | 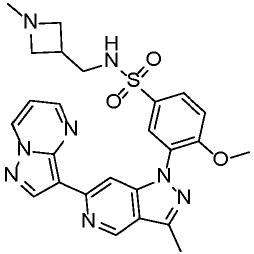 4-methoxy-3-(3-methyl-6-(pyrazolo[1,5-*a*]pyrimidin-3-yl)-1*H-*pyrazolo[4,3-*c*]pyridin-1-yl)-*N*-((1-methylazetidin-3-yl)methyl)benzenesulfon amide | Intermediate 18ag | (300 MHz, *DMSO-d₆*) δ: 9.23 (dd, J=7.0, 1.6 Hz, 1H), 9.17 (d, J=1.0 Hz, 1H), 8.89 (s, 1H), 8.71 (dd, J=4.0, 1.7 Hz, 1H), 8.30 (d, J=1.0 Hz, 1H), 7.91 (m, 2H), 7.66-7.77 (m, 1H), 7.58 (d, J=8.5 Hz, 1H), 7.16 (dd, J=7.0, 4.2 Hz, 1H), 3.97 (s, 3H), 3.10 (t, J=7.5 Hz, 2H), 2.89-2.99 (m, 2H), 2.77 (t, J=6.4 Hz, 2H), 2.67 (s, 3H), 2.29-2.41 (m, 1H), 2.10 (s, 3H). | (Method 7): Rt = 3.34 min, ES⁺ *m*/*z* 519.1 [M+H]⁺ |
| Ex. 18 | 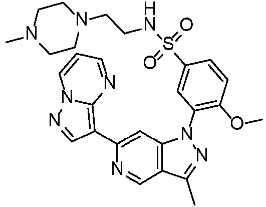 4-methoxy-3-(3-methyl-6-(pyrazolo[1,5-*a*]pyrimidin-3-yl)-1*H-*pyrazolo[4,3-*c*]pyridin-1-yl)-*N*-(2-(4-methylpiperazin-1-yl)ethyl)benzenesulfona mide | Intermediate 18ah | (500 MHz, *DMSO-d₆,-*7.97 (m, 2H), 7.55 (d, J=8.5 Hz, 1H), 7.26 (br s, 1H), 7.12 (dd, J=6.9 Hz, J=4.1 Hz, 1H), 3.98 (s, 3H), 2.95 (t, J=6.4 Hz, 2H), 2.68 (s, 3H), 2.33 (t, J=6.6 Hz, 2H), 2.26 (m, 4H), 2.17 (m, 4H), 2.04 (s, 3H). | (Method 7): Rt = 3.27 min, ES⁺ *m*/*z* 562.2 [M+H]⁺ |
| Ex. 19 | 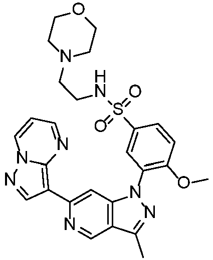 4-methoxy-3-(3-methyl-6-(pyrazolo[1,5-*a*]pyrimidin-3-yl)-1*H-*pyrazolo[4,3-*c*]pyridin-1-yl)-*N*-(2-morpholinoethyl)benzene sulfonamide | Intermediate 18ai | (500 MHz, *DMSO-d₆*) δ: 9.23 (dd, J=7.0, 1.5 Hz, 1H), 9.17 (s, 1H), 8.89 (s. 1H), 8.71 (dd, J=4.1, 1.5 Hz, 1H), 8.30 (s, 1H), 7.97-9.92 (m, 2H), 7.58 (d, J=8.5 Hz, 2H), 7.17 (dd, J=7.1, 4.1 Hz, 1H), 3.97 (s, 3H), 3.42 (t, J=4.4 Hz, 4H), 2.92 (q, J=5.5 Hz, 2H), 2.67 (s, 3H), 2.28 (t, J=6.8 Hz, 2H), 2.22 (bs, 4H). | (Method 7): Rt = 3.29 min, ES⁺ *m*/*z* 549.2 [M+H]⁺ |
| Ex. 20 | 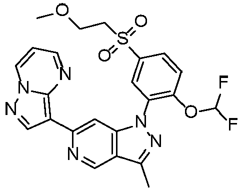 1-(2-(difluoromethoxy)-5-((2-methoxyethyl)sulfonyl)p henyl)-3-methyl-6-(pyrazolo[1,5-*a*]pyrimidin-3-yl)-1*H-*pyrazolo[4,3-*c*]pyridine | Intermediate 35a | (500 MHz, *DMSO-d₆*) δ: 9.23 (dd, J=6.87, 1.37 Hz, 1H), 9.21 (s, 1H), 8.91 (s, 1H), 8.66-8.68 (m, 1H), 8.37 (s, 1H), 8.17 (d, J=1.83 Hz, 1H), 8.11-8.16 (m, 1H), 7.79 (d, J=8.85 Hz, 1H), 7.49 (t, J=72.60 Hz, 1H), 7.16 (dd, J=6.87, 4.12 Hz, 1H), 3.71-3.78 (m, 2H), 3.64-3.70 (m, 2H), 3.10 (s, 3H), 2.69 (s, 3H). | (Method 7): Rt = 4.46 min, ES⁺ *m*/*z* 515.1 |
| Ex. 21 | 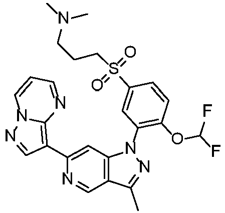 3-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)-N,N-dimethylpropan-1-amine | Intermediate 35b | (500 MHz, *DMSO-d₆*) *δ* 9.23 (dd, J=7.0, 1.5 Hz, 1H), 9.21 (d, J=0.9 Hz, 1H), 8.91 (s, 1H), 8.68 (dd, J=4.1, 1.7 Hz, 1H), 8.39 (d, J=0.9 Hz, 1H), 8.18 (d, J=2.1 Hz, 1H), 8.16 (dd, J=8.7, 2.3 Hz, 1H), 7.82 (d, J=8.5 Hz, 1H), 7.50 (t, J=72.9 Hz, 1H), 7.17 (dd, J=6.9, 4.1 Hz, 1H), 3.41-3.46 (m, 2H), 2.69 (s, 3H), 2.23 (t, J=6.9 Hz, 2H), 2.03 (s, 6H), 1.73 (quin, J=7.2 Hz, 2H). | (Method 7): Rt = 3.61 min, ES⁺ *m*/*z* 542.2 |
| Ex. 22 | 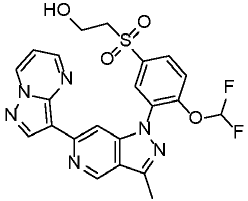 2-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)ethan-1-ol | Intermediate 35c | (*DMSO-d₆,-*8.18 (m, 2H), 7.78 (d, J=8.4 Hz, 1H), 7.55 (t, J=72.3 Hz, 1H), 7.16 (dd, J=7.1, 4.1 Hz, 1H), 4.93 (t, J=5.3 Hz, 1H), 3.71-3.79 (m, 2H), 3.56-3.62 (m, 2H), 2.68 (s, 3H) | (Method 7): Rt = 4.14 min, ES⁺ *m*/*z* 501.1 [M+H]⁺ |
| Ex. 23 | 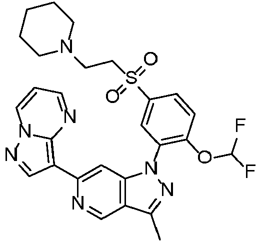 11-(2-(difluoromethoxy)-5-((2-(piperidin-1-yl)ethyl)sulfonyl)phenyl) -3-methyl-6-(pyrazolo[1,5-a]pyrimidin-3-yl)-1H-pyrazolo[4,3-c]pyridine | Intermediate 35d | (300 MHz, *DMSO-d₆*) *δ* = 9.22 (dd, J=1.7, 7.1 Hz, 1H), 9.20 (d, J=1.0 Hz, 1H), 8.91-8.88 (m, 1H), 8.69-8.62 (m, 1H), 8.40 (d, J=0.7 Hz, 1H), 8.19 (d, J=2.3 Hz, 1H), 8.12 (dd, J=2.3, 8.7 Hz, 1H), 7.77 (d, J =9.1 Hz, 1H), 7.46 (t, J=72.8 Hz, 1H), 7.15 (dd, J=3.9, 6.9 Hz, 1H), 3.62 (t, J=6.3 Hz, 2H), 2.57 (t, J=6.1 Hz, 1H), 2.11 (br s, 4H), 1.06 (br s, 6H) | (Method 7): Rt = 3.92 min, ES⁺ *m*/*z* 568.2 [M+H]⁺ |
| Ex. 24 | idin-1-yl)ethan-1-one 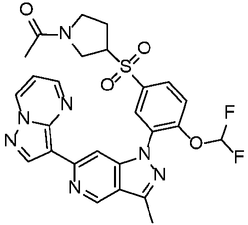 1-(3-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)pyrrol idin-1-yl)ethan-1-one | Intermediate 35e | (400 MHz, *DMSO-d₆*, 80°C): δ = 9.18 (s, 1H), 9.13 (d, J=7.0 Hz, 1H), 8.87 (s, 1H), 8.62-8.67 (m, 1H), 8.37 (d, J=1.0 Hz, 1H), 8.12-8.22 (m, 2H), 7.82 (d, J=8.4 Hz, 1H), 7.37 (t, J=71.8 Hz, 1H), 7.11 (dd, J=6.9, 4.2 Hz, 1H), 4.21-4.37 (m, 1H), 3.81 (s, 1H), 3.43-3.64 (m, 2H), 2.69 (s, 3H), 2.50 (m, 1H), 2.20-2.42 (m, 2H), 1.89 (br s, 3H). | (Method 7): Rt = 4.35 min, ES⁺ *m*/*z* 568.2 [M+H]⁺ |
| Ex. 25 | 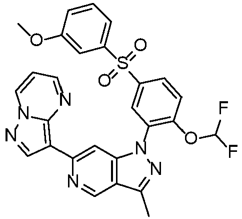 1-(2-(difluoromethoxy)-5-((3-methoxyphenyl)sulfonyl) phenyl)-3-methyl-6-(pyrazolo[1,5-a]pyrimidin-3-yl)-1H-pyrazolo[4,3-c]pyridine | Intermediate 35f | (*DMSO-d₆,-*7.64 (m, 3H), 7.45 (t, J=72 Hz, 1H), 7.23-7.28 (m, 1H), 7.17 (dd, J=7.0, 4.0 Hz, 1H), 3.78 (s, 3H), 2.68 (s, 3H) | (Method 7): Rt = 3.66 min, ES⁺ *m*/*z* 563.1 [M+H]⁺ |
| Ex. 26 | 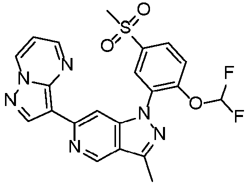 1-(2-(difluoromethoxy)-5-(methylsulfonyl)phenyl)-3-methyl-6-(pyrazolo[1,5-a]pyrimidin-3-yl)-1H-pyrazolo[4,3-c]pyridine | Intermediate 35g | (500 MHz, *DMSO-d₆*) δ: 9.22-9.25 (m, 1H), 9.21 (d, J=0.9 Hz, 1H), 8.91 (s, 1H), 8.68 (dd, J=4.1, 1.7 Hz, 1H), 8.40 (s, 1H), 8.24 (d, J=2.4 Hz, 1H), 8.16-8.20 (m, 1H), 7.82 (d, J=8.9 Hz, 1H), 7.49 (t, J=72.0 Hz, 1H), 7.15-7.19 (m, 1H), 3.35-3.37 (m, 3H), 2.69 (s, 3H). | (Method 6): Rt = 3.66 min, ES⁺ *m*/*z* 470.9 [M+H]⁺ |
| Ex. 27 | 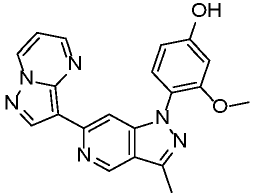 3-methoxy-4-(3-methyl-6-(pyrazolo[1,5-*a*]pyrimidin-3-yl)-1*H-*pyrazolo[4,3-*c*]pyridin-1-yl)phenol | Intermediate 19 | (*DMSO-d₆,* | (Method 6): Rt = 0.96 min, ES⁺ *m*/*z* 373.2 [M+H]⁺ |
| Ex. 28 | 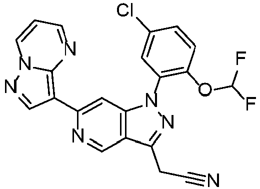 2-(1-(5-chloro-2-(difluoromethoxy)phenyl )-6-(pyrazolo[1,5-*a*]pyrimidin-3-yl)-1*H-*pyrazolo[4,3-*c*]pyridin-3-yl)acetonitrile | Intermediate 22 | (500 MHz, *DMSO-d₆*) δ: 9.30-9.21 (m, 2H); 8.92 (s, 1H); 8.71 (dd, J=3.8, 1.5 Hz, 1H); 8.35 (s, 1H); 7.90 (d, J=2.4 Hz, 1H); 7.79 (dd, J=9.1, 2.6 Hz, 1H); 7.62 (d, J=8.9 Hz, 1H); 7.26 (t, J=72.7 Hz, 1H); 7.17 (dd. J=7.0, 4.3 Hz, 1H); 4.66 (s, 2H). | (Method 5): Rt = 4.10 min, ES⁺ 452.0/454. 0 [M+H]⁺ |
| Ex. 29 | 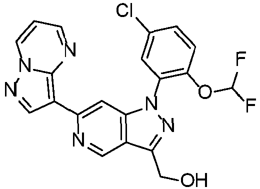 (1-(5-chloro-2-(difluoromethoxy)phenyl )-6-(pyrazolo[1,5-*a*]pyrimidin-3-yl)-1*H-*pyrazolo[4,3-*c*]pyridin-3-yl)methanol | Intermediate 23a | (600 MHz, *DMSO-d₆*) δ: 9.31 (s. 1H); 9.24 (bd, J=6.9 Hz, 1H); 8.91 (s, 1H); 8.72 (bd, J=4.1 Hz, 1H); 8.35 (s. 1H); 7.83 (d, J=2.6 Hz. 1H); 7.76 (dd, J=8.9, 2.6 Hz, 1H); 7.61 (d, J=8.9 Hz, 1H); 7.27 (t, J=72.5 Hz, 1H); 7.17 (dd, J=6.9, 4.1 Hz, 1H); 5.71 (t, J=5.5 Hz, 1H); 4.94 (d, J=5.5 Hz, 2H | (Method 6): Rt = 3.80 min, ES⁺ *m*/*z* 443.1/445. 1 [M+H]⁺ |
| Ex. 30 | 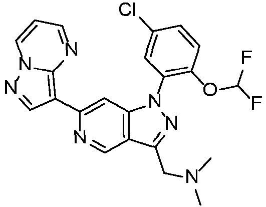 1-(1-(5-chloro-2-(difluoromethoxy)phenyl )-6-(pyrazolo[1,5-*a*]pyrimidin-3-yl)-1*H-*pyrazolo[4,3-*c*]pyridin-3-*yl)-N,N-*dimethylmethanamine | Intermediate 27b | (300 MHz, *DMSO-d₆*) δ:9.26 (d, J=1.2 Hz, 1H), 9.22 (dd, J=7.0, 1.7 Hz, 1H), 8.88 (s, 1H), 8.69 (dd, J=4.0, 1.7 Hz, 1H), 8.32 (d, J=1.0 Hz, 1H), 7.87 (d, J=2.6 Hz, 1H), 7.75 (dd, J=8.9, 2.6 Hz, 1H), 7.59 (d, J=8.9 Hz, 1H), 7.21 (t, J=72.8 Hz, 1H) 7.16 (dd, J=7.1, 4.1 Hz, 1H), 3.88 (s, 2H), 2.27 (s, 6H). | (Method 5): Rt = 3.06 min, ES⁺ *m*/*z* 470.5/472. 5 [M+H]⁺ |
| | | (Palladium source: Pd(PPh₃)₄; solvent: 1,4-dioxane/wate r; base: Cs₂CO₃) | | |
| Ex. 31 | 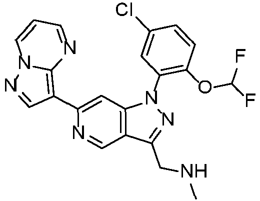 1-(1-(5-chloro-2-(difluoromethoxy)phenyl )-6-(pyrazolo[1,5-*a*]pyrimidin-3-yl)-1*H-*pyrazolo[4,3-*c*]pyridin-3-yl)-*N-*methylmethanamine | Intermediate 27c | (300 MHz, *DMSO-d₆*) δ: 9.32 (d, J=1.1 Hz, 1H), 9.23 (dd, J=7.1, 1.7 Hz, 1H), 8.89 (s, 1H), 8.69 (dd, J=4.1, 1.77 Hz, 1H), 8.33 (d, J=09 Hz, 1H), 7.84 (d, J=2.6 Hz, 1H), 7.74 (dd, J=8.9, 2.6 Hz, 1H), 7.59 (d, J=9.1 Hz, 1H), 7.5 (s, 1H), 7.24 (t, J=72.8 Hz, 1H), 7.15 (dd, J=7.0, 4.14 Hz, 1H), 4.13 (s, 2H), 2.36 (s, 3H) | (Method 6): Rt = 4.14 min, ES⁺ *m*/*z* 456.2/458. 1 [M+H]⁺ |
| | | (Palladium source: Pd(PPh₃)₄; solvent: 1,4-dioxane/wate r; base: Cs₂CO₃) | | |
| Ex. 32 | 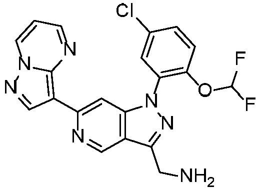 (1-(5-chloro-2-(difluoromethoxy)phenyl )-6-(pyrazolo[1,5-*a*]pyrimidin-3-yl)-1*H-*pyrazolo[4,3-*c*]pyridin-3-yl)methanamine | Intermediate 27d (Palladium source: Pd(PPh₃)₄; solvent: 1,4-dioxane/wate r; base: Cs₂CO₃) | (300 MHz, *DMSO-d₆*) δ:9.37 (d, J=0.9 Hz, 1H), 9.23 (dd, J=7.0, 1.6 Hz, 1H), 8.90 (s, 1H), 8.70 (dd, J=4.0, 1.7 Hz, 1H), 8.35 (d, J=0.9 Hz, 1H), 7.82 (d, J=2.6 Hz, 1H), 7.76 (dd, J=9.1, 2.6 Hz, 1H), 7.60 (d, J=8.9 Hz, 1H), 7.29 (t, J=72.8 Hz, 1H), 7.16 (dd, J=7.0, 4.2 Hz, 1H), 5.11 (br. s, 2H), 4.34 (s, 2H). | (Method 5): Rt = 3.32 min, ES⁺ *m*/*z* 442.5/444. 5 [M+H]⁺ |
| Ex. 33 | 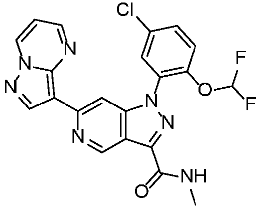 1-(5-chloro-2-(difluoromethoxy)phenyl )-N-methyl-6-(pyrazolo[1,5-*a*]pyrimidin-3-yl)-1*H-*pyrazolo[4,3-*c*]pyridine-3-carboxamide | Intermediate 26a | (600 MHz, *DMSO-d₆*) δ: 9.52 (d, J=1.1 Hz. 1H); 9.24 (dd, J=6.9, 1.7 Hz, 1H); 8.89 (s, 1H); 8.72 (dd, J=4.1, 1.7 Hz, 1H); 8.68 (q, J=4.8 Hz, 1H); 8.38 (d, J=1.1 Hz. 1H); 8.04 (d, J=2.6 Hz. 1H); 7.83 (dd, J=8.9, 2.6 Hz, 1H); 7.65 (d, J=8.9 Hz, 1H); 7.28 (t, J=72.5 Hz, 1H); 7.18 (dd, J=6.9, 4.1 Hz, 1H); 2.87 (d, J=4.7 Hz, 3H). | (Method 4): Rt = 1.61 min, ES⁺ *m*/*z* 470.1/472. 1 [M+H]⁺ |
| Ex. 34 | 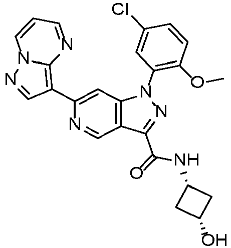 1-(5-chloro-2-methoxyphenyl)-*N-((1s,3s)-3-*hydroxycyclobutyl)-6-(pyrazolo[1,5-*a*]pyrimidin-3-yl)-1*H-*pyrazolo[4,3-c]pyridine-3-carboxamide | Intermediate 26d | (300 MHz, *DMSO-d₆*) δ:9.45 (d, J=1.2 Hz, 1H), 9.23 (dd, J=7.1, 1.7 Hz, 1H), 8.90 (s, 1H), 8.85 (d, J=7.8 Hz, 1H), 8.72 (dd, J=4.1, 1.7 Hz, 1H), 8.32 (d, J=1.2 Hz, 1H), 7.80 (d, J=2.6 Hz, 1H), 7.69 (dd, J=9.0, 2.7 Hz, 1H), 7.45 (d, J=9.1 Hz, 1H), 7.17 (dd, J=7.0, 4.2 Hz, 1H), 5.07 (d, J=5.7 Hz, 1H), 3.93-4.04 (m, 1H), 3.80-3.93 (m, 1H), 3.85 (s, 3H), 2.51-2.63 (m, 2H), 2.03 (qd, J=8.6, 2.7 Hz, 2H). | (Method 5): Rt = 3.43 min, ES⁺ *m*/*z* 489.9/491. 9 [M+H]⁺ |
| | | (Palladium source: Pd(PPh₃)₄; solvent: 1,4-dioxane/wate r; base: Cs₂CO₃) | | |
| Ex. 35 | 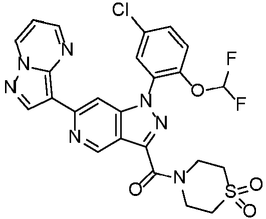 (1-(5-chloro-2-(difluoromethoxy)phenyl )-6-(pyrazolo[1,5-*a*]pyrimidin-3-yl)-1*H-*pyrazolo[4,3-*c*]pyridin-3-yl)(1,1-dioxidothiomorpholino) methanone | Intermediate 26b | (300 MHz, *DMSO-d₆*) δ:9.38-9.42 (m, 1H), 9.25 (dd, J=7.0, 1.6 Hz, 1H), 8.92 (s, 1H), 8.71 (dd, J=4.2, 1.7 Hz, 1H), 8.40-8.41 (m, 1H), 8.04 (d, J=2.6 Hz, 1H), 7.83 (dd, J=9.1, 2.6 Hz, 1H), 7.65 (d, J=8.9 Hz, 1H), 7.28 (t, J=72.6 Hz, 1H), 7.18 (dd, J=7.4, 4.2 Hz, 1H), 4.42-4.52 (m, 2H), 4.12-4.20 (m, 2H). | (Method 5): Rt = 3.97 min, ES⁺ *m*/*z* 574.4/576. 3 [M+H]⁺ |
| | | (Palladium source: Pd(PPh₃)₄; solvent: 1,4-dioxane/wate r; base: Cs₂CO₃) | | |
| Ex.36 | 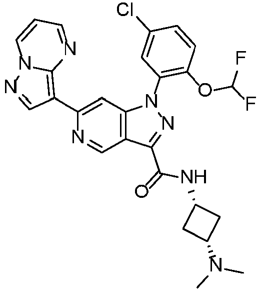 1-(5-chloro-2-(difluoromethoxy)phenyl )-*N*-((1*s*,3*s*)-3-(dimethylamino)cyclobut yl)-6-(pyrazolo[1,5-*a*]pyrimidin-3-yl)-1*H-*pyrazolo[4,3-*c*]pyridine-3-carboxamide | Intermediate 26g | (300 MHz, *DMSO-d₆*) δ: 9.48 (d, J=1.2 Hz, 1H), 9.24 (dd, J=7.1, 1.7 Hz, 1H), 8.87-8.94 (m, 2H), 8.71 (dd, J=4.2, 1.7 Hz, 1H), 8.38 (d, J=1.2 Hz, 1H), 8.03 (d, J=2.6 Hz, 1H), 7.83 (dd, J=8.9, 2.7 Hz, 1H), 7.64 (d, J=8.9 Hz, 1H), 7.28 (t, J=72.6 Hz, 1H), 7.17 (dd, J=7.0, 4.2 Hz, 1H), 4.10-4.28 (m, 1H), 2.31-2.44 (m, 3H), 2.04 (s, 6H), 1.90-2.02 (m, 2H). | (Method 5): Rt = 2.95 min, ES⁺ *m*/*z* 553.2/555. 1 [M+H]⁺ |
| | | (Palladium source: Pd(PPh₃)₄; solvent: 1,4-dioxane/wate r; base: Cs₂CO₃) | | |
| Ex. 37 | 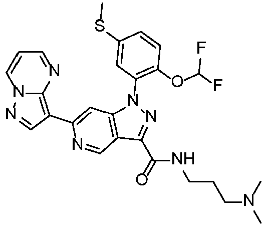 1-(2-(difluoromethoxy)-5-(methylthio)phenyl)-N-(3-(dimethylamino)propyl)-6-(pyrazolo[1,5-*a*]pyrimidin-3-yl)-1*H-*pyrazolo[4,3-*c*]pyridine-3-carboxamide | Intermediate 26e | (500 MHz, *DMSO-d₆*) δ7 : 9.52 (d, J=0.9 Hz, 1H), 9.27-9.22 (m, 1H), 8.93 (s, 1H), 8.80 (t, J=5.8 Hz, 1H), 8.73-8.69 (m, 1H), 8.40 (d, J=0.6 Hz, 1H), 7.74 (d, J=2.1 Hz, 1H), 7.65-7.59 (m, 1H), 7.57-7.52 (m, 1H), 7.19 (t, J=73.0 Hz, 1H), 7.18-7.16 (m, 1H), 3.37 (q, J=6.7 Hz, 2H), 2.58 (s, 3H), 2.29 (t, J=7.0 Hz, 2H), 2.14 (s, 6H), 1.71 (t, J=7.0 Hz, 2H) | (Method 7): Rt = 4.67 min, ES⁺ *m*/*z* 553.1 [M+H]⁺ |
| Ex. 38 | 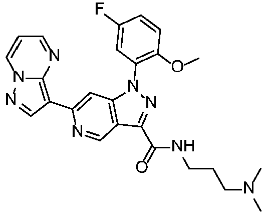 *N-(3-*(dimethylamino)propyl)-1-(5-fluoro-2-methoxyphenyl)-6-(pyrazolo[1,5-*a*]pyrimidin-3-yl)-1*H-*pyrazolo[4,3-*c*]pyridine-3-carboxamide | Intermediate 26f | (300 MHz, *DMSO-d₆*) δ: 9.49 (d, J=1.2 Hz, 1H); 9.23 (dd, J=7.1, 1.7 Hz, 1H); 8.91 (s, 1H); 8.80 (t, J=5.7 Hz, 1H); 8.72 (dd, J=4.1, 1.7 Hz, 1H); 8.34 (d, J=1.2 Hz, 1H); 7.62 (dd, J=8.7, 3.0 Hz, 1H); 7.40-7.56 (m, 2H); 7.17 (dd, J=7.1, 4.1 Hz, 1H); 3.84 (s, 3H); 3.34-3.40 (m, 2H); 2.27 (t, J=7.0 Hz, 2H); 2.13 (s, 6H); 1.70 (quin, J=7.1 Hz, 2H) | (Method 7): Rt = 4.00 min, ES⁺ *m*/*z* 489.1 [M+H]⁺ |
| Ex. 39 | 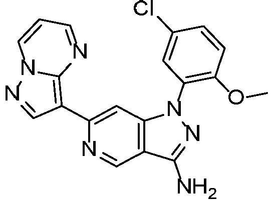 1-(5-chloro-2-methoxyphenyl)-6-(pyrazolo[1,5-*a*]pyrimidin-3-yl)-1*H-*pyrazolo[4,3-*c*]pyridin-3-amine | Intermediate 181 | (500 MHz, *DMSO-d₆*) δ: 9.21 (dd, J=6.7, 1.5 Hz, 1H), 9.06 (s, 1H), 8.86 (s, 1H), 8.70 (dd, J=4.1, 1.7 Hz, 1H), 8.17 (s, 1H), 7.49 - 7.45 (m, 2H), 7.35 (d, J=8.5 Hz, 1H), 7.15 (dd, J=7.0, 4.0 Hz, 1H), 6.20 (s, 2H), 3.90 (s, 3H). | (Method 5): Rt = 2.98 min, ES⁺ *m*/*z* 391.9/393. 9 [M+H]⁺ |
| | | (Palladium source: Pd(PPh₃)₄; solvent: 1,4-dioxane/wate r; base: Cs₂CO₃) | | |
| Ex. 40 | 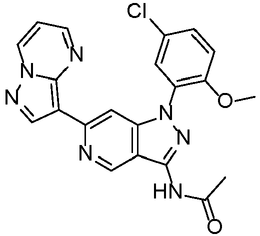 N-(1-(5-chloro-2-methoxyphenyl)-6-(pyrazolo[1,5-a]pyrimidin-3-yl)-1H-pyrazolo[4,3-*c*]pyridin-3-yl)acetamide | Intermediate 18am | (500 MHz, *DMSO-d₆*) δ: 11.02 (br s, 1H), 9.38 (s, 1H), 9.23 (dd, J=7.0, 1.8 Hz, 1H), 8.89 (s, 1H), 8.71 (dd, J=4.1, 1.7 Hz, 1H), 8.21 (d, J=0.9 Hz, 1H), 7.64 - 7.59 (m, 2H), 7.42 (d, J=8.5 Hz, 1H), 7.16 (dd, J=7.0, 4.0 Hz, 1H), 3.87 (s, 3H), 2.20 (s, 3H). | (Method 6): Rt = 3.80 min, ES⁺ *m*/*z* 433.9/435. 9 [M+H]⁺ |
| Ex. 41 | 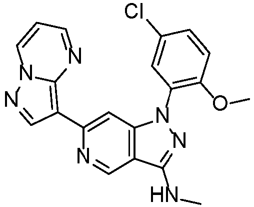 1-(5-chloro-2-methoxyphenyl)-*N-*methyl-6-(pyrazolo[1,5-*a*]pyrimidin-3-yl)-1*H-*pyrazolo[4,3-*c*]pyridin-3-amine | Intermediate 18r | (300 MHz, *DMSO-d₆*) δ: 9.22 (dd,J=7.2, 1.7 Hz, 1H), 9.03 (d, J=1.0 Hz, 1H), 8.85 (s, 1H), 8.71 (dd, J=4.1, 1.7 Hz, 1H), 8.15 (d, J=1.0 Hz, 1H), 7.54 (d, J=2.6 Hz, 1H), 7.48 (dd, J=9.0, 2.7 Hz, 1H), 7.36 (d, J=8.9 Hz, 1H), 7.15 (dd, J=7.3, 4.2 Hz, 1H), 6.88-6.82 (m, 1H), 3.9 (s, 3H), 2.93 (d, J=4.9 Hz, 3H) | (Method 5): Rt = 3.33 min, ES⁺ *m*/*z* 405.9/407. 2 [M+H]⁺ |
| Ex. 42 | 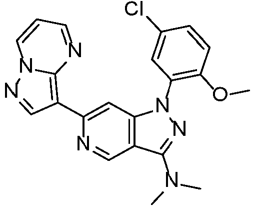 1-(5-chloro-2-methoxyphenyl)-*N,N-*dimethyl-6-(pyrazolo[1,5-*a*]pyrimidin-3-yl)-1*H-*pyrazolo[4,3-*c*]pyridin-3-amine | Intermediate 18aj | (600 MHz, *DMSO-d₆*) δ: 9.23-9.21 (m, 2H), 8.85 (s, 1H), 8.85 (s, 1H), 8.71 (dd, J=4.1, 1.7 Hz, 1H), 8.14 (d, J=1.0 Hz, 1H), 7.55 (d, J=2.7 Hz, 1H), 7.53 (dd, J=8.8, 2.7 Hz), 7.38 (d, J=8.8 Hz, 1H), 7.16 (dd, J=6.9, 4.1 Hz, 1H), 3.88 (s, 3H), 3.28 (s, 6H) | (Method 5): Rt = 3.58 min, ES⁺ *m*/*z* 420.1/420. 2 [M+H]⁺ |
| Ex. 43 | 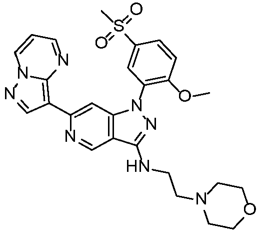 1-(2-methoxy-5-(methylsulfonyl)phenyl)-*N*-(2-morpholinoethyl)-6-(pyrazolo[1,5-*a*]pyrimidin-3-yl)-1*H-*pyrazolo[4,3-*c*]pyridin-3-amine | Intermediate 18q | (*DMSO-d₆*) δ: 9.21 (dd, J=7.1, 1.7 Hz, 1H), 9.10 (d, J=0.7 Hz, 1H), 8.85 (s, 1H), 8.70 (dd, J=3.9, 1.7 Hz, 1H), 8.19 (d, J=0.9 Hz, 1H), 7.91-8.01 (m, 2H), 7.57 (d, J=8.7 Hz, 1H), 7.15 (dd, J=7.0, 4.2 Hz, 1H), 6.86 (t, J=5.5 Hz, 1H), 4.02 (s, 3H), 3.56-3.62 (m, 4H), 3.42-3.52 (m, 2H), 3.24 (s, 3H), 2.59-2.68 (m, 2H), 2.54 (m, 4H, overlap with dmso). | (Method 3): Rt = 1.34 min, ES⁺ *m*/*z* 549.55 [M+H]⁺ |
| Ex. 44 | 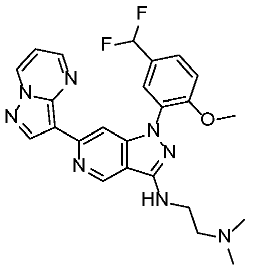 *N*¹-(1-(5-(difluoromethyl)-2-methoxyphenyl)-6-(pyrazolo[1,5-*a*]pyrimidin-3-yl)-1*H-*pyrazolo[4,3-*c*]pyridin-3-yl)*-N²,N²-*dimethylethane-1,2-diamine | Intermediate 18m | (300 MHz, *DMSO-d₆*) δ: 9.20 (dd, J=7.1, 1.5 Hz, 1H); 9.06-9.13 (m, 1H); 8.85 (s, 1H); 8.69 (dd, J=4.1, 1.7 Hz, 1H); 8.11-8.17 (m, 1H); 7.68 (br s, 1H); 7.63 (br d, J=8.5 Hz, 1H); 7.45 (d, J=8.7 Hz, 1H); 7.14 (dd, J=7.0, 4.0 Hz, 1H); 7.07 (t, J=55.6 Hz, 1H); 6.75 (t, J=5.6 Hz, 1H); 3.94 (s, 3H); 3.41 (q, J=6.3 Hz, 2H); 2.55 (t, J=6.8 Hz, 2H); 2.21 (s, 6H). | (Method 7): Rt = 3.67 min, ES⁺ *m*/*z* 479.2 [M+H]⁺ |
| Ex. 45 | 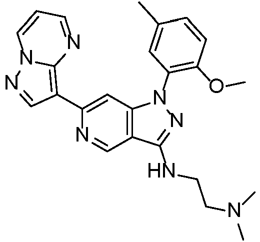 N1-(1-(2-methoxy-5-methylphenyl)-6-(pyrazolo[1,5-a]pyrimidin-3-yl)-1H-pyrazolo[4,3-c]pyridin-3-yl)-N²,N²-dimethylethane-1,2-diamine | Intermediate 18n | (500 MHz, *DMSO-d₆*) δ: 9.20 (dd, J=7.02, 1.53 Hz, 1H); 9.08 (s, 1H); 8.85 (s, 1H); 8.68 (dd, J=4.12, 1.68 Hz, 1H); 8.08 (s, 1H); 7.28 (s, 1H); 7.18-7.25 (m, 2H); 7.13 (dd, J=7.02, 3.97 Hz, 1H); 6.63 (t, J=5.49 Hz, 1H); 3.82 (s, 3H); 3.36-3.44 (m, 2H); 2.52-2.58 (m, 2H); 2.28-2.35 (m, 3H); 2.23 (s, 6H). | (Method 7): Rt = 3.69 min, ES⁺ *m*/*z* 443.2 [M+H]⁺ |
| Ex. 46 | 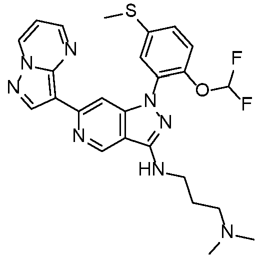 *N*¹-(1-(2-(difluoromethoxy)-5-(methylthio)phenyl)-6-(pyrazolo[1,5-*a*]pyrimidin-3-yl)-1*H-*pyrazolo[4,3-*c*]pyridin-3-yl)-*N*³,*N*³-dimethylpropane-1,3-diamine | Intermediate 18ak | (500 MHz, *DMSO-d₆*) *δ* = 9.22 (d, J=7.0 Hz, 1H), 9.09 (s, 1H), 8.87 (s, 1H), 8.68 (d, J=4.0 Hz, 1H), 8.24 (s, 1H), 7.51 (s, 1H), 7.42 (br s, 2H), 7.16 (br d, J=4.0 Hz, 1H), 7.14 (t, J=73.0 Hz, 1H), 6.89 (br t, J=5.2 Hz, 1H), 3.34 (ov, 2H), 2.55 (s, 3H), 2.40-2.34 (m, 2H), 2.18 (s, 6H), 1.82 (br t, J=7.0 Hz, 2H) | (Method 7): Rt = 4.13 min, ES⁺ *m*/*z* 525.2 [M+H]⁺ |
| Ex. 47 | 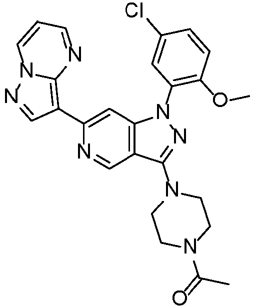 1-(4-(1-(5-chloro-2-methoxyphenyl)-6-(pyrazolo[1,5-*a*]pyrimidin-3-yl)-1*H-*pyrazolo[4,3-*c*]pyridin-3-yl)piperazin-1-yl)ethan-1-one | Intermediate 18s | (500 MHz, *CDCl₃*) δ: 9.13 (s, 1H), 8.97 (s, 1H), 8.75 (dd, J=7.0, 1.8 Hz, 1H), 8.57 (dd, J=4.0, 1.5 Hz, 1H), 8.20 (s, 1H), 7.55 (d, J=2.7 Hz, 1H), 7.38 (dd, J=8.9, 2.4 Hz, 1H), 7.08 (d, J=8.9 Hz, 1H), 6.91 (dd, J=6.9, 4.1 Hz, 1H), 3.90 (s, 3H), 3.89 (br d, J=5.5 Hz, 2H), 3.69-3.77 (m, 2H), 3.62 (dt, J=10.4, 5.2 Hz, 4H), 2.19 (s, 3H). | (Method 6): Rt = 4.26 min, ES⁺ *m*/*z* 503.3/505. 3 |
| Ex. 48 | 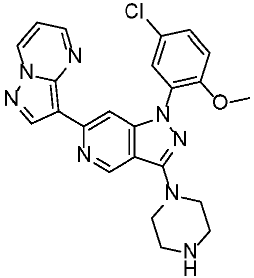 1-(5-chloro-2-methoxyphenyl)-3-(piperazin-1-yl)-6-(pyrazolo[1,5-*a*]pyrimidin-3-yl)-1*H-*pyrazolo[4,3-*c*]pyridine | Intermediate 18al | (500 MHz, *DMSO-d₆*) δ: 9.26 (s, 1H), 9.23 (d, J=6.8 Hz, 1H), 8.86 (s, 1H), 8.71 (dd, J=4.0, 1.8 Hz, 1H), 8.18 (s, 1H), 7.57 (d, J=2.4 Hz, 1H), 7.54 (dd, J=9.0, 2.6 Hz, 1H), 7.37-7.41 (m, 1H), 7.16 (dd, J=7.0, 4.0 Hz, 1H), 3.89 (s, 3H), 3.57 (br s, 3H), 3.51 (br s, 1H), 2.76-2.81 (m, 1H), 2.74 (br s, 3H). | (Method 5): Rt = 2.55 min, ES⁺ *m*/*z* 461.1/463. 1 |
| Ex. 49 | 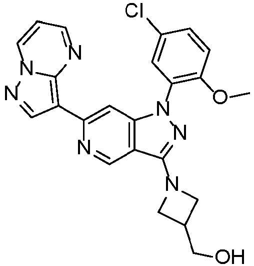 (1-(1-(5-chloro-2-methoxyphenyl)-6-(pyrazolo[1,5-a]pyrimidin-3-yl)-1H-pyrazolo[4,3-c]pyridin-3-yl)azetidin-3-yl)methanol | Intermediate 18t | (*DMSO-d₆,-*3.03 (m, 1H). | (Method 6): Rt = 3.93 min, ES⁺ *m*/*z* 462.0/463. 9 [M+H]⁺C_{2 3}H₂₀ClN₇ O₂ |
| Ex. 50 | 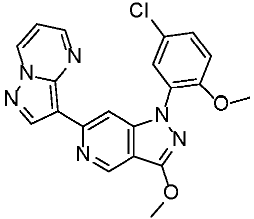 1-(5-chloro-2-methoxyphenyl)-3-methoxy-6-(pyrazolo[1,5-*a*]pyrimidin-3-yl)-1*H-*pyrazolo[4,3-*c*]pyridine | methoxyphenyl)-3-Intermediate 18y | (500 MHz, *DMSO-d₆*) *δ* 9.22-9.26 (m, 1H), 8.99-9.01 (m, 1H), 8.85-8.89 (m, 1H), 8.70-8.74 (m, 1H), 8.21 (d, J=0.9 Hz, 1H), 7.60-7.62 (m, 1H), 7.54-7.59 (m, 1H), 7.37-7.43 (m, 1H), 7.14-7.20 (m, 1H), 4.10 (s, 3H), 3.90 (s, 3H) | (Method 5): Rt = 3.78 min, ES⁺ *m*/*z* 407.4/409. 4 [M+H]⁺ |
| Ex. 51 | 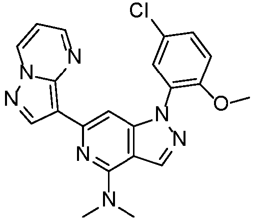 1-(5-chloro-2-methoxyphenyl)-*N,N-*dimethyl-6-(pyrazolo[1,5-*a*]pyrimidin-3-yl)-1*H-*pyrazolo[4,3-*c*]pyridin-4-amine | Intermediate 36a | (500 MHz, *DMSO-d₆*) δ: 9.17 (dd, J=7.0, 1.6 Hz, 1H), 8.82 (s, 1H), 8.64 (dd, J=4.1, 1.8 Hz, 1H), 8.44 (d, J=0.8 Hz, 1H), 7.62 (dd, J=8.6, 2.9 Hz, 1H), 7.55 (d, J=2.9 Hz, 1H), 7. 50 (s, 1H), 7.40 (d, J=8.7 Hz, 1H), 7.09 (dd, J=7.0, 4.1 Hz, 1H), 3.83-3.80 (s, 3H), 3.43 (s, 6H) | (Method 5): Rt = 3.23 min, ES⁺ *m*/*z* 420.1/421. 9 |
| Ex. 52 | 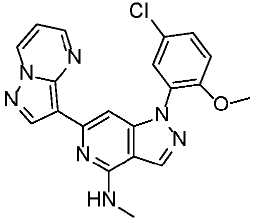 1-(5-chloro-2-methoxyphenyl)-N-methyl-6-(pyrazolo[1,5-*a*]pyrimidin-3-yl)-1*H-*pyrazolo[4,3-*c*]pyridin-4-amine | Intermediate 36b | (500 MHz, *DMSO-d₆*) δ: 9.17 (dd, J=7.1, 1.5 Hz, 1H), 8.83 (s, 1H), 8.64 (dd, J=4.0, 1.5 Hz, 1H), 8.33 (s, 1H), 7.60 (dd, J=8.9, 2.8 Hz, 1H), 7.53 (d, J=2.6 Hz, 1H), 7.52 (s, 1H), 7.43 (q, J=4.7 Hz, 1H), 7.38 (d, J=9.0 Hz, 1H), 7.10 (dd, J=7.0, 4.2 Hz, 1H), 3.81 (s, 3H), 3.13-3.07 (d, J=4.5 Hz, 3H) | (Method 5): Rt = 3.17 min, ES⁺ *m*/*z* 406.3/408. 3 [M+H]⁺ |
| Ex. 53 | 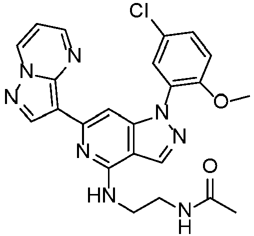 *N*-(2-((1-(5-chloro-2-methoxyphenyl)-6-(pyrazolo[1,5-*a*]pyrimidin-3-yl)-1*H-*pyrazolo[4,3-*c*]pyridin-4-yl)amino)ethyl)acetamid e | Intermediate 36c | (400 MHz, *DMSO-d₆*) δ: 9.16 (d, J=7.2 Hz, 1H), 8.92 (s, 1H), 8.63 (dd, J=4.2,1.8 Hz, 1H), 8.34 (d, J=0.8 Hz, 1H), 8.11-8.09 (t, J=5.1 Hz, 1H), 7.59 (dd, J=9.0,2.8 Hz, 1H), 7.52 (m, 1H), 7.38 (d, J=8.5 Hz, 1H), 7.08 (dd, J=6.9,4.1 Hz, 1H), 3.82 (s, 3H), 3.66-3.59 (m, 2H), 3.44-3.39 (m, 2H), 1.83 (s, 3H) | (Method 5): Rt = 3.03 min, ES⁺ *m*/*z* 477.1/479. 0 [M+H]⁺ |
| Ex. 54 | 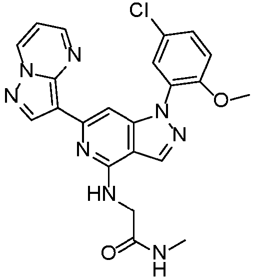 2-((1-(5-chloro-2-methoxyphenyl)-6-(pyrazolo[1,5-*a*]pyrimidin-3-yl)-1*H-*pyrazolo[4,3-*c*]pyridin-4-yl)amino)-*N-*methylacetamide | Intermediate 36d | (500 MHz, *DMSO-d₆*) δ:9.16 (d, J=6.7 Hz, 1H), 8.78 (s, 1H), 8.64 (d, J=2.1 Hz, 1H), 8.41 (s, 1H), 8.02 (d, J=4.3 Hz, 1H), 7.84-7.94 (m, 1H), 7.60 (d, J=8.9 Hz, 1H), 7.54 (s, 2H), 7.39 (d, J=8.9 Hz, 1H), 7.09 (dd, J=6.4, 4.3 Hz, 1H), 4.10 (d, J=5.2 Hz, 2H), 3.82 (s, 3H), 2.63 (d, J=4.0 Hz, 3H). | (Method 5): Rt = 3.09 min, ES⁺ *m*/*z* 463.0/465. 0 [M+H]⁺ |
| Ex. 55 | 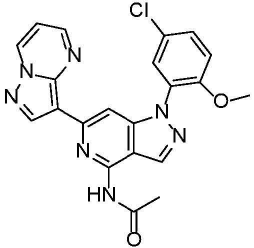 N-(1-(5-chloro-2-methoxyphenyl)-6-(pyrazolo[1,5-a]pyrimidin-3-yl)-1H-pyrazolo[4,3-c]pyridin-4-yl)acetamide | Intermediate 36e | (500 MHz, *DMSO-d₆*) δ: 10.79 (s, 1H), 9.22 (dd, J=6.9,1.7 Hz, 1H), 8.80 (s, 1H), 8.71 (dd, J=4.1,1.7 Hz, 1H), 8.48 (s, 1H), 8.04 (d, J=0.6 Hz, 1H), 7.69-7.61 (m, 2H), 7.43 (d, J=8.9 Hz, 1H), 7.16 (dd, J=7.0,4.0 Hz, 1H), 3.85 (s, 3H), 2.31 (s, 3H) | (Method 5): Rt = 3.38 min, ES⁺ *m*/*z* 434.6/436. 5 [M+H]⁺ |
| Ex. 56 | 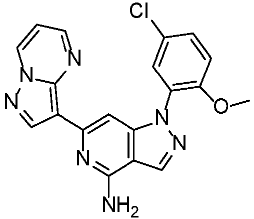 1-(5-chloro-2-methoxyphenyl)-6-(pyrazolo[1,5-a]pyrimidin-3-yl)-1H-pyrazolo[4,3-c]pyridin-4-amine | Intermediate 36f | (600 MHz, *DMSO-d₆*) δ: 9.17 (dd, J=7.0,1.8, Hz, 1H), 8.71 (s, 1H), 8.65 (dd, J=4.1,1.7 Hz, 1H), 8.38 (d, J=0.9 Hz, 1H), 7.61 (dd, J=8.9,2.7 Hz, 1H), 7.56 (d, J=0.9 Hz, 1H), 7.55 (d, J=2.6 Hz, 1H), 7.40 (d, J=9.0 Hz, 1H), 7.11 (dd, J=7.0, 4.0 Hz, 1H), 6.87 (s, 2H), 3.84 (s, 3H) | (Method 5): Rt = 3.11 min, ES⁺ *m*/*z* 392.1/394. 1 [M+H]⁺ |
| Ex. 57 | 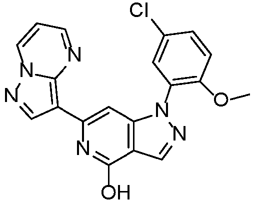 1-(5-chloro-2-methoxyphenyl)-6-(pyrazolo[1,5-*a*]pyrimidin-3-yl)-1*H-*pyrazolo[4,3-*c*]pyridin-4-ol | Intermediate 36g | (600 MHz, *DMSO-d₆*) δ/ 11.24 (br s, 1H), 9.27 (dd, J=1.7, 7.0 Hz, 1H), 9.05 (s, 1H), 8.77 (dd, J=1.7, 4.2 Hz, 1H), 8.29 (d, J=0.7 Hz, 1H), 7.65 (dd, J=2.8, 9.0 Hz, 1H), 7.61 (d, J=2.8 Hz, 1H), 7.40 (d, J=9.0 Hz, 1H), 7.25 (dd, J=4.2, 7.0 Hz, 1H), 7.10 (d, J=0.7 Hz, 1H), 3.85 (s, 3H) | (Method 5): Rt = 3.81 min, ES⁺ *m*/*z* 393.1/395. 2 [M+H]⁺ |

### Example 58

### Step 1

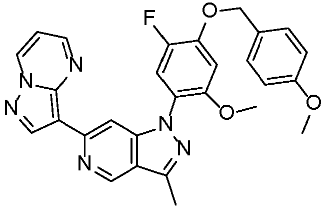

### 1-(5-Fluoro-2-methoxy-4-((4-methoxybenzyl)oxy)phenyl)-3-methyl-6-(pyrazolo[1,5-a]pyrimidin-3-yl)-1H-pyrazolo[4,3-c]pyridine (Example 58-step 1)

To a mixture of intermediate 18u (75.0 mg, 0.18 mmol), 3-(4,4,5,5-tetramethyl-1,3,2-dioxaborolan-2-yl)pyrazolo[1,5-a]pyrimidine (77.3 mg, 0.32 mmol), and solution of K₃PO₄( 0.5 M, 701 µL, 0.35 mmol) in degassed THF/water (2.7 mL), XPhos Pd G3 (7.42 mg, 8.8 µmol) was added and the RM was stirred at 50 °C 1 h under argon. After cooling to RT, RM was diluted with water (5 mL). The precipitate formed was collected by filtration and purified by flash chromatography on a Si cartridge by eluting with 0-100% EtOAc in DCM to afford the title product (63 mg).
LCMS (Method 2), Rt = 1.22 min, ES⁺ *m*/*z* 511.2 [M+H]⁺

### Step 2

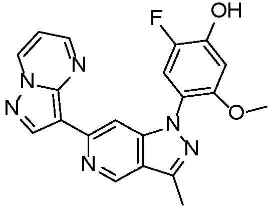

### 2-Fluoro-5-methoxy-4-(3-methyl-6-(pyrazolo[1,5-a]pyrimidin-3-yl)-1H-pyrazolo[4,3-c]pyridin-1-yl)phenol (Example 58)

An ice chilled solution of intermediate example 58-step 1 (57.0 mg, 0.112 mmol) in dry DCM (1 mL) was treated with TFA (1 mL, 13.4 mmol), warmed up to RT and stirred for 1 h. RM was neutralized with sat. aq. NaHCO₃ to form a precipitate that was collected by filtration and followed by washings with a small amount of water/DCM. The crude was purified by chromatography on a Si cartridge by eluting with 0-100 % DCM/MeCN/NH₄OH (10:10:1) in DCM to afford the title compound(18 mg).
LCMS (Method 5), Rt = 2.61 min, ES⁺ *m*/*z* 390.9 [M+H]⁺
¹H-NMR (300 MHz, *DMSO-d₆*) δ: 10.44 (br. s, 1H), 9.21 (dd, J=7.1, 1.7 Hz, 1H), 9.11 (d, J=1.2 Hz, 1H), 8.87 (s, 1H), 8.71 (dd, J=4.2, 1.7 Hz, 1H), 8.17 (d, J=1.2 Hz, 1H), 7.36 (d, J=11.1 Hz, 1H), 7.14 (dd, J=7.1, 4.1 Hz, 1H), 6.87 (d, J=8.0 Hz, 1H), 3.71 (s, 3H), 2.61 (s, 3H)

### Example 59 to 60

The following examples were prepared in a similar manner to example 58 by replacing intermediate 18u with the starting material indicated in the table below. When minor modifications on base, solvent, temperature, ligand and/or palladium source were made, they were stated in brackets.

| Example | Structure/Name | *Step 1* | | *Step 2* | |
|---|---|---|---|---|---|
| | | Starting Material | Name of intermediate Step 1 / LCMS | ¹H-NMR | LCMS |
| Example 59 | 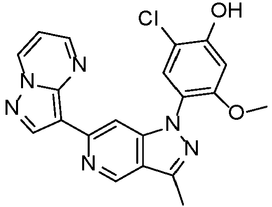 2-chloro-5-methoxy-4-(3-methyl-6-(pyrazolo[1, 5-a]pyrimidin-3-yl)-1H-pyrazolo[4,3-c]pyridin-1-yl)phenol | Intermediate 18v (Palladium source: / Pd(PPh3)4; solvent: in 1,4-dioxane/water; base:, Cs2CO3 )as a base | 1-(5-chloro-2-methoxy-4-((4-methoxybenzyl)oxy)p henyl)-3-methyl-6-(pyrazolo[1,5-a]pyrimidin-3-yl)-1H-pyrazolo[4,3-c]pyridine | (500 MHz, *DMSO-d₆*) δ: 10.82 (s, 1H), 9.25 (d, J=7.0 Hz, 1H), 9.22 (s, 1H), 8.93 (s, 1H), 8.75 (br d, J=2.7 Hz, 1H), 8.21 (s, 1H), 7.51 (s, 1H), 7.19 (dd, J=6.9, 4.1 Hz, 1H), 6.90 (s, 1H). 3.74 (s, 3H) 2.65 (s, 3H). | (Method 5): Rt = 2.85 min, ES+ m/z 406.9/408.9 |
| | | | (Method 2): Rt = 1.28, ES+ m/z 527.3/529.2 [M+H]⁺ | | |
| Example 60 | 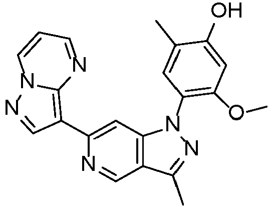 5-methoxy-2-methyl-4-(3-methyl-6-(pyrazolo[1, 5-a]pyrimidin-3-yl)-1H-pyrazolo[4,3-c]pyridin-1-yl)phenol | Intermediate 18w | 1-(2-methoxy-4-((4-methoxybenzyl)oxy)-5-methylphenyl)-3-methyl-6-(pyrazolo[1,5-a]pyrimidin-3-yl)-1H-pyrazolo[4,3-c]pyridine | (600 MHz, *DMSO-d₆*) δ: 9.72-10.18 (m, 1H), 9.18-9.28 (m, 1H), 9.11 (s, 1H), 8.89 (s, 1H), 8.71 (br d, J=2.4 Hz, 1H), 8.15 (s, 1H), 7.09-7.24 (m, 2H), 6.69-6.76 (m, 1H), 3.69 (s, 3H), 2.63 (s, 3H), 2.13 (s, 3H). | (Method 5): Rt = 2.85 min, ES+ m/z 406.9/408.9 |
| | | | (Method 2): Rt = 1.26, ES+ m/z 507.3 [M+H]⁺ | | |

### Example 61

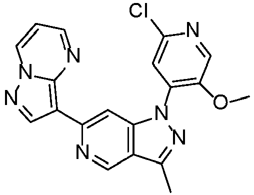

### 1-(2-Chloro-5-methoxypyridin-4-yl)-3-methyl-6-(pyrazolo[1,5-a]pyrimidin-3-yl)-1H-pyrazolo[4,3-c]pyridine (Example 61)

The title compound was prepared in a similar manner to intermediate 18a starting from intermediate 6 and 4-bromo-2-chloro-5-methoxy-pyridine.
LCMS (Method 5), Rt = 3.31 min, ES⁺ *m*/*z* 392.4 [M+H]⁺
¹H-NMR (500 MHz, *CDCl₃*) δ:9.08 (d, J=0.9 Hz, 1H), 8.98 (s, 1H), 8.78 (dd, J=7.0,1.8 Hz, 1H), 8.62 (dd, J=4.0, 1.8 Hz, 1H), 8.46-8.48 (m, 1H), 8.31 (s, 1H), 7.65 (s, 1H), 6.94 (dd, J=7.0, 4.0 Hz, 1H), 4.07 (s, 3H), 2.73 (s, 3H)

### Example 62

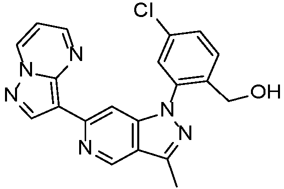

### (4-Chloro-2-(3-methyl-6-(pyrazolo[1,5-a]pyrimidin-3-yl)-1H-pyrazolo[4,3-c]pyridin-1-yl)phenyl)methanol (Example 62)

Intermediate 5 (60.0 mg, 0.220 mmol) and intermediate 8c (20.0 %, 377 mg, 0.36 mmol) in NMP (0.8 mL) were stirred at 60 °C for 1h and at 100 °C for 2 h. After cooling to RT, RM was diluted with water (10 mL) then extracted with EtOAc (2x10 mL) and DCM (2x10 mL). Combined organic layers were washed with water (4x10 mL), sat. aq. NaCl (10 mL) and evaporated under reduced pressure. The crude material was triturated in DCM to afford the title product (6.3 mg).
LCMS (Method 6), Rt = 3.93 min, ES⁺ *m*/*z* 391.44 [M+H]⁺
¹H-NMR (500 MHz, *CDCl₃*) δ: 9.22-9.25 (m, 1H), 9.20 (d, J=1.2 Hz, 1H), 8.90 (s, 1H), 8.67-8.70 (m, 1H), 8.27 (d, J=1.2 Hz, 1H), 7.80 (d, J=8.2 Hz, 1H), 7.68 (dd, J=8.5, 2.1 Hz, 1H), 7.64 (d, J=2.1 Hz, 1H), 7.13-7.18 (m, 1H), 5.27-5.32 (m, 1H), 4.33-4.40 (m, 2H), 2.66 (s, 3H)

### Example 63

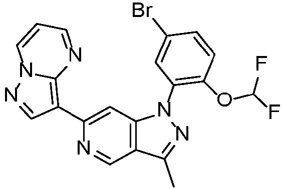

### 1-(5-Bromo-2-(difluoromethoxy)phenyl)-3-methyl-6-(pyrazolo[1,5-a]pyrimidin-3-yl)-1H-pyrazolo[4,3-c]pyridine (Example 63)

Intermediate 5 (30.0 mg, 0.110 mmol) and intermediate 8b (35.0 mg, 0.121 mmol) were suspended in NMP (0.7 mL) and stirred at 60 °C under MW irradiation for 1h, then at 120 °C for 2 h. After cooling to RT, RM was diluted with water (6 mL) and extracted with EtOAc (2x8 mL). Combined organic layers were washed with water (3x8 mL), sat. aq. NaCl (8 mL), dried over Na₂SO₄ and evaporated under reduced pressure. The residue was purified by chromatography on a Si cartridge by eluting with 0-1% MeOH in DCM to afford the title product (15 mg).
LCMS (Method 6): Rt = 4.88 min, ES⁺ *m*/*z* 471.0/473.0 [M+H]⁺
¹H-NMR (400 MHz, *DMSO-d₆*) δ / 9.20-9.26 (m, 1H), 9.19 (d, J=1.0 Hz, 1H), 8.90 (s, 1H), 8.70 (dd, J=4.1, 1.8 Hz, 1H), 8.32 (d, J=1.1 Hz, 1H), 7.93 (d, J=2.4 Hz, 1H), 7.85 (dd, J=8.9, 2.5 Hz, 1H), 7.51 (s, 1H), 7.24 (t, J=73.0 Hz, 1H), 7.16 (dd, J=7.1, 4.2 Hz, 1H), 2.66 (s, 3H)

### Example 64

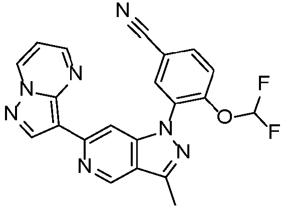

### 4-(difluoromethoxy)-3-(3-methyl-6-(pyrazolo[1,5-a]lpyrimidin-3-yl)-1H-pyrazolo[4,3-c]pyridin-1-yl)benzonitrile (Example 64)

tBuXPhos (9.04 mg, 0.02 mmol) was added to a solution of intermediate 28 (60.0 mg, 0.13 mmol) and zinc cyanide (17.9 mg, 0.15 mmol) in NMP (0.5 mL). RM was stirred at 120°C under MW irradiation for 15 min, then allylpalladium chloride dimer (9.32 mg, 0.03 mmol) added and stirring continued under the same conditions for 1 h. After cooling to RT, RM was diluted with water (5 mL) and the formed precipitate collected by filtration. The crude material was purified by flash chromatography on a Si cartridge by eluting with 0-45 % DCM/MeOH/NH₄OH (90:9:0.5) in DCM to afford the title product (21 mg).
LCMS (Method 5): Rt = 3.19 min, ES⁺ *m*/*z* 418.4 [M+H]⁺
¹H-NMR (500 MHz, *DMSO-d₆*) δ / 9.22-9.27 (m, 1H), 9.20 (s, 1H), 8.91 (s, 1H), 8.68-8.73 (m, 1H), 8.34 (s, 1H), 8.26-8.30 (m, 1H), 8.15-8.19 (m, 1H), 7.74 (br d, J=8.9 Hz, 1H), 7.48 (t, J=73.0 Hz, 1H), 7.17 (dd, J=6.9, 4.1 Hz, 1H), 2.67 (s, 3H)

### Example 65

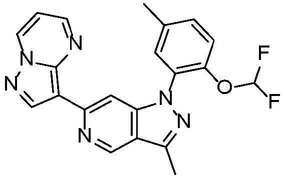

### 1-(2-(Difluoromethoxy)-5-methylphenyl)-3-methyl-6-(pyrazolo[1,5-a]pyrimidin-3-yl)-1H-pyrazolo[4,3-c]pyridine (Example 65)

To a mixture of intermediate 28 (50.0 mg, 0.11 mmol), 2,4,6-trimethyl-1,3,5,2,4,6-trioxatriborinane (74.2 µL, 0.53 mmol), Cs₂CO₃ (70 mg, 0.21 mmol) in degassed 1,4-dioxane/H₂O (2:1, 7.2 mL) , Pd(PPh₃)₄ (12.3 mg, 0.01 mmol) was added and RM was stirred at 80 °C overnight under argon atmosphere. After cooling to RT, RM was diluted with water (5 mL), the formed precipitate collected by filtration and submitted to a flash chromatography on Si cartridge by eluting with 0-100 % EtOAc in DCM to afford the title product (10 mg).
LCMS (Method 5): Rt = 3.41 min, ES⁺ *m*/*z* 407.0 [M+H]⁺
¹H-NMR (300 MHz, *DMSO-d₆*) δ: 9.22 (dd, J=6.97, 1.74 Hz, 1H), 9.16 (d, J=1.05 Hz, 1H), 8.89 (s, 1H), 8.68 (dd, J=4.01, 1.74 Hz, 1H), 8.28 (d, J=1.05 Hz, 1H), 7.47-7.52 (m, 1H), 7.38-7.48 (m, 2H), 7.14 (dd, J=7.14, 4.18 Hz, 1H), 7.11 (t, J=73.36 Hz, 1H), 2.65 (s, 3H), 2.40 (s, 3H).

### Example 66

### Step 1

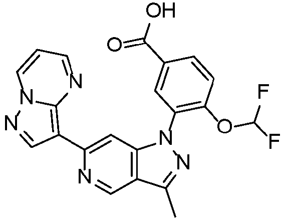

### 4-(Difluoromethoxy)-3-(3-methyl-6-(pyrazolo[1,5-a]pyrimidin-3-yl)-1H-pyrazolo[4,3-c]pyridin-1-yl)benzoic acid (Example 66-step 1)

To a mixture of intermediate 28 (500.0 mg, 1.06 mmol), Pd(OAc)₂ (7.15 mg, 32 µmol), Xantphos (18.4 mg, 32 µmol) and formic acid (280 µl, 7.43 mmol) in DMF (5 mL), DCC (438 mg, 2.12 mmol) and TEA (296 µl, 2.12 mmol) were added, and RM stirred at 80 °C for 6 h. RM was cooled to RT and diluted with EtOAc (15 mL) to form a precipitate that was collected by filtration, washed with a small amount of MeOH and dried. The crude was dissolved in aq.1M NaOH (20 mL), filtered, the filtrate acidified with aq. 2M HCl to form a precipitate that was collected by filtration to afford the title product (200 mg).
LCMS (Method 2): Rt = 0.51 min, ES⁺ *m*/*z* 437.1 [M+H]⁺

### Step 2

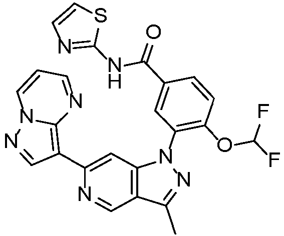

### 4-(Difluoromethoxy)-3-(3-methyl-6-(pyrazolo[1,5-a]pyrimidin-3-yl)-1H-pyrazolo[4,3-c]pyridin-1-yl)-N-(thiazol-2-yl)benzamide (Example 66)

To a mixture of intermediate example 66-step 1 (30.0 mg, 0.07 mmol), thiazol-2-amine (8.26 mg, 0.08 mmol), DIPEA (24 µL, 0.14 mmol) in dry DMF (1 mL), HATU (28.8 mg, 0.08 mmol) was added and RM stirred at 50 °C for 1 h. RM was cooled to RT and diluted with water (5 mL) to form a precipitate that was collected by filtration. The crude was triturated with MeOH to afford the title product (19.5 mg).
LCMS (Method 5): Rt = 3.24 min, ES⁺ *m*/*z* 519.1 [M+H]⁺
¹H-NMR (500 MHz, *DMSO-d₆*) δ:12.86 (br s, 1H), 9.17-9.28 (m, 2H), 8.91 (s, 1H), 8.64-8.69 (m, 1H), 8.47 (d, J=1.8 Hz, 1H), 8.37 (dd, J=8.8, 2.14 Hz, 1H), 8.35 (s, 1H), 7.72 (d, J=8.5 Hz, 1H), 7.56 (d, J=3.4 Hz, 1H), 7.45 (t, J=72.4 Hz, 1H), 7.27-7.32 (m, 1H), 7.15 (dd, J=7.0, 4.0 Hz, 1H), 2.70 (s, 3H)

### Example 67

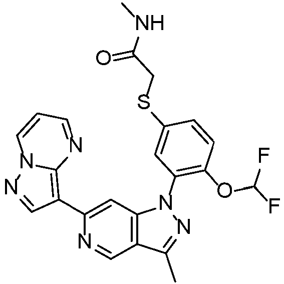

### 2-((4-(Difluoromethoxy)-3-(3-methyl-6-(pyrazolo[1,5-a]pyrimidin-3-yl)-1H-pyrazolo[4,3-c]pyridin-1-yl)phenyl)thio)-N-methylacetamide (Example 67)

To a mixture of intermediate 29 (55.0 mg, 0.11 mmol) in DMF (1 mL), HATU (52.0 mg, 0.14 mmol) and DIPEA (80 µL, 0.46 mmol) were added and RM stirred at RT for 45 min. Methylamine (2M in THF, 171 µL, 0.342 mmol) was added and stirring continued at RT for 1 h. RM was partitioned between EtOAc (8 mL) and water (10 mL), aqueous layer further extracted with EtOAc (8 mL) and combined organic layers washed with sat. aq. NaHCO₃ (2x10 mL), sat. aq. NaCl (10 mL) and evaporated under reduced pressure. The residue was purified by flash chromatography on a Si cartridge by eluting with 0-100 % DCM/MeOH (19:1) in DCM to afford the title product (3.57 mg).
LCMS (Method 5): Rt = 3.57 min, ES⁺ *m*/*z* 496.5 [M+H]⁺
¹H-NMR (600 MHz, *DMSO-d₆*) δ / 9.22-9.26 (m, 1H), 9.17-9.21 (m, 1H), 8.88 (br d, J=1.7 Hz, 1H), 8.68-8.73 (m, 1H), 8.29-8.33 (m, 1H), 8.06-8.12 (m, 1H), 7.66-7.70 (m, 1H), 7.59-7.62 (m, 1H), 7.47-7.52 (m, 1H), 7.18 (t, J=73.0 Hz, 1H), 7.13-7.17 (m, 1H), 3.73 (s, 2H), 2.67 (s, 3H), 2.56 (d, J=4.6 Hz, 3H)

### Example 68

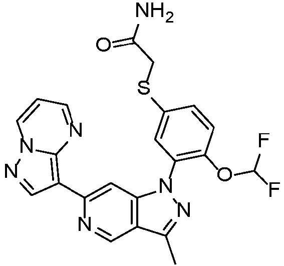

### 2-((4-(Difluoromethoxy)-3-(3-methyl-6-(pyrazolo[1,5-a]pyrimidin-3-yl)-1H-pyrazolo[4,3-c]pyridin-1-yl)phenyl)thio)acetamide (Example 68)

The title compound was prepared in a similar manner to example 67 starting from intermediate 29 and ammonia (0.5 M in 1,4-dioxane).
LCMS (Method 6): Rt = 3.44 min, ES⁺ *m*/*z* 482.6 [M+H]⁺
¹H-NMR (600 MHz, *DMSO-d₆*) δ: 9.22 (dd, J=7.0, 1.3 Hz, 1H), 9.18 (d, J=1.0 Hz, 1H), 8.90 (s, 1H), 8.71 (dd, J=4.0, 1.5 Hz, 1H), 8.32 (d, J=0.9 Hz, 1H), 7.68 (d, J=2.3 Hz, 1H), 7.61 (dd, J=8.7, 2.3 Hz, 1H), 7.60 (bs, 1H), 7.50 (d, J=8.6 Hz, 1H), 7.18 (t, J=73 Hz, 1H), 7.18 (bs, 1H), 7.16 dd, J=7.1, 4.0 Hz, 1H), 3.73 (s, 2H), 2.67 (s, 3H)

### Example 69

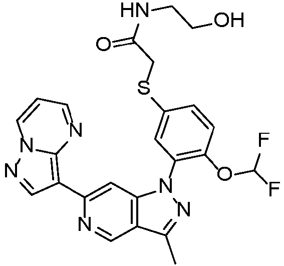

### 2-((4-(difluoromethoxy)-3-(3-methyl-6-(pyrazolo[1,5-a]pyrimidin-3-yl)-1H-pyrazolo[4,3-c]pyridin-1-yl)phenyl)thio)-N-(2-hydroxyethyl)acetamide (Example 69)

The title compound was prepared in a similar manner to example 67 starting from intermediate 29 and ethanolamine.
LCMS (Method 6): Rt = 3.35 min, ES⁺ *m*/*z* 526.5 [M+H]⁺
¹H-NMR (600 MHz, *DMSO-d₆,* 1.3 Hz, 1H), 8.85 (s, 1H), 8.66 (dd, J=3.9, 1.5 Hz, 1H), 8.29 (d, J=0.8 Hz, 1H), 7.82 (bs, 1H), 7.68 (d, J=2.3 Hz, 1H), 7.60 (dd, J=7.6, 2.3 Hz, 1H), 7.46 (d, J=7.6 Hz, 1H), 7.09 (dd, J=7.1, 4.0 Hz, 1H), 7.04 (t, J=73.0 Hz, 1H), 4.31 (bs, 1H), 3.71 (s, 2H), 3.37 (q, J=5.2 Hz, 2H), 3.12 (q, J=5.4 Hz, 2H), 2.66 (s, 3H)

### Example 70

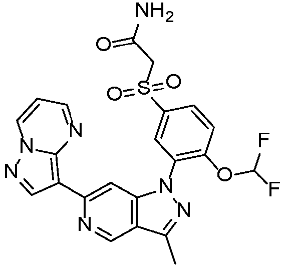

### 2-((4-(Difluoromethoxy)-3-(3-methyl-6-(pyrazolo[1,5-a]pyrimidin-3-yl)-1H-pyrazolo[4,3-c]pyridin-1-yl)phenyl)sulfonyl)acetamide (Example 70)

To a mixture of example 68 (33.0 mg, 0.07 mmol) in EtOH (1.2 mL), a suspension of oxone^{®} (169 mg, 0.27 mol) in water (0.85 mL) was added and RM stirred at 60 °C for 1h. RM was diluted with water (2 mL) and DCM (2 mL) and organic layer concentrated under reduced pressure at RT. The residue was purified by flash chromatography on a Si cartridge by eluting with 0-80 % DCM/MeOH/NH₄OH (90:9:0.5) in DCM to afford the title product (3.6 mg).
LCMS (Method 6): Rt = 3.32 min, ES⁺ *m*/*z* 514.2 [M+H]⁺
¹H-NMR (*DMSO-d₆*, 600 MHz) δ: 9.24 (dd, J=7.0, 1.7 Hz, 1H), 9.22 (d, J=1.1 Hz, 1H), 8.91 (s, 1H), 8.70 (dd, J=4.1, 1.7 Hz, 1H), 8.40 (d, J=1.1 Hz, 1H), 8.16 (d, J=2.4 Hz, 1H), 8.11 (dd, J=8.8, 2.4 Hz, 1H), 7.81 (d, J=8.8 Hz, 1H), 7.65 (s, 1H), 7.52 (t, J=72.0 Hz, 1H), 7.40 (br s, 1H), 7.17 (dd, J=7.0, 4.0 Hz, 1H), 4.39 (s, 2H), 2.70 (s, 3H)

### Example 71

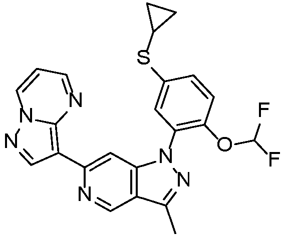

### 1-(5-(cyclopropylthio)-2-(difluoromethoxy)phenyl)-3-methyl-6-(pyrazolo[1,5-a]pyrimidin-3-yl)-1H-pyrazolo[4,3-c]pyridine (Example 71)

Intermediate 31 (20.0 mg, 0.05 mmol), cyclopropyl boronic acid (5.77 mg, 0.07 mmol), copper (II) acetate (8.13 mg, 0.05 mmol), 2,2'-Dipyridyl (6.99 mg, 0.05 mmol) and Cs₂CO₃ (14.6 mg, 0.05 mmol) in DCE (0.5 mL) were stirred at 70 °C overnight. After cooling to RT, RM was diluted with DCM (5 mL), added with aq. ammonia (24%, 5 mL) and stirred at RT for 10 min. Organic layer was separated and washed with aq. ammonia (24%, 5 mL), water (5 mL), sat. aq. NaCl (5 mL) and evaporated under reduced pressure. The residue was purified by flash chromatography on a Si cartridge by eluting with 0-75 % EtOAc in cyclohexane to afford the title product (4.1 mg).
LCMS (Method 5): Rt = 4.12 min, ES⁺ *m*/*z* 465.5 [M+H]⁺
¹H-NMR (400 MHz, *DMSO-d₆*) δ 9.22-9.25 (m, 1H), 9.17 (s, 1H), 8.90 (s, 1H), 8.68 (dd, J=4.1, 1.8 Hz, 1H), 8.35 (d, J=1.2 Hz, 1H), 7.66 (d, J=2.4 Hz, 1H), 7.56-7.59 (m, 1H), 7.48-7.52 (m, 1H), 7.14-7.18 (m, 1H), 7.14 (t, J=73.0 Hz, 1H), 2.65 (s, 3H), 2.37 (ddd, J=7.3, 4.3, 3.1 Hz, 1H), 1.04-1.11 (m, 2H), 0.62-0.67 (m, 2H)

### Example 72

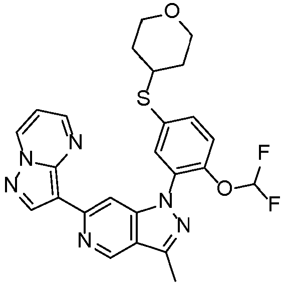

### 1-(2-(Difluoromethoxy)-5-((tetrahydro-2H-pyran-4-yl)thio)phenyl)-3-methyl-6-(pyrazolo[1,5-a]pyrimidin-3-yl)-1H-pyrazolo[4,3-c]pyridine (Example 72)

To a mixture of intermediate 31 (35.0 mg, 0.08 mmol) in DMF (1.5 mL), NaI (12.4 mg, 0.08 mmol), K₂CO₃ (22.8 mg, 0.17 mmol) and 4-bromotetrahydropyran (10.2 µL, 0.09 mmol) were added and RM stirred at RT for 60 h. RM was partitioned between EtOAc (8 mL) and water (8 mL). Organic layer was washed with water (8 mL), sat.aq. NaHCO₃ (8 mL), sat. aq. NaCl (8 mL) and evaporated under reduced pressure. The residue was purified by MDAP preparative HPLC (Method prep 1) to afford the title product (2.5 mg).
LCMS (Method 5): Rt = 3.74 min, ES⁺ *m*/*z* 509.1 [M+H]⁺
¹H-NMR (500 MHz, *DMSO-d₆*) δ: 9.22-9.27 (m, 1H), 9.18 (d, J=1.2 Hz, 1H), 8.90 (s, 1H), 8.65-8.71 (m, 1H), 8.30-8.33 (m, 1H), 7.69-7.73 (m, 1H), 7.66 (dd, J=8.7, 2.3 Hz, 1H), 7.49-7.53 (m, 1H), 7.22 (t, J=73.0 Hz, 1H), 7.17 (dd, J=7.0, 4.3 Hz, 1H), 3.75-3.82 (m, 2H), 3.56-3.65 (m, 1H), 3.34-3.40 (m, 2H), 2.66-2.68 (m, 3H), 1.86-1.94 (m, 2H), 1.48-1.58 (m, 2H)

### Example 73

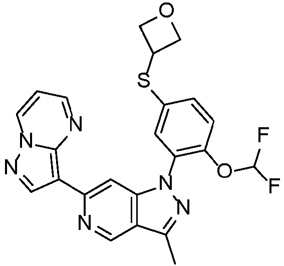

### 1-(2-(Difluoromethoxy)-5-(oxetan-3-ylthio)phenyl)-3-methyl-6-(pyrazolo[1,5-a]pyrimidin-3-yl)-1H-pyrazolo[4,3-c]pyridine (Example 73)

To a mixture of intermediate 31 (33.0 mg, 0.08 mmol) in acetone (3 mL), oxetane-3-yl 4-methylbenzenesulfonate (19.5 mg, 0.09 mmol), NaI (11.7 mg, 0.08 mmol) and K₂CO₃ (21.5 mg, 0.16 mmol) were added. RM was stirred at reflux for 3 h and at RT overnight. RM was partitioned between EtOAc (15 mL) and water (10 mL). Organic layer was washed with sat. aq. NaCl (10 mL) and evaporated under reduced pressure. The residue was purified by MDAP preparative HPLC (Method prep 2) to afford the title product (8 mg).
LCMS (Method 7): Rt = 5.25 min, ES⁺ *m*/*z* 481.1 [M+H]⁺
¹H-NMR (400 MHz, *DMSO-d₆*) δ 9.22 (dd, J=7.0, 1.7 Hz, 1 H), 9.17 (s, 1 H), 8.90 (s, 1 H), 8.66-8.70 (m, 1 H), 8.31 (s, 1 H), 7.53-7.56 (m, 1 H), 7.50 (s, 2 H), 7.18 (t, J=73.0 Hz, 1 H), 7.13-7.17 (m, 1 H), 5.00 (t, J=7.0 Hz, 2 H), 4.67-4.78 (m, 1 H), 4.50 (t, J=6.4 Hz, 2 H), 2.65 (s, 3 H)

### Example 74

### Step 1

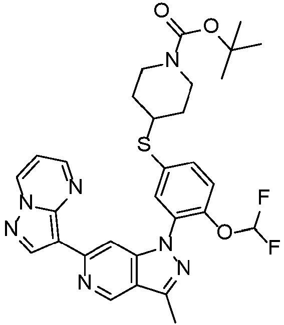

### tert-Butyl 4-((4-(difluoromethoxy)-3-(3-methyl-6-(pyrazolo[1,5-a]pyrimidin-3-yl)-1H-pyrazolo[4,3-c]pyridin-1-yl)phenyl)thio)piperidine-1-carboxylate (Example 74-step 1)

To a mixture of intermediate 31 (85.0 %, 60.0 mg, 0.12 mmol) in acetone (4 mL), *N*-Boc-4-bromopiperidine (38.1 mg, 0.14 mmol), NaI (18.0 mg, 0.12 mmol) and K₂CO₃ (33.2 mg, 0.240 mmol) were added, and RM refluxed for 2.5 h. After cooling to RT, RM was diluted with water (10 mL) to form a precipitate that was collected by filtration to afford the title product (66 mg).
LCMS (Method 2): Rt = 1.38 min, ES⁺ *m*/*z* 608.4 [M+H]⁺

### Step 2

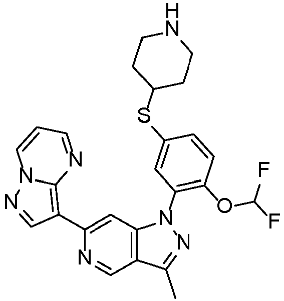

### 1-(2-(Difluoromethoxy)-5-(piperidin-4-ylthio)phenyl)-3-methyl-6-(pyrazolo[1,5-a]pyrimidin-3-yl)-1H-pyrazolo[4,3-c]pyridine (Example 74)

To a mixture of intermediate example 74-step 1 (70.0 %, 66.0 mg, 0.08 mmol) in DCM (2 mL), TFA (113 µL, 1.52 mmol) was added and RM stirred at RT overnight. RM was evaporated under reduced pressure, and the residue partitioned between EtOAc (10 mL) and water (10 mL). Aqueous layer was neutralized with sat. aq. NaHCO₃ (10 mL) and extracted with DCM (2x10 mL). Combined organic layers were washed with sat. aq. NaCl (10 mL), dried over Na₂SO₄ and evaporated under reduced pressure to give a crude that was purified by flash chromatography on a Si cartridge by eluting with 0-30 % DCM/MeOH/NH₄OH (90:9:1.5) in DCM affording the title compound (22 mg).
LCMS (Method 6): Rt = 4.51 min, ES⁺ *m*/*z* 508.5 [M+H]⁺
¹H-NMR (500 MHz, *DMSO-d₆*) δ 9.22-9.27 (m, 1 H), 9.18 (s, 1 H), 8.90 (s, 1 H), 8.67-8.71 (m, 1 H), 8.33 (s, 1 H), 7.67 (d, J=2.1 Hz, 1 H), 7.60-7.65 (m, 1 H), 7.47-7.52 (m, 1 H), 7.21 (t, J=73.0 Hz, 1 H), 7.13-7.18 (m, 1 H), 3.38-3.47 (m, 1 H), 2.83-2.91 (m, 2 H), 2.66 (s, 3 H), 2.41-2.49 (m, 2 H), 1.83-1.92 (m, 2 H), 1.34-1.45 (m, 2 H)

### Example 75

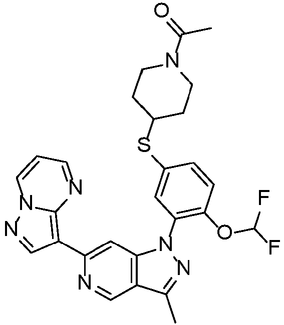

### 1-(4-((4-(difluoromethoxy)-3-(3-methyl-6-(pyrazolo[1,5-a]pyrimidin-3-yl)-1H-pyrazolo[4,3-c]pyridin-1-yl)phenyl)thio)piperidin-1-yl)ethan-1-one (Example 75)

To a mixture of example 74 (14.0 mg, 0.03 mmol) in DCM (0.4 mL), TEA (11.5 µL, 0.08 mmol) was added. RM was cooled in an ice bath followed by the addition of acetic anhydride (3.1 µL, 0.03 mmol), and RM stirred for 2 h. RM was dried under reduced pressure and the residue partitioned between EtOAc (6 mL) and aq. 0.1M HCl. Aqueous layer was neutralized to pH 8 and extracted with EtOAc (2x8 mL). Combined organic layers were washed with sat. aq. NaHCO₃ (2x5 mL), sat. aq. NaCl (5 mL) and evaporated under reduced pressure. The crude material was triturated with EtOAc/hexanes to afford the desired product (10 mg).
LCMS (Method 6): Rt = 4.21 min, ES⁺ *m*/*z* 550.4 [M+H]⁺
¹H-NMR (500 MHz, *DMSO-d₆*) δ 9.22-9.26 (m, 1H), 9.19 (d, J=1.2 Hz, 1H), 8.91 (s, 1H), 8.67-8.72 (m, 1H), 8.33 (s, 1H), 7.72 (d, J=2.1 Hz, 1H), 7.67 (dd, J=8.7, 2.3 Hz, 1H), 7.50-7.54 (m, 1H), 7.23 (t, J=73.0 Hz, 1H), 7.13-7.19 (m, 1H), 4.10-4.16 (m, 1H), 3.68-3.74 (m, 1H), 3.61-3.67 (m, 1H), 3.09-3.17 (m, 1H), 2.77-2.84 (m, 1H), 2.67 (s, 3H), 1.93-2.02 (m, 4H), 1.45-1.54 (m, 1H), 1.31-1.40 (m, 1H), 1.22-1.29 (m, 1H)

### Example 76

### Step 1

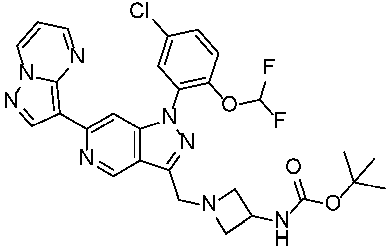

### tert-Butyl (1-((1-(5-chloro-2-(difluoromethoxy)phenyl)-6-(pyrazolo[1,5-a]pyrimidin-3-yl)-1H-pyrazolo[4,3-c]pyridin-3-yl)methyl)azetidin-3-yl)carbamate (Example 76-step 1)

To a mixture of intermediate 27a (60.0 mg, 0.12 mmol), 3-(4,4,5,5-tetramethyl-1,3,2-dioxaborolan-2-yl)pyrazolo[1,5-*a*]pyrimidine (45.7 mg, 0.19 mmol), Cs₂CO₃ (76 mg, 0.23 mmol) in degassed 1,4-dioxane/water (2:1, 1.95 mL), Pd(PPh₃)₄ (13.5mg, 0.01 mmol) was added and RM stirred at 80 °C for 1.5 h under argon atmosphere. After cooling to RT, RM was diluted with EtOAc (15 mL), washed with sat. aq. NaHCO₃ (3x10 mL) and sat. aq. NaCl (10 mL). The organic layer was dried over Na₂SO₄ and concentrated *in vacuo*. The residue was purified by flash chromatography on a Si cartridge by eluting with 0-30 % DCM/MeCN/NH₄OH (10:10:1) in DCM. The material obtained was triturated with acetonitrile to afford the title compound (36 mg).
LCMS (Method 2): Rt = 1.19 min, ES⁺ *m*/*z* 597.2/599.2 [M+H]⁺

### Step 2

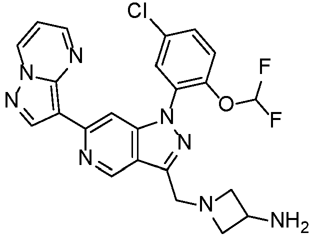

### 1-((1-(5-Chloro-2-(difluoromethoxy)phenyl)-6-(pyrazolo[1,5-a]pyrimidin-3-yl)-1H-pyrazolo[4,3-c]pyridin-3-yl)methyl)azetidin-3-amine (Example 76)

An ice chilled solution of intermediate example 76-step 1 (26.0 mg, 0.0435 mmol) in dry DCM (2 ml) was treated with TFA (100 µl, 1.31 mmol), then RM warmed up to RT and stirred for 3 h. RM was loaded on an SCX cartridge, washed with methanol and eluted with methanolic ammonia (7N). The crude was submitted to a flash chromatography on Si cartridge by eluting with 0-100 % DCM/MeOH/NH₄OH (90:9:1.5) in DCM. The material obtained was triturated with n-hexane/DCM to afford the desired compound (6 mg).
LCMS (Method 4): Rt = 1.57 min, ES⁺ *m*/*z* 497.1/499.1 [M+H]⁺
¹H-NMR (600 MHz, *DMSO-d₆*) δ:9.16-9.35 (m, 2H), 8.83-8.97 (m, 1H), 8.71 (br s, 1H), 8.28-8.40 (m, 1H), 7.82-7.93 (m, 1H), 7.71-7.81 (m, 1H), 7.54-7.66 (m, 1H), 7.06-7.40 (m, 1H), 7.14-7.21 (m, 1H), 3.93-4.03 (m, 2H), 3.49-3.63 (m, 2H), 3.42-3.47 (m, 1H), 2.72-2.90 (m, 2H)

### Example 77

### Step 1

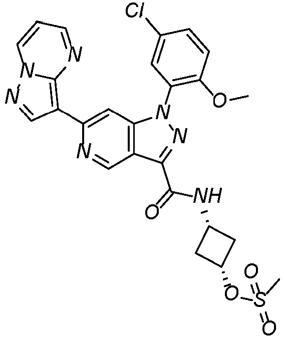

### (1s,3s)-3-(1-(5-Chloro-2-methoxyphenyl)-6-(pyrazolo[1,5-a]pyrimidin-3-yl)-1H-pyrazolo[4,3-c]pyridine-3-carboxamido)cyclobutyl methanesulfonate (Example 77-step 1)

To a cooled mixture at 0 °C of example 34 (240 mg, 0.49 mmol) in DCM (8 mL), methanesulfonyl chloride (49.3 µL, 0.64 mmol) and TEA (205 µL, 0.09 mmol) were added and RM stirred for 1.5 h from 0°C to RT. RM was evaporated under reduced pressure and the crude triturated with water to afford the title product (285 mg).
LCMS (Method 2): Rt = 1.06 min, ES⁺ *m*/*z* 568.1/570.1 [M+H]⁺

### Step 2

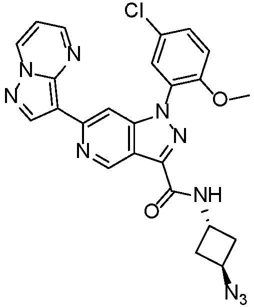

### N-((1r,3r)-3-Azidocyclobutyl)-1-(5-chloro-2-methoxyphenyl)-6-(pyrazolo[1,5-a]pyrimidin-3-yl)-1H-pyrazolo[4,3-c]pyridine-3-carboxamide (Example 77-step 2)

To a mixture of intermediate example 76-step 1 (60.0 mg, 0.11 mmol) in dry DMF (1.5 mL), NaN₃ (13.7 mg, 0.21 mmol) was added and RM stirred at 85 °C overnight. After cooling to RT, RM was diluted with EtOAc (25 mL), then washed with sat.aq. NaHCO₃ (3x15 mL) and sat. aq. NaCl (15 mL). Organic layer was dried over Na₂SO₄ and concentrated *in vacuo* to provide the title product (55 mg) that was used in the next steps without further purification.
LCMS (Method 2): Rt = 1.23 min, ES⁺ *m*/*z* 515.2/517.1 [M+H]⁺.

### Step 3

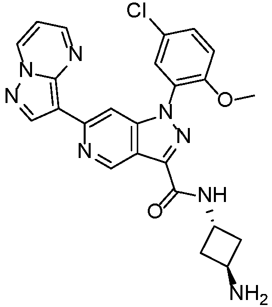

### N-((1r,3r)-3-Aminocyclobutyl)-1-(5-chloro-2-methoxyphenyl)-6-(pyrazolo[1,5-a]pyrimidin-3-yl)-1H-pyrazolo[4,3-c]pyridine-3-carboxamide (Example 77)

Triphenylphosphine (84.0 mg, 0.32 mmol) was added to a solution of intermediate example 77-step 2 (55.0 mg, 0.11 mmol) in THF/water 15:1 (1.6 mL). RM was stirred at RT for 4 h. RM was loaded on a SCX cartridge, washed with MeOH and eluted with methanolic ammonia (7M). Pooled fractions containing the product were evaporated and submitted to flash chromatography purification on a Si cartridge by eluting with 0-65 % DCM/MeOH/NH₄OH (90:15:1.5) in DCM to afford the title product (16 mg).
LCMS (Method 5): Rt =2.82 min, ES⁺ *m*/*z* 489.1/491.3 [M+H]⁺
¹H-NMR (300 MHz, *DMSO-d₆*) δ: 9.46 (s, 1H), 9.23 (d, J=7.0 Hz, 1H), 8.91 (s, 1H), 8.87 (d, J=7.5 Hz, 1H), 8.67-8.75 (m, 1H), 8.31 (s, 1H), 7.80 (d, J=1.9 Hz, 1H), 7.69 (dd, J=8.7, 2.1 Hz, 1H), 7.45 (d, J=8.9 Hz, 1H), 7.17 (dd, J=6.9, 4.1 Hz, 1H), 4.53-4.69 (m, 1H), 3.85 (s, 3H), 3.46-3.56 (m, 1H), 2.28-2.41 (m, 2H), 1.91-2.09 (m, 2H).

### Example 78

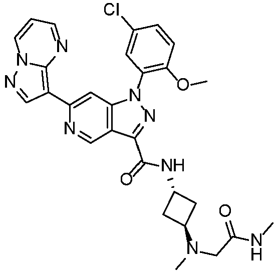

### 1-(5-chloro-2-methoxyphenyl)-N-((1r,3r)-3-(methyl(2-(methylamino)-2-oxoethyl)amino)cyclobutyl)-6-(pyrazolo[1,5-a]pyrimidin-3-yl)-1H-pyrazolo[4,3-clpyridine-3-carboxamide (Example 78)

Intermediate example 77-step 1 (30.0 mg, 0.05 mmol), N-methyl-2-(methylamino)acetamide (0.5 mL, 4.57 mmol) and DMAP (1.29 mg, 0.01 mmol) in DMSO (100 µL) were reacted at 120 °C under MW irradiation for 3 h. After cooling to RT, RM was diluted with EtOAc (10 mL), washed with sat.aq. NaHCO₃ (3x5 mL) and sat. aq. NaCl (5 mL). Organic layer was dried over Na₂SO₄ and concentrated *in vacuo* to provide a crude product that was chromatographed on a Si cartridge by eluting with 0-100% DCM/MeCN/MeOH (10:10:2) in DCM to afford the title product (2 mg).
LCMS (Method 5): Rt =2.89 min, ES⁺ *m*/*z* 574.2/576.1 [M+H]⁺
¹H-NMR (300 MHz, *DMSO-d₆,* 1H), 8.59-8.80 (m, 2H), 8.32 (s, 1H), 7.73-7.80 (m, 1H), 7.65-7.71 (m, 1H), 7.49-7.64 (m, 1H), 7.46 (br d, J=8.9 Hz, 1H), 7.10-7.18 (m, 1H), 4.47-4.58 (m, 1H), 3.88 (s, 3H), 3.18-3.23 (m, 1H), 2.82-2.90 (m, 2H), 2.64-2.70 (m, 3H), 2.23-2.34 (m, 4H), 2.13-2.21 (m, 3H)

### Example 79

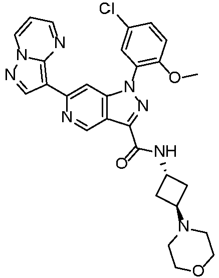

### 1-(5-Chloro-2-methoxyphenyl)-N-((1r,3r)-3-morpholinocyclobutyl)-6-(pyrazolo[1,5-a]pyrimidin-3-yl)-1H-pyrazolo[4,3-c]pyridine-3-carboxamide (Example 79)

The title product was prepared in a similar manner to example 78 starting from intermediate example 77-step 1 and morpholine.
LCMS (Method 5): Rt =2.93 min, ES⁺ *m*/*z* 559.2/561.1 [M+H]⁺
¹H-NMR (300 MHz, *DMSO-d₆*) δ: 9.46 (d, J=1.2 Hz, 1H), 9.24 (dd, J=6.9, 1.7 Hz, 1H), 8.97 (d, J=7.3 Hz, 1H), 8.91 (s, 1H), 8.72 (dd, J=4.0, 1.5 Hz, 1H), 8.31 (d, J=0.9 Hz, 1H), 7.81 (d, J=2.7 Hz, 1H), 7.70 (dd, J=9.0, 2.6 Hz, 1H), 7.46 (d, J=9.2 Hz, 1H), 7.17 (dd, J=6.9, 4.1 Hz, 1H), 4.50 (sxt, J=7.2 Hz, 1H), 3.86 (s, 3H), 3.60 (br t, J=4.3 Hz, 4H), 2.77-2.87 (m, 1H), 2.18-2.34 (m, 8H)

### Example 80

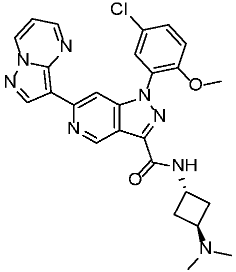

### 1-(5-Chloro-2-methoxyphenyl)-N-((1r,3r)-3-(dimethylamino)cyclobutyl)-6-(pyrazolo[1,5-a]pyrimidin-3-yl)-1H-pyrazolo[4,3-c]pyridine-3-carboxamide (Example 80)

Example 77 (12.0 mg, 0.0245 mmol) was dissolved in a mixture of formic acid (254 µl, 6.72 mmol) / aq. formaldehyde (37.0 %, 500 µl, 6.72 mmol) and stirred at 60 °C for 4 h. RM was diluted with EtOAc (15 mL), washed with sat.aq. NaHCO₃ (3x15 mL), sat. aq. NaCl (15 mL), dried over Na₂SO₄ and evaporated under reduced pressure. The residue was purified by flash chromatography on a Si cartridge by eluting with 0-65% DCM/MeOH/NH₄OH (90:9:1.5) in DCM to afford the title product (10 mg).
LCMS (Method 5): Rt =2.91 min, ES⁺ *m*/*z* 517.2/519.0 [M+H]⁺
¹H-NMR (300 MHz, *DMSO-d₆*) δ:9.46 (s, 1H), 9.23 (dd, J=6.9, 1.3 Hz, 1H), 8.95 (d, J=7.1 Hz, 1H), 8.91 (s, 1H), 8.67-8.78 (m, 1H), 8.31 (s, 1H), 7.81 (d, J=2.4 Hz, 1H), 7.70 (dd, J=8.8, 2.5 Hz, 1H), 7.45 (d, J=9.1 Hz, 1H), 7.17 (dd, J=6.8, 4.2 Hz, 1H), 4.38-4.53 (m, 1H), 3.85 (s, 3H), 2.67-2.81 (m, 1H), 2.22 (br t, J=6.4 Hz, 4H), 2.06 (s, 6H)

### Example 81

### Step 1

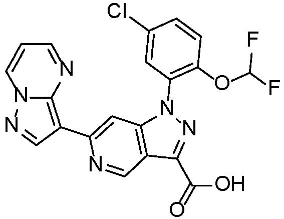

### 1-(5-Chloro-2-(difluoromethoxy)phenyl)-6-(pyrazolo[1,5-a]pyrimidin-3-yl)-1H-pyrazolo[4,3-c]pyridine-3-carboxylic acid (Example 81-step 1)

To a mixture of intermediate 23c (100 mg, 0.27 mmol), 3-(4,4,5,5-tetramethyl-1,3,2-dioxaborolan-2-yl)pyrazolo[1,5-a]pyrimidine (105 mg, 0.43 mmol) and Cs₂CO₃ (174 mg, 0.54 mmol) in a degassed mixture of dioxane/water (2:1, 4.5 mL), Pd(PPh₃)₄ (30.9 mg, 0.03 mmol) was added and RM stirred at 80 °C overnight under argon. After cooling to RT, RM was diluted with EtOAc and extracted with water. Aqueous layer was acidified with aq. 2M HCl and the formed precipitate collected by filtration. The crude material was purified by flash chromatography on a Si cartridge by eluting with DCM/MeOH/formic acid (90:5:0. 3) in DCM to afford the title product (15 mg).
LCMS (Method 2): Rt = 0.57 min, ES⁺ *m*/*z* 456.9/458.9 [M+H]⁺

### Step 2

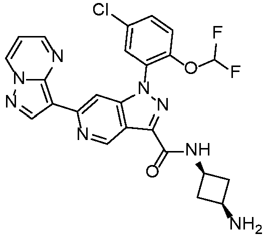

### N-((1s,3s)-3-aminocyclobutyl)-1-(5-chloro-2-(difluoromethoxy)phenyl)-6-(pyrazolo[1,5-a]pyrimidin-3-yl)-1H-pyrazolo[4,3-c]pyridine-3-carboxamide (Example 81)

To a mixture of intermediate example 81-step 1 (15.0 mg, 32.8 µmol), *tert*-butyl *N-*(3-aminocyclobutyl) carbamate (7.34 mg, 39.4 µmol) and DIPEA (12 µL, 65 µmol) in dry DMF (1 mL), HATU (14 mg, 36.1 µmol) was added and RM stirred at 50 °C for 1 h. After cooling to RT, RM was diluted with EtOAc (15 mL), washed with sat. aq. NaHCO₃ (3x10 mL) and sat. aq. NaCl (10 mL). Organic layer was dried over Na₂SO₄ and concentrated *in vacuo.* The residue was purified by flash chromatography on a Si cartridge by eluting with 0-40 % DCM/MeCN/MeOH (10:10:1) in DCM. The material obtained was dissolved in DCM (2 mL) and cooled in an ice-bath prior to dropwise addition of TFA (122 µl, 50 eq). RM was allowed to warm to RT over 2 h and then loaded on a SCX cartridge, washed with MeOH and eluted with methanolic ammonia (1.5 N) to afford the title product (10 mg).
LCMS (Method 5): Rt = 2.88 min, ES⁺ *m*/*z* 525.1/527.1 [M+H]⁺
¹H-NMR (500 MHz, *DMSO-d₆*) δ:9.50 (s, 1H), 9.25 (br d, J=6.7 Hz, 1H), 8.93 (s, 1H), 8.76 (br d, J=7.9 Hz, 1H), 8.72 (br d, J=3.1 Hz, 1H), 8.38 (s, 1H), 8.04 (d, J=2.1 Hz, 1H), 7.84 (dd, J=9.0, 2.3 Hz, 1H), 7.65 (d, J=9.1 Hz, 1H), 7.28 (t, J=72.5 Hz, 1H), 7.18 (dd, J=6.9, 4.1 Hz, 1H), 4.04-4.16 (m, 1H), 3.05 (quin, J=7.8 Hz, 1H), 2.53-2.62 (m, 2H), 1.82-1.94 (m, 2H)

### Example 82

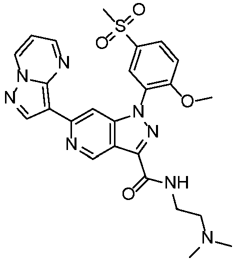

### N-(2-(Dimethylamino)ethyl)-1-(2-methoxy-5-(methylsulfonyl)phenyl)-6-(pyrazolo[1,5-a]pyrimidin-3-yl)-1H-pyrazolo[4,3-c]pyridine-3-carboxamide (Example 82)

Intermediate 25 (40.0 mg, 0.08 mmol), DABAL-Me3 (32.1 mg, 0.13 mmol),THF (2 mL) and *N',N'*-dimethylethane-1,2-diamine (13.7 µL, 0.13 mmol) were heated at 130 °C under MW irradiation for 10 min in nitrogen atmosphere. A second equivalent of DABAL-Me3 (32.1 mg, 0.125 mmol) was added and RM further heated for 10 min under the same conditions. RM was carefully quenched with aq. 1M HCl (2 mL) and washed with DCM (10 mL). Aqueous layer was brought to pH 9.4 using aq. 2M NaOH and extracted with DCM (5 x 5 mL). Combined organic layers were passed through a phase separator and evaporated to dryness. The crude was purified by flash chromatography on a Si cartridge by eluting with 0-75 % DCM/MeOH/NH₄OH (90:9:0.5) in DCM to afford the title compound (25 mg).
LCMS (Method 5): Rt = 2.26 min, ES⁺ *m*/*z* 535.3 [M+H]⁺
¹H-NMR (600 MHz, *DMSO-d₆*) δ: 9.52 (d, J=1.9 Hz, 1H); 9.25 (dd, J=6.9, 1.7 Hz, 1H); 8.93 (s, 1H); 8.72 (dd, J=4.1, 1.8 Hz, 1H); 8.59 (t, J=5.7 Hz, 1H); 8.34 (d, J=0.9 Hz, 1H); 8.22 (d, J=2.3 Hz, 1H); 8.18 (dd, J=8.8, 2.3 Hz, 1H); 7.69 (d, J=8.8 Hz, 1H); 7.18 (dd, J=6.9, 4.1 Hz, 1H); 3.98 (s, 3H); 3.45 (q, 6.5 Hz, 2H); 3.31 (s, 3H); 2.45 (t, J=6.8 Hz, 2H); 2.20 (s, 6H)

### Example 83 to 84

The following examples were prepared in a similar manner to example 82 from the indicated starting materials.

| **Example** | Structure/name | Starting material | NMR | LCMS |
|---|---|---|---|---|
| Example 83 | 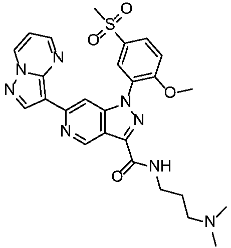 *N*-(3-(dimethylamino)propyl)-1-(2-methoxy-5-(methylsulfonyl)phenyl)-6-(pyrazolo[1,5-*a*]pyrimidin-3-yl)-1*H*-pyrazolo[4,3-c]pyridine-3-carboxamide | Intermediate 25 / *N',N'-*dimethylpropane-1,3-diamine | ¹H-NMR (600 MHz, *DMSO-d₆*) δ: 9.52 (d, J=1.3 Hz, 1H), 9.25 (dd, J=7.1, 1.7 Hz, 1H), 8.90-8.94 (m, 2H), 8.72 (dd, J=4.1, 1.7 Hz, 1H), 8.37 (d, J=1.3 Hz, 1H), 8.22 (d, J=2.2 Hz, 1H), 8.18 (dd, J=8.9, 2.3 Hz, 1H), 7.69 (d, J=9.0 Hz, 1H), 7.19 (dd, J=7.0, 4.0 Hz, 1H), 4.00 (s, 3H), 3.35-3.41 (m, 2H), 3.30-3.31 (m, 3H), 2.30 (t, J=6.9 Hz, 2H), 2.15 (s, 6H), 1.72 (quin, J=7.1 Hz, 2H). | (Method 6): Rt = 3.46 min, ES⁺ *m*/*z* 549.3 [M+H]⁺ |
| Example 84 | 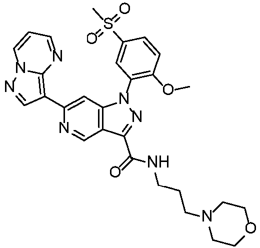 1-(2-methoxy-5-(methylsulfonyl)phenyl)-*N*-(3-morpholinopropyl)-6-(pyrazolo[1,5-*a*]pyrimidin-3-yl)-1*H*-pyrazolo[4,3-c]pyridine-3-carboxamide | Intermediate 25 / 3-morpholinopropan-1-amine | ¹H-NMR (500 MHz, *DMSO-d₆*) δ: 9.52 (d, J=1.2 Hz, 1H), 9.25 (dd, J=7.0, 1.8 Hz, 1H), 8.98 (t, J=5.6 Hz, 1H), 8.93 (s, 1H), 8.72 (dd, J=4.1, 1.7 Hz, 1H), 8.35 (d, J=1.2 Hz, 1H), 8.21 (d, J=2.4 Hz, 1H), 8.17-8.20 (m, 1H), 7.69 (d, J=8.9 Hz, 1H), 7.18 (dd, J=7.0, 4.3 Hz, 1H), 3.98 (s, 3H), 3.56 (t, J=4.6 Hz, 4H), 3.39-3.44 (m, 2H), 3.29-3.30 (m, 3H), 2.33-2.41 (m, 6H), 1.75 (quin, J=6.7 Hz, 2H). | (Method 6): Rt = 3.55 min, ES⁺ *m*/*z* 591.4 [M+H]⁺ |

### Example 85

### Step 1

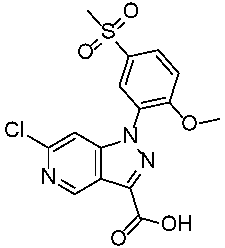

### 6-Chloro-1-(2-methoxy-5-(methylsulfonyl)phenyl)-1H-pyrazolo[4,3-c]pyridine-3-carboxylic acid (Example 85-step 1)

To a mixture of intermediate 24c (543 mg, 1.37 mmol) in THF (11.3 mL), a solution of LiOH (165 mg, 6.86 mmol) in water (3.75 mL) was added and RM stirred at RT overnight. RM was concentrated *in vacuo.* The residue was taken in water and pH adjusted to 2.5 using aq.1M HCl to form a precipitate that was collected by filtration, washed with water and dried to afford the title product (485 mg).
LCMS (Method 2): Rt = 0.44, ES⁺ *m*/*z* 382.0/383.9 [M+H]⁺

### Step 2

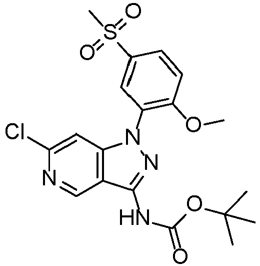

### tert-butyl (6-chloro-1-(2-methoxy-5-(methylsulfonyl)phenyl)-1H-pyrazolo[4,3-c]pyridin-3-yl)carbamate (Example-85-step 2)

Intermediate example 85-step 1 (330 mg, 0.86 mmol) was dissolved in t-BuOH (15 mL) and TEA (361 µL, 2.6 mmol) and refluxed for 30 min. RM was allowed to cool to RT, added with DPPA (279 µL, 1.3 mmol) and refluxed for 10 h. RM was concentrated under reduced pressure and the residue purified by flash chromatography on a Si cartridge by eluting with 0-100 % EtOAc in cyclohexane. The isolated material was triturated with ethyl ether to afford the title product (224 mg).
LCMS (Method 2): Rt = 1.04, ES⁺ *m*/*z* 453.1/455.1 [M+H]⁺

### Step 3

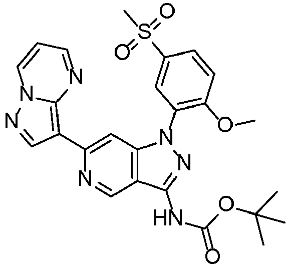

### tert-Butyl (1-(2-methoxy-5-(methylsulfonyl)phenyl)-6-(pyrazolo[1,5-a]pyrimidin-3-yl)-1H-pyrazolo[4,3-c]pyridin-3-yl)carbamate (Example 85-step 3)

To a degassed mixture of intermediate example 85-step 2 (224 mg, 0.50 mmol), 3-(4,4,5,5-tetramethyl-1,3,2-dioxaborolan-2-yl)pyrazolo[1,5-*a*]pyrimidine (182 mg, 0.742 mmol) and K₃PO₄ (262 mg, 1.24 mmol) in a water (4.4 mL)/ THF (8.8 mL) mixture, XPhos PdG3 (25 mg, 0.06 mmol) was added and RM stirred at 75 °C for 2 h under argon. After cooling to RT, RM was diluted with water (15 mL) and sat. aq. NaHCO₃ (15 mL) and extracted with DCM (4x15 mL). Combined organic layers were washed with sat. aq. NaCl, dried over Na₂SO₄ and concentrated *in vacuo.* The residue was purified by flash chromatography on a Si cartridge by eluting with 0-100 % DCM/MeOH/NH₄OH (90:40:1) in DCM to afford the title product (45 mg).
LCMS (Method 2): Rt = 1.01, ES⁺ *m*/*z* 536.1 [M+H]⁺

### Step 4

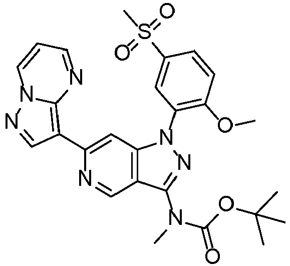

### tert-Butyl (1-(2-methoxy-5-(methylsulfonyl)phenyl)-6-(pyrazolo[1,5-a]pyrimidin-3-yl)-1H-pyrazolo[4,3-c]pyridin-3-yl)(methyl)carbamate (Example 85-step 4)

To a mixture of intermediate 85-step 3 (45.0 mg, 0.08 mmol) in dry DMF (1.5 mL), NaH, (60 % dispersion in mineral oil, 3.4 mg, 0.08 mmol) was added at 0 °C. RM was stirred at 0 °C for 1 h, then iodomethane (5.75µl, 0.09 mmol) was added. RM was allowed to warm to RT. After stirring for 2h, RM was quenched with water and extracted with EtOAc (3x). Combined organic layers were washed with sat. aq. NaCl, dried and concentrated *in vacuo.* The residue was purified by flash chromatography on a Si cartridge by eluting with 0-100 % EtOAc in cyclohexane to afford the title product (30 mg).
LCMS (Method 2): Rt = 1.07, ES⁺ *m*/*z* 550.9 [M+H]⁺

### Step 5

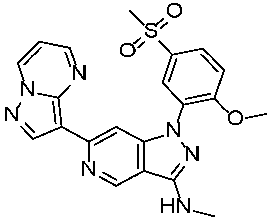

### 1-(2-Methoxy-5-(methylsulfonyl)phenyl)-N-methyl-6-(pyrazolo[1,5-a]pyrimidin-3-yl)-1H-pyrazolo[4,3-c]pyridin-3-amine (Example 85)

To a mixture of intermediate example 85-step 4 (30.0 mg, 0.05 mmol) in DCM (3 mL), TFA (243 µL, 3.28 mmol) was added and RM stirred at RT overnight. RM was dried under reduced pressure and the residue taken in MeOH, loaded onto an SCX cartridge, washed with MeOH and eluted with 2M methanolic ammonia. The material obtained was submitted to flash chromatography on a Si cartridge by eluting with 0-100 % DCM/MeOH/NH₄OH (90:9:1.5) in DCM to afford the title product (15 mg).
LCMS (Method 7): Rt = 3.74 min, ES⁺ *m*/*z* 450.0 [M+H]⁺
¹HNMR (500 MHz, *DMSO-d₆*) δ: 9.21-9.23 (m, 1H); 9.06 (d, J=0.6 Hz, 1H); 8.86 (s, 1H); 8.71 (dd, J=4.1 Hz, J=1.7 Hz, 1H); 8.18 (d, J=0.9 Hz, 1H); 8.01 (d, J=2.4 Hz, 1H); 7.97 (dd, J=8.9 Hz, J=2.4 Hz, 1H); 7.58 (d, J=8.9 Hz, 1H); 7.16 (dd, J=7.0 Hz, J=4.1 Hz, 1H); 6.91 (q, J=4.8 Hz, 1H); 4.02 (s, 3H); 3.26 (s, 3H); 2.95 (d, J=5.2 Hz, 3H)

### Example 86

### Step 1

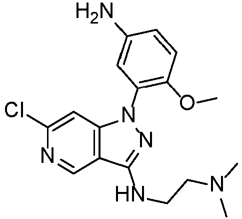

### N¹-(1-(5-amino-2-methoxyphenyl)-6-chloro-1H-pyrazolo[4,3-c]pyridin-3-yl)-N²,N²-dimethylethane-1,2-diamine (Example 86-step 1)

Intermediate 18o (77 mg, 0.12 mmol) in EtOH (1.8 mL), ammonium formate (46 mg, 0.73 mmol) and Pt/C (3% on activated carbon, sulfided, 50 %, 9.5 mg, 0.02 mmol) were refluxed for 2 h. RM was diluted with DCM, filtered through a diacemateous earth pad and washed with DCM. The filtrate was evaporated and the residue partitioned between water and DCM. Aqueous layer was further extracted with DCM (2 × 15 mL). Combined organic layers were washed with sat. aq. NaCl, dried over Na₂SO₄ and evaporated under reduced pressure. The residue was purified by flash chromatography on a Si cartridge by eluting with 0-100 % DCM/MeOH/NH₄OH (90:9:0.5) in DCM to afford the title compound (18 mg).
LCMS (Method 2): Rt = 0.83, ES⁺ *m*/*z* 361.1/363.1 [M+H]⁺

### Step 2

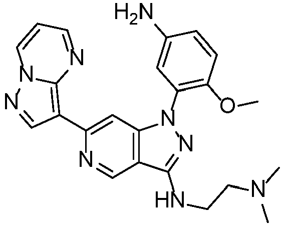

### N¹-(1-(5-Amino-2-methoxyphenyl)-6-(pyrazolo[1,5-a]pyrimidin-3-yl)-1H-pyrazolo[4,3-c]pyridin-3-yl)-N²,N²-dimethylethane-1,2-diamine (Example 86-step 2)

The title product was prepared on a similar manner as example 85-step 3 starting from intermediate example 86-step 1.
LCMS (Method 1): Rt = 0.76, ES⁺ *m*/*z* 443.9 [M+H]⁺

### Step 3

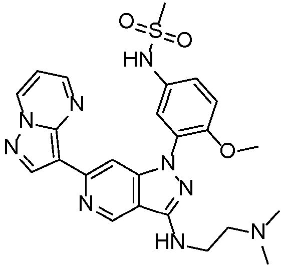

### N-(3-(3-((2-(Dimethylamino)ethyl)amino)-6-(pyrazolo[1,5-a]pyrimidin-3-yl)-1H-pyrazolo[4,3-c]pyridin-1-yl)-4-methoxyphenyl)methanesulfonamide (Example 86)

To a cooled (at 0 °C) mixture of intermediate example 86-step 2 (14 mg, 0.03 pyridine (2.8 mg, 0.03 mmol) followed by methanesulfonyl chloride (2.2 µL, 0.03 mmol) were added. RM was stirred at 0°C for 15 min and at RT overnight. RM was diluted with sat. aq. NaHCO₃ and extracted with DCM (4x15 mL). Combined organic layers were washed with sat. aq. NaCl, dried over Na₂SO₄ and solvent removed under reduced pressure. The residue was purified by flash chromatography on Si cartridge by eluting with 0-100 % DCM/MeOH/NH₄OH (90:9:1.5) in DCM to afford the title product (5 mg).
LCMS (Method 7): Rt = 3.14 min, ES⁺ *m*/*z* 522.2 [M+H]⁺
¹H-NMR (600 MHz, *DMSO-d₆*) δ: 9.61 (bs, 1H), 9.21 (dd, J=7.1, 1.7 Hz, 1H), 9.09 (d, J=1.0 Hz, 1H), 8.86 (s. 1H), 8.70 (dd, J=4.0, 1.7 Hz, 1H), 8.15 (d, J=1.0 Hz, 1H), 7.37 (d, J=2.6 Hz, 1H), 7.31 (d, J=8.9 Hz, 1H), 7.28 (dd, J=8.9, 2.6 Hz, 1H), 7.15 (dd, J=7.1, 4.1 Hz, 1H), 6.74 (t, J=5.6 Hz, 1H), 3.87 (s, 3H), 3.42 (q, J=6.1 Hz, 2H), 2.98 (s, 3H), 2.60-2.56 (m, 2H), 2.24 (s, 6H).

### Example 87

### Step 1

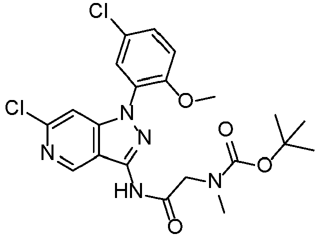

### tert-Butyl (2-((6-chloro-1-(5-chloro-2-methoxyphenyl)-1H-pyrazolo[4,3-c]pyridin-3-yl)amino)-2-oxoethyl)(methyl) (Example 87-step 1)

A suspension of BOC-Sarcosine (42.8 mg, 0.23 mmol) and EEDQ (56.0 mg, 0.23 mmol) in DCE (1 mL) was stirred for 10 min at RT. Intermediate 18l (35.0 mg, 0.11 mmol) in DCE (2 mL) was added and RM was stirred at 80°C overnight. RM was diluted with DCM and washed with sat. aq. NaHCO₃ (2x5 mL). Organic layer was dried over Na₂SO₄ and concentrated to give a residue that was purified by flash chromatography on a Si cartridge by eluting with 0.100 % DCM/MeOH (20:1) in DCM. The material obtained was triturated with diethyl ether and cyclohexane to afford the title product (29 mg).
LCMS (Method 2): Rt = 1.20 min, ES⁺ *m*/*z* 479.1/481.0

### Step 2

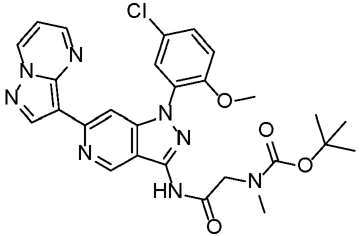

### tert-butyl (2-((1-(5-Chloro-2-methoxyphenyl)-6-(pyrazolo[1,5-a]pyrimidin-3-yl)-1H-pyrazolo[4,3-c]pyridin-3-yl)amino)-2-oxoethyl)(methyl)carbamate (Example 87-step 2)

A mixture of 1,4-dioxane/water (2:1, 0.9 mL) and Pd(PPh₃)₄ (6.98 mg, 6.0 µmol) were added to a vial charged with intermediate example 87-step 1 (29.0 mg, 0.06 mmol), 3-(4,4,5,5-tetramethyl-1,3,2-dioxaborolan-2-yl)pyrazolo[1,5-a]pyrimidine (22.2 mg, 0.09 mmol) and Cs₂CO₃ (39.3 mg, 0.12 mmol). RM was stirred at 80 °C for 2 h under argon. After cooling to RT, RM was diluted with EtOAc (15 mL), then washed with sat.aq. NaHCO₃ (3x5 mL) and sat. aq. NaCl (5 mL). Organic layer was dried over Na₂SO₄ and concentrated *in vacuo.* The residue was purified by flash chromatography on a Si cartridge by eluting with 0-10 % MeOH in EtOAc to afford the title product (15 mg).
LCMS (Method 2): Rt = 1.12 min, ES⁺ *m*/*z* 563.3/565.2.

### Step 3

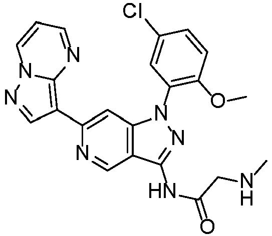

### N-(1-(5-chloro-2-methoxyphenyl)-6-(pyrazolo[1,5-a]pyrimidin-3-yl)-1H-pyrazolo[4,3-c]pyridin-3-yl)-2-(methylamino)acetamide (Example 87)

A solution of intermediate 87-step 2 (15.0 mg, 26.6 µmol) in DCE (0.5 mL) was treated with TFA (69.3 µL, 0.93 mmol) and RM stirred at RT for 1 h. RM was evaporated under reduced pressure and the residue purified by flash chromatography on a Si cartridge by eluting with 0-100 % DCM/MeOH/NH₄OH (90:1:0.1), to afford the title product (11.5 mg).
LCMS (Method 3): Rt = 0.97 min, ES⁺ *m*/*z* 462.9 [M+H]⁺
¹H-NMR (300 MHz, *DMSO-d₆*) δ: 9.41 (d, J=1.0 Hz, 1H), 9.23 (dd, J=7.1, 1.7 Hz, 1H), 8.88 (s, 1H), 8.71 (dd, J=4.1, 1.7 Hz, 1H), 8.22 (d, J=1.0 Hz, 1H), 7.67 - 7.56 (m, 2H), 7.45 - 7.39 (m, 1H), 7.16 (dd, J=7.0, 4.0 Hz, 1H), 6.73 (br s, 1H), 3.86 (s, 3H), 3.43 (s, 2H), 2.38 (s, 3H).

### Example 88

### Step 1

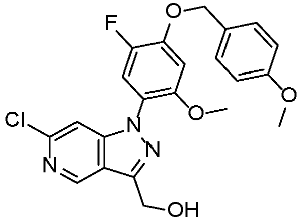

### (6-Chloro-1-(5-fluoro-2-methoxy-4-((4-methoxybenzyl)oxy)phenyl)-1H-pyrazolo[4,3-c]pyridin-3-yl)methanol (Example 88-step 1)

Intermediate 37 (193 mg, 1.05 mmol), intermediate 13a (538 mg, 1.58 mmol), K₂CO₃ (291 mg, 2.10 mmol), copper(I)iodide (100 mg, 0.526 mmol) and DMCHA (166 µL 1.05 mmol) in DMF (2.9 mL) were stirred at 100°C for 10 h under argon atmosphere. After cooling to RT, RM was diluted with EtOAc and washed multiple times with aqueous ammonia (1M). The organic layer wasdried over Na₂SO₄ and evaporated to dryness. The residue was cromatographed on a silica gel column by eluting with 0-50 % EtOAc in DCM to afford the title product was (108 mg).
LCMS (Method 2): Rt = 1.02 min, ES⁺ *m*/*z* 444.1/446.1 [M+H]⁺

### Step 2

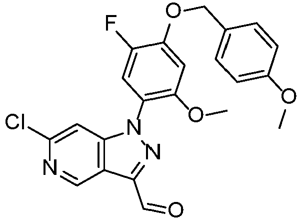

### 6-Chloro-1-(5-fluoro-2-methoxy-4-((4-methoxybenzyl)oxy)phenyl)-1H-pyrazolo[4,3-c]pyridine-3-carbaldehyde (Example 88-step 2)

DMP (115 mg, 0.272 mmol) was added to a suspension of intermediate example 88-step 1 (107 mg, 0.23 mmol) in DCM (10 mL). RM was stirred at RT for 1 h, then quenched with a mixture of sat. aq. Na₂S₂O₃ / sat. aq. NaHCO₃ (1:1, 10 mL) and stirred for further 30 min. Organic layer was dried over Na₂SO₄ and solvent removed under reduced pressure to afford the title product (102 mg) that was used in the next steps without further purification.
LCMS (Method 2): Rt = 1.33, ES⁺ *m*/*z* 442.1/444.1 [M+H]⁺

### Step 3

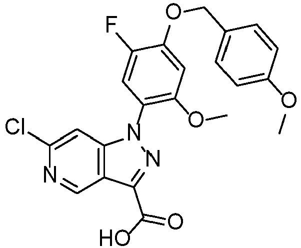

### 6-Chloro-1-(5-fluoro-2-methoxy-4-((4-methoxybenzyl)oxy)phenyl)-1H-pyrazolo[4,3-c]pyridine-3-carboxylic acid (Example 88-step 3)

A solution of NaClO₂ (207 mg, 2.3 mmol), NaH₂PO₄ (274 mg, 32.3 mmol) in water (0.61 mL) was added to a solution of intermediate example 88- step 2 (101 mg, 0.23 mmol) in THF (3.7 mL), then 2-Methyl-2-butene (1.1 mL, 10 mmol) was added and RM stirred at 40°C overnight. After cooling to RT, RM was concentrated under reduced pressure and diluted with water. The pH of aqueous mixture was adjusted to 3 using aq. 1N HCl to form a precipitate was collected by filtration, washed with water and dried to afford the desired product (104 mg) that was used in the next step without further purification.
LCMS (Method 2): Rt = 0.72, ES⁺ *m*/*z* 458.1/460.0 [M+H]⁺

### Step 4

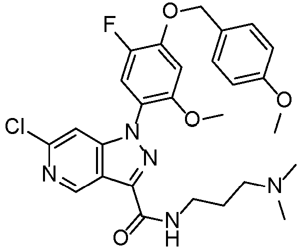

### 6-Chloro-N-(3-(dimethylamino)propyl)-1-(5-fluoro-2-methoxy-4-((4-methoxybenzyl)oxy)phenyl)-1H-pyrazolo[4,3-c]pyridine-3-carboxamide (Example 88-step 4)

To a solution of intermediate example 88-step 3 (72.0 mg, 0.14 mmol), *N',N'-*dimethylpropane-1,3-diamine (34.0 µL, 0.27 mmol) and DIPEA (70.7 µL, 0.41 mmol) in dry DMF (0.7 mL), HATU (56.6 mg, 0.15 mmol) was added. RM was stirred at 60°C for 1 h. A second equivalent of of HATU (56.6 mg, 0.15 mmol) was added and RM stirred for further 50 min . RM was diluted with EtOAc and washed with sat. aq. NH₄Cl, sat. aq. NaHCO₃, water and sat. aq. NaCl. Organic layer was dried over Na₂SO₄ and evaporated to dryness. The residue was purified by flash chromatography on a Si cartridge by eluting with 5 to 10% of MeOH in DCM to afford the title product (42mg).
LCMS (Method 2): Rt = 1.28, ES⁺ *m*/*z* 542.3/544.2 [M+H]⁺

### Step 5

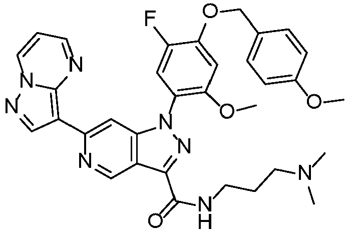

### N-(3-(Dimethylamino)propyl)-1-(5-fluoro-2-methoxy-4-((4-methoxybenzyl)oxy)phenyl)-6-(pyrazolo[1,5-a]pyrimidin-3-yl)-1H-pyrazolo[4,3-c]pyridine-3-carboxamide (Example 88-step 5)

THF (1.58 mL) and water (0. 55 mL) were added to a vial with intermediate example 88-step 4 (40.7 mg, 0.07 mmol), 3-(4,4,5,5-tetramethyl-1,3,2-dioxaborolan-2-yl)pyrazolo[1,5-*a*]pyrimidine (26.2 mg, 0.11 mmol) and K₃PO₄ (30.3 mg, 0.14 mmol). XPhos Pd G3 (6.04 mg, 7.1 µmol) was added and RM stirred at 60°C for 75 min under argon atmosphere. After cooling to RT, RM was partitioned between DCM and water. Organic layer was washed with water, and sat. aq. NaCl, dried over Na₂SO₄ and evaporated to dryness. The residue was chromatographed on silica gel column by eluting with DCM/MeOH/NH₄OH (90:9:1.5) in DCM to afford the title product (24.5 mg).
LCMS (Method 2): Rt = 1.20, ES⁺ *m*/*z* 625.4 [M+H]⁺

### Step 6

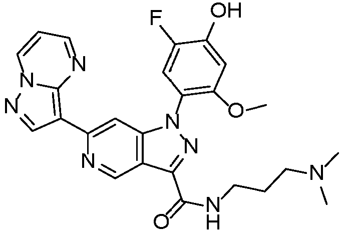

### N-(3-(dimethylamino)propyl)-1-(5-fluoro-4-hydroxy-2-methoxyphenyl)-6-(pyrazolo[1,5-a]pyrimidin-3-yl)-1H-pyrazolo[4,3-c]pyridine-3-carboxamide (Example 88)

A suspension of intermediate example 87-step 5 (22.3 mg, 0.04 mmol) in dichloromethane (0.5 mL) was cooled in an ice-bath and treated with TFA (318 µL, 4.28 mmol). RM was stirred at RT for 20 min. RM was evaporated to dryness and partitioned between DCM and water (pH adjusted to ≈8 using sat. aq. NaHCO₃). The formed precipitate was collected by filtration, washed with water and dried to afford the title product (6.20 mg).
LCMS (Method 3): Rt = 0.94 min, ES⁺ *m*/*z* 505.3
¹H-NMR (500 MHz, *DMSO-d₆*) δ: 9.48 (s, 1H), 9.23 (br d, J=7.0 Hz, 1H), 8.91 (s, 1H), 8.76 (br t, J=5.5 Hz, 1H), 8.72 (br d, J=3.1 Hz, 1H), 8.26 (s, 1H), 7.53 (br d, J=11.0 Hz, 1H), 7.15 (dd, J=6.7, 4.0 Hz, 1H), 6.90 (br d, J=7.6 Hz, 1H), 3.72 (s, 3H), 3.35 (m, 2H, overlap with HDO), 2.28 (br t, J=6.9 Hz, 2H), 2.14 (s, 6H), 1.70 (quin, J=6.9 Hz, 2H).

### Example 89

### Step 1

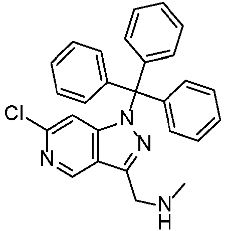

### 1-(6-Chloro-1-trityl-1H-pyrazolo[4,3-c]pyridin-3-yl)-N-methylmethanamine (Example 89-step 1)

A solution of intermediate 37-1a/intermediate 37-1b (mixture obtained after step 1 of intermediate 37) (563 mg, 1.15 mmol) in THF (11.7 mL) was added dropwise to a solution of methylamine (2.0M in THF, 5.76 mL, 11.5 mmol) and stirred at RT for 45 min. RM was dried under reduced pressure and the residue partitioned between EtOAc and sat. aq. NaHCO₃. Organic layer was dried over Na₂SO₄ and evaporated to dryness. The residue was chromatographed on silica gel column by eluting with DCM/MeOH (20:1) in DCM to afford the title product.
LCMS (Method 2): Rt = 1.37, ES⁺ *m*/*z* 439.2/441.1 [M+H]⁺

### Step 2

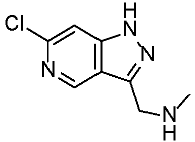

### 1-(6-Chloro-1H-pyrazolo[4,3-c]pyridin-3-yl)-N-methylmethanamine (Example 89-step 2)

TFA (5.91 mL, 79.6 mmol) was added to a solution of intermediate 89-step 1 (520 mg, 1.18 mmol) and triethylsilane (568 µL, 3.55 mmol) in dichloromethane (5.91 mL). RM was stirred at RT for 2 h . RM was evaporated under reduced pressure and the residue chromatographed on silica gel column by eluting with DCM/MeOH/NH₄OH (90:9:1.5) to afford the title product.
LCMS (Method 2): Rt = 0.50, ES⁺ *m*/*z* 197.0/199.0 [M+H]⁺.

### Step 3

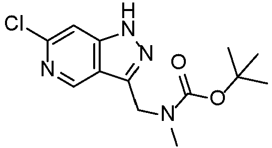

### tert-Butyl ((6-chloro-1H-pyrazolo[4,3-c]pyridin-3-yl)methyl)(methyl)carbamate (Example 89-step 3)

Triethylamine (417 µL, 2.99 mmol) and Boc₂O (256 mg, 1.17 mmol) were added to a solution of intermediate example 89-step 2 (210 mg, 1.07 mmol) in DCM (5 mL). RM was stirred at RT for 100 min, then partitioned between DCM and sat. aq. NaHCO₃. Organic layer was washed with water and sat. aq. NaCl, dried over Na₂SO₄ and evaporated under reduced pressure. The residue was cromatographed on silica gel column by eluting with DCM/MeOH (20:1) to afford the title product (199 mg).
LCMS (Method 2): Rt = 0.91, ES⁺ *m*/*z* 297.1/299.1 [M+H]⁺.

### Step 4

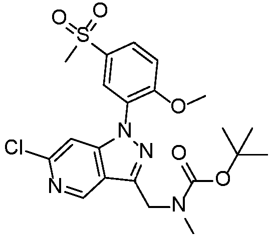

### tert-Butyl ((6-chloro-1-(2-methoxy-5-(methylsulfonyl)phenyl)-1H-pyrazolo[4,3-c]pyridin-3-yl)methyl)(methyl)carbamate (Example 89-step 4)

Intermediate example 89-step 3 (70.0 mg, 0.24 mmol), intermediate 11c (93.8 mg, 0.35 mmol), K₂CO₃ (65.2 mg, 0.472 mmol), copper(I)iodide (33.7 mg, 0.18 mmol) and DMCHA (50.3 mg, 0.35 mmol) in DMF (0.8 mL) were stirred at 100 °C overnight under argon atmosphere. After cooling to RT, RM was partitioned between EtOAc and water. Organic layer was washed with water, sat. aq. NaCl, dried over Na₂SO₄ and solvent removed under reduced pressure. The residue was chromatographed on silica gel column by eluting with DCM/MeOH (30:1) in DCM, followed by a second purification on silica gel column by eluting with EtOAc in n-hexane (2:1) to afford the title product (29.0 mg).
LCMS (Method 2): Rt = 1.11, ES⁺ *m*/*z* 481.0/482.9 [M+H]⁺.

### Step 5

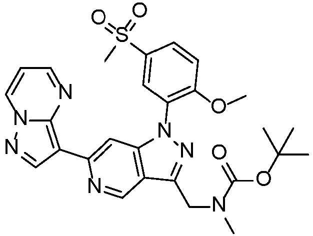

### tert-Butyl ((1-(2-methoxy-5-(methylsulfonyl)phenyl)-6-(pyrazolo[1,5-a]pyrimidin-3-yl)-1H-pyrazolo[4,3-c]pyridin-3-yl)methyl)(methyl)carbamate (Example 89-step 5)

Intermediate example 89-step 4 (27.0 mg, 0.06 mmol), 3-(4,4,5,5-tetramethyl-1,3,2-dioxaborolan-2-yl)pyrazolo[1,5-a]pyrimidine (24.8 mg, 0.10 mmol), K₃PO₄ (23.8 mg, 0.11 mmol) in THF (1.18 mL) and water (0.41 mL) were degassed with argon, then XPhos Pd G3 (4.75 mg, 5.6 µmol) was added. RM was stirred at 60°C for 1 h under argon. After cooling to RT, RM was partitioned between DCM and water. Organic layer was washed with water, sat. aq. NaCl, dried over Na₂SO₄ and solvent removed under reduced pressure. The residue was chromatographed on silica gel column by eluting with MeOH in DCM from 0 to 1:30 followed by further purification on silica gel column eluting with DCM/EtOAc, (1: 1) to afford the title product (26.0 mg).
LCMS (Method 5): Rt = 4.28, ES⁺ *m*/*z* 564.3 [M+H]⁺.

### Step 6

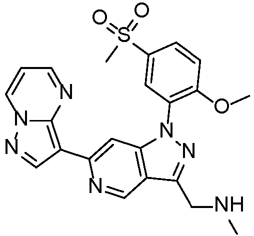

### 1-(1-(2-Methoxy-5-(methylsulfonyl)phenyl)-6-(pyrazolo[1,5-a]pyrimidin-3-yl)-1H-pyrazolo[4,3-c]pyridin-3-yl)-N-methylmethanamine (Example 89)

A suspension of intermediate example 88-step 5 (23.0 mg, 0.04 mmol) in DCM (0.5 mL) was treated with TFA (106 µL, 1.43 mmol) at RT for 1 h. RM was evaporated under reduced pressure and the residue was chromatographed on a silica gel column by eluting with DCM/MeOH/NH₄OH (90:9:1.5) to afford the title product (17.5 mg).
LCMS (Method 3): Rt = 0.78 min, ES⁺ *m*/*z* 464.2 [M+H]⁺
¹H-NMR (500 MHz, *DMSO-d₆*) δ: 9.32 (d, J=0.9 Hz, 1H), 9.23 (dd, J=7.0, 1.8 Hz, 1H), 8.89 (s, 1H), 8.71 (dd, J=4.0, 1.8 Hz, 1H), 8.33 (d, J=0.9 Hz, 1H), 8.09 (dd, J=8.9, 2.1 Hz, 1H), 8.05 (d, J=2.1 Hz, 1H), 7.65 (d, J=8.9 Hz, 1H), 7.17 (dd, J=7.0, 4.3 Hz, 1H), 4.13 (s, 2H), 4.00 (s, 3H), 3.28 (s, 3H), 2.38 (s, 3H)

### Example 90

### Step 1

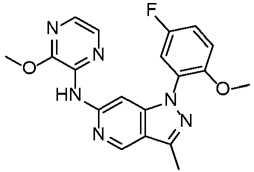

### 1-(5-Fluoro-2-methoxyphenyl)-N-(3-methoxypyrazin-2-yl)-3-methyl-1H-pyrazolo[4,3-c]pyridin-6-amine (Example 90-step 1)

To a degassed mixture of intermediate 18a (70 mg, 0.24 mmol), 2-amino-3-methoxypyrazine (39 mg, 0.31 mmol), sodium *t*-butoxide (35 mg, 0.36 mmol) in 1,4-dioxane (2.1 mL), RuPhos-Pd-G3 (30 mg, 0.04 mmol) was added and RM stirred at 100 °C for 3 h under argon atmosphere. After cooling to RT, RM was diluted with water (5 mL) to form a precipitate that was collected by filtration to afford the title product (110 mg) that was used in the next steps without further purification.
LCMS (Method 2): Rt = 1.21, ES⁺ *m*/*z* 381.1 [M+H]⁺

### Step 2

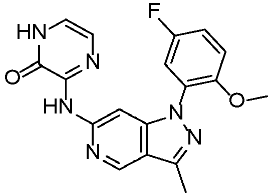

### 3-((1-(5-Fluoro-2-methoxyphenyl)-3-methyl-1H-pyrazolo[4,3-c]pyridin-6-yl)amino)pyrazin-2(1H)-one (Example 90)

TMS-Cl (110 µL, 0.84 mmol) and NaI (126 mg, 0.84 mmol) were added to a mixture of intermediate example 90-step 1 (107 mg, 0.28 mmol) in acetonitrile (8 mL). RM was stirred at 85 °C for 2 h, then cooled to RT and evaporated under reduced pressure. The residue was purified by flash chromatography on a Si cartridge by eluting with 0-40 % DCM/MeOH/NH₄OH (90:9:0.5) in DCM to afford the title compound (51 mg).
LCMS (Method 5): Rt = 3.26 min, ES⁺ *m*/*z* 367.1[M+H]⁺
¹H-NMR (300 MHz, *DMSO-d₆*) δ *=* 12.19 (br s, 1H), 8.87 (d, J=0.9 Hz, 1H), 8.75 (s, 1H), 8.19 (d, J=1.0 Hz, 1H), 7.30-7.44 (m, 3H), 6.83-6.97 (m, 2H), 3.82 (s, 3H), 2.58 (s, 3H)

### Example 91 to 99

The following examples were prepared in a similar manner to example 90 from the indicated starting materials. When minor modifications on ligand / palladium source were made, they were reported in brackets.

| Example | Structure/Name | *Step 1* | | *Step 2* | |
|---|---|---|---|---|---|
| | | Starting Material | Name of intermediate Step 1 / LCMS | ¹H-NMR | LCMS |
| Example 91 | 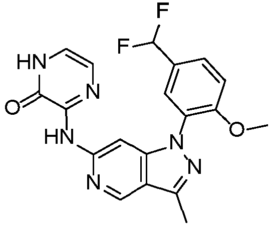 3-((1-(5-(difluoromethyl)-2-methoxyphenyl)-3-methyl-1*H-*pyrazolo[4,3-*c*]pyridin-6-yl)amino)pyrazin-2(1*H*)-one | Intermediate 18c | 1-(5-(difluoromethyl)-2-methoxyphenyl)-N-(3-methoxypyrazin-2-yl)-3-methyl-1H-pyrazolo[4,3-c]pyridin-6-amine | (300 MHz, *DMSO-d₆*) δ: 12.19 (br s, 1H), 8.88 (s, 1H), 8.75 (s, 1H), 8.20 (s, 1H), 7.72 (d, J=8.5 Hz, 1H), 7.68 (s, 1H), 7.48 (d, J=8.5 Hz, 1H), 7.08 (t, J=56.3 Hz, 1H), 6.90-6.92 (m, 1H), 6.85-6.90 (m, 1H), 3.89 (s, 3H), 2.59 (s, 3H) | (Method 7): Rt = 4.64 min, ES⁺ *m*/*z* 399.1 [M+H]⁺ |
| | | | (Method 2): Rt = 1.22, ES+ m/z 413. 1[M+H]+ | | |
| Example 92 | 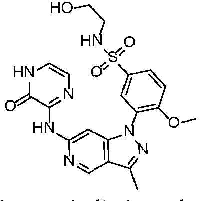 *N*-(2-hydroxyethyl)-4-methoxy-3-(3-methyl-6-((3-oxo-3,4-dihydropyrazin-2-yl)amino)-1*H*-pyrazolo[4,3-*c*]pyridin-1-yl)benzenesulfonamide | Intermediate 18ae (Pd source/ligand: XPhos Pd-G3) | N-(2-hydroxyethyl)-4-methoxy-3-(6-((3-methoxypyrazin-2-yl)amino)-3-methyl-1H-pyrazolo[4,3-c]pyridin-1-yl)benzenesulfonamide | (500 MHz, *DMSO-d₆*) δ 8.90 (s, 1H), 8.78 (s, 1H), 8.24 (s, 1H), 7.87-7.93 (m, 2H), 7.64 (s, 1H), 7.55 (d, J=8.9 Hz, 1H), 6.95 (d, 4.4 Hz, 1H), 6.90 (d, 4.4 Hz, 1H), 4.69 (br s, 1H), 3.95 (s, 3H), 3.38 (m, 2H, overlapping with HDO), 2.81 (t, J=6.1 Hz, 2H), 2.62 (s, 3H) | (Method 7): Rt = 3.55 min, ES⁺ *m*/*z 472.1* |
| | | | (Method 2): Rt = 0.73, ES+ m/z 486.1 [M+H]+ | | |
| Example 93 | 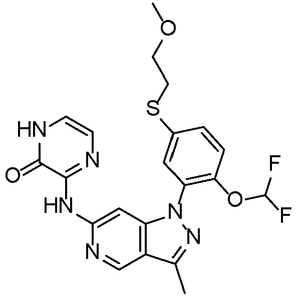 3-((1-(2-(difluoromethoxy)-5-((2-methoxyethyl)thio)phenyl)-3-methyl-1H-pyrazolo[4,3-c]pyridin-6-yl)amino)pyrazin-2(1H)-one | Intermediate 34a (Pd source/ligand: XPhos Pd-G3) | 1-(2-(difluoromethoxy)-5-((2-methoxyethyl)sulfonyl) phenyl)-N-(3-methoxypyrazin-2-yl)-3-methyl-1H-pyrazolo[4,3-c]pyridin-6-amine | (500 MHz, *DMSO-d₆*) δ 12.21 (br s, 1H), 8.91 (s, 1H), 8.79 (s, 1H), 8.27 (s, 1H), 7.60 (s, 1H), 7.58 (br d, J=8.54 Hz, 1H), 7.45 (br d, J=8.54 Hz, 1H), 7.18 (t, J =74.50 Hz, 1H), 6.86-6.95 (m, 2H), 3.56 (t, J=6.26 Hz, 2H), 3.19-3.27 (m, 5H), 2.60 (s, 3H) | (Method 7): Rt = 5.23 min, ES⁺ *m*/*z* 475.1 [M+H]⁺ |
| Example 94 | 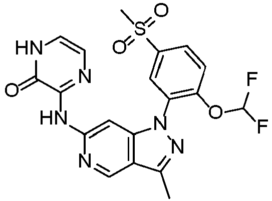 3-((1-(2-(difluoromethoxy)-5-(methylsulfonyl)phenyl)-3-methyl-1*H-*pyrazolo[4,3-*c*]pyridin-6-yl)amino)pyrazin-2(1*H*)-one | Intermediate 35g | 1-(2-(difluoromethoxy)-5-(methylsulfonyl)phenyl )-N-(3-methoxypyrazin-2-yl)-3-methyl-1H-pyrazolo[4,3-c]pyridin-6-amine | (300 MHz, *DMSO-d₆*) δ: 12.21 (br s, 1H), 8.93 (s, 1H), 8.79 (s, 1H), 8.36 (s, 1H), 8.16-8.20 (m, 1H), 8.09-8.16 (m, 1H), 7.77 (d, J=8.7 Hz, 1H), 7.48 (t, J=71.9 Hz, 1H), 6.90 (s, 2H), 3.32 (s, 3H), 2.63 (s, 3H) | (Method 5) Rt = 2.83 min, ES⁺ *m*/*z* 462.8 [M+H]⁺ |
| | | | (Method 2): Rt = 1.05, ES+ m/z 477.1 [M+H]+ | | |
| Example 95 | 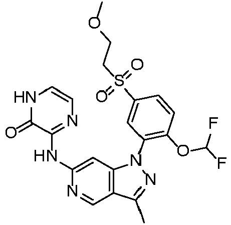 3-((1-(2-(difluoromethoxy)-5-((2-methoxyethyl)sulfonyl)phenyl)-3-methyl-1H-pyrazolo[4,3-c]pyridin-6-yl)amino)pyrazin-2(1H)-one | Intermediate 35a (Pd source/ligand: XPhos Pd-G3) | 1-(2-(difluoromethoxy)-5-((2-methoxyethyl)sulfonyl) phenyl)-N-(3-methoxypyrazin-2-yl)-3-methyl-1H-pyrazolo[4,3-c]pyridin-6-amine | (500 MHz, *DMSO-d₆*) δ 12.21 (br s, 1H), 8.94 (s, 1H), 8.79 (s, 1H), 8.34 (s, 1H), 8.08-8.14 (m, 2H), 7.76 (d, J=8.24 Hz, 1H), 7.49 (t, J=74.20 Hz, 1H), 6.90 (m, 2H), 3.70-3.77 (m, 2H), 3.65-3.68 (m, 2H), 3.09 (s, 3H), 2.63 (s, 3H) | (Method 7): Rt = 4.47 min, ES+ m/z 507.1 [M+H]+ |
| | | | (Method 1): Rt = 1.13, ES+ m/z 489.2 [M+H]+ | | |
| Example 96 | 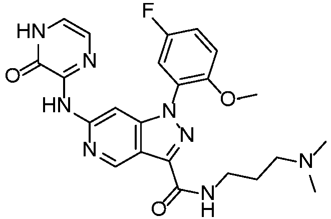 *N*-(3-(dimethylamino)propyl)-1-(5-fluoro-2-methoxyphenyl)-6-((3-oxo-3,4-dihydropyrazin-2-yl)amino)-1*H-*pyrazolo[4,3-*c*]pyridine-3-carboxamide | Intermediate 26f | N-(3-(dimethylamino)propyl) -1-(5-fluoro-2-methoxyphenyl)-6-((3-methoxypyrazin-2-yl)amino)-1H-pyrazolo[4,3-c]pyridine-3-carboxamide | (300 MHz, *DMSO-d₆*) δ: 12.24 (br s, 1H); 9.19 (d, J=1.0 Hz, 1H); 8.83 (s, 1H); 8.76 (t, J=5.9 Hz, 1H); 8.26 (d, J=1.0 Hz, 1H); 7.57 (dd, J=8.7, 3.1 Hz, 1H); 7.36-7.53 (m, 2H); 6.93 (d, J=4.3 Hz, 1H); 6.91 (d, J=4.3 Hz, 1H); 3.82 (s, 3H); 3.32-3.41 (m, 2H); 2.27 (t, J=7.1 Hz, 2H) | (Method 7): Rt = 3.78 min, ES+ m/z 481.1 [M+H]+ |
| | | | (Method 2): Rt = 1.27, ES+ m/z 495.2 [M+H]+ | | |
| Example 97 | 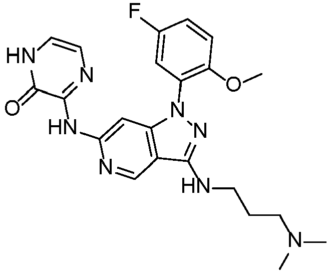 3-((3-((3-(dimethylamino)propyl)amino)-1-(5-fluoro-2-methoxyphenyl)-1*H-*pyrazolo[4,3-*c*]pyridin-6-yl)amino)pyrazin-2(1*H*)-one | Intermediate 18p | N3-(3-(dimethylamino)propyl) -1-(5-fluoro-2-methoxyphenyl)-N6-(3-methoxypyrazin-2-yl)-1H-pyrazolo[4,3-c]pyridine-3,6-diamine | (500 MHz, *DMSO-d₆*) δ: 12.16 (bs, 1H), 10.0 (bs, 1H), 8.77 (s, 1H), 8.67 (s, 1H), 8.09 (s, 1H), 7.32- 7.22 (m, 3H), 6.94 (d, J=4.5 Hz, 1H), 6.88 (d, J=4.5 Hz, 1H), 6.78 (t, J=5.5 Hz, 1H), 3.86 (s, 3H), 3.30- 3.28 (m, 2H, overlap with HDO), 2.37-2.32 (m, 2H, overlap with DMSO), 2.16 (s, 6H), 1.83 - 1.76 (m, 2H) | (Method 7): Rt = 3.30 min, ES⁺ *m*/*z* 453.2 [M+H]⁺ |
| | | | (Method 1): Rt = 0.66, ES+ m/z 467.2 [M+H]+ | | |
| Example 98 | 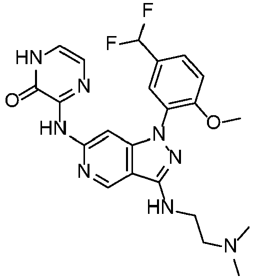 3-((1-(5-(difluoromethyl)-2-methoxyphenyl)-3-((2-(dimethylamino)ethyl)amino)-1H-pyrazolo[4,3-*c*]pyridin-6-yl)amino)pyrazin-2(1*H*)-one | Intermediate 18m | 1-(5-(difluoromethyl)-2-methoxyphenyl)-N3-(2-(dimethylamino)ethyl)-N6-(3-methoxypyrazin-2-yl)-1H-pyrazolo[4,3-c]pyridine-3,6-diamine | (500 MHz, *DMSO-d₆,* | (Method 7): Rt = 3.51 min, ES+ m/z 471.2 [M+H]+ |
| | | | (Method 2): Rt = 1.17, ES+ m/z 485.2 [M+H]+ | | |
| Example 99 | 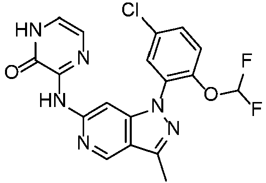 3-((1-(5-chloro-2-(difluoromethoxy)phenyl)-3-methyl-1H-pyrazolo[4,3-c]pyridin-6-yl)amino)pyrazin-2(1H)-one | Intermediate 18z | 1-(5-chloro-2-(difluoromethoxy)phen yl)-N-(3-methoxypyrazin-2-yl)-3-methyl-1H-pyrazolo[4,3-c]pyridin-6-amine | (500 MHz, *DMSO-d₆*) δ: 2.38 (br s, 1H), 9.93 (br s, 1H), 9.03 (s, 1H), 8.13 (s, 1H), 7.80 (d, J=2.4 Hz, 1H), 7.73 (dd, J=2.4, 8.9 Hz, 1H), 7.58 (d, J=8.9 Hz, 1H), 7.24 (t, J=72.6Hz, 1H), 7.00 (t, J=5.0 Hz, 1H), 6.96 (d, J=5.0 Hz, 1H), 2.65 (s, 3H) | (Method 7): Rt = 3.51 min, m/z 418.9/420.0 [M+H]+ |
| | | | (Catalyst: XPhos Pd-G3) | | |
| | | | (Method 2): Rt = 1.38, ES+ m/z 433.2 [M+H]+ | | |

### PHARMACOLOGICAL ACTIVITY OF THE COMPOUNDS OF THE INVENTION (1-99).

### Biochemical Potency on JAK1, JAK2 , JAK3 and Tyk2

### Assay principle

The objective of this study was to assess the capability of compounds to inhibit all 4 JAK isoforms activity in a cell-free environment. Assay for JAK 1, JAK 2, JAK 3 and TYK2 were performed by Time-resolved fluorescence resonance energy transfer (TR-FRET) technology. It consists in the interaction of two labelled binding partners detected by the energy transfer from an excited donor to an acceptor dye and measurement of light emission by the acceptor dye. LANCE Ultra kinase assay was used. In presence of JAK 1, JAK 2, JAK 3 and TYK2 kinases and ATP (corresponding to Km), the ULight peptide substrate (LANCE Ulight-JAK-1 (Tyr1023) Peptide, Perkin Elmer, TRF0121) is phosphorylated. It is then captured by Eu-anti-phospho-substrate antibody (LANCE Eu-W1024 Anti-phosphotyrosine (PT66), Perkin Elmer, AD0069), which bring the Eu-chelate donor and ULight acceptor dyes into close proximity. Upon excitation at 320 nm, the Eu-chelate transfers its energy to the ULight dye, resulting in a fluorescent light emission at 665 nm.

### Compound testing

Serial dilutions of compounds in pure DMSO are prepared from 10 mM DMSO stock solutions. Compounds were tested in 384-well plate for 11 consecutive 5-fold dilutions starting from 20 µM highest concentration (20 µM - 2 pM). 200 nL of compound were transferred from mother plate to test plate by using Mosquito (TTP labtech). Assay was performed in 384-well Perkin Elmer test plate in 20 µL assay volume (kinase reaction) and 40 µL total volume (stopping reagent and antibody detection reagents). In 10 µL of substrate solution (peptide + ATP) 30/50/20/10nM of peptide and 20/0.7/0.2/12µM of ATP were added for JAK 1, JAK 2, JAK 3 and TYK2 respectively. 10 µL of enzyme solution was added to kinase reaction at these concentrations: 0.15/0.083/0.025/0.144 ng/µL of JAK 1, JAK 2, JAK 3 and TYK2 respectively. After shaking and 1.5h of incubation at r.t., 20 µL of Stop (10 µL EDTA) and Detection mixture (10 µL Europium-anti-phospho antibody, final: 0.5 nM) were added. Reading was performed after 1h of incubation on a EnVision 2104 reader (Perkin Elmer).

Calculation of IC50 data, curves and QC analysis was performed by using Excel tool and GraphPadPrism software, v9. Briefly, individual concentration-effect curves are generated by plotting the logarithm of the tested concentration of tested compounds (X) vs. corresponding percent inhibition values (Y) using least squares (ordinary) fit. Best fit IC50 values are calculated using Log(inhibitor) vs. normalized response - Variable slope equation, where Y=100/(1+10^((LogIC50-X)*HillSlope)). QC criteria parameters (Z', S:B, R2, HillSlope) were checked for every IC50 curve. Calculation of IC50 data, curves and QC analysis were made using Excel tools and GraphPadPrism software. QC criteria parameters: Z' ≥ 0.5, Hill Slope range 0.5 to 5, S:B > 2.

Compounds according to the invention (including example 1a-10a and 1-99) show pIC50 values higher than 6 with respect to their inhibitory activity on all JAK isoforms corresponding to < 1 µM in terms of inhibitory concentration. Most compounds preferably showed values equal to or higher than 7.3 even more preferably higher than 8.3 at least with respect to their inhibitory activity on JAK1; corresponding to ≤ 50nM in terms of inhibitory concentration.

Data for compounds 1-99 are reported in the table hereinbelow

| **Example** | **JAK1** | **JAK2** | **JAK3** | **Tyk2** |
|---|---|---|---|---|
| 1 | ++ | +++ | ++ | ++ |
| 2 | +++ | +++ | ++ | ++ |
| 3 | ++ | +++ | ++ | ++ |
| 4 | ++ | +++ | ++ | + |
| 5 | ++ | +++ | ++ | ++ |
| 6 | ++ | ++ | +++ | ++ |
| 7 | ++ | +++ | +++ | ++ |
| 8 | ++ | +++ | ++ | + |
| 9 | ++ | +++ | ++ | + |
| 10 | ++ | ++ | +++ | + |
| 11 | +++ | +++ | +++ | ++ |
| 12 | ++ | +++ | ++ | ++ |
| 13 | +++ | +++ | +++ | ++ |
| 14 | ++ | +++ | +++ | ++ |
| 15 | ++ | +++ | ++ | + |
| 16 | + | ++ | + | + |
| 17 | ++ | ++ | + | + |
| 18 | ++ | ++ | + | + |
| 19 | ++ | ++ | ++ | + |
| 20 | ++ | +++ | +++ | ++ |
| 21 | ++ | ++ | ++ | + |
| 22 | ++ | +++ | +++ | ++ |
| 23 | ++ | ++ | ++ | + |
| 24 | ++ | +++ | +++ | + |
| 25 | ++ | +++ | +++ | ++ |
| 26 | +++ | +++ | +++ | ++ |
| 27 | ++ | +++ | ++ | + |
| 28 | +++ | +++ | +++ | ++ |
| 29 | ++ | +++ | +++ | ++ |
| 30 | ++ | ++ | ++ | + |
| 31 | ++ | ++ | ++ | + |
| 32 | ++ | +++ | ++ | ++ |
| 33 | +++ | +++ | +++ | ++ |
| 34 | ++ | ++ | ++ | + |
| 35 | ++ | ++ | ++ | ++ |
| 36 | ++ | ++ | ++ | + |
| 37 | +++ | +++ | +++ | ++ |
| 38 | ++ | ++ | ++ | + |
| 39 | +++ | +++ | +++ | ++ |
| 40 | +++ | +++ | +++ | ++ |
| 41 | +++ | +++ | +++ | ++ |
| 42 | ++ | ++ | ++ | ++ |
| 43 | ++ | ++ | ++ | + |
| 44 | ++ | +++ | ++ | ++ |
| 45 | ++ | ++ | ++ | + |
| 46 | ++ | +++ | +++ | ++ |
| 47 | ++ | ++ | ++ | + |
| 48 | ++ | ++ | ++ | ++ |
| 49 | ++ | ++ | ++ | ++ |
| 50 | ++ | ++ | ++ | ++ |
| 51 | ++ | ++ | ++ | + |
| 52 | + | ++ | + | + |
| 53 | ++ | + | + | + |
| 54 | ++ | ++ | ++ | + |
| 55 | ++ | ++ | + | + |
| 56 | ++ | ++ | ++ | ++ |
| 57 | ++ | +++ | ++ | ++ |
| 58 | +++ | +++ | +++ | ++ |
| 59 | +++ | +++ | +++ | ++ |
| 60 | +++ | +++ | +++ | ++ |
| 61 | ++ | ++ | ++ | + |
| 62 | + | ++ | ++ | + |
| 63 | +++ | +++ | +++ | ++ |
| 64 | ++ | +++ | +++ | ++ |
| 65 | ++ | ++ | +++ | ++ |
| 66 | ++ | ++ | +++ | + |
| 67 | ++ | ++ | ++ | + |
| 68 | ++ | +++ | +++ | + |
| 69 | ++ | ++ | ++ | + |
| 70 | ++ | +++ | +++ | + |
| 71 | ++ | ++ | ++ | + |
| 72 | ++ | ++ | +++ | + |
| 73 | +++ | +++ | +++ | ++ |
| 74 | + | ++ | ++ | + |
| 75 | ++ | ++ | ++ | + |
| 76 | ++ | ++ | ++ | + |
| 77 | ++ | ++ | ++ | ++ |
| 78 | ++ | ++ | ++ | + |
| 79 | ++ | ++ | ++ | + |
| 80 | ++ | ++ | ++ | ++ |
| 81 | ++ | ++ | ++ | + |
| 82 | ++ | ++ | ++ | + |
| 83 | ++ | ++ | ++ | + |
| 84 | ++ | ++ | ++ | + |
| 85 | ++ | +++ | ++ | + |
| 86 | + | ++ | ++ | + |
| 87 | ++ | +++ | ++ | ++ |
| 88 | +++ | ++ | ++ | ++ |
| 89 | ++ | ++ | + | + |
| 90 | ++ | +++ | +++ | ++ |
| 91 | ++ | +++ | +++ | ++ |
| 92 | ++ | +++ | +++ | + |
| 93 | ++ | +++ | +++ | ++ |
| 94 | +++ | +++ | +++ | ++ |
| 95 | ++ | +++ | +++ | ++ |
| 96 | ++ | ++ | ++ | ++ |
| 97 | ++ | +++ | +++ | ++ |
| 98 | ++ | ++ | ++ | ++ |
| 99 | +++ | +++ | +++ | ++ |

The compounds are classified in the table above, in terms of potency with respect to their inhibitory activity on JAK1, JAK2, JAK3 and Tyk2 isoforms according to the following classification criterion:
+ + + : pIC₅₀ ≥ 8.3
+ + : 8.3 > pIC₅₀ ≥ 7.3
+ : pIC₅₀ < 7.3

Where a numerical limit or range is stated herein, the endpoints are included. Also, all values and subranges within a numerical limit or range are specifically included when not explicitly written out.

As used herein the words "a" and "an" and the like carry the meaning of "one or more."

Obviously, numerous modifications and variations of the present invention are possible in light of the above teachings. It is therefore to be understood that, within the scope of the appended claims, the invention may be practiced otherwise than as specifically described herein.

## Claims

1. A compound of formula I 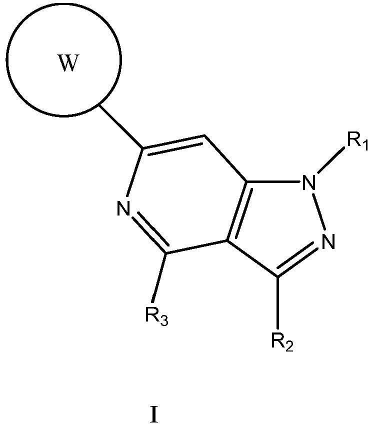 Wherein,
**W is** a heteroaryl selected from pyrazolo[1,5-a]pyrimidin-3-yl, imidazo[1,2-b]pyridazin-3-yl and (3-oxo-3,4-dihydropyrazin-2-yl)amino;
**R₁ is** selected in the group of pyridinyl, piperidinyl, phenyl or benzyl substituted by at least 2 or 3 groups, independently selected from
halogen,
-OH,
-CN
-NO₂
-(CH₂)ₘNR₄R₅,
(C₁-C₆)alkyl,
(C₁-C₆)hydroxyalkyl,
(C₁-C₆)alkoxy,
(C₁-C₆)alkylthio-
(C₁-C₆)haloalkyl,
(C₁-C₆)haloalkoxy,
(C₁-C₆)haloalkylthio-
and
a group of formula **K** 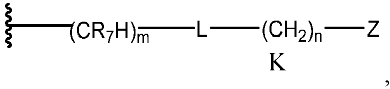 in meta position with respect to the point of attachment of R₁ with the rest of the molecule;
wherein
**L** is absent or is a divalent group selected from O, S, S(O)₂, (CO), C(O)O, O(O)C, C(O)N(R₆), N(R₆)C(O), NHCONH, N(R₆)S(O)₂, S(O)₂N(R₆);
**Z** is selected from the group consisting of H, -OH, -CN, -NO₂, (C₁-C₆)alkyl, (C₁-C₆)hydroxyalkyl, (C₁-C₆)haloalkyl, (C₁-C₆)alkoxy, (C₁-C₆)alkoxy (C₁-C₆)alkyl, (C₁-C₆)alkoxycarbonyl, -(CH₂)ₘNR₄R₅, -C(O)NH(R₆), (C₃-C₈)cycloalkyl, aryl, heteroaryl and (C₃-C₆)heterocycloalkyl; wherein said (C₃-C₈)cycloalkyl, aryl, heteroaryl and (C₃-C₆)heterocycloalkyl are further optionally substituted by one or more substituents selected from the group consisting of
(C₁-C₁₀)alkyl, (C₁-C₆)alkoxy, alkanoyl, (C₁-C₆)alkoxycarbonyl, oxo, - C(O)NH(R₆), (C₁-C₆)alkoxy(C₁-C₆)alkyl;
**R₃** is selected independently from the group consisting of H, (C₁-C₆)alkyl, and
**R₂ is** selected independently from the group consisting of H, (C₁-C₆)alkyl, and a group of formula **J** 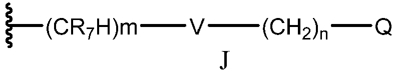
wherein
**V** is absent or is a divalent group selected from O, S, S(O)₂, C(O), C(O)O, O(O)C, C(O)N(R₆), N(R₆)C(O); N(R₆)-(CH₂)ₘ-N(R₆),-N(R₆)-,
**Q** is selected from the group consisting of H, -CN, -OH, (C₁-C₆)alkyl, (C₁-C₆)hydroxyalkyl, (C₁-C₆)haloalkyl, (C₁-C₆)alkoxy, (C₁-C₆)alkoxycarbonyl, hydroxycarbonyl, -(CH₂)ₘNR₄R₅, -C(O)NR₄R₅, -N(R₆)C(O)R₆, - CH(CN)NR₄R₅, (C₃-C₈)cycloalkyl, aryl, heteroaryl and (C₃-C₆)heterocycloalkyl; wherein said (C₃-C₈)cycloalkyl, aryl, heteroaryl and (C₃-C₆)heterocycloalkyl are further optionally substituted by one or more substituents selected from
-OH, oxo, (C₁-C₁₀)alkyl, (C₁-C₁₀)alkyl-S(O)₂-O-, alkanoyl, (C₁-C₆)hydroxyalkyl, (C₁-C₆)alkoxy(C₁-C₆)alkyl, (C₁-C₆)alkoxycarbonyl, (C₁-C₆)alkoxycarbonyl-NH-, -N(R₆)(CH₂)ₘC(O)NR₄R₅, -NR₄R₅, (C₃-C₆)heterocycloalkyl;
**n and m are in each occurrence independently** 0 or an integer selected from 1, 2, 3 and 4;
**R₄ and R₅,** the same or different, are selected from the group consisting of
-H,
(C₁-C₆)alkyl,
(C₁-C₆)haloalkyl,
(C₁-C₆)hydroxyalkyl,
alkanoyl, (C₁-C₆)alkoxycarbonyl, and
(C₃-C₆)heterocycloalkyl;
**R₆** is in each occurrence independently selected from the group consisting of H, (C₁-C₆)alkyl, (C₁-C₆)hydroxyalkyl, and alkanoyl,
**R₇** is in each occurrence independently selected from the group consisting of H, (C₁-C₆)alkyl, -NR₄R₅
single enantiomers, diastereoisomers and mixtures thereof in any proportion,
or pharmaceutically acceptable salts and solvates thereof.

2. A compound according to claim 1
wherein R₁ is phenyl substituted by 2 or 3 groups, independently selected from halogen, preferably Cl and F,
-OH,
(C₁-C₆)alkoxy, preferably methoxy,
(C₁-C₆)alkylthio- preferably methylthio
(C₁-C₆)haloalkoxy, preferably difluoromethoxy

3. A compound according to claim 1 represented by the formula (Ib) 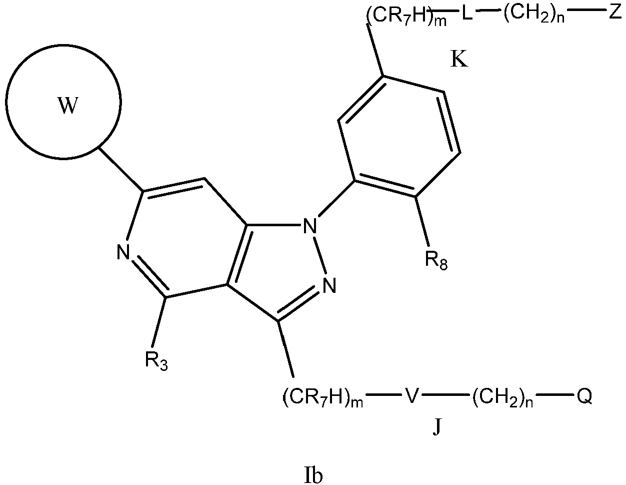 **wherein**
**R₈** is selected in the group consisting of
(C₁-C₆)alkoxy,
(C₁-C₆)haloalkoxy;
**L** is selected from the divalent group consisting of O, S, S(O)₂, C(O)O, O(O)C, C(O)N(R₆), N(R₆)C(O);
**Z** is selected from the group consisting of H, (C₁-C₆)alkyl, (C₁-C₆)hydroxyalkyl, (C₁-C₆)haloalkyl, (C₁-C₆)alkoxy, (C₁-C₆)alkoxycarbonyl, -(CH₂)ₘNR₄R₅, - C(O)NH(R₆), (C₃-C₈)cycloalkyl, aryl, heteroaryl and (C₃-C₆)heterocycloalkyl wherein said (C₃-C₈)cycloalkyl, aryl, heteroaryl and (C₃-C₆)heterocycloalkyl are further optionally substituted by one or more substituents selected from the group consisting of
(C₁-C₁₀)alkyl, alkanoyl, (C₁-C₆)alkoxycarbonyl, -C(O)NH(R₆), (C₁-C₆)alkoxy(C₁-C₆)alkyl;
**R₃** is -H or (C₁-C₆)alkyl;
**V** is absent or is selected from the divalent group consisting of O, S, S(O)₂, C(O), C(O)O, O(O)C, C(O)N(R₆), N(R₆)C(O); N(R₆)-(CH₂)ₘ-N(R₆), -N(R₆)-,
**Q** is selected from the group consisting of H, -CN, -OH, (C₁-C₆)alkyl, (C₁-C₆)hydroxyalkyl, (C₁-C₆)haloalkyl, (C₁-C₆)alkoxy, (C₁-C₆)alkoxycarbonyl, - (CH₂)ₘNR₄R₅, -C(O)NR₄R₅, -N(R₆)C(O)R₆, -CH(CN)NR₄R₅, (C₃-C₈)cycloalkyl, aryl, heteroaryl and (C₃-C₆)heterocycloalkyl, wherein said (C₃-C₈)cycloalkyl, aryl, heteroaryl and (C₃-C₆)heterocycloalkyl are further optionally substituted by one or more substituents selected from the group consisting of -OH, oxo, (C₁-C₁₀)alkyl, (C₁-C₁₀)alkyl-S(O)₂-O-, alkanoyl, (C₁-C₆)hydroxyalkyl, (C₁-C₆)alkoxy(C₁-C₆)alkyl, (C₁-C₆)alkoxycarbonyl, (C₁-C₆)alkoxycarbonyl-NH-, -N(R₆)(CH₂)ₘC(O)NR₄R₅, -NR₄R₅, (C₃-C₆)heterocycloalkyl;
**n and m are in each occurrence independently** 0 or an integer from 1 to 4;
**R₄ and R₅,** the same or different, are selected from the group consisting of
-H,
(C₁-C₆)alkyl,
(C₁-C₆)haloalkyl,
(C₁-C₆)hydroxyalkyl,
alkanoyl,
(C₁-C₆)alkoxycarbonyl, and
(C₃-C₆)heterocycloalkyl;
**R₆** is in each occurrence independently selected from the group consisting of H, (C₁-C₆)alkyl, (C₁-C₆)hydroxyalkyl,
**R₇** is in each occurrence independently selected from the group consisting of H, (C₁-C₆)alkyl,
single enantiomers, diastereoisomers and mixtures thereof
or a pharmaceutically acceptable salt or solvate thereof.

4. A compound according to claim 1 or 2 represented in formula (Ib1) 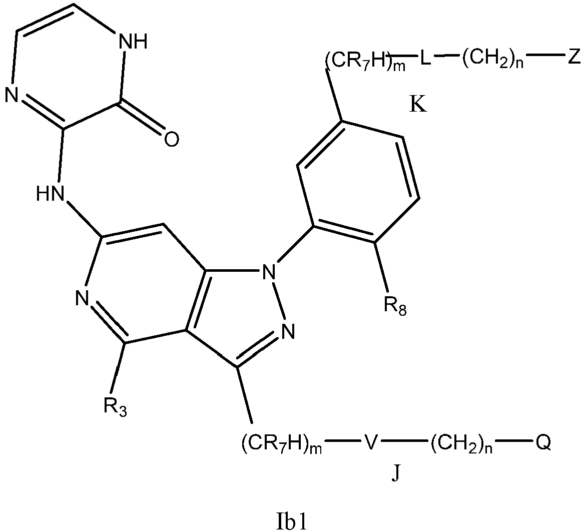 wherein
**R₈** is selected in the group consisting of
(C₁-C₆)alkoxy,
(C₁-C₆)haloalkoxy;
**L** is selected from the divalent group consisting of O, S, S(O)₂, C(O)O, O(O)C, C(O)N(R₆), N(R₆)C(O);
**Z** is selected from the group consisting of H, (C₁-C₆)alkyl, (C₁-C₆)hydroxyalkyl, (C₁-C₆)haloalkyl, (C₁-C₆)alkoxy, (C₁-C₆)alkoxycarbonyl, -(CH₂)ₘNR₄R₅, - C(O)NH(R₆),
(C₃-C₈)cycloalkyl, aryl, heteroaryl and (C₃-C₆)heterocycloalkyl optionally substituted by one or more substituents selected from the group consisting of
(C₁-C₁₀)alkyl, alkanoyl, (C₁-C₆)alkoxycarbonyl, -C(O)NH(R₆), (C₁-C₆)alkoxy(C₁-C₆)alkyl;
**R₃** is -H or (C₁-C₆)alkyl;
**V** is absent or is selected from the divalent group consisting of O, S, S(O)₂, C(O), C(O)O, O(O)C, C(O)N(R₆), N(R₆)C(O); N(R₆)-(CH₂)ₘ-N(R₆), -N(R₆)-,
**Q** is selected from the group consisting of H, -CN, -OH, (C₁-C₆)alkyl, (C₁-C₆)hydroxyalkyl, (C₁-C₆)haloalkyl, (C₁-C₆)alkoxy, (C₁-C₆)alkoxycarbonyl, - (CH₂)ₘNR₄R₅, -C(O)NR₄R₅, -N(R₆)C(O)R₆, -CH(CN)NR₄R₅,
(C₃-C₈)cycloalkyl, aryl , heteroaryl and (C₃-C₆)heterocycloalkyl optionally substituted by one or more substituents selected from the group consisting of -OH, oxo, (C₁-C₁₀)alkyl, (C₁-C₁₀)alkyl-S(O)₂-O-, alkanoyl, (C₁-C₆)hydroxyalkyl, (C₁-C₆)alkoxy(C₁-C₆)alkyl, (C₁-C₆)alkoxycarbonyl, (C₁-C₆)alkoxycarbonyl-NH-, -N(R₆)(CH₂)ₘC(O)NR₄R₅, -NR₄R₅, (C₃-C₆)heterocycloalkyl;
**n and m are in each occurrence independently** 0 or an integer from 1 to 4;
**R₄ and R₅**, the same or different, are selected from the group consisting of
-H,
(C₁-C₆)alkyl,
(C₁-C₆)haloalkyl,
(C₁-C₆)hydroxyalkyl,
alkanoyl,(C₁-C₆)alkoxycarbonyl, and
(C₃-C₆)heterocycloalkyl;
**R₆** is in each occurrence independently selected from the group consisting of H, (C₁-C₆)alkyl, (C₁-C₆)hydroxyalkyl,
**R₇** is in each occurrence independently selected from the group consisting of H, (C₁-C₆)alkyl,
single enantiomers, diastereoisomers and mixtures thereof in any proportion or pharmaceutically acceptable salts and solvates thereof.

5. A compound according to claim 1 selected from:
(1-(5-chloro-2-(difluoromethoxy)phenyl)-6-(pyrazolo[1,5-a]pyrimidin-3-yl)-1H-pyrazolo[4,3-b]pyridin-3-yl)methanol;
methyl 1-(5-chloro-2-(difluoromethoxy)phenyl)-6-(pyrazolo[1,5-a]pyrimidin-3-yl)-1H-pyrazolo[4,3-b]pyridine-3-carboxylate;
2-(1-(5-chloro-2-(difluoromethoxy)phenyl)-6-(pyrazolo[1,5-a]pyrimidin-3-yl)-1H-pyrazolo[4,3-b]pyridin-3-yl)-N,N-dimethylacetamide;
2-(1-(5-chloro-2-(difluoromethoxy)phenyl)-6-(pyrazolo[1,5-a]pyrimidin-3-yl)-1H-pyrazolo[4,3-b]pyridin-3-yl)acetic acid;
1-(5-chloro-2-(difluoromethoxy)phenyl)-6-( imidazo[1,2-b]pyridazin-3-yl)-3-methyl-1H-pyrazolo[4,3-c]pyridine;
1-(5-chloro-2-(difluoromethoxy)phenyl)-3-methyl-6-(pyrazolo[1,5-a]pyrimidin-3-yl)-1H-pyrazolo[4,3-c]pyridine;
1-(4-chloro-2-fluorobenzyl)-6-( imidazo[1,2-b]pyridazin-3-yl )-3-methyl-1H-pyrazolo[4,3-b]pyridine;
1-(5-chloro-2-(difluoromethoxy)phenyl)-3-methyl-6-(pyrazolo[1,5-a]pyrimidin-3-yl)-1H-pyrazolo[4,3-b]pyridine;
3-(3-(3-methyl-6-(pyrazolo[1,5-a]pyrimidin-3-yl)-1H-pyrazolo[4,3-b]pyridin-1-yl)piperidin-1-yl)-3-oxopropanenitrile;
1-(5-fluoro-2-methoxyphenyl)-3-methyl-6-(pyrazolo[1,5-a]pyrimidin-3-yl)-1H-pyrazolo[4,3-c]pyridine;
1-(5-chloro-2-methoxyphenyl)-3-methyl-6-(pyrazolo[1,5-a]pyrimidin-3-yl)-1H-pyrazolo[4,3-c]pyridine;
1-(5-(difluoromethyl)-2-methoxyphenyl)-3-methyl-6-(pyrazolo[1,5-a]pyrimidin-3-yl)-1H-pyrazolo[4,3-c]pyridine;
1-(2-methoxy-5-(trifluoromethyl)phenyl)-3-methyl-6-(pyrazolo[1,5-a]pyrimidin-3-yl)-1H-pyrazolo[4,3-c]pyridine;
1-(5-chloro-2-(methylthio)phenyl)-3-methyl-6-(pyrazolo[1,5-a]pyrimidin-3-yl)-1H-pyrazolo[4,3-c]pyridine;
1-(5-chloro-2-((difluoromethyl)thio)phenyl)-3-methyl-6-(pyrazolo[1,5-a]pyrimidin-3-yl)-1H-pyrazolo[4,3-c]pyridine;
1-(5-chloro-2-cyclopropoxyphenyl)-3-methyl-6-(pyrazolo[1,5-a]pyrimidin-3-yl)-1H-pyrazolo[4,3-c]pyridine;
1-(2,5-dimethoxyphenyl)-3-methyl-6-(pyrazolo[1,5-a]pyrimidin-3-yl)-1H-pyrazolo[4,3-c]pyridine;
3-((4-methoxy-3-(3-methyl-6-(pyrazolo[1,5-a]pyrimidin-3-yl)-1H-pyrazolo[4,3-c]pyridin-1-yl)phenyl)sulfonyl)propan-1-ol;
1-(2-methoxy-5-(propylsulfonyl)phenyl)-3-methyl-6-(pyrazolo[1,5-a]pyrimidin-3-yl)-1H-pyrazolo[4,3-c]pyridine;
1-(5-chloro-2-(difluoromethoxy)phenyl)-3-methyl-6-(pyrazolo[1,5-a]pyrimidin-3-yl)-1H-pyrazolo[4,3-c]pyridine;
4-chloro-2-(3-methyl-6-(pyrazolo[1,5-a]pyrimidin-3-yl)-1H-pyrazolo[4,3-c]pyridin-1-yl)phenol;
1-(2-(difluoromethoxy)-5-(methylthio)phenyl)-3-methyl-6-(pyrazolo[1,5-a]pyrimidin-3-yl)-1H-pyrazolo[4,3-c]pyridine;
4-methoxy-N-methyl-3-(3-methyl-6-(pyrazolo[1,5-a]pyrimidin-3-yl)-1H-pyrazolo[4,3-c]pyridin-1-yl)benzenesulfonamide;
N-(2-hydroxyethyl)-4-methoxy-3-(3-methyl-6-(pyrazolo[1,5-a]pyrimidin-3-yl)-1H-pyrazolo[4,3-c]pyridin-1-yl)benzenesulfonamide;
4-methoxy-3-(3-methyl-6-(pyrazolo[1,5-a]pyrimidin-3-yl)-1H-pyrazolo[4,3-c]pyridin-1-yl)-N-(3-(4-methylpiperazin-1-yl)propyl)benzenesulfonamide;
4-methoxy-3-(3-methyl-6-(pyrazolo[1,5-a]pyrimidin-3-yl)-1H-pyrazolo[4,3-c]pyridin-1-yl)-N-((1-methylazetidin-3-yl)methyl)benzenesulfonamide;
4-methoxy-3-(3-methyl-6-(pyrazolo[1,5-a]pyrimidin-3-yl)-1H-pyrazolo[4,3-c]pyridin-1-yl)-N-(2-(4-methylpiperazin-1-yl)ethyl)benzenesulfonamide;
4-methoxy-3-(3-methyl-6-(pyrazolo[1,5-a]pyrimidin-3-yl)-1H-pyrazolo[4,3-c]pyridin-1-yl)-N-(2-morpholinoethyl)benzenesulfonamide;
1-(2-(difluoromethoxy)-5-((2-methoxyethyl)sulfonyl)phenyl)-3-methyl-6-(pyrazolo[1,5-a]pyrimidin-3-yl)-1H-pyrazolo[4,3-c]pyridine;
3-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)-N,N-dimethylpropan-1-amine;
2-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)ethan-1-ol;
1-(2-(difluoromethoxy)-5-((2-(piperidin-1-yl)ethyl)sulfonyl)phenyl)-3-methyl-6-(pyrazolo[1,5-a]pyrimidin-3-yl)-1H-pyrazolo[4,3-c]pyridine;
1-(3-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)pyrrolidin-1-yl)ethan-1-one;
1-(2-(difluoromethoxy)-5-((3-methoxyphenyl)sulfonyl)phenyl)-3-methyl-6-(pyrazolo[1,5-a]pyrimidin-3-yl)-1H-pyrazolo[4,3-c]pyridine;
1-(2-(difluoromethoxy)-5-(methylsulfonyl)phenyl)-3-methyl-6-(pyrazolo[1,5-a]pyrimidin-3-yl)-1H-pyrazolo[4,3-c]pyridine;
3-methoxy-4-(3-methyl-6-(pyrazolo[1,5-a]pyrimidin-3-yl)-1H-pyrazolo[4,3-c]pyridin-1-yl)phenol;
2-(1-(5-chloro-2-(difluoromethoxy)phenyl)-6-(pyrazolo[1,5-a]pyrimidin-3-yl)-1H-pyrazolo[4,3-c]pyridin-3-yl)acetonitrile;
(1-(5-chloro-2-(difluoromethoxy)phenyl)-6-(pyrazolo[1,5-a]pyrimidin-3-yl)-1H-pyrazolo[4,3-c]pyridin-3-yl)methanol;
1-(1-(5-chloro-2-(difluoromethoxy)phenyl)-6-(pyrazolo[1,5-a]pyrimidin-3-yl)-1H-pyrazolo[4,3-c]pyridin-3-yl)-N,N-dimethylmethanamine;
1-(1-(5-chloro-2-(difluoromethoxy)phenyl)-6-(pyrazolo[1,5-a]pyrimidin-3-yl)-1H-pyrazolo[4,3-c]pyridin-3-yl)-N-methylmethanamine;
(1-(5-chloro-2-(difluoromethoxy)phenyl)-6-(pyrazolo[1,5-a]pyrimidin-3-yl)-1H-pyrazolo[4,3-c]pyridin-3-yl)methanamine;
1-(5-chloro-2-(difluoromethoxy)phenyl)-N-methyl-6-(pyrazolo[1,5-a]pyrimidin-3-yl)-1H-pyrazolo[4,3-c]pyridine-3-carboxamide;
1-(5-chloro-2-methoxyphenyl)-N-((1s,3s)-3-hydroxycyclobutyl)-6-(pyrazolo[1,5-a]pyrimidin-3-yl)-1H-pyrazolo[4,3-c]pyridine-3-carboxamide;
(1-(5-chloro-2-(difluoromethoxy)phenyl)-6-(pyrazolo[1,5-a]pyrimidin-3-yl)-1H-pyrazolo[4,3-c]pyridin-3-yl)(1,1-dioxidothiomorpholino)methanone;
1-(5-chloro-2-(difluoromethoxy)phenyl)-N-((1s,3s)-3-(dimethylamino)cyclobutyl)-6-(pyrazolo[1,5-a]pyrimidin-3-yl)-1H-pyrazolo[4,3-c]pyridine-3-carboxamide;
1-(2-(difluoromethoxy)-5-(methylthio)phenyl)-N-(3-(dimethylamino)propyl)-6-(pyrazolo[1,5-a]pyrimidin-3-yl)-1H-pyrazolo[4,3-c]pyridine-3-carboxamide;
N-(3-(dimethylamino)propyl)-1-(5-fluoro-2-methoxyphenyl)-6-(pyrazolo[1,5-a]pyrimidin-3-yl)-1H-pyrazolo[4,3-c]pyridine-3-carboxamide;
1-(5-chloro-2-methoxyphenyl)-6-(pyrazolo[1,5-a]pyrimidin-3-yl)-1H-pyrazolo[4,3-c]pyridin-3-amine;
N-(1-(5-chloro-2-methoxyphenyl)-6-(pyrazolo[1,5-a]pyrimidin-3-yl)-1H-pyrazolo[4,3-c]pyridin-3-yl)acetamide;
1-(5-chloro-2-methoxyphenyl)-N-methyl-6-(pyrazolo[1,5-a]pyrimidin-3-yl)-1H-pyrazolo[4,3-c]pyridin-3-amine;
1-(5-chloro-2-methoxyphenyl)-N,N-dimethyl-6-(pyrazolo[1,5-a]pyrimidin-3-yl)-1H-pyrazolo[4,3-c]pyridin-3-amine;
1-(2-methoxy-5-(methylsulfonyl)phenyl)-N-(2-morpholinoethyl)-6-(pyrazolo[1,5-a]pyrimidin-3-yl)-1H-pyrazolo[4,3-c]pyridin-3-amine;
N1-(1-(5-(difluoromethyl)-2-methoxyphenyl)-6-(pyrazolo[1,5-a]pyrimidin-3-yl)-1H-pyrazolo[4,3-c]pyridin-3-yl)-N2,N2-dimethylethane-1,2-diamine;
N1-(1-(2-methoxy-5-methylphenyl)-6-(pyrazolo[1,5-a]pyrimidin-3-yl)-1H-pyrazolo[4,3-c]pyridin-3-yl)-N2,N2-dimethylethane-1,2-diamine;
N1-(1-(2-(difluoromethoxy)-5-(methylthio)phenyl)-6-(pyrazolo[1,5-a]pyrimidin-3-yl)-1H-pyrazolo[4,3-c]pyridin-3-yl)-N3,N3-dimethylpropane-1,3-diamine;
1-(4-(1-(5-chloro-2-methoxyphenyl)-6-(pyrazolo[1,5-a]pyrimidin-3-yl)-1H-pyrazolo[4,3-c]pyridin-3-yl)piperazin-1-yl)ethan-1-one;
1-(5-chloro-2-methoxyphenyl)-3-(piperazin-1-yl)-6-(pyrazolo[1,5-a]pyrimidin-3-yl)-1H-pyrazolo[4,3-c]pyridine;
(1-(1-(5-chloro-2-methoxyphenyl)-6-(pyrazolo[1,5-a]pyrimidin-3-yl)-1H-pyrazolo[4,3-c]pyridin-3-yl)azetidin-3-yl)methanol;
1-(5-chloro-2-methoxyphenyl)-3-methoxy-6-(pyrazolo[1,5-a]pyrimidin-3-yl)-1H-pyrazolo[4,3-c]pyridine;
1-(5-chloro-2-methoxyphenyl)-N,N-dimethyl-6-(pyrazolo[1,5-a]pyrimidin-3-yl)-1H-pyrazolo[4,3-c]pyridin-4-amine;
1-(5-chloro-2-methoxyphenyl)-N-methyl-6-(pyrazolo[1,5-a]pyrimidin-3-yl)-1H-pyrazolo[4,3-c]pyridin-4-amine;
N-(2-((1-(5-chloro-2-methoxyphenyl)-6-(pyrazolo[1,5-a]pyrimidin-3-yl)-1H-pyrazolo[4,3-c]pyridin-4-yl)amino)ethyl)acetamide;
2-((1-(5-chloro-2-methoxyphenyl)-6-(pyrazolo[1,5-a]pyrimidin-3-yl)-1H-pyrazolo[4,3-c]pyridin-4-yl)amino)-N-methylacetamide;
N-(1-(5-chloro-2-methoxyphenyl)-6-(pyrazolo[1,5-a]pyrimidin-3-yl)-1H-pyrazolo[4,3-c]pyridin-4-yl)acetamide;
1-(5-chloro-2-methoxyphenyl)-6-(pyrazolo[1,5-a]pyrimidin-3-yl)-1H-pyrazolo[4,3-c]pyridin-4-amine;
1-(5-chloro-2-methoxyphenyl)-6-(pyrazolo[1,5-a]pyrimidin-3-yl)-1H-pyrazolo[4,3-c]pyridin-4-ol;
2-fluoro-5-methoxy-4-(3-methyl-6-(pyrazolo[1,5-a]pyrimidin-3-yl)-1H-pyrazolo[4,3-c]pyridin-1-yl)phenol;
2-chloro-5-methoxy-4-(3-methyl-6-(pyrazolo[1,5-a]pyrimidin-3-yl)-1H-pyrazolo[4,3-c]pyridin-1-yl)phenol;
5-methoxy-2-methyl-4-(3-methyl-6-(pyrazolo[1,5-a]pyrimidin-3-yl)-1H-pyrazolo[4,3-c]pyridin-1-yl)phenol;
1-(2-chloro-5-methoxypyridin-4-yl)-3-methyl-6-(pyrazolo[1,5-a]pyrimidin-3-yl)-1H-pyrazolo[4,3-c]pyridine;
(4-chloro-2-(3-methyl-6-(pyrazolo[1,5-a]pyrimidin-3-yl)-1H-pyrazolo[4,3-c]pyridin-1-yl)phenyl)methanol;
1-(5-bromo-2-(difluoromethoxy)phenyl)-3-methyl-6-(pyrazolo[1,5-a]pyrimidin-3-yl)-1H-pyrazolo[4,3-c]pyridine;
4-(difluoromethoxy)-3-(3-methyl-6-(pyrazolo[1,5-a]pyrimidin-3-yl)-1H-pyrazolo[4,3-c]pyridin-1-yl)benzonitrile;
1-(2-(difluoromethoxy)-5-methylphenyl)-3-methyl-6-(pyrazolo[1,5-a]pyrimidin-3-yl)-1H-pyrazolo[4,3-c]pyridine;
4-(difluoromethoxy)-3-(3-methyl-6-(pyrazolo[1,5-a]pyrimidin-3-yl)-1H-pyrazolo[4,3-c]pyridin-1-yl)-N-(thiazol-2-yl)benzamide;
2-((4-(difluoromethoxy)-3-(3-methyl-6-(pyrazolo[1,5-a]pyrimidin-3-yl)-1H-pyrazolo[4,3-c]pyridin-1-yl)phenyl)thio)-N-methylacetamide;
2-((4-(difluoromethoxy)-3-(3-methyl-6-(pyrazolo[1,5-a]pyrimidin-3-yl)-1H-pyrazolo[4,3-c]pyridin-1-yl)phenyl)thio)acetamide;
2-((4-(difluoromethoxy)-3-(3-methyl-6-(pyrazolo[1,5-a]pyrimidin-3-yl)-1H-pyrazolo[4,3-c]pyridin-1-yl)phenyl)thio)-N-(2-hydroxyethyl)acetamide;
2-((4-(difluoromethoxy)-3-(3-methyl-6-(pyrazolo[1,5-a]pyrimidin-3-yl)-1H-pyrazolo[4,3-c]pyridin-1-yl)phenyl)sulfonyl)acetamide;
1-(5-(cyclopropylthio)-2-(difluoromethoxy)phenyl)-3-methyl-6-(pyrazolo[1,5-a]pyrimidin-3-yl)-1H-pyrazolo[4,3-c]pyridine;
1-(2-(difluoromethoxy)-5-((tetrahydro-2H-pyran-4-yl)thio)phenyl)-3-methyl-6-(pyrazolo[1,5-a]pyrimidin-3-yl)-1H-pyrazolo[4,3-c]pyridine;
1-(2-(difluoromethoxy)-5-(oxetan-3-ylthio)phenyl)-3-methyl-6-(pyrazolo[1,5-a]pyrimidin-3-yl)-1H-pyrazolo[4,3-c]pyridine;
1-(2-(difluoromethoxy)-5-(piperidin-4-ylthio)phenyl)-3-methyl-6-(pyrazolo[1,5-a]pyrimidin-3-yl)-1H-pyrazolo[4,3-c]pyridine;
1-(4-((4-(difluoromethoxy)-3-(3-methyl-6-(pyrazolo[1,5-a]pyrimidin-3-yl)-1H-pyrazolo[4,3-c]pyridin-1-yl)phenyl)thio)piperidin-1-yl)ethan-1-one;
1-((1-(5-chloro-2-(difluoromethoxy)phenyl)-6-(pyrazolo[1,5-a]pyrimidin-3-yl)-1H-pyrazolo[4,3-c]pyridin-3-yl)methyl)azetidin-3-amine;
N-((1r,3r)-3-aminocyclobutyl)-1-(5-chloro-2-methoxyphenyl)-6-(pyrazolo[1,5-a]pyrimidin-3-yl)-1H-pyrazolo[4,3-c]pyridine-3-carboxamide;
1-(5-chloro-2-methoxyphenyl)-N-((1r,3r)-3-(methyl(2-(methylamino)-2-oxoethyl)amino)cyclobutyl)-6-(pyrazolo[1,5-a]pyrimidin-3-yl)-1H-pyrazolo[4,3-c]pyridine-3-carboxamide;
1-(5-chloro-2-methoxyphenyl)-N-((1r,3r)-3-morpholinocyclobutyl)-6-(pyrazolo[1,5-a]pyrimidin-3-yl)-1H-pyrazolo[4,3-c]pyridine-3-carboxamide;
1-(5-chloro-2-methoxyphenyl)-N-((1r,3r)-3-(dimethylamino)cyclobutyl)-6-(pyrazolo[1,5-a]pyrimidin-3-yl)-1H-pyrazolo[4,3-c]pyridine-3-carboxamide;
N-((1s,3s)-3-aminocyclobutyl)-1-(5-chloro-2-(difluoromethoxy)phenyl)-6-(pyrazolo[1,5-a]pyrimidin-3-yl)-1H-pyrazolo[4,3-c]pyridine-3-carboxamide;
N-(2-(dimethylamino)ethyl)-1-(2-methoxy-5-(methylsulfonyl)phenyl)-6-(pyrazolo[1,5-a]pyrimidin-3-yl)-1H-pyrazolo[4,3-c]pyridine-3-carboxamide;
N-(3-(dimethylamino)propyl)-1-(2-methoxy-5-(methylsulfonyl)phenyl)-6-(pyrazolo[1,5-a]pyrimidin-3-yl)-1H-pyrazolo[4,3-c]pyridine-3-carboxamide;
1-(2-methoxy-5-(methylsulfonyl)phenyl)-N-(3-morpholinopropyl)-6-(pyrazolo[1,5-a]pyrimidin-3-yl)-1H-pyrazolo[4,3-c]pyridine-3-carboxamide;
1-(2-methoxy-5-(methylsulfonyl)phenyl)-N-methyl-6-(pyrazolo[1,5-a]pyrimidin-3-yl)-1H-pyrazolo[4,3-c]pyridin-3-amine;
N-(3-(3-((2-(dimethylamino)ethyl)amino)-6-(pyrazolo[1,5-a]pyrimidin-3-yl)-1H-pyrazolo[4,3-c]pyridin-1-yl)-4-methoxyphenyl)methanesulfonamide;
N-(1-(5-chloro-2-methoxyphenyl)-6-(pyrazolo[1,5-a]pyrimidin-3-yl)-1H-pyrazolo[4,3-c]pyridin-3-yl)-2-(methylamino)acetamide;
N-(3-(dimethylamino)propyl)-1-(5-fluoro-4-hydroxy-2-methoxyphenyl)-6-(pyrazolo[1,5-a]pyrimidin-3-yl)-1H-pyrazolo[4,3-c]pyridine-3-carboxamide;
1-(1-(2-methoxy-5-(methylsulfonyl)phenyl)-6-(pyrazolo[1,5-a]pyrimidin-3-yl)-1H-pyrazolo[4,3-c]pyridin-3-yl)-N-methylmethanamine;
3-((1-(5-fluoro-2-methoxyphenyl)-3-methyl-1H-pyrazolo[4,3-c]pyridin-6-yl)amino)pyrazin-2(1H)-one;
3-((1-(5-(difluoromethyl)-2-methoxyphenyl)-3-methyl-1H-pyrazolo[4,3-c]pyridin-6-yl)amino)pyrazin-2(1H)-one;
N-(2-hydroxyethyl)-4-methoxy-3-(3-methyl-6-((3-oxo-3,4-dihydropyrazin-2-yl)amino)-1H-pyrazolo[4,3-c]pyridin-1-yl)benzenesulfonamide;
3-((1-(2-(difluoromethoxy)-5-((2-methoxyethyl)thio)phenyl)-3-methyl-1H-pyrazolo[4,3-c]pyridin-6-yl)amino)pyrazin-2(1H)-one;
3-((1-(2-(difluoromethoxy)-5-(methylsulfonyl)phenyl)-3-methyl-1H-pyrazolo[4,3-c]pyridin-6-yl)amino)pyrazin-2(1H)-one;
3-((1-(2-(difluoromethoxy)-5-((2-methoxyethyl)sulfonyl)phenyl)-3-methyl-1H-pyrazolo[4,3-c]pyridin-6-yl)amino)pyrazin-2(1H)-one;
N-(3-(dimethylamino)propyl)-1-(5-fluoro-2-methoxyphenyl)-6-((3-oxo-3,4-dihydropyrazin-2-yl)amino)-1H-pyrazolo[4,3-c]pyridine-3-carboxamide;
3-((3-((3-(dimethylamino)propyl)amino)-1-(5-fluoro-2-methoxyphenyl)-1H-pyrazolo[4,3-c]pyridin-6-yl)amino)pyrazin-2(1H)-one;
3-((1-(5-(difluoromethyl)-2-methoxyphenyl)-3-((2-(dimethylamino)ethyl)amino)-1H-pyrazolo[4,3-c]pyridin-6-yl)amino)pyrazin-2(1H)-one;
3-((1-(5-chloro-2-(difluoromethoxy)phenyl)-3-methyl-1H-pyrazolo[4,3-c]pyridin-6-yl)amino)pyrazin-2(1H)-one;
single enantiomers, diastereoisomers and mixtures thereof or a pharmaceutically acceptable salt or solvate thereof.

6. A pharmaceutical composition comprising a compound as defined in any one of claims 1 to 5, or a pharmaceutically acceptable salt thereof, in admixture with one or more pharmaceutically acceptable carrier or excipient.

7. A pharmaceutical composition according to claim 6 suitable to be administered by inhalation, selected from inhalable powders, propellant-containing metering aerosols or propellant-free inhalable formulations.

8. A device comprising the pharmaceutical composition according to claim 7, which may be a single- or multi-dose dry powder inhaler, a metered dose inhaler or a soft mist nebulizer.

9. A compound or a pharmaceutical composition according to any one of claims 1 to 7 for use as a medicament.

10. A compound or a pharmaceutical composition for use according to claim 9 in the prevention and /or treatment of a pulmonary disease selected from the group consisting of asthma, chronic obstructive pulmonary disease COPD, idiopathic pulmonary fibrosis (IPF), acute lung injury and acute respiratory distress syndrome (ARDS).

11. A combination of a compound as defined in any one of the claims 1 to 5 with one or more active ingredients selected from the classes consisting of with other pharmaceutical active ingredients selected from the group consisting of beta2-agonists, antimuscarinic agents, corticosteroids, mitogen-activated kinases (P38 MAP kinases) inhibitors, nuclear factor kappa-B kinase subunit beta inhibitors (IKK2), human neutrophil elastase (HNE) inhibitors, phosphodiesterase 4 (PDE4) inhibitors, leukotriene modulators, non-steroidal anti-inflammatory agents (NSAIDs) and mucus regulators.

## Patentansprüche

1. Verbindung der Formel I 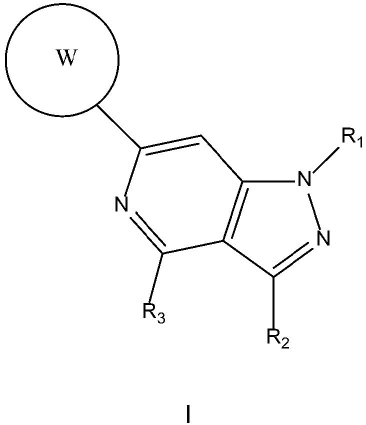 wobei
**W** ein Heteroaryl ist, das ausgewählt ist aus Pyrazolo[1,5-a]pyrimidin-3-yl, Imidazo[1,2-b]pyridazin-3-yl und (3-Oxo-3,4-dihydropyrazin-2-yl)amino;
**R₁** ausgewählt ist aus der Gruppe von Pyridinyl, Piperidinyl, Phenyl oder Benzyl, das durch mindestens 2 oder 3 Gruppen substituiert ist, die unabhängig ausgewählt sind aus
Halogen,
-OH,
-CN
-NO₂
-(CH₂)ₘNR₄R₅,
(C₁-C₆)-Alkyl,
(C₁-C₆)-Hydroxyalkyl,
(C₁-C₆)-Alkoxy,
(C₁-C₆)-Alkylthio-
(C₁-C₆)-Halogenalkyl,
(C₁-C₆)-Haloalkoxy,
(C₁-C₆)-Halogenalkylthio-
und
einer Gruppe der Formel **K** 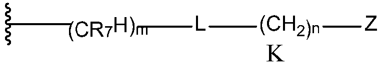 , in meta-Position in Bezug auf die Verknüpfungsstelle von R₁ mit dem Rest des Moleküls;
wobei
**L** fehlt oder eine zweiwertige Gruppe ist, die ausgewählt ist aus O, S, S(O)₂, (CO), C(O)O, O(O)C, C(O)N(R₆), N(R₆)C(O), NHCONH, N(R₆)S(O)₂, S(O)₂N(R₆);
**Z** ausgewählt ist aus der Gruppe bestehend aus H, -OH, -CN, -NO₂, (C₁-C₆)Alkyl, (C₁-C₆)Hydroxyalkyl, (C₁-C₆)Halogenalkyl, (C₁-C₆)Alkoxy, (C₁-C₆)Alkoxy(C₁-C₆)alkyl, (C₁-C₆)Alkoxycarbonyl, -(CH₂)ₘNR₄R₅, -C(O)NH(R₆), (C₃-C₈)Cycloalkyl, Aryl, Heteroaryl und (C₃-C₆)Heterocycloalkyl; wobei das (C₃-C₈)Cycloalkyl, Aryl, Heteroaryl und (C₃-C₆)Heterocycloalkyl ferner gegebenenfalls durch einen oder mehrere Substituenten substituiert sind, die ausgewählt sind aus der Gruppe bestehend aus
(C₁-C₁₀)Alkyl, (C₁-C₆)Alkoxy, Alkanoyl, (C₁-C₆)Alkoxycarbonyl, Oxo, -C(O)NH(R₆), (C₁-C₆)Alkoxy(C₁-C₆)alkyl;
**R₃** unabhängig ausgewählt ist aus der Gruppe bestehend aus H, (C₁-C₆)-Alkyl und
**R₂** unabhängig ausgewählt ist aus der Gruppe bestehend aus H, (C₁-C₆)Alkyl und einer Gruppe der Formel **J** 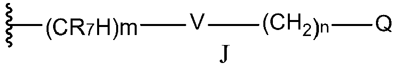
wobei
**V** fehlt oder eine zweiwertige Gruppe ist, die ausgewählt ist aus O, S, S(O)₂, C(O), C(O)O, O(O)C, C(O)N(R₆), N(R₆)C(O); N(R₆)-(CH₂)ₘ-N(R₆),-N(R₆)-,
**Q** ausgewählt ist aus der Gruppe bestehend aus H, -CN, -OH, (C₁-C₆)Alkyl, (C₁-C₆)Hydroxyalkyl, (C₁-C₆)Halogenalkyl, (C₁-C₆)Alkoxy, (C₁-C₆)Alkoxycarbonyl, Hydroxycarbonyl, -(CH₂)ₘNR₄R₅, -C(O)NR₄R₅, -N(R₆)C(O)R₆, -CH(CN)NR₄R₅, (C₃-C₈)Cycloalkyl, Aryl, Heteroaryl und (C₃-C₆)Heterocycloalkyl; wobei das (C₃-C₈)Cycloalkyl, Aryl, Heteroaryl und (C₃-C₆)Heterocycloalkyl ferner gegebenenfalls durch einen oder mehrere Substituenten substituiert sind, die ausgewählt sind aus
-OH, Oxo, (C₁-C₁₀)Alkyl, (C₁-C₁₀)Alkyl-S(O)₂-O-, Alkanoyl, (C₁-C₆)Hydroxyalkyl, (C₁-C₆)Alkoxy(C₁-C₆)alkyl, (C₁-C₆)Alkoxycarbonyl, (C₁-C₆)Alkoxycarbonyl-NH-, -N(R₆)(CH₂)ₘC(O)NR₄R₅, -NR₄R₅, (C₃-C₆)Heterocycloalkyl;
**n und m bei jedem Vorkommen unabhängig** 0 oder eine ganze Zahl sind, die ausgewählt ist aus 1, 2, 3 und 4;
**R₄ und R₅**, gleich oder verschieden, ausgewählt sind aus der Gruppe bestehend aus
-H,
(C₁-C₆)-Alkyl,
(C₁-C₆)-Halogenalkyl,
(C₁-C₆)-Hydroxyalkyl,
Alkanoyl, (C₁-C₆)Alkoxycarbonyl und
(C₃-C₆)-Heterocycloalkyl;
**R₆** bei jedem Vorkommen unabhängig ausgewählt ist aus der Gruppe bestehend aus H, (C₁-C₆)-Alkyl, (C₁-C₆)-Hydroxyalkyl und Alkanoyl,
**R₇** bei jedem Vorkommen unabhängig ausgewählt ist aus der Gruppe bestehend aus H, (C₁-C₆)Alkyl, -NR₄R₅
einzelne Enantiomere, Diastereoisomere und Mischungen davon in beliebigem Verhältnis
oder pharmazeutisch verträgliche Salze und Solvate davon.

2. Verbindung nach Anspruch 1
wobei R₁ Phenyl ist, das durch 2 oder 3 Gruppen substituiert ist, die unabhängig ausgewählt sind aus Halogen, vorzugsweise Cl und F,
-OH,
(C₁-C₆)-Alkoxy, vorzugsweise Methoxy,
(C₁-C₆)Alkylthio- vorzugsweise Methylthio
(C₁-C₆)-Halogenalkoxy, vorzugsweise Difluormethoxy

3. Verbindung nach Anspruch 1, dargestellt durch die Formel (Ib) 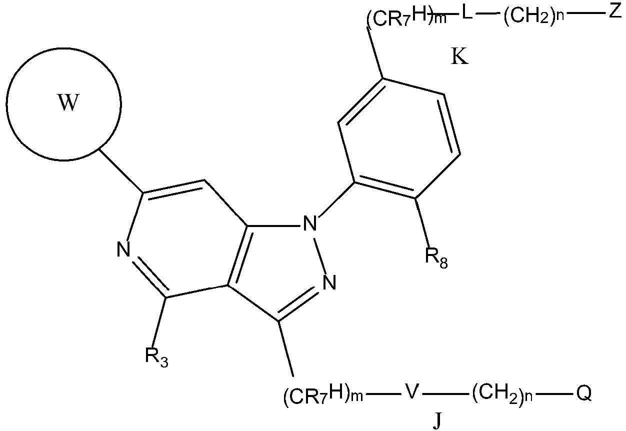 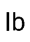 wobei
**R₈** ausgewählt ist aus der Gruppe bestehend aus
(C₁-C₆)-Alkoxy,
(C₁-C₆)-Halogenalkoxy;
**L** ausgewählt ist aus der zweiwertigen Gruppe bestehend aus O, S, S(O)₂, C(O)O, O(O)C, C(O)N(R₆), N(R₆)C(O);
**Z** ausgewählt ist aus der Gruppe bestehend aus H, (C₁-C₆)Alkyl, (C₁-C₆)Hydroxyalkyl, (C₁-C₆)Halogenalkyl, (C₁-C₆)Alkoxy, (C₁-C₆)Alkoxycarbonyl, - (CH₂)ₘNR₄R₅, -C(O)NH(R₆), (C₃-C₃)Cycloalkyl, Aryl, Heteroaryl und (C₃-C₆)Heterocycloalkyl, wobei das (C₃-C₈)Cycloalkyl, Aryl, Heteroaryl und (C₃-C₆)Heterocycloalkyl ferner gegebenenfalls durch einen oder mehrere Substituenten substituiert sind, die ausgewählt sind aus der Gruppe bestehend aus
(C₁-C₁₀)Alkyl, Alkanoyl, (C₁-C₆)Alkoxycarbonyl, -C(O)NH(R₆), (C₁-C₆)Alkoxy(C₁-C₆)alkyl;
**R₃** H oder (C₁-C₆)Alkyl ist;
**V** fehlt oder ausgewählt ist aus der zweiwertigen Gruppe bestehend aus O, S, S(O)₂, C(O), C(O)O, O(O)C, C(O)N(R₆), N(R₆)C(O); N(R₆)-(CH₂)ₘ-N(R₆), -N(R₆)-,
**Q** ausgewählt ist aus der Gruppe bestehend aus H, -CN, -OH, (C₁-C₆)Alkyl, (C₁-C₆)Hydroxyalkyl, (C₁-C₆)Halogenalkyl, (C₁-C₆)Alkoxy, (C₁-C₆)Alkoxycarbonyl, - (CH₂)ₘNR₄R₅, -C(O)NR₄R₅, -N(R₆)C(O)R₆, -CH(CN)NR₄R₅, (C₃-C₈)Cycloalkyl, Aryl, Heteroaryl und (C₃-C₆)Heterocycloalkyl, wobei das (C₃-C₈)Cycloalkyl, Aryl, Heteroaryl und (C₃-C₆)Heterocycloalkyl ferner gegebenenfalls durch einen oder mehrere Substituenten substituiert sind, die ausgewählt sind aus der Gruppe bestehend aus -OH, Oxo, (C₁-C₁₀)Alkyl, (C₁-C₁₀)Alkyl-S(O)₂-O-, Alkanoyl, (C₁-C₆)Hydroxyalkyl, (C₁-C₆)Alkoxy(C₁-C₆)alkyl, (C₁-C₆)Alkoxycarbonyl, (C₁-C₆)Alkoxycarbonyl-NH-, -N(R₆)(CH₂)ₘC(O)NR₄R₅, -NR₄R₅, (C₃-C₆)Heterocycloalkyl;
**n und m bei jedem Vorkommen unabhängig** 0 oder eine ganze Zahl von 1 bis 4 sind;
**R₄ und R₅**, gleich oder verschieden, ausgewählt sind aus der Gruppe, bestehend aus
-H,
(C₁-C₆)-Alkyl,
(C₁-C₆)-Halogenalkyl,
(C₁-C₆)-Hydroxyalkyl,
Alkanoyl,
(C₁-C₆)Alkoxycarbonyl und
(C₃-C₆)-Heterocycloalkyl;
**R₆** bei jedem Vorkommen unabhängig ausgewählt ist aus der Gruppe bestehend aus H, (C₁-C₆)-Alkyl, (C₁-C₆)-Hydroxyalkyl,
**R₇** bei jedem Vorkommen unabhängig ausgewählt ist aus der Gruppe bestehend aus H, (C₁-C₆)Alkyl,
einzelne Enantiomere, Diastereoisomere und Mischungen davon
oder ein pharmazeutisch annehmbares Salz oder Solvat davon.

4. Verbindung nach Anspruch 1 oder 2, dargestellt in Formel (Ib1) 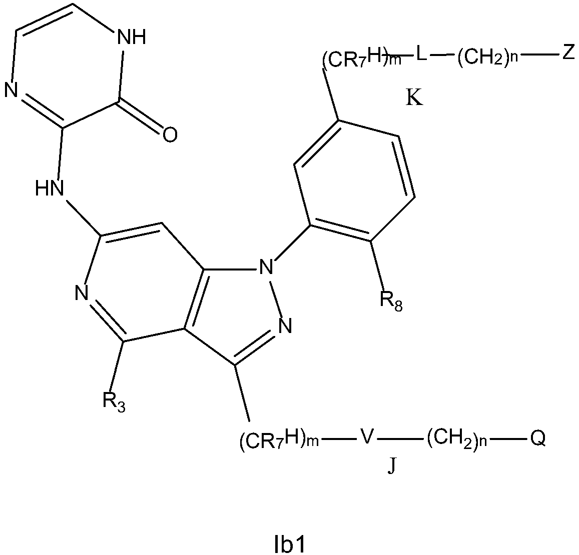 wobei
**R₈** ausgewählt ist aus der Gruppe bestehend aus
(C₁-C₆)-Alkoxy,
(C₁-C₆)-Halogenalkoxy;
**L** ausgewählt ist aus der zweiwertigen Gruppe bestehend aus O, S, S(O)₂, C(O)O, O(O)C, C(O)N(R₆), N(R₆)C(O);
**Z** ausgewählt ist aus der Gruppe bestehend aus H, (C₁-C₆)Alkyl, (C₁-C₆)Hydroxyalkyl, (C₁-C₆)Halogenalkyl, (C₁-C₆)Alkoxy, (C₁-C₆)Alkoxycarbonyl, - (CH₂)ₘNR₄R₅, -C(O)NH(R₆),
(C₃-C₈)Cycloalkyl, Aryl, Heteroaryl und (C₃-C₆)Heterocycloalkyl, die gegebenenfalls durch einen oder mehrere Substituenten substituiert sind, die ausgewählt sind aus der Gruppe bestehend aus
(C₁-C₁₀)Alkyl, Alkanoyl, (C₁-C₆)Alkoxycarbonyl, -C(O)NH(R₆), (C₁-C₆)Alkoxy(C₁-C₆)alkyl;
**R₃** H oder (C₁-C₆)Alkyl ist;
**V** fehlt oder ausgewählt ist aus der zweiwertigen Gruppe bestehend aus O, S, S(O)₂, C(O), C(O)O, O(O)C, C(O)N(R₆), N(R₆)C(O); N(R₆)-(CH₂)ₘ-N(R₆), -N(R₆)-,
**Q** ausgewählt ist aus der Gruppe bestehend aus H, -CN, -OH, (C₁-C₆)Alkyl, (C₁-C₆)Hydroxyalkyl, (C₁-C₆)Halogenalkyl, (C₁-C₆)Alkoxy, (C₁-C₆)Alkoxycarbonyl, -(CH₂)ₘNR₄R₅, -C(O)NR₄R₅, -N(R₆)C(O)R₆, -CH(CN)NR₄R₅,
(C₃-C₈)Cycloalkyl, Aryl, Heteroaryl und (C₃-C₆)Heterocycloalkyl, die gegebenenfalls durch einen oder mehrere Substituenten substituiert sind, die ausgewählt sind aus der Gruppe bestehend aus -OH, Oxo, (C₁-C₁₀)Alkyl, (C₁-C₁₀)Alkyl-S(O)₂-O-, Alkanoyl, (C₁-C₆)Hydroxyalkyl, (C₁-C₆)Alkoxy(C₁-C₆)alkyl, (C₁-C₆)Alkoxycarbonyl, (C₁-C₆)Alkoxycarbonyl-NH-, -N(R₆)(CH₂)ₘC(O)NR₄R₅, -NR₄R₅, (C₃-C₆)Heterocycloalkyl;
**n und m bei jedem Vorkommen unabhängig** 0 oder eine ganze Zahl von 1 bis 4 sind;
**R₄ und R₅**, gleich oder verschieden, ausgewählt sind aus der Gruppe bestehend aus
-H,
(C₁-C₆)-Alkyl,
(C₁-C₆)-Halogenalkyl,
(C₁-C₆)-Hydroxyalkyl,
Alkanoyl, (C₁-C₆)Alkoxycarbonyl und
(C₃-C₆)-Heterocycloalkyl;
**R₆** bei jedem Vorkommen unabhängig ausgewählt ist aus der Gruppe bestehend aus H, (C₁-C₆)-Alkyl, (C₁-C₆)-Hydroxyalkyl,
**R₇** bei jedem Vorkommen unabhängig ausgewählt ist aus der Gruppe bestehend aus H, (C₁-C₆)Alkyl,
einzelne Enantiomere, Diastereoisomere und Mischungen davon in beliebigem Verhältnis oder pharmazeutisch annehmbare Salze und Solvate davon.

5. Verbindung nach Anspruch 1, ausgewählt aus:
(1-(5-Chlor-2-(difluormethoxy)phenyl)-6-(pyrazolo[1,5-a]pyrimidin-3-yl)-1H-pyrazolo[4,3-b]pyridin-3-yl)methanol;
Methyl-1-(5-chlor-2-(difluormethoxy)phenyl)-6-(pyrazolo[1,5-a]pyrimidin-3-yl)-1H-pyrazolo[4,3-b]pyridin-3-carboxylat;
2-(1-(5-Chlor-2-(difluormethoxy)phenyl)-6-(pyrazolo[1,5-a]pyrimidin-3-yl)-1H-pyrazolo[4,3-b]pyridin-3-yl)-N,N-dimethylacetamid;
2-(1-(5-Chlor-2-(difluormethoxy)phenyl)-6-(pyrazolo[1,5-a]pyrimidin-3-yl)-1H-pyrazolo[4,3-b]pyridin-3-yl)essigsäure;
1-(5-Chlor-2-(difluormethoxy)phenyl)-6-(imidazo[1,2-b]pyridazin-3-yl)-3-methyl-1H-pyrazolo[4,3-c]pyridin;
1-(5-Chlor-2-(difluormethoxy)phenyl)-3-methyl-6-(pyrazolo[1,5-a]pyrimidin-3-yl)-1H-pyrazolo[4,3-c]pyridin;
1-(4-Chlor-2-fluorbenzyl)-6-(imidazo[1,2-b]pyridazin-3-yl)-3-methyl-1H-pyrazolo[4,3-b]pyridin;
1-(5-Chlor-2-(difluormethoxy)phenyl)-3-methyl-6-(pyrazolo[1,5-a]pyrimidin-3-yl)-1H-pyrazolo[4,3-b]pyridin;
3-(3-(3-Methyl-6-(pyrazolo[1,5-a]pyrimidin-3-yl)-1H-pyrazolo[4,3-b]pyridin-1-yl)piperidin-1-yl)-3-oxopropannitril;
1-(5-Fluor-2-methoxyphenyl)-3-methyl-6-(pyrazolo[1,5-a]pyrimidin-3-yl)-1H-pyrazolo[4,3-c]pyridin;
1-(5-Chlor-2-methoxyphenyl)-3-methyl-6-(pyrazolo[1,5-a]pyrimidin-3-yl)-1H-pyrazolo[4,3-c]pyridin;
1-(5-(Difluormethyl)-2-methoxyphenyl)-3-methyl-6-(pyrazolo[1,5-a]pyrimidin-3-yl)-1H-pyrazolo[4,3-c]pyridin;
1-(2-Methoxy-5-(trifluormethyl)phenyl)-3-methyl-6-(pyrazolo[1,5-a]pyrimidin-3-yl)-1H-pyrazolo[4,3-c]pyridin;
1-(5-Chlor-2-(methylthio)phenyl)-3-methyl-6-(pyrazolo[1,5-a]pyrimidin-3-yl)-1H-pyrazolo[4,3-c]pyridin;
1-(5-Chlor-2-((difluormethyl)thio)phenyl)-3-methyl-6-(pyrazolo[1,5-a]pyrimidin-3-yl)-1H-pyrazolo[4,3-c]pyridin;
1-(5-Chlor-2-cyclopropoxyphenyl)-3-methyl-6-(pyrazolo[1,5-a]pyrimidin-3-yl)-1H-pyrazolo[4,3-c]pyridin;
1-(2,5-Dimethoxyphenyl)-3-methyl-6-(pyrazolo[1,5-a]pyrimidin-3-yl)-1H-pyrazolo[4,3-c]pyridin;
3-((4-Methoxy-3-(3-methyl-6-(pyrazolo[1,5-a]pyrimidin-3-yl)-1H-pyrazolo[4,3-c]pyridin-1-yl)phenyl)sulfonyl)propan-1-ol;
1-(2-Methoxy-5-(propylsulfonyl)phenyl)-3-methyl-6-(pyrazolo[1,5-a]pyrimidin-3-yl)-1H-pyrazolo[4,3-c]pyridin;
1-(5-Chlor-2-(difluormethoxy)phenyl)-3-methyl-6-(pyrazolo[1,5-a]pyrimidin-3-yl)-1H-pyrazolo[4,3-c]pyridin;
4-Chlor-2-(3-methyl-6-(pyrazolo[1,5-a]pyrimidin-3-yl)-1H-pyrazolo[4,3-c]pyridin-1-yl)phenol;
1-(2-(Difluormethoxy)-5-(methylthio)phenyl)-3-methyl-6-(pyrazolo[1,5-a]pyrimidin-3-yl)-1H-pyrazolo[4,3-c]pyridin;
4-Methoxy-N-methyl-3-(3-methyl-6-(pyrazolo[1,5-a]pyrimidin-3-yl)-1H-pyrazolo[4,3-c]pyridin-1-yl)benzolsulfonamid;
N-(2-Hydroxyethyl)-4-methoxy-3-(3-methyl-6-(pyrazolo[1,5-a]pyrimidin-3-yl)-1H-pyrazolo[4,3-c]pyridin-1-yl)benzolsulfonamid;
4-Methoxy-3-(3-methyl-6-(pyrazolo[1,5-a]pyrimidin-3-yl)-1H-pyrazolo[4,3-c]pyridin-1-yl)-N-(3-(4-methylpiperazin-1-yl)propyl)benzolsulfonamid;
4-Methoxy-3-(3-methyl-6-(pyrazolo[1,5-a]pyrimidin-3-yl)-1H-pyrazolo[4,3-c]pyridin-1-yl)-N-((1-methylazetidin-3-yl)methyl)benzolsulfonamid;
4-Methoxy-3-(3-methyl-6-(pyrazolo[1,5-a]pyrimidin-3-yl)-1H-pyrazolo[4,3-c]pyridin-1-yl)-N-(2-(4-methylpiperazin-1-yl)ethyl)benzolsulfonamid;
4-Methoxy-3-(3-methyl-6-(pyrazolo[1,5-a]pyrimidin-3-yl)-1H-pyrazolo[4,3-c]pyridin-1-yl)-N-(2-morpholinoethyl)benzolsulfonamid;
1-(2-(Difluormethoxy)-5-((2-methoxyethyl)sulfonyl)phenyl)-3-methyl-6-(pyrazolo[1,5-a]pyrimidin-3-yl)-1H-pyrazolo[4,3-c]pyridin;
3-((4-(Difluormethoxy)-3-(3-methyl-6-(pyrazolo[1,5-a]pyrimidin-3-yl)-1H-pyrazolo[4,3-c]pyridin-1-yl)phenyl)sulfonyl)-N,N-dimethylpropan-1-amin;
2-((4-(Difluormethoxy)-3-(3-methyl-6-(pyrazolo[1,5-a]pyrimidin-3-yl)-1H-pyrazolo[4,3-c]pyridin-1-yl)phenyl)sulfonyl)ethan-1-ol;
1-(2-(Difluormethoxy)-5-((2-(piperidin-1-yl)ethyl)sulfonyl)phenyl)-3-methyl-6-(pyrazolo[1,5-a]pyrimidin-3-yl)-1H-pyrazolo[4,3-c]pyridin;
1-(3-((4-(Difluormethoxy)-3-(3-methyl-6-(pyrazolo[1,5-a]pyrimidin-3-yl)-1H-pyrazolo[4,3-c]pyridin-1-yl)phenyl)sulfonyl)pyrrolidin-1-yl)ethan-1-on;
1-(2-(Difluormethoxy)-5-((3-methoxyphenyl)sulfonyl)phenyl)-3-methyl-6-(pyrazolo[1,5-a]pyrimidin-3-yl)-1H-pyrazolo[4,3-c]pyridin;
1-(2-(Difluormethoxy)-5-(methylsulfonyl)phenyl)-3-methyl-6-(pyrazolo[1,5-a]pyrimidin-3-yl)-1H-pyrazolo[4,3-c]pyridin;
3-Methoxy-4-(3-methyl-6-(pyrazolo[1,5-a]pyrimidin-3-yl)-1H-pyrazolo[4,3-c]pyridin-1-yl)phenol;
2-(1-(5-Chlor-2-(difluormethoxy)phenyl)-6-(pyrazolo[1,5-a]pyrimidin-3-yl)-1H-pyrazolo[4,3-c]pyridin-3-yl)acetonitril;
(1-(5-Chlor-2-(difluormethoxy)phenyl)-6-(pyrazolo[1,5-a]pyrimidin-3-yl)-1H-pyrazolo[4,3-c]pyridin-3-yl)methanol;
1-(1-(5-Chlor-2-(difluormethoxy)phenyl)-6-(pyrazolo[1,5-a]pyrimidin-3-yl)-1H-pyrazolo[4,3-c]pyridin-3-yl)-N,N-dimethylmethanamin;
1-(1-(5-Chlor-2-(difluormethoxy)phenyl)-6-(pyrazolo[1,5-a]pyrimidin-3-yl)-1H-pyrazolo[4,3-c]pyridin-3-yl)-N-methylmethanamin;
(1-(5-Chlor-2-(difluormethoxy)phenyl)-6-(pyrazolo[1,5-a]pyrimidin-3-yl)-1H-pyrazolo[4,3-c]pyridin-3-yl)methanamin;
1-(5-Chlor-2-(difluormethoxy)phenyl)-N-methyl-6-(pyrazolo[1,5-a]pyrimidin-3-yl)-1H-pyrazolo[4,3-c]pyridin-3-carboxamid;
1-(5-Chlor-2-methoxyphenyl)-N-((1s,3s)-3-hydroxycyclobutyl)-6-(pyrazolo[1,5-a]pyrimidin-3-yl)-1H-pyrazolo[4,3-c]pyridin-3-carboxamid;
(1-(5-Chlor-2-(difluormethoxy)phenyl)-6-(pyrazolo[1,5-a]pyrimidin-3-yl)-1H-pyrazolo[4,3-c]pyridin-3-yl)(1,1-dioxidothiomorpholino)methanon;
1-(5-Chlor-2-(difluormethoxy)phenyl)-N-((1s,3s)-3-(dimethylamino)cyclobutyl)-6-(pyrazolo[1,5-a]pyrimidin-3-yl)-1H-pyrazolo[4,3-c]pyridin-3-carboxamid;
1-(2-(Difluormethoxy)-5-(methylthio)phenyl)-N-(3-(dimethylamino)propyl)-6-(pyrazolo[1,5-a]pyrimidin-3-yl)-1H-pyrazolo[4,3-c]pyridin-3-carboxamid;
N-(3-(Dimethylamino)propyl)-1-(5-fluor-2-methoxyphenyl)-6-(pyrazolo[1,5-a]pyrimidin-3-yl)-1H-pyrazolo[4,3-c]pyridin-3-carboxamid;
1-(5-Chlor-2-methoxyphenyl)-6-(pyrazolo[1,5-a]pyrimidin-3-yl)-1H-pyrazolo[4,3-c]pyridin-3-amin;
N-(1-(5-Chlor-2-methoxyphenyl)-6-(pyrazolo[1,5-a]pyrimidin-3-yl)-1H-pyrazolo[4,3-c]pyridin-3-yl)acetamid;
1-(5-Chlor-2-methoxyphenyl)-N-methyl-6-(pyrazolo[1,5-a]pyrimidin-3-yl)-1H-pyrazolo[4,3-c]pyridin-3-amin;
1-(5-Chlor-2-methoxyphenyl)-N,N-dimethyl-6-(pyrazolo[1,5-a]pyrimidin-3-yl)-1H-pyrazolo[4,3-c]pyridin-3-amin;
1-(2-Methoxy-5-(methylsulfonyl)phenyl)-N-(2-morpholinoethyl)-6-(pyrazolo[1,5-a]pyrimidin-3-yl)-1H-pyrazolo[4,3-c]pyridin-3-amin;
N1-(1-(5-(Difluormethyl)-2-methoxyphenyl)-6-(pyrazolo[1,5-a]pyrimidin-3-yl)-1H-pyrazolo[4,3-c]pyridin-3-yl)-N2,N2-dimethylethan-1,2-diamin;
N1-(1-(2-Methoxy-5-methylphenyl)-6-(pyrazolo[1,5-a]pyrimidin-3-yl)-1H-pyrazolo[4,3-c]pyridin-3-yl)-N2,N2-dimethylethan-1,2-diamin;
N 1-(1-(2-(Difluormethoxy)-5-(methylthio)phenyl)-6-(pyrazolo[1,5-a]pyrimidin-3-yl)-1H-pyrazolo[4,3-c]pyridin-3-yl)-N3,N3-dimethylpropan-1,3-diamin;
1-(4-(1-(5-Chlor-2-methoxyphenyl)-6-(pyrazolo[1,5-a]pyrimidin-3-yl)-1H-pyrazolo[4,3-c]pyridin-3-yl)piperazin-1-yl)ethan-1-on;
1-(5-Chlor-2-methoxyphenyl)-3-(piperazin-1-yl)-6-(pyrazolo[1,5-a]pyrimidin-3-yl)-1H-pyrazolo[4,3-c]pyridin;
(1-(1-(5-Chlor-2-methoxyphenyl)-6-(pyrazolo[1,5-a]pyrimidin-3-yl)-1H-pyrazolo[4,3-c]pyridin-3-yl)azetidin-3-yl)methanol;
1-(5-Chlor-2-methoxyphenyl)-3-methoxy-6-(pyrazolo[1,5-a]pyrimidin-3-yl)-1H-pyrazolo[4,3-c]pyridin;
1-(5-Chlor-2-methoxyphenyl)-N,N-dimethyl-6-(pyrazolo[1,5-a]pyrimidin-3-yl)-1H-pyrazolo[4,3-c]pyridin-4-amin;
1-(5-Chlor-2-methoxyphenyl)-N-methyl-6-(pyrazolo[1,5-a]pyrimidin-3-yl)-1H-pyrazolo[4,3-c]pyridin-4-amin;
N-(2-((1-(5-Chlor-2-methoxyphenyl)-6-(pyrazolo[1,5-a]pyrimidin-3-yl)-1H-pyrazolo[4,3-c]pyridin-4-yl)amino)ethyl)acetamid;
2-((1-(5-Chlor-2-methoxyphenyl)-6-(pyrazolo[1,5-a]pyrimidin-3-yl)-1H-pyrazolo[4,3-c]pyridin-4-yl)amino)-N-methylacetamid;
N-(1-(5-Chlor-2-methoxyphenyl)-6-(pyrazolo[1,5-a]pyrimidin-3-yl)-1H-pyrazolo[4,3-c]pyridin-4-yl)acetamid;
1-(5-Chlor-2-methoxyphenyl)-6-(pyrazolo[1,5-a]pyrimidin-3-yl)-1H-pyrazolo[4,3-c]pyridin-4-amin;
1-(5-Chlor-2-methoxyphenyl)-6-(pyrazolo[1,5-a]pyrimidin-3-yl)-1H-pyrazolo[4,3-c]pyridin-4-ol;
2-Fluor-5-methoxy-4-(3-methyl-6-(pyrazolo[1,5-a]pyrimidin-3-yl)-1H-pyrazolo[4,3-c]pyridin-1-yl)phenol;
2-Chlor-5-methoxy-4-(3-methyl-6-(pyrazolo[1,5-a]pyrimidin-3-yl)-1H-pyrazolo[4,3-c]pyridin-1-yl)phenol;
5-Methoxy-2-methyl-4-(3-methyl-6-(pyrazolo[1,5-a]pyrimidin-3-yl)-1H-pyrazolo[4,3-c]pyridin-1-yl)phenol;
1-(2-Chlor-5-methoxypyridin-4-yl)-3-methyl-6-(pyrazolo[1,5-a]pyrimidin-3-yl)-1H-pyrazolo[4,3-c]pyridin;
(4-Chlor-2-(3-methyl-6-(pyrazolo[1,5-a]pyrimidin-3-yl)-1H-pyrazolo[4,3-c]pyridin-1-yl)phenyl)methanol;
1-(5-Brom-2-(difluormethoxy)phenyl)-3-methyl-6-(pyrazolo[1,5-a]pyrimidin-3-yl)-1H-pyrazolo[4,3-c]pyridin;
4-(Difluormethoxy)-3-(3-methyl-6-(pyrazolo[1,5-a]pyrimidin-3-yl)-1H-pyrazolo[4,3-c]pyridin-1-yl)benzonitril;
1-(2-(Difluormethoxy)-5-methylphenyl)-3-methyl-6-(pyrazolo[1,5-a]pyrimidin-3-yl)-1H-pyrazolo[4,3-c]pyridin;
4-(Difluormethoxy)-3-(3-methyl-6-(pyrazolo[1,5-a]pyrimidin-3-yl)-1H-pyrazolo[4,3-c]pyridin-1-yl)-N-(thiazol-2-yl)benzamid;
2-((4-(Difluormethoxy)-3-(3-methyl-6-(pyrazolo[1,5-a]pyrimidin-3-yl)-1H-pyrazolo[4,3-c]pyridin-1-yl)phenyl)thio)-N-methylacetamid;
2-((4-(Difluormethoxy)-3-(3-methyl-6-(pyrazolo[1,5-a]pyrimidin-3-yl)-1H-pyrazolo[4,3-c]pyridin-1-yl)phenyl)thio)acetamid;
2-((4-(Difluormethoxy)-3-(3-methyl-6-(pyrazolo[1,5-a]pyrimidin-3-yl)-1H-pyrazolo[4,3-c]pyridin-1-yl)phenyl)thio)-N-(2-hydroxyethyl)acetamid;
2-((4-(Difluormethoxy)-3-(3-methyl-6-(pyrazolo[1,5-a]pyrimidin-3-yl)-1H-pyrazolo[4,3-c]pyridin-1-yl)phenyl)sulfonyl)acetamid;
1-(5-(Cyclopropylthio)-2-(difluormethoxy)phenyl)-3-methyl-6-(pyrazolo[1,5-a]pyrimidin-3-yl)-1H-pyrazolo[4,3-c]pyridin;
1-(2-(Difluormethoxy)-5-((tetrahydro-2H-pyran-4-yl)thio)phenyl)-3-methyl-6-(pyrazolo[1,5-a]pyrimidin-3-yl)-1H-pyrazolo[4,3-c]pyridin;
1-(2-(Difluormethoxy)-5-(oxetan-3-ylthio)phenyl)-3-methyl-6-(pyrazolo[1,5-a]pyrimidin-3-yl)-1H-pyrazolo[4,3-c]pyridin;
1-(2-(Difluormethoxy)-5-(piperidin-4-ylthio)phenyl)-3-methyl-6-(pyrazolo[1,5-a]pyrimidin-3-yl)-1H-pyrazolo[4,3-c]pyridin;
1-(4-((4-(Difluormethoxy)-3-(3-methyl-6-(pyrazolo[1,5-a]pyrimidin-3-yl)-1H-pyrazolo[4,3-c]pyridin-1-yl)phenyl)thio)piperidin-1-yl)ethan-1-on;
1-((1-(5-Chlor-2-(difluormethoxy)phenyl)-6-(pyrazolo[1,5-a]pyrimidin-3-yl)-1H-pyrazolo[4,3-c]pyridin-3-yl)methyl)azetidin-3-amin;
N-((1r,3r)-3-Aminocyclobutyl)-1-(5-chlor-2-methoxyphenyl)-6-(pyrazolo[1,5-a]pyrimidin-3-yl)-1H-pyrazolo[4,3-c]pyridin-3-carboxamid;
1-(5-Chlor-2-methoxyphenyl)-N-((1r,3r)-3-(methyl(2-(methylamino)-2-oxoethyl)amino)cyclobutyl)-6-(pyrazolo[1,5-a]pyrimidin-3-yl)-1H-pyrazolo[4,3-c]pyridin-3-carboxamid;
1-(5-Chlor-2-methoxyphenyl)-N-((1r,3r)-3-morpholinocyclobutyl)-6-(pyrazolo[1,5-a]pyrimidin-3-yl)-1H-pyrazolo[4,3-c]pyridin-3-carboxamid;
1-(5-Chlor-2-methoxyphenyl)-N-((1r,3r)-3-(dimethylamino)cyclobutyl)-6-(pyrazolo[1,5-a]pyrimidin-3-yl)-1H-pyrazolo[4,3-c]pyridin-3-carboxamid;
N-((1s,3s)-3-Aminocyclobutyl)-1-(5-chlor-2-(difluormethoxy)phenyl)-6-(pyrazolo[1,5-a]pyrimidin-3-yl)-1H-pyrazolo[4,3-c]pyridin-3-carboxamid;
N-(2-(Dimethylamino)ethyl)-1-(2-methoxy-5-(methylsulfonyl)phenyl)-6-(pyrazolo[1,5-a]pyrimidin-3-yl)-1H-pyrazolo[4,3-c]pyridin-3-carboxamid;
N-(3-(Dimethylamino)propyl)-1-(2-methoxy-5-(methylsulfonyl)phenyl)-6-(pyrazolo[1,5-a]pyrimidin-3-yl)-1H-pyrazolo[4,3-c]pyridin-3-carboxamid;
1-(2-Methoxy-5-(methylsulfonyl)phenyl)-N-(3-morpholinopropyl)-6-(pyrazolo[1,5-a]pyrimidin-3-yl)-1H-pyrazolo[4,3-c]pyridin-3-carboxamid;
1-(2-Methoxy-5-(methylsulfonyl)phenyl)-N-methyl-6-(pyrazolo[1,5-a]pyrimidin-3-yl)-1H-pyrazolo[4,3-c]pyridin-3-amin;
N-(3-(3-((2-(Dimethylamino)ethyl)amino)-6-(pyrazolo[1,5-a]pyrimidin-3-yl)-1H-pyrazolo[4,3-c]pyridin-1-yl)-4-methoxyphenyl)methansulfonamid;
N-(1-(5-Chlor-2-methoxyphenyl)-6-(pyrazolo[1,5-a]pyrimidin-3-yl)-1H-pyrazolo[4,3-c]pyridin-3-yl)-2-(methylamino)acetamid;
N-(3-(Dimethylamino)propyl)-1-(5-fluor-4-hydroxy-2-methoxyphenyl)-6-(pyrazolo[1,5-a]pyrimidin-3-yl)-1H-pyrazolo[4,3-c]pyridin-3-carboxamid;
1-(1-(2-Methoxy-5-(methylsulfonyl)phenyl)-6-(pyrazolo[1,5-a]pyrimidin-3-yl)-1H-pyrazolo[4,3-c]pyridin-3-yl)-N-methylmethanamin;
3-((1-(5-Fluor-2-methoxyphenyl)-3-methyl-1H-pyrazolo[4,3-c]pyridin-6-yl)amino)pyrazin-2(1H)-on;
3-((1-(5-(Difluormethyl)-2-methoxyphenyl)-3-methyl-1H-pyrazolo[4,3-c]pyridin-6-yl)amino)pyrazin-2(1H)-on;
N-(2-Hydroxyethyl)-4-methoxy-3-(3-methyl-6-((3-oxo-3,4-dihydropyrazin-2-yl)amino)-1H-pyrazolo[4,3-c]pyridin-1-yl)benzolsulfonamid;
3-((1-(2-(Difluormethoxy)-5-((2-methoxyethyl)thio)phenyl)-3-methyl-1H-pyrazolo[4,3-c]pyridin-6-yl)amino)pyrazin-2(1H)-on;
3-((1-(2-(Difluormethoxy)-5-(methylsulfonyl)phenyl)-3-methyl-1H-pyrazolo[4,3-c]pyridin-6-yl)amino)pyrazin-2(1H)-on;
3-((1-(2-(Difluormethoxy)-5-((2-methoxyethyl)sulfonyl)phenyl)-3-methyl-1H-pyrazolo[4,3-c]pyridin-6-yl)amino)pyrazin-2(1H)-on;
N-(3-(Dimethylamino)propyl)-1-(5-fluor-2-methoxyphenyl)-6-((3-oxo-3,4-dihydropyrazin-2-yl)amino)-1H-pyrazolo[4,3-c]pyridin-3-carboxamid;
3-((3-((3-(Dimethylamino)propyl)amino)-1-(5-fluor-2-methoxyphenyl)-1H-pyrazolo[4,3-c]pyridin-6-yl)amino)pyrazin-2(1H)-on;
3-((1-(5-(Difluormethyl)-2-methoxyphenyl)-3-((2-(dimethylamino)ethyl)amino)-1H-pyrazolo[4,3-c]pyridin-6-yl)amino)pyrazin-2(1H)-on;
3-((1-(5-Chlor-2-(difluormethoxy)phenyl)-3-methyl-1H-pyrazolo[4,3-c]pyridin-6-yl)amino)pyrazin-2(1H)-on;
einzelne Enantiomere, Diastereoisomere und Mischungen davon oder ein pharmazeutisch annehmbares Salz oder Solvat davon.

6. Pharmazeutische Zusammensetzung, umfassend eine Verbindung nach einem der Ansprüche 1 bis 5 oder ein pharmazeutisch annehmbares Salz davon, in Beimischung mit einem oder mehreren pharmazeutisch annehmbaren Träger(n) oder Hilfsstoff(en).

7. Pharmazeutische Zusammensetzung nach Anspruch 6, die zur Verabreichung durch Inhalation geeignet und ausgewählt ist aus inhalierbaren Pulvern, treibgashaltigen Dosieraerosolen oder treibgasfreien inhalierbaren Formulierungen.

8. Vorrichtung, umfassend die pharmazeutische Zusammensetzung nach Anspruch 7, die ein Trockenpulverinhalator für Einzel- oder Mehrfachdosen, ein Dosierinhalator oder ein Soft-Mist-Inhalator sein kann.

9. Verbindung oder pharmazeutische Zusammensetzung nach einem der Ansprüche 1 bis 7 zur Verwendung als Medikament.

10. Verbindung oder pharmazeutische Zusammensetzung zur Verwendung nach Anspruch 9 bei der Vorbeugung und/oder Behandlung einer Lungenkrankheit, die ausgewählt ist aus der Gruppe bestehend aus Asthma, chronisch obstruktiver Lungenkrankheit COPD, idiopathischer Lungenfibrose (IPF), akuter Lungenschädigung und akutem Atemnotsyndrom (ARDS).

11. Kombination einer Verbindung nach einem der Ansprüche 1 bis 5 mit einem oder mehreren Wirkstoff(en), der/die ausgewählt ist/sind aus den Klassen bestehend aus mit anderen pharmazeutischen Wirkstoffen, die ausgewählt sind aus der Gruppe bestehend aus Beta2-Agonisten, Antimuskarinika, Kortikosteroiden, Inhibitoren von Mitogen-aktivierten Kinasen (P38 MAP-Kinasen), Inhibitoren von Nuklearfaktor Kappa-B-Kinase Untereinheit Beta (IKK2), Inhibitoren von humaner neutrophiler Elastase (HNE), Inhibitoren von Phosphodiesterase 4 (PDE4), Leukotrienmodulatoren, nicht-steroidalen Antiphlogistika (NSAID) und Schleimregulatoren.

## Revendications

1. Composé de formule I 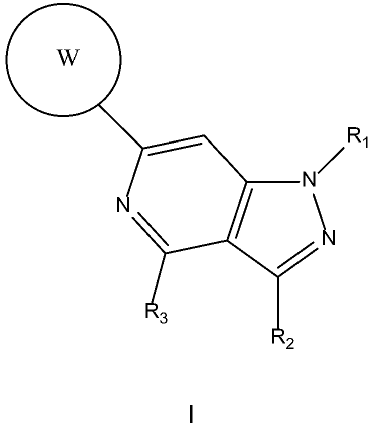 dans lequel,
**W est** un hétéroaryle choisi parmi pyrazolo[1,5-a]pyrimidin-3-yle, imidazo[1,2-b]pyridazin-3-yle et (3-oxo-3,4-dihydropyrazin-2-yl)amino ;
**R₁ est** choisi dans le groupe de pyridinyle, pipéridinyle, phényle ou benzyle substitué par au moins 2 ou 3 groupes, indépendamment choisis parmi
halogène,
-OH,
-CN
-NO₂
-(CH₂)ₘNR₄R₅,
alkyle en (C₁-C₆),
hydroxyalkyle en (C₁-C₆),
alcoxy en (C₁-C₆),
alkylthio en (C₁-C₆)-
haloalkyle en (C₁-C₆),
haloalcoxy en (C₁-C₆),
haloalkylthio en (C₁-C₆)-
et
un groupe de formule **K** 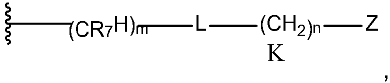 en position méta par rapport au point d'attachement de R₁ avec le reste de la molécule ;
dans lequel
**L** est absent ou est un groupe divalent choisi parmi O, S, S(O)₂, (CO), C(O)O, O(O)C, C(O)N(R₆), N(R₆)C(O), NHCONH, N(R₆)S(O)₂, S(O)₂N(R₆) ;
**Z** est choisi dans le groupe constitué de H, -OH, -CN, -NO₂, alkyle en (C₁-C₆), hydroxyalkyle en (C₁-C₆), haloalkyle en (C₁-C₆), alcoxy en (C₁-C₆), alcoxy en (C₁-C₆)-alkyle en (C₁-C₆), alcoxycarbonyle en (C₁-C₆), -(CH₂)ₘNR₄R₅, - C(O)NH(R₆), cycloalkyle en (C₃-C₈), aryle, hétéroaryle et hétérocycloalkyle en (C₃-C₆) ; dans lequel lesdits cycloalkyle en (C₃-C₈), aryle, hétéroaryle et hétérocycloalkyle en (C₃-C₆) sont facultativement en outre substitués par un ou plusieurs substituants choisis dans le groupe constitué de
alkyle en (C₁-C₁₀), alcoxy en (C₁-C₆), alcanoyle, alcoxycarbonyle en (C₁-C₆), oxo, -C(O)NH(R₆), alcoxy en (C₁-C₆)-alkyle en (C₁-C₆) ;
**R₃** est choisi indépendamment dans le groupe constitué de H, alkyle en (C₁-C₆), et
**R₂ est** choisi indépendamment dans le groupe constitué de H, d'alkyle en (C₁-C₆), et d'un groupe de formule **J** 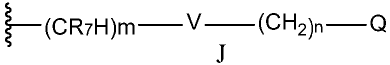
dans lequel
**V** est absent ou est un groupe divalent choisi parmi O, S, S(O)₂, C(O), C(O)O, O(O)C, C(O)N(R₆), N(R₆)C(O) ; N(R₆)-(CH₂)ₘ-N(R₆),-N(R₆)-,
**Q** est choisi dans le groupe constitué de H, -CN, -OH, alkyle en (C₁-C₆), hydroxyalkyle en (C₁-C₆), haloalkyle en (C₁-C₆), alcoxy en (C₁-C₆), alcoxycarbonyle en (C₁-C₆), hydroxycarbonyle, -(CH₂)ₘNR₄R₅, -C(O)NR₄R₅, - N(R₆)C(O)R₆, -CH(CN)NR₄R₅, cycloalkyle en (C₃-C₈), aryle, hétéroaryle et hétérocycloalkyle en (C₃-C₆) ; dans lequel lesdits cycloalkyle en (C₃-C₈), aryle, hétéroaryle et hétérocycloalkyle en (C₃-C₆) sont facultativement en outre substitués par un ou plusieurs substituants choisis parmi
-OH, oxo, alkyle en (C₁-C₁₀), alkyle en (C₁-C₁₀)-S(O)₂-O-, alcanoyle, hydroxyalkyle en (C₁-C₆), alcoxy en (C₁-C₆)-alkyle en(C₁-C₆), alcoxycarbonyle en (C₁-C₆), alcoxycarbonyle en (C₁-C₆)-NH-, -N(R₆)(CH₂)ₘC(O)NR₄R₅, -NR₄R₅, hétérocycloalkyle en (C₃-C₆) ;
**n et m valent dans chaque occurrence indépendamment** 0 ou un nombre entier choisi parmi 1, 2, 3 et 4 ;
**R₄ et R₅**, identiques ou différents, sont choisis dans le groupe constitué de
-H,
alkyle en (C₁-C₆),
haloalkyle en (C₁-C₆),
hydroxyalkyle en (C₁-C₆),
alcanoyle, alcoxycarbonyle en (C₁-C₆), et
hétérocycloalkyle en (C₃-C₆) ;
**R₆** est dans chaque occurrence indépendamment choisi dans le groupe constitué de H, alkyle en (C₁-C₆), hydroxyalkyle en (C₁-C₆), et alcanoyle,
**R₇** est dans chaque occurrence indépendamment choisi dans le groupe constitué de H, alkyle en (C₁-C₆), -NR₄R₅
énantiomères simples, diastéréo-isomères et mélanges de ceux-ci dans l'une quelconque proportion,
ou sels et solvates pharmaceutiquement acceptables de ceux-ci.

2. Composé selon la revendication 1
dans lequel R₁ est phényle substitué par 2 ou 3 groupes, indépendamment choisis parmi halogène, de préférence Cl et F,
-OH,
alcoxy en (C₁-C₆), de préférence méthoxy,
alkylthio en (C₁-C₆)- de préférence méthylthio
haloalcoxy en (C₁-C₆), de préférence difluorométhoxy

3. Composé selon la revendication 1 représenté par la formule (Ib) 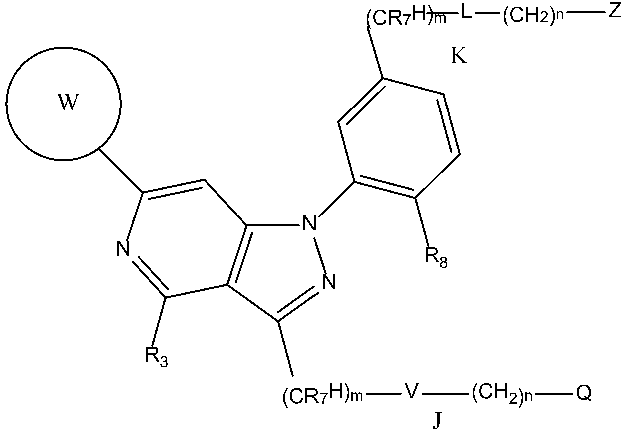 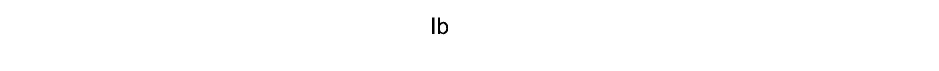 dans lequel
**R₈** est choisi dans le groupe constitué de
alcoxy en (C₁-C₆),
haloalcoxy en (C₁-C₆) ;
**L** est choisi parmi le groupe divalent constitué de O, S, S(O)₂, C(O)O, O(O)C, C(O)N(R₆), N(R₆)C(O) ;
**Z** est choisi dans le groupe constitué de H, alkyle en (C₁-C₆), hydroxyalkyle en (C₁-C₆), haloalkyle en (C₁-C₆), alcoxy en (C₁-C₆), alcoxycarbonyle en (C₁-C₆), -(CH₂)ₘNR₄R₅, -C(O)NH(R₆), cycloalkyle en (C₃-C₈), aryle, hétéroaryle et hétérocycloalkyle en (C₃-C₆) dans lequel lesdits cycloalkyle en (C₃-C₈), aryle, hétéroaryle et hétérocycloalkyle en (C₃-C₆) sont facultativement en outre substitués par un ou plusieurs substituants choisis dans le groupe constitué de
alkyle en (C₁-C₁₀), alcanoyle, alcoxycarbonyle en (C₁-C₆), -C(O)NH(R₆), alcoxy en (C₁-C₆)-alkyle en (C₁-C₆)alkyle ;
**R₃** est -H ou alkyle en (C₁-C₆) ;
**V** est absent ou est choisi parmi le groupe divalent constitué de O, S, S(O)₂, C(O), C(O)O, O(O)C, C(O)N(R₆), N(R₆)C(O) ; N(R₆)-(CH₂)ₘ-N(R₆), -N(R₆)-,
**Q** est choisi dans le groupe constitué de H, -CN, -OH, alkyle en (C₁-C₆), hydroxyalkyle en (C₁-C₆), haloalkyle en (C₁-C₆), alcoxy en (C₁-C₆), alcoxycarbonyle en (C₁-C₆), -(CH₂)ₘNR₄R₅, -C(O)NR₄R₅, -N(R₆)C(O)R₆, - CH(CN)NR₄R₅, cycloalkyle en (C₃-C₈), aryle, hétéroaryle et hétérocycloalkyle en (C₃-C₆), dans lequel lesdits cycloalkyle en (C₃-C₈), aryle, hétéroaryle et hétérocycloalkyle en (C₃-C₆) sont facultativement en outre substitués par un ou plusieurs substituants choisis dans le groupe constitué de -OH, oxo, alkyle en (C₁-C₁₀), alkyle en (C₁-C₁₀)-S(O)₂-O-, alcanoyle, hydroxyalkyle en (C₁-C₆), alcoxy en (C₁-C₆)- alkyle en(C₁-C₆), alcoxycarbonyle en (C₁-C₆), alcoxycarbonyle en (C₁-C₆)-NH-, -N(R₆)(CH₂)ₘC(O)NR₄R₅, -NR₄R₅, hétérocycloalkyle en (C₃-C₆) ;
**n et m valent dans chaque occurrence indépendamment** 0 ou un nombre entier allant de 1 à 4 ;
**R₄ et R₅**, identiques ou différents, sont choisis dans le groupe constitué de
-H,
alkyle en (C₁-C₆),
haloalkyle en (C₁-C₆),
hydroxyalkyle en (C₁-C₆),
alcanoyle,
alcoxycarbonyle en (C₁-C₆), et
hétérocycloalkyle en (C₃-C₆) ;
**R₆** est dans chaque occurrence indépendamment choisi dans le groupe constitué de H, alkyle en (C₁-C₆), hydroxyalkyle en (C₁-C₆),
**R₇** est dans chaque occurrence indépendamment choisi dans le groupe constitué de H, alkyle en (C₁-C₆),
énantiomères simples, diastéréo-isomères et mélanges de ceux-ci
ou sel ou solvate pharmaceutiquement acceptable de ceux-ci.

4. Composé selon la revendication 1 ou 2 représenté dans la formule (Ib1) 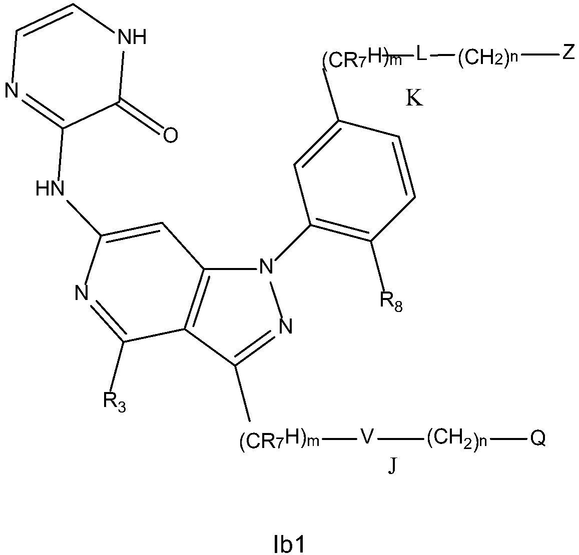 dans lequel
**R₈** est choisi dans le groupe constitué de
alcoxy en (C₁-C₆),
haloalcoxy en (C₁-C₆) ;
**L** est choisi parmi le groupe divalent constitué de O, S, S(O)₂, C(O)O, O(O)C, C(O)N(R₆), N(R₆)C(O) ;
**Z** est choisi dans le groupe constitué de H, alkyle en (C₁-C₆), hydroxyalkyle en (C₁-C₆), haloalkyle en (C₁-C₆), alcoxy en (C₁-C₆), alcoxycarbonyle en (C₁-C₆), -(CH₂)ₘNR₄R₅, -C(O)NH(R₆),
cycloalkyle en (C₃-C₈), aryle, hétéroaryle et hétérocycloalkyle en (C₃-C₆) facultativement substitués par un ou plusieurs substituants choisis dans le groupe constitué de
alkyle en (C₁-C₁₀), alcanoyle, alcoxycarbonyle en (C₁-C₆), -C(O)NH(R₆), alcoxy en (C₁-C₆)-alkyle en (C₁-C₆)alkyle ;
**R₃** est -H ou alkyle en (C₁-C₆) ;
**V** est absent ou est choisi parmi le groupe divalent constitué de O, S, S(O)₂, C(O), C(O)O, O(O)C, C(O)N(R₆), N(R₆)C(O) ; N(R₆)-(CH₂)ₘ-N(R₆), -N(R₆)-,
**Q** est choisi dans le groupe constitué de H, -CN, -OH, alkyle en (C₁-C₆), hydroxyalkyle en (C₁-C₆), haloalkyle en (C₁-C₆), alcoxy en (C₁-C₆), alcoxycarbonyle en (C₁-C₆), -(CH₂)ₘNR₄R₅, -C(O)NR₄R₅, -N(R₆)C(O)R₆, - CH(CN)NR₄R₅,
cycloalkyle en (C₃-C₈), aryle, hétéroaryle et hétérocycloalkyle en (C₃-C₆) facultativement substitué par un ou plusieurs substituants choisis dans le groupe constitué de -OH, oxo, alkyle en (C₁-C₁₀), alkyle en (C₁-C₁₀)-S(O)₂-O-, alcanoyle, hydroxyalkyle en (C₁-C₆), alcoxy en (C₁-C₆)-alkyle en (C₁-C₆), alcoxycarbonyle en (C₁-C₆), alcoxycarbonyle en (C₁-C₆)-NH-, -N(R₆)(CH₂)ₘC(O)NR₄R₅, -NR₄R₅, hétérocycloalkyle en (C₃-C₆) ;
**n et m valent dans chaque occurrence indépendamment** 0 ou un nombre entier allant de 1 à 4 ;
**R₄ et R₅**, identiques ou différents, sont choisis dans le groupe constitué de
-H,
alkyle en (C₁-C₆),
haloalkyle en (C₁-C₆),
hydroxyalkyle en (C₁-C₆),
alcanoyle, alcoxycarbonyle en (C₁-C₆), et
hétérocycloalkyle en (C₃-C₆) ;
**R₆** est dans chaque occurrence indépendamment choisi dans le groupe constitué de H, alkyle en (C₁-C₆), hydroxyalkyle en (C₁-C₆),
**R₇** est dans chaque occurrence indépendamment choisi dans le groupe constitué de H, alkyle en (C₁-C₆),
énantiomères simples, diastéréo-isomères et mélanges de ceux-ci dans l'une quelconque proportion, ou sels et solvates pharmaceutiquement acceptables de ceux-ci.

5. Composé selon la revendication 1 choisi parmi :
(1-(5-chloro-2-(difluorométhoxy)phényl)-6-(pyrazolo[1,5-a]pyrimidin-3-yl)-1H-pyrazolo[4,3-b]pyridin-3-yl)méthanol ;
1-(5-chloro-2-(difluorométhoxy)phényl)-6-(pyrazolo[1,5-a]pyrimidin-3-yl)-1H-pyrazolo[4,3-b]pyridin-3-carboxylate de méthyle ;
2-(1-(5-chloro-2-(difluorométhoxy)phényl)-6-(pyrazolo[1,5-a]pyrimidin-3-yl)-1H-pyrazolo[4,3-b]pyridin-3-yl)-N,N-diméthylacétamide ;
acide 2-(1-(5-chloro-2-(difluorométhoxy)phényl)-6-(pyrazolo[1,5-a]pyrimidin-3-yl)-1H-pyrazolo[4,3-b]pyridin-3-yl)acétique ;
1-(5-chloro-2-(difluorométhoxy)phényl)-6-(imidazo[1,2-b]pyridazin-3-yl)-3-méthyl-1H-pyrazolo[4,3-c]pyridine ;
1-(5-chloro-2-(difluorométhoxy)phényl)-3-méthyl-6-(pyrazolo[1,5-a]pyrimidin-3-yl)-1H-pyrazolo[4,3-c]pyridine ;
1-(4-chloro-2-fluorobenzyl)-6-(imidazo[1,2-b]pyridazin-3-yl)-3-méthyl-1H-pyrazolo[4,3-b]pyridine ;
1-(5-chloro-2-(difluorométhoxy)phényl)-3-méthyl-6-(pyrazolo[1,5-a]pyrimidin-3-yl)-1H-pyrazolo[4,3-b]pyridine ;
3-(3-(3-méthyl-6-(pyrazolo[1,5-a]pyrimidin-3-yl)-1H-pyrazolo[4,3-b]pyridin-1-yl)pipéridin-1-yl)-3-oxopropanenitrile ;
1-(5-fluoro-2-méthoxyphényl)-3-méthyl-6-(pyrazolo[1,5-a]pyrimidin-3-yl)-1H-pyrazolo[4,3-c]pyridine ;
1-(5-chloro-2-méthoxyphényl)-3-méthyl-6-(pyrazolo[1,5-a]pyrimidin-3-yl)-1H-pyrazolo[4,3-c]pyridine ;
1-(5-(difluorométhyl)-2-méthoxyphényl)-3-méthyl-6-(pyrazolo[1,5-a]pyrimidin-3-yl)-1H-pyrazolo[4,3-c]pyridine ;
1-(2-méthoxy-5-(trifluorométhyl)phényl)-3-méthyl-6-(pyrazolo[1,5-a]pyrimidin-3-yl)-1H-pyrazolo[4,3-c]pyridine ;
1-(5-chloro-2-(méthylthio)phényl)-3-méthyl-6-(pyrazolo[1,5-a]pyrimidin-3-yl)-1H-pyrazolo[4,3-c]pyridine ;
1-(5-chloro-2-((difluorométhyl)thio)phényl)-3-méthyl-6-(pyrazolo[1,5-a]pyrimidin-3-yl)-1H-pyrazolo[4,3-c]pyridine ;
1-(5-chloro-2-cyclopropoxyphényl)-3-méthyl-6-(pyrazolo[1,5-a]pyrimidin-3-yl)-1H-pyrazolo[4,3-c]pyridine ;
1-(2,5-diméthoxyphényl)-3-méthyl-6-(pyrazolo[1,5-a]pyrimidin-3-yl)-1H-pyrazolo[4,3-c]pyridine ;
3-((4-méthoxy-3-(3-méthyl-6-(pyrazolo[1,5-a]pyrimidin-3-yl)-1H-pyrazolo[4,3-c]pyridin-1-yl)phényl)sulfonyl)propan-1-ol ;
1-(2-méthoxy-5-(propylsulfonyl)phényl)-3-méthyl-6-(pyrazolo[1,5-a]pyrimidin-3-yl)-1H-pyrazolo[4,3-c]pyridine ;
1-(5-chloro-2-(difluorométhoxy)phényl)-3-méthyl-6-(pyrazolo[1,5-a]pyrimidin-3-yl)-1H-pyrazolo[4,3-c]pyridine ;
4-chloro-2-(3-méthyl-6-(pyrazolo[1,5-a]pyrimidin-3-yl)-1H-pyrazolo[4,3-c]pyridin-1-yl)phénol ;
1-(2-(difluorométhoxy)-5-(méthylthio)phényl)-3-méthyl-6-(pyrazolo[1,5-a]pyrimidin-3-yl)-1H-pyrazolo[4,3-c]pyridine ;
4-méthoxy-N-méthyl-3-(3-méthyl-6-(pyrazolo[1,5-a]pyrimidin-3-yl)-1H-pyrazolo[4,3-c]pyridin-1-yl)benzènesulfonamide ;
N-(2-hydroxyéthyl)-4-méthoxy-3-(3-méthyl-6-(pyrazolo[1,5-a]pyrimidin-3-yl)-1H-pyrazolo[4,3-c]pyridin-1-yl)benzènesulfonamide ;
4-méthoxy-3-(3-méthyl-6-(pyrazolo[1,5-a]pyrimidin-3-yl)-1H-pyrazolo[4,3-c]pyridin-1-yl)-N-(3-(4-méthylpipérazin-1-yl)propyl)benzènesulfonamide ;
4-méthoxy-3-(3-méthyl-6-(pyrazolo[1,5-a]pyrimidin-3-yl)-1H-pyrazolo[4,3-c]pyridin-1-yl)-N-((1-méthylazetidin-3-yl)méthyl)benzènesulfonamide ;
4-méthoxy-3-(3-méthyl-6-(pyrazolo[1,5-a]pyrimidin-3-yl)-1H-pyrazolo[4,3-c]pyridin-1-yl)-N-(2-(4-méthylpipérazin-1-yl)éthyl)benzènesulfonamide ;
4-méthoxy-3-(3-méthyl-6-(pyrazolo[1,5-a]pyrimidin-3-yl)-1H-pyrazolo[4,3-c]pyridin-1-yl)-N-(2-morpholinoéthyl)benzènesulfonamide ;
1-(2-(difluorométhoxy)-5-((2-méthoxyéthyl)sulfonyl)phényl)-3-méthyl-6-(pyrazolo[1,5-a]pyrimidin-3-yl)-1H-pyrazolo[4,3-c]pyridine ;
3-((4-(difluorométhoxy)-3-(3-méthyl-6-(pyrazolo[1,5-a]pyrimidin-3-yl)-1H-pyrazolo[4,3-c]pyridin-1-yl)phényl)sulfonyl)-N,N-diméthylpropan-1-amine ;
2-((4-(difluorométhoxy)-3-(3-méthyl-6-(pyrazolo[1,5-a]pyrimidin-3-yl)-1H-pyrazolo[4,3-c]pyridin-1-yl)phényl)sulfonyl)éthan-1-ol ;
1-(2-(difluorométhoxy)-5-((2-(pipéridin-1-yl)éthyl)sulfonyl)phényl)-3-méthyl-6-(pyrazolo[1,5-a]pyrimidin-3-yl)-1H-pyrazolo[4,3-c]pyridine ;
1-(3-((4-(difluorométhoxy)-3-(3-méthyl-6-(pyrazolo[1,5-a]pyrimidin-3-yl)-1H-pyrazolo[4,3-c]pyridin-1-yl)phényl)sulfonyl)pyrrolidin-1-yl)éthan-1-one ;
1-(2-(difluorométhoxy)-5-((3-méthoxyphényl)sulfonyl)phényl)-3-méthyl-6-(pyrazolo[1,5-a]pyrimidin-3-yl)-1H-pyrazolo[4,3-c]pyridine ;
1-(2-(difluorométhoxy)-5-(méthylsulfonyl)phényl)-3-méthyl-6-(pyrazolo[1,5-a]pyrimidin-3-yl)-1H-pyrazolo[4,3-c]pyridine ;
3-méthoxy-4-(3-méthyl-6-(pyrazolo[1,5-a]pyrimidin-3-yl)-1H-pyrazolo[4,3-c]pyridin-1-yl)phénol ;
2-(1-(5-chloro-2-(difluorométhoxy)phényl)-6-(pyrazolo[1,5-a]pyrimidin-3-yl)-1H-pyrazolo[4,3-c]pyridin-3-yl)acétaonitrile ;
(1-(5-chloro-2-(difluorométhoxy)phényl)-6-(pyrazolo[1,5-a]pyrimidin-3-yl)-1H-pyrazolo[4,3-c]pyridin-3-yl)méthanol ;
1-(1-(5-chloro-2-(difluorométhoxy)phényl)-6-(pyrazolo[1,5-a]pyrimidin-3-yl)-1H-pyrazolo[4,3-c]pyridin-3-yl)-N,N-diméthylméthanamine ;
1-(1-(5-chloro-2-(difluorométhoxy)phényl)-6-(pyrazolo[1,5-a]pyrimidin-3-yl)-1H-pyrazolo[4,3-c]pyridin-3-yl)-N-méthylméthanamine ;
(1-(5-chloro-2-(difluorométhoxy)phényl)-6-(pyrazolo[1,5-a]pyrimidin-3-yl)-1H-pyrazolo[4,3-c]pyridin-3-yl)méthanamine ;
1-(5-chloro-2-(difluorométhoxy)phényl)-N-méthyl-6-(pyrazolo[1,5-a]pyrimidin-3-yl)-1H-pyrazolo[4,3-c]pyridin-3-carboxamide ;
1-(5-chloro-2-méthoxyphényl)-N-((1s,3s)-3-hydroxycyclobutyl)-6-(pyrazolo[1,5-a]pyrimidin-3-yl)-1H-pyrazolo[4,3-c]pyridin-3-carboxamide ;
(1-(5-chloro-2-(difluorométhoxy)phényl)-6-(pyrazolo[1,5-a]pyrimidin-3-yl)-1H-pyrazolo[4,3-c]pyridin-3-yl)(1,1-dioxidothiomorpholino)méthanone ;
1-(5-chloro-2-(difluorométhoxy)phényl)-N-((1s,3s)-3-(diméthylamino)cyclobutyl)-6-(pyrazolo[1,5-a]pyrimidin-3-yl)-1H-pyrazolo[4,3-c]pyridin-3-carboxamide ;
1-(2-(difluorométhoxy)-5-(méthylthio)phényl)-N-(3-(diméthylamino)propyl)-6-(pyrazolo[1,5-a]pyrimidin-3-yl)-1H-pyrazolo[4,3-c]pyridin-3-carboxamide ;
N-(3-(diméthylamino)propyl)-1-(5-fluoro-2-méthoxyphényl)-6-(pyrazolo[1,5-a]pyrimidin-3-yl)-1H-pyrazolo[4,3-c]pyridin-3-carboxamide ;
1-(5-chloro-2-méthoxyphényl)-6-(pyrazolo[1,5-a]pyrimidin-3-yl)-1H-pyrazolo[4,3-c]pyridin-3-amine ;
N-(1-(5-chloro-2-méthoxyphényl)-6-(pyrazolo[1,5-a]pyrimidin-3-yl)-1H-pyrazolo[4,3-c]pyridin-3-yl)acétamide ;
1-(5-chloro-2-méthoxyphényl)-N-méthyl-6-(pyrazolo[1,5-a]pyrimidin-3-yl)-1H-pyrazolo[4,3-c]pyridin-3-amine ;
1-(5-chloro-2-méthoxyphényl)-N,N-diméthyl-6-(pyrazolo[1,5-a]pyrimidin-3-yl)-1H-pyrazolo[4,3-c]pyridin-3-amine ;
1-(2-méthoxy-5-(méthylsulfonyl)phényl)-N-(2-morpholinoéthyl)-6-(pyrazolo[1,5-a]pyrimidin-3-yl)-1H-pyrazolo[4,3-c]pyridin-3-amine ;
N1-(1-(5-(difluorométhyl)-2-méthoxyphényl)-6-(pyrazolo[1,5-a]pyrimidin-3-yl)-1H-pyrazolo[4,3-c]pyridin-3-yl)-N2,N2-diméthyléthane-1,2-diamine ;
N1-(1-(2-méthoxy-5-méthylphényl)-6-(pyrazolo[1,5-a]pyrimidin-3-yl)-1H-pyrazolo[4,3-c]pyridin-3-yl)-N2,N2-diméthyléthane-1,2-diamine ;
N 1-(1-(2-(difluorométhoxy)-5-(méthylthio)phényl)-6-(pyrazolo[1,5-a]pyrimidin-3-yl)-1H-pyrazolo[4,3-c]pyridin-3-yl)-N3,N3-diméthylpropane-1,3-diamine ;
1-(4-(1-(5-chloro-2-méthoxyphényl)-6-(pyrazolo[1,5-a]pyrimidin-3-yl)-1H-pyrazolo[4,3-c]pyridin-3-yl)pipérazin-1-yl)éthan-1-one ;
1-(5-chloro-2-méthoxyphényl)-3-(pipérazin-1-yl)-6-(pyrazolo[1,5-a]pyrimidin-3-yl)-1H-pyrazolo[4,3-c]pyridine ;
(1-(1-(5-chloro-2-méthoxyphényl)-6-(pyrazolo[1,5-a]pyrimidin-3-yl)-1H-pyrazolo[4,3-c]pyridin-3-yl)azetidin-3-yl)méthanol ;
1-(5-chloro-2-méthoxyphényl)-3-méthoxy-6-(pyrazolo[1,5-a]pyrimidin-3-yl)-1H-pyrazolo[4,3-c]pyridine ;
1-(5-chloro-2-méthoxyphényl)-N,N-diméthyl-6-(pyrazolo[1,5-a]pyrimidin-3-yl)-1H-pyrazolo[4,3-c]pyridin-4-amine ;
1-(5-chloro-2-méthoxyphényl)-N-méthyl-6-(pyrazolo[1,5-a]pyrimidin-3-yl)-1H-pyrazolo[4,3-c]pyridin-4-amine ;
N-(2-((1-(5-chloro-2-méthoxyphényl)-6-(pyrazolo[1,5-a]pyrimidin-3-yl)-1H-pyrazolo[4,3-c]pyridin-4-yl)amino)éthyl)acétamide ;
2-((1-(5-chloro-2-méthoxyphényl)-6-(pyrazolo[1,5-a]pyrimidin-3-yl)-1H-pyrazolo[4,3-c]pyridin-4-yl)amino)-N-méthylacétamide ;
N-(1-(5-chloro-2-méthoxyphényl)-6-(pyrazolo[1,5-a]pyrimidin-3-yl)-1H-pyrazolo[4,3-c]pyridin-4-yl)acétamide ;
1-(5-chloro-2-méthoxyphényl)-6-(pyrazolo[1,5-a]pyrimidin-3-yl)-1H-pyrazolo[4,3-c]pyridin-4-amine ;
1-(5-chloro-2-méthoxyphényl)-6-(pyrazolo[1,5-a]pyrimidin-3-yl)-1H-pyrazolo[4,3-c]pyridin-4-ol ;
2-fluoro-5-méthoxy-4-(3-méthyl-6-(pyrazolo[1,5-a]pyrimidin-3-yl)-1H-pyrazolo[4,3-c]pyridin-1-yl)phénol ;
2-chloro-5-méthoxy-4-(3-méthyl-6-(pyrazolo[1,5-a]pyrimidin-3-yl)-1H-pyrazolo[4,3-c]pyridin-1-yl)phénol ;
5-méthoxy-2-méthyl-4-(3-méthyl-6-(pyrazolo[1,5-a]pyrimidin-3-yl)-1H-pyrazolo[4,3-c]pyridin-1-yl)phénol ;
1-(2-chloro-5-méthoxypyridin-4-yl)-3-méthyl-6-(pyrazolo[1,5-a]pyrimidin-3-yl)-1H-pyrazolo[4,3-c]pyridine ;
(4-chloro-2-(3-méthyl-6-(pyrazolo[1,5-a]pyrimidin-3-yl)-1H-pyrazolo[4,3-c]pyridin-1-yl)phényl)méthanol ;
1-(5-bromo-2-(difluorométhoxy)phényl)-3-méthyl-6-(pyrazolo[1,5-a]pyrimidin-3-yl)-1H-pyrazolo[4,3-c]pyridine ;
4-(difluorométhoxy)-3-(3-méthyl-6-(pyrazolo[1,5-a]pyrimidin-3-yl)-1H-pyrazolo[4,3-c]pyridin-1-yl)benzonitrile ;
1-(2-(difluorométhoxy)-5-méthylphényl)-3-méthyl-6-(pyrazolo[1,5-a]pyrimidin-3-yl)-1H-pyrazolo[4,3-c]pyridine ;
4-(difluorométhoxy)-3-(3-méthyl-6-(pyrazolo[1,5-a]pyrimidin-3-yl)-1H-pyrazolo[4,3-c]pyridin-1-yl)-N-(thiazol-2-yl)benzamide ;
2-((4-(difluorométhoxy)-3-(3-méthyl-6-(pyrazolo[1,5-a]pyrimidin-3-yl)-1H-pyrazolo[4,3-c]pyridin-1-yl)phényl)thio)-N-méthylacétamide ;
2-((4-(difluorométhoxy)-3-(3-méthyl-6-(pyrazolo[1,5-a]pyrimidin-3-yl)-1H-pyrazolo[4,3-c]pyridin-1-yl)phényl)thio)acétamide ;
2-((4-(difluorométhoxy)-3-(3-méthyl-6-(pyrazolo[1,5-a]pyrimidin-3-yl)-1H-pyrazolo[4,3-c]pyridin-1-yl)phényl)thio)-N-(2-hydroxyéthyl)acétamide ;
2-((4-(difluorométhoxy)-3-(3-méthyl-6-(pyrazolo[1,5-a]pyrimidin-3-yl)-1H-pyrazolo[4,3-c]pyridin-1-yl)phényl)sulfonyl)acétamide ;
1-(5-(cyclopropylthio)-2-(difluorométhoxy)phényl)-3-méthyl-6-(pyrazolo[1,5-a]pyrimidin-3-yl)-1H-pyrazolo[4,3-c]pyridine ;
1-(2-(difluorométhoxy)-5-((tétrahydro-2H-pyran-4-yl)thio)phényl)-3-méthyl-6-(pyrazolo[1,5-a]pyrimidin-3-yl)-1H-pyrazolo[4,3-c]pyridine ;
1-(2-(difluorométhoxy)-5-(oxétan-3-ylthio)phényl)-3-méthyl-6-(pyrazolo[1,5-a]pyrimidin-3-yl)-1H-pyrazolo[4,3-c]pyridine ;
1-(2-(difluorométhoxy)-5-(pipéridin-4-ylthio)phényl)-3-méthyl-6-(pyrazolo[1,5-a]pyrimidin-3-yl)-1H-pyrazolo[4,3-c]pyridine ;
1-(4-((4-(difluorométhoxy)-3-(3-méthyl-6-(pyrazolo[1,5-a]pyrimidin-3-yl)-1H-pyrazolo[4,3-c]pyridin-1-yl)phényl)thio)pipéridin-1-yl)éthan-1-one ;
1-((1-(5-chloro-2-(difluorométhoxy)phényl)-6-(pyrazolo[1,5-a]pyrimidin-3-yl)-1H-pyrazolo[4,3-c]pyridin-3-yl)méthyl)azetidin-3-amine ;
N-((1r,3r)-3-aminocyclobutyl)-1-(5-chloro-2-méthoxyphényl)-6-(pyrazolo[1,5-a]pyrimidin-3-yl)-1H-pyrazolo[4,3-c]pyridin-3-carboxamide ;
1-(5-chloro-2-méthoxyphényl)-N-((1r,3r)-3-(méthyl(2-(méthylamino)-2-oxoéthyl)amino)cyclobutyl)-6-(pyrazolo[1,5-a]pyrimidin-3-yl)-1H-pyrazolo[4,3-c]pyridin-3-carboxamide ;
1-(5-chloro-2-méthoxyphényl)-N-((1r,3r)-3-morpholinocyclobutyl)-6-(pyrazolo[1,5-a]pyrimidin-3-yl)-1H-pyrazolo[4,3-c]pyridin-3-carboxamide ;
1-(5-chloro-2-méthoxyphényl)-N-((1r,3r)-3-(diméthylamino)cyclobutyl)-6-(pyrazolo[1,5-a]pyrimidin-3-yl)-1H-pyrazolo[4,3-c]pyridin-3-carboxamide ;
N-((1s,3s)-3-aminocyclobutyl)-1-(5-chloro-2-(difluorométhoxy)phényl)-6-(pyrazolo[1,5-a]pyrimidin-3-yl)-1H-pyrazolo[4,3-c]pyridin-3-carboxamide ;
N-(2-(diméthylamino)éthyl)-1-(2-méthoxy-5-(méthylsulfonyl)phényl)-6-(pyrazolo[1,5-a]pyrimidin-3-yl)-1H-pyrazolo[4,3-c]pyridin-3-carboxamide ;
N-(3-(diméthylamino)propyl)-1-(2-méthoxy-5-(méthylsulfonyl)phényl)-6-(pyrazolo[1,5-a]pyrimidin-3-yl)-1H-pyrazolo[4,3-c]pyridin-3-carboxamide ;
1-(2-méthoxy-5-(méthylsulfonyl)phényl)-N-(3-morpholinopropyl)-6-(pyrazolo[1,5-a]pyrimidin-3-yl)-1H-pyrazolo[4,3-c]pyridin-3-carboxamide ;
1-(2-méthoxy-5-(méthylsulfonyl)phényl)-N-méthyl-6-(pyrazolo[1,5-a]pyrimidin-3-yl)-1H-pyrazolo[4,3-c]pyridin-3-amine ;
N-(3-(3-((2-(diméthylamino)éthyl)amino)-6-(pyrazolo[1,5-a]pyrimidin-3-yl)-1H-pyrazolo[4,3-c]pyridin-1-yl)-4-méthoxyphényl)méthanesulfonamide ;
N-(1-(5-chloro-2-méthoxyphényl)-6-(pyrazolo[1,5-a]pyrimidin-3-yl)-1H-pyrazolo[4,3-c]pyridin-3-yl)-2-(méthylamino)acétamide ;
N-(3-(diméthylamino)propyl)-1-(5-fluoro-4-hydroxy-2-méthoxyphényl)-6-(pyrazolo[1,5-a]pyrimidin-3-yl)-1H-pyrazolo[4,3-c]pyridin-3-carboxamide ;
1-(1-(2-méthoxy-5-(méthylsulfonyl)phényl)-6-(pyrazolo[1,5-a]pyrimidin-3-yl)-1H-pyrazolo[4,3-c]pyridin-3-yl)-N-méthylméthanamine ;
3-((1-(5-fluoro-2-méthoxyphényl)-3-méthyl-1H-pyrazolo[4,3-c]pyridin-6-yl)amino)pyrazin-2(1H)-one ;
3-((1-(5-(difluorométhyl)-2-méthoxyphényl)-3-méthyl-1H-pyrazolo[4,3-c]pyridin-6-yl)amino)pyrazin-2(1H)-one ;
N-(2-hydroxyéthyl)-4-méthoxy-3-(3-méthyl-6-((3-oxo-3,4-dihydropyrazin-2-yl)amino)-1H-pyrazolo[4,3-c]pyridin-1-yl)benzènesulfonamide ;
3-((1-(2-(difluorométhoxy)-5-((2-méthoxyéthyl)thio)phényl)-3-méthyl-1H-pyrazolo[4,3-c]pyridin-6-yl)amino)pyrazin-2(1H)-one ;
3-((1-(2-(difluorométhoxy)-5-(méthylsulfonyl)phényl)-3-méthyl-1H-pyrazolo[4,3-c]pyridin-6-yl)amino)pyrazin-2(1H)-one ;
3-((1-(2-(difluorométhoxy)-5-((2-méthoxyéthyl)sulfonyl)phényl)-3-méthyl-1H-pyrazolo[4,3-c]pyridin-6-yl)amino)pyrazin-2(1H)-one ;
N-(3-(diméthylamino)propyl)-1-(5-fluoro-2-méthoxyphényl)-6-((3-oxo-3,4-dihydropyrazin-2-yl)amino)-1H-pyrazolo[4,3-c]pyridin-3-carboxamide ;
3-((3-((3-(diméthylamino)propyl)amino)-1-(5-fluoro-2-méthoxyphényl)-1H-pyrazolo[4,3-c]pyridin-6-yl)amino)pyrazin-2(1H)-one ;
3-((1-(5-(difluorométhyl)-2-méthoxyphényl)-3-((2-(diméthylamino)éthyl)amino)-1H-pyrazolo[4,3-c]pyridin-6-yl)amino)pyrazin-2(1H)-one ;
3-((1-(5-chloro-2-(difluorométhoxy)phényl)-3-méthyl-1H-pyrazolo[4,3-c]pyridin-6-yl)amino)pyrazin-2(1H)-one ;
énantiomères simples, diastéréo-isomères et mélanges de ceux-ci ou sel ou solvate pharmaceutiquement acceptable de ceux-ci.

6. Composition pharmaceutique comprenant un composé selon l'une quelconque des revendications 1 à 5, ou un sel pharmaceutiquement acceptable de celui-ci, en mélange avec un ou plusieurs supports ou excipients pharmaceutiquement acceptables.

7. Composition pharmaceutique selon la revendication 6, apte à être administrée par inhalation, choisie parmi des poudres inhalables, aérosols-doseurs contenant un agent propulseur ou formulations inhalables sans agent propulseur.

8. Dispositif comprenant la composition pharmaceutique selon la revendication 7, qui peut être un inhalateur de poudre sèche à dose unique ou à doses multiples, un aérosol-doseur ou un nébuliseur à brouillard doux.

9. Composé ou composition pharmaceutique selon l'une quelconque des revendications 1 à 7, pour une utilisation en tant que médicament.

10. Composé ou composition pharmaceutique pour utilisation selon la revendication 9 dans la prévention et/ou le traitement de maladie pulmonaire choisie dans le groupe constitué d'asthme, maladie pulmonaire obstructive chronique MPOC, fibrose pulmonaire idiopathique (IPF), lésion pulmonaire aiguë et syndrome de détresse respiratoire aiguë (ARDS).

11. Combinaison d'un composé selon l'une quelconque des revendications 1 à 5 avec un ou plusieurs ingrédients actifs choisis parmi les classes constituées avec d'autres ingrédients actifs pharmaceutiques choisis dans le groupe constitué de bêta-2-agonistes, agents antimuscariniques, corticostéroïdes, inhibiteurs de kinases activées par mitogène (kinases MAP P38), inhibiteurs de sous-unité bêta de kinase du facteur nucléaire kappa-B (IKK2), inhibiteurs d'élastase neutrophile humaine (HNE), inhibiteurs de phosphodiestérase 4 (PDE4), modulateurs de leucotriènes, anti-inflammatoires non stéroïdiens (AINS) et régulateurs de mucus.
